(19) Europäisches Patentamt
European Patent Office
Office européen des brevets

(11) EP 1 716 128 B1

(12) **EUROPÄISCHE PATENTSCHRIFT**

(45) Veröffentlichungstag und Bekanntmachung des Hinweises auf die Patenterteilung:
**31.03.2010 Patentblatt 2010/13**

(21) Anmeldenummer: **05715296.9**

(22) Anmeldetag: **11.02.2005**

(51) Int Cl.:
***C07D 277/60*** *(2006.01)*

(86) Internationale Anmeldenummer:
**PCT/EP2005/001369**

(87) Internationale Veröffentlichungsnummer:
**WO 2005/077924 (25.08.2005 Gazette 2005/34)**

(54) **SUBSTITUIERTE 4,5,6,7-TETRAHYDRO-BENZOTHIAZOL-2-YLAMIN-VERBINDUNGEN**

SUBSTITUTED 4,5,6,7-TETRAHYDRO-BENZOTHIAZOL-2-YLAMINE COMPOUNDS

COMPOSES 4,5,6,7-TETRAHYDRO-BENZOTHIAZOL-2-YLAMINE SUBSTITUES

(84) Benannte Vertragsstaaten:
**AT BE BG CH CY CZ DE DK EE ES FI FR GB GR HU IE IS IT LI LT LU MC NL PL PT RO SE SI SK TR**
Benannte Erstreckungsstaaten:
**LV**

(30) Priorität: **11.02.2004 DE 102004006808**

(43) Veröffentlichungstag der Anmeldung:
**02.11.2006 Patentblatt 2006/44**

(73) Patentinhaber: **Grünenthal GmbH**
**52078 Aachen (DE)**

(72) Erfinder:
- **OBERBÖRSCH, Stefan**
  **52074 Aachen (DE)**
- **SUNDERMANN, Corinna**
  **52074 Aachen (DE)**
- **SUNDERMANN, Bernd**
  **52074 Aachen (DE)**
- **BIJSTERVELD, Edward**
  **NL-6503 CB Nijmegen (NL)**

(74) Vertreter: **Brosch, Oliver et al**
**Kutzenberger & Wolff**
**Theodor-Heuss-Ring 23**
**50668 Köln (DE)**

(56) Entgegenhaltungen:
**EP-A- 0 445 026** **WO-A-01/43740**
**US-A- 5 286 735**

- **A.N.NITU ET AL.: "Emerging Trends in the Pharmacotherapy of Chronic Pain" EXPERT OPIN.INVESTIG.DRUGS, Bd. 12, Nr. 4, 2003, Seiten 545-559, XP002335568**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 716 128 B1

Printed by Jouve, 75001 PARIS (FR)

## Beschreibung

Die vorliegende Erfindung betrifft substituierte 4,5,6,7-Tetrahydro-benzothiazol-2-ylamin-Verbindungen, Verfahren zu deren Herstellung, Arzneimittel enthaltend diese Verbindungen sowie die Verwendung dieser Verbindungen zur Herstellung von Arzneimitteln.

Depression ist eine Störung der Affektivität, bei der ein depressives Syndrom im Vordergrund steht, wobei depressiv mit Verstimmung verbunden bzw. traurig gestimmt heißt. Die zur Therapie verwendeten Antidepressiva sind auch wichtige Adjuvantien für die Schmerztherapie (Tzschentke, NA and 5-HT Reuptake Inhibitors and $\alpha 2$ agonists, in Analgesics: From Chemistry and Pharmacology to Clinical Application, S. 265-284, Wiley 2002), insbesondere bei chronischen Schmerzzuständen, da die andauernde Schmerzbelastung zu einer depressiven Verstimmung der Patienten führen kann. Dies ist besonders häufig bei Krebs-Schmerzpatienten der Fall (Berard, Int. Med. J. 1996, 3/4, 257-259). Da es bisher keine Schmerzmittel mit einer klinisch relevanten antidepressiven Wirkkomponente gibt, müssen die Antidepressiva als Zusatzmedikation zur Analgetika-Gabe hinzugefügt werden. Da chronische Schmerzpatienten häufig eine Vielzahl von verschiedenen Medikamenten benötigen, führt die zusätzliche Gabe des Antidepressivums zu einer weiteren Belastung des Organismus. Aus diesem Grund sowie zur Verbesserung der Compliance besteht ein großer Bedarf nach einem pharmakologischen Wirkstoff, der neben seiner analgetischen Wirkung vorzugsweise auch eine antidepressive Wirkkomponente aufweist. Grundlage für eine solche antidepressive Wirkung eines pharmakologischen Wirkstoffs ist dessen Hemmung der Wiederaufnahme von Serotonin.

Eine Aufgabe der vorliegenden Erfindung bestand daher darin, Verbindungen zur Verfügung zu stellen, die sich insbesondere als pharmazeutische Wirkstoffe in Arzneimitteln eignen, bevorzugt in Arzneimitteln zur vorzugsweise gleichzeitigen Therapie von Schmerzen und Depressionen. Des weiteren sollen sich die Arzneimittel ferner zur Regulierung von 5-HT-Rezeptoren, Noradrenalin-Rezeptoren, zur Behandlung von Akohol- und/oder Drogen- und/oder Medikamentenmißbrauch, zur Prophylaxe und/oder Behandlung von Akohol- und/oder Drogen- und/oder Medikamentenabhängigkeit, zur Prophylaxe und/oder Behandlung von Entzündungen, zur Prophylaxe und/oder Behandlung von Antriebslosigkeit, zur Prophylaxe und/oder Behandlung von Störungen der Nahrungsmittelaufnahme, vorzugsweise solchen ausgewählt aus der Gruppe bestehend aus Bulimie, Anorexie, Fettsucht und Kachexie, zur Prophylaxe und/oder Behandlung von Katalepsie, zur Vigilanzsteigerung, zur Libidosteigerung oder zur Anxiolyse eignen.

Diese Aufgabe wurde gelöst durch die Bereitstellung substituierter 4,5,6,7-Tetrahydro-benzothiazol-2-ylamin-Verbindungen der nachstehend angegebenen allgemeinen Formel I.

Es wurde überraschenderweise gefunden, daß diese Verbindungen eine Affinität zu 5-HT- sowie zu Noradrenalin-Rezeptoren aufweisen und zur Inhibierung des Noradrenalin-Uptakes (der Noradrenalin-Wiederaufnahme) sowie zur Inhibierung des 5-Hydroxytryptamin-(5-HT)-Uptakes (der 5HT-Wiederaufnahme) führen.

Die erfindungsgemäßen substituierten 4,5,6,7-Tetrahydro-benzothiazol-2-ylamin-Verbindungen der nachstehend angegebenen allgemeinen Formel I eignen sich daher insbesondere als pharmazeutische Wirkstoffe in Arzneimitteln, vorzugsweise in solchen zur Prophylaxe und/oder Behandlung von Depressionen, zur Behandlung von Akohol- und/oder Drogen- und/oder Medikamentenmißbrauch, zur Prophylaxe und/oder Behandlung von Akohol- und/oder Drogen- und/oder Medikamentenabhängigkeit, zur Prophylaxe und/oder Behandlung von Entzündungen, zur Prophylaxe und/oder Behandlung von Antriebslosigkeit, zur Prophylaxe und/oder Behandlung von Störungen der Nahrungsmittelaufnahme, vorzugsweise ausgewählt aus der Gruppe bestehend aus Bulimie, Anorexie, Fettsucht und Kachexie, zur Prophylaxe und/oder Behandlung von Katalepsie, zur Vigilanzsteigerung, zur Libidosteigerung oder zur Anxiolyse. Darüber hinaus zeigen die erfindungsgemäßen Verbindungen auch eine ausgeprägte analgetische Wirksamkeit, so daß sich entsprechende Arzneimittel insbesondere auch zur gleichzeitigen Therapie von Schmerzen und Depressionen eignen.

Ein Gegenstand der vorliegenden Erfindung sind daher substituierte 4,5,6,7-Tetrahydro-benzothiazol-2-ylamin-Verbindungen der allgemeinen Formel I,

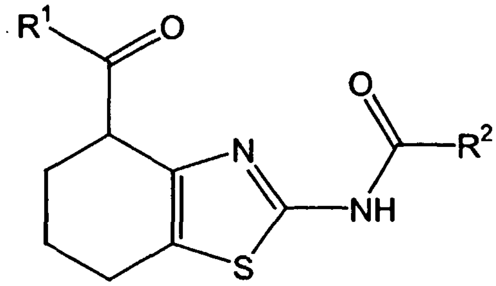

I

worin
$R^1$ für eine Gruppe $-NR^3R^4$ oder für eine Gruppe $-NR^5R^6$ steht,
$R^2$ für einen linearen oder verzweigten, gesättigten oder ungesättigten, ggf. wenigstens einfach substituierten aliphatischen Rest, für einen gesättigten oder ungesättigten, ggf. wenigstens einfach substituierten, ggf. wenigstens ein Heteroatom als Ringglied aufweisenden cycloaliphatischen Rest, der über eine ggf. wenigstens einfach substituierte, ggf. wenigstens ein Heteroatom als Kettenglied aufweisende Alkylen-, Alkenylen- oder Alkinylen-Gruppe gebunden sein kann, oder für einen ggf. wenigstens einfach substituierten Aryl- oder Heteroaryl-Rest steht, der über eine ggf. wenigstens einfach substituierte, ggf. wenigstens ein Heteroatom als Kettenglied aufweisende Alkylen-, Alkenylen- oder Alkinylen-Gruppe gebunden sein kann,
$R^3$ für Wasserstoff, einen linearen oder verzweigten, gesättigten oder ungesättigten, ggf. wenigstens einfach substituierten aliphatischen Rest, für einen gesättigten oder ungesättigten, ggf. wenigstens einfach substituierten, ggf. wenigstens ein Heteroatom als Ringglied aufweisenden cycloaliphatischen Rest, der über eine ggf. wenigstens einfach substituierte, ggf. wenigstens ein Heteroatom als Kettenglied aufweisende Alkylen-, Alkenylen- oder Alkinylen-Gruppe gebunden sein kann, oder für einen ggf. wenigstens einfach substituierten Aryl- oder Heteroaryl-Rest steht, der über eine ggf. wenigstens einfach substituierte, ggf. wenigstens ein Heteroatom als Kettenglied aufweisende Alkylen-, Alkenylen- oder Alkinylen-Gruppe gebunden sein kann,
$R^4$ für Wasserstoff, einen linearen oder verzweigten, gesättigten oder ungesättigten, ggf. wenigstens einfach substituierten aliphatischen Rest, für einen gesättigten oder ungesättigten, ggf. wenigstens einfach substituierten, ggf. wenigstens ein Heteroatom als Ringglied aufweisenden cycloaliphatischen Rest, der über eine ggf. wenigstens einfach substituierte, ggf. wenigstens ein Heteroatom als Kettenglied aufweisende Alkylen-, Alkenylen- oder Alkinylen-Gruppe gebunden sein kann, oder für einen ggf. wenigstens einfach substituierten Aryl- oder Heteroaryl-Rest steht, der über eine ggf. wenigstens einfach substituierte, ggf. wenigstens ein Heteroatom als Kettenglied aufweisende Alkylen-, Alkenylen- oder Alkinylen-Gruppe gebunden sein kann,
$R^5$ und $R^6$ gemeinsam mit dem sie verbrückenden Stickstoffatom als Ringglied einen gesättigten, ungesättigten oder aromatischen, ggf. wenigstens einfach substituierten, ggf. wenigstens ein weiteres Heteroatom als Ringglied aufweisenden heterocyclischen Rest bilden,
ggf. in Form ihrer reinen Stereoisomeren, insbesondere Enantiomeren oder Diastereomeren, ihrer Racemate oder in Form von Mischungen der Stereoisomeren, insbesondere der Enantiomeren und/oder Diastereomeren, in einem beliebigen Mischungsverhältnis, oder jeweils ggf. in Form entsprechender Salze oder jeweils ggf. in Form entsprechender Solvate.

Sofern einer der Reste $R^1$ bis $R^6$ für einen aliphatischen Rest steht oder einen aliphatischen Rest aufweist, kann dieser aliphatische Rest- sofern nicht anders angegeben - einfach oder mehrfach, beispielsweise 1-, 2-, 3-, 4- oder 5-fach, substituiert sein, wobei die Substituenten jeweils unabhängig voneinander bevorzugt ausgewählt werden können aus der Gruppe bestehend aus F, Cl, Br, $C_{1-6}$-Alkoxy, Hydroxy, CN, $CF_3$, $CHF_3$, $CH_2F$, unsubstituiertem Phenyl und $-NR^aR^b$, worin $R^a$ und $R^b$ jeweils unabhängig voneinander ausgewählt werden können aus der Gruppe bestehend aus H, $C_{1-3}$-Alkyl und unsubstituiertem Phenyl.

Geeignete aliphatische Reste, die einfach oder mehrfach, beispielsweise 1-, 2-, 3-, 4-oder 5-fach, substituiert sein können, können beispielsweise ausgewählt werden aus der Gruppe bestehend aus Methyl, Ethyl, n-Propyl, Isopropyl, n-Butyl, Iso-butyl, sec-Butyl, tert-Butyl, n-Pentyl, 2,2-Dimethyl-propyl, penta-1,3-dienyl, n-Hexyl, n-Heptyl, n-Octyl, n-Nonyl, n-Decyl, Vinyl, Ethinyl, Propenyl, Propinyl, Butenyl und Butinyl.

Sofern einer der Reste $R^1$ bis $R^6$ für einen cycloaliphatischen Rest steht oder einen cycloaliphatischen Rest

aufweist, kann dieser cycloalphatische Rest - sofern nicht anders angegeben- einfach oder mehrfach, beispielsweise 1-, 2-, 3-, 4- oder 5-fach, substituiert sein, wobei die Substituenten jeweils unabhängig voneinander vorzugsweise ausgewählt werden können aus der Gruppe bestehend aus F, Cl, Br, $C_{1-6}$-Alkoxy, $C_{1-6}$-Alkyl, Hydroxy, CN, $CF_3$, $CHF_3$, $CH_2F$, unsubstituiertem Phenyl, - $NR^aR^b$, worin $R^a$ und $R^b$ jeweils unabhängig voneinander ausgewählt werden können aus der Gruppe bestehend aus H, $C_{1-3}$-Alkyl und unsubstituiertem Phenyl, und einem ggf. über eine Methylen-Gruppe gebundenen Phenyl- oder Benzofuranyl-Rest, der einfach oder mehrfach, beispielsweise 1-, 2-, 3-, 4- oder 5-fach, mit einem Substituenten ausgewählt aus der Gruppe bestehend aus F, Cl, Br, $CF_3$, $CHF_2$, $CH_2F$, Phenoxy, Benzyloxy, Phenyl, $C_{1-4}$-Alkoxy, $C_{1-4}$-Alkyl und $C_{1-4}$ Alkylthio substituiert sein kann.

Geeignete cycloaliphatische Reste, die einfach oder mehrfach, beispielsweise 1-, 2-, 3-, 4- oder 5-fach, substituiert sein können, können beispielsweise ausgewählt werden aus der Gruppe bestehend aus Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl.

Sofern einer der Reste $R^1$ bis $R^6$ für einen Aryl- oder Heteroaryl-Rest steht oder einen Aryl- oder Heteroaryl-Rest aufweist, kann dieser Aryl- oder Heteroaryl-Rest - sofern nicht anders angegeben - einfach oder mehrfach, beispielsweise 1-, 2-, 3-, 4-oder 5-fach, substituiert sein, wobei die Substituenten jeweils unabhängig voneinander vorzugsweise ausgewählt werden können aus der Gruppe bestehend aus F, Cl, Br, $C_{1-6}$-Alkoxy, $C_{1-6}$-Alkyl, Hydroxy, CN, $CF_3$, $CHF_3$, $CH_2F$, unsubstituiertem Phenyl, unsubstituiertem Morpholinyl und -$NR^aR^b$, worin $R^a$ und $R^b$ jeweils unabhängig voneinander ausgewählt werden können aus der Gruppe bestehend aus H, $C_{1-3}$-Alkyl und unsubstituiertem Phenyl.

Geeignete Aryl-Reste, die einfach oder mehrfach, beispielsweise 1-, 2-, 3-, 4- oder 5-fach, substituiert sein können, sind insbesondere Phenyl bzw. Naphthyl. Geeignete Heteroaryl-Reste können insbesondere ausgewählt werden aus der Gruppe bestehend aus Pyridyl, Furanyl, und Thiophenyl.

Sofern die Reste $R^5$ und $R^6$ gemeinsam mit dem sie verbrückenden Stickstoffatom als Ringglied einen gesättigten, ungesättigten oder aromatischen, ggf. wenigstens ein weiteres Heteroatom als Ringglied aufweisenden heterocyclischen Rest bilden, der einfach oder mehrfach, beispielsweise 1-, 2-, 3-, 4- oder 5-fach, substituiert ist, können die Substituenten bevorzugt ausgewählt werden aus der Gruppe bestehend aus einem linearen oder verzweigten, gesättigten oder ungesättigten, ggf. wenigstens einfach substituierten aliphatischen $C_{1-6}$ Rest, einem ggf. über eine ggf. wenigstens einfach substituierte, ggf. wenigstens ein Heteroatom oder ggf. wenigstens eine Carbonyl-(C=O)-Gruppe als Kettenglied aufweisende $C_{1-6}$-Alkylen-, $C_{2-6}$-Alkenylen- oder $C_{2-6}$-Alkinylen-Gruppe gebundenen, gesättigten oder ungesättigten, ggf. wenigstens einfach substituierten, ggf. wenigstens ein Heteroatom als Ringglied enthaltenden 5-, 6- oder 7-gliedrigen cycloaliphatischen Rest, der mit einem ggf. wenigstens einfach, beispielsweise 1-, 2-, 3-, 4- oder 5-fach, substituierten, mono- oder polyzyklischen Ringsystem kondensiert sein kann, und einem ggf. über eine ggf. wenigstens einfach substituierte, ggf. wenigstens ein Heteroatom als Kettenglied aufweisende $C_{1-6}$-Alkylen-, $C_{2-6}$-Alkenylen- oder $C_{2-6}$-Alkinylen-Gruppe gebundenen, ggf. wenigstens einfach substituierten 5- oder 6-gliedrigen Aryl- oder Heteroaryl-Rest, der mit einem ggf. wenigstens einfach, beispielsweise 1-, 2-, 3-, 4- oder 5-fach, substituierten, mono- oder polyzyklischen Ringsystem kondensiert sein kann. Sofern der mit $R^5$ und $R^6$ gebildete Heterozyklus eines oder mehrere weitere, beispielsweise 1, 2 oder 3, Heteroatome aufweist, können diese bevorzugt ausgewählt werden aus der Gruppe bestehend aus Sauerstoff, Stickstoff und Schwefel.

Unter einem mono- bzw. polyzyklischen Ringsystem werden im Sinne der vorliegenden Erfindung mono- bzw. polyzyklische Kohlenwasserstoffreste verstanden, die gesättigt, ungesättigt oder aromatisch sein und ggf. ein oder mehrere, beispielsweise 1, 2 oder 3, Heteroatom als Ringglieder aufweisen können. Ein solches mono- bzw. polyzyklisches Ringsystem kann beispielsweise mit einem cycloaliphatischen Rest, einem Aryl-Rest oder einem Heteroaryl-Rest kondensiert, d.h. anneliert bzw. verbunden sein.

Sofern ein polyzyklisches Ringsystem vorliegt, können die verschiedenen Ringe, jeweils unabhängig voneinander, einen unterschiedlichen Sättigungsgrad aufweisen, d.h. gesättigt, ungesättigt oder aromatisch sein. Die Heteroatome können jeweils bevorzugt ausgewählt werden aus der Gruppe bestehend aus Sauerstoff, Stickstoff und Schwefel. Vorzugsweise sind die jeweiligen Ringe des Ringsystems 5- oder 6-gliedrig.

Sofern einer der Reste $R^1$ bis $R^6$ eine Alkylen-, Alkenylen- oder Alkinylen-Gruppe aufweist, können diese jeweils verzweigt oder unverzweigt und ggf. einfach oder mehrfach, beispielsweise 1-, 2-, 3-, 4- oder 5-fach, substituiert sein, wobei die Substituenten unabhängig voneinander vorzugsweise ausgewählt werden können aus der Gruppe bestehend aus F, Cl, Br, $C_{1-6}$-Alkoxy, Hydroxy, CN, $CF_3$, $CHF_3$, $CH_2F$, unsubstituiertem Phenyl und -$NR^aR^b$, worin $R^a$ und $R^b$ jeweils unabhängig voneinander ausgewählt werden können aus der Gruppe bestehend aus H, $C_{1-3}$-Alkyl und unsubstituiertem Phenyl.

Sofern einer der Reste $R^1$ bis $R^6$ für einen wenigstens ein Heteroatom aufweisenden cycloaliphatischen Rest oder einen Heteroaryl-Rest steht oder eine Alkylen-, Alkenylen- oder Alkinylen-Gruppe mit wenigstens einem Heteroatom als Kettenglied aufweist oder $R^5$ und $R^6$ gemeinsam mit dem sie verbrückenden Stickstoffatom einen Heterozyklus bilden, der ein oder mehr weitere Heteroatome aufweist, können die jeweiligen Heteroatome bevorzugt ausgewählt werden aus der Gruppe bestehend aus Sauerstoff, Stickstoff und Schwefel. Bevorzugt können die vorstehend genannten Reste 1, 2 oder 3 Heteroatome als Ring- bzw. Kettenglied(er) aufweisen, die jeweils unabhängig voneinander ausgewählt

werden können aus der Gruppe bestehend aus Sauerstoff, Stickstoff und Schwefel.

Bevorzugt sind substituierte 4,5,6,7-Tetrahydro-benzothiazol-2-ylamin-Verbindungen der allgemeinen Formel I, in denen $R^2$ für einen linearen oder verzweigten, gesättigten oder ungesättigten, ggf. wenigstens einfach substituierten aliphatischen $C_1$-$C_{10}$-Rest steht, für einen gesättigten oder ungesättigten, ggf. wenigstens einfach substituierten, ggf. wenigstens ein Heteroatom als Ringglied aufweisenden drei- bis siebengliedrigen, cycloaliphatischen Rest, der über eine ggf. wenigstens einfach substituierte, ggf. wenigstens ein Heteroatom als Kettenglied aufweisende $C_1$-$C_{10}$-Alkylen-, $C_2$-$C_{10}$-Alkenylen- oder $C_2$-$C_{10}$-Alkinylen-Gruppe gebunden sein kann, oder für einen ggf. wenigstens einfach substituierten fünf- bis zwölfgliedrigen Aryl- oder Heteroaryl-Rest steht, der über eine ggf. wenigstens einfach substituierte, ggf. wenigstens ein Heteroatom als Kettenglied aufweisende $C_1$-$C_{10}$-Alkylen-, $C_2$-$C_{10}$-Alkenylen- oder $C_2$-$C_{10}$-Alkinylen-Gruppe gebunden sein kann,

vorzugsweise für einen linearen oder verzweigten $C_1$-$C_{10}$ Alkyl-Rest, einen linearen oder verzweigten $C_2$-$C_{10}$ Alkenyl-Rest, für einen gesättigten oder ungesättigten, ggf. wenigstens einfach substituierten, ggf. wenigstens ein Heteroatom als Ringglied aufweisenden fünf- oder sechs-gliedrigen cycloaliphatischen Rest, der über eine ggf. wenigstens einfach substituierte, ggf. wenigstens ein Heteroatom als Kettenglied aufweisende $C_1$-$C_5$-Alkylen- oder $C_2$-$C_5$-Alkenylen-Gruppe gebunden sein kann, oder für einen Phenyl-, 1-Naphthyl-, 2-Naphthyl, 2-Furanyl-(2-Furyl-), 3-Furanyl-(3-Furyl-), 2-Thiophenyl- (2-Thienyl-) oder 3-Thiophenyl- (3-Thienyl-) Rest steht, der jeweils wenigstens einfach substituiert und/ oder über eine ggf. wenigstens einfach substituierte, ggf. wenigstens ein Heteroatom als Kettenglied aufweisende $C_1$-$C_5$-Alkylen- oder $C_2$-$C_5$-Alkenylen-Gruppe gebunden sein kann,

besonders bevorzugt für einen linearen oder verzweigten $C_1$-$C_5$ Alkyl-Rest, einen linearen oder verzweigten $C_2$-$C_5$ Alkenyl-Rest, für einen gesättigten oder ungesättigten, ggf. wenigstens einfach substituierten, ggf. wenigstens ein Heteroatom als Ringglied aufweisenden sechs-gliedrigen cycloaliphatischen Rest, der über eine $C_1$-$C_3$-Alkylen-Gruppe gebunden sein kann, oder für einen Phenyl-, 1-Naphthyl-, 2-Naphthyl, 2-Furanyl-, 3-Furanyl-, 2-Thiophenyl- oder 3-Thiophenyl-Rest steht, der jeweils wenigstens einfach substituiert und/oder über eine ggf. wenigstens einfach substituierte, ggf. wenigstens ein Heteroatom als Kettenglied aufweisende $C_1$-$C_5$-Alkylen- oder $C_2$-$C_5$-Alkenylen-Gruppe gebunden sein kann, und jeweils die übrigen Reste $R^1$ und $R^3$ bis $R^6$, die vorstehend angegebenene Bedeutung haben, ggf. in Form ihrer reinen Stereoisomeren, insbesondere Enantiomeren oder Diastereomeren, ihrer Racemate oder in Form von Mischungen der Stereoisomeren, insbesondere der Enantiomeren und/oder Diastereomeren, in einem beliebigen Mischungsverhältnis, oder ggf. in Form entsprechender Salze oder jeweils in Form entsprechender Solvate.

Weiterhin bevorzugt sind substituierte 4,5,6,7-Tetrahydro-benzothiazol-2-ylamin-Verbindungen der allgemeinen Formel I, in denen Rest $R^3$ für Wasserstoff, einen linearen oder verzweigten, gesättigten oder ungesättigten, ggf. wenigstens einfach substituierten aliphatischen $C_1$-$C_{10}$-Rest steht, für einen gesättigten oder ungesättigten, ggf. wenigstens einfach substituierten, ggf. wenigstens ein Heteroatom als Ringglied aufweisenden drei- bis siebengliedrigen cycloaliphatischen Rest, der über eine ggf. wenigstens einfach substituierte, ggf. wenigstens ein Heteroatom als Kettenglied aufweisende $C_1$-$C_{10}$-Alkylen-, $C_2$-$C_{10}$-Alkenylen- oder $C_2$-$C_{10}$-Alkinylen-Gruppe gebunden sein kann, oder für einen ggf. wenigstens einfach substituierten fünf- bis zwölfgliedrigen Aryl- oder Heteroaryl-Rest steht, der über eine ggf. wenigstens einfach substituierte, ggf. wenigstens ein Heteroatom als Kettenglied aufweisende $C_1$-$C_{10}$-Alkylen-, $C_2$-$C_{10}$-Alkenylen- oder $C_2$-$C_{10}$-Alkinylen-Gruppe gebunden sein kann,

vorzugsweise für Wasserstoff, einen linearen oder verzweigten, ggf. wenigstens einfach substituierten $C_1$-$C_{10}$-Alkyl-Rest, für einen gesättigten oder ungesättigten, ggf. wenigstens einfach substituierten, ggf. wenigstens ein Heteroatom als Ringglied aufweisenden fünf-, sechs- oder siebengliedrigen cycloaliphatischen Rest, der über eine ggf. wenigstens einfach substituierte, ggf. wenigstens ein Heteroatom als Kettenglied aufweisende $C_1$-$C_{10}$-Alkylen-, $C_2$-$C_{10}$-Alkenylen- oder $C_2$-$C_{10}$-Alkinylen-Gruppe gebunden sein kann, oder für einen ggf. wenigstens einfach substituierten fünf- bis zwölfgliedrigen Aryl- oder Heteroaryl-Rest steht, der über eine ggf. wenigstens einfach substituierte, ggf. wenigstens ein Heteroatom als Kettenglied aufweisende $C_1$-$C_{10}$-Alkylen-, $C_2$-$C_{10}$-Alkenylen- oder $C_2$-$C_{10}$-Alkinylen-Gruppe gebunden sein kann,

besonders bevorzugt für Wasserstoff, einen linearen oder verzweigten, ggf. wenigstens einfach substituierten $C_{1-3}$ Alkyl-Rest, für einen über eine $C_{1-3}$-AlkylenGruppe gebundenen cycloaliphatischen Rest ausgewählt aus der Gruppe bestehend aus

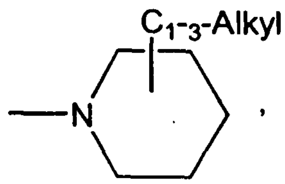, 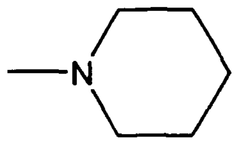, 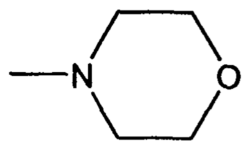,

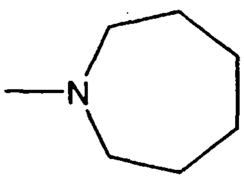, 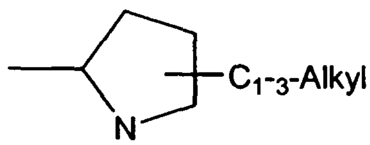, 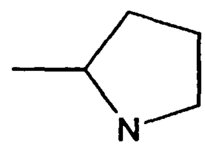,

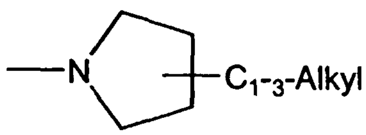 und 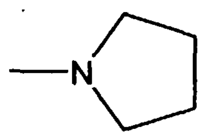,

oder für einen cycloaliphatischen Rest

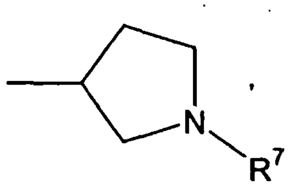,

wobei der Rest $R^7$ für einen über eine Methylen-Gruppe gebundenen Phenyl- oder Benzofuranyl-Rest steht, der einfach oder mehrfach, gleich oder verschieden, mit einem Substituenten ausgewählt aus der Gruppe bestehend aus F, Cl, Br, $CF_3$, $CHF_2$, $CH_2F$, Phenoxy, Benzyloxy, Phenyl, $C_{1-4}$-Alkoxy oder $C_{1-4}$ Alkylthio substituiert sein kann,
oder der Rest $R^3$ für einen ggf. über eine Methylen-Gruppe gebundenen Phenyl Rest steht, der einfach oder mehrfach, gleich oder verschieden, mit einem Substituenten ausgewählt aus der Gruppe bestehend aus Di-($C_{1-3}$)-Alkylamino. $C_{1-3}$-Methoxy und Morpholinyl substituiert sein kann, und jeweils die Reste $R^1$, $R^2$ und $R^4$ bis $R^6$ die vorstehend genannte Bedeutung haben, ggf. in Form ihrer reinen Stereoisomeren, insbesondere Enantiomeren oder Diastereomeren, ihrer Racemate oder in Form von Mischungen der Stereoisomeren, insbesondere der Enantiomeren und/oder Diastereomeren, in einem beliebigen Mischungsverhältnis, oder ggf. in Form entsprechender Salze oder jeweils in Form entsprechender Solvate

Ebenfalls bevorzugt sind substituierte 4,5,6,7-Tetrahydro-benzothiazol-2-ylamin-Verbindungen der allgemeinen Formel I, in denen der Rest $R^4$ für Wasserstoff, einen linearen oder verzweigten, gesättigten oder ungesättigten, ggf. wenigstens einfach substituierten aliphatischen $C_1$-$C_{10}$-Rest steht, für einen gesättigten oder ungesättigten, ggf. wenigstens einfach substituierten, ggf. wenigstens ein Heteroatom als Ringglied aufweisenden drei- bis siebengliedrigen cycloaliphatischen Rest, der über eine ggf. wenigstens einfach substituierte, ggf. wenigstens ein Heteroatom als Kettenglied aufweisende $C_1$-$C_{10}$-Alkylen-, $C_2$-$C_{10}$-Alkenylen- oder $C_2$-$C_{10}$-Alkinylen-Gruppe gebunden sein kann, oder für einen ggf. wenigstens einfach substituierten fünf- bis zwölfgliedrigen Aryl- oder Heteroaryl-Rest steht, der über eine ggf. wenigstens einfach substituierte, ggf. wenigstens ein Heteroatom als Kettenglied aufweisende $C_1$-$C_{10}$-Alkylen-, $C_2$-$C_{10}$-Al-

kenylen- oder $C_2$-$C_{10}$-Alkinylen-Gruppe gebunden sein kann,
vorzugsweise für Wasserstoff, einen linearen oder verzweigten, ggf. wenigstens einfach substituierten $C_1$-$C_{10}$-Alkyl-Rest, für einen gesättigten oder ungesättigten, ggf. wenigstens einfach substituierten, ggf. wenigstens ein Heteroatom als Ringglied aufweisenden fünf-, sechs- oder siebengliedrigen cycloaliphatischen Rest, der über eine ggf. wenigstens einfach substituierte, ggf. wenigstens ein Heteroatom als Kettenglied aufweisende $C_1$-$C_{10}$-Alkylen-, $C_2$-$C_{10}$-Alkenylen- oder $C_2$-$C_{10}$-Alkinylen-Gruppe gebunden sein kann, oder für einen ggf. wenigstens einfach substituierten fünf- bis zwölfgliedrigen Aryl- oder Heteroaryl-Rest steht, der über eine ggf. wenigstens einfach substituierte, ggf. wenigstens ein Heteroatom als Kettenglied aufweisende $C_1$-$C_{10}$-Alkylen-, $C_2$-$C_{10}$-Alkenylen- oder $C_2$-$C_{10}$-Alkinylen-Gruppe gebunden sein kann,
besonders bevorzugt für einen linearen oder verzweigten, ggf. wenigstens einfach substituierten $C_{1-3}$ Alkyl-Rest, für einen über eine $C_{1-3}$-Alkylen-Gruppe gebundenen cycloaliphatischen Rest ausgewählt aus der Gruppe bestehend aus

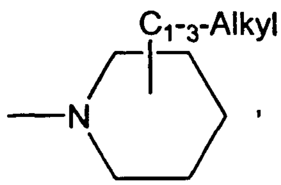 , 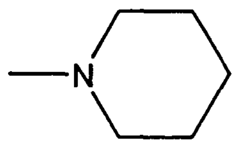 , 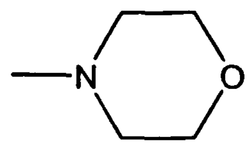 ,

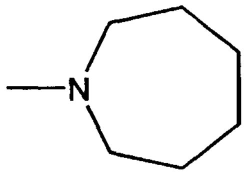 , 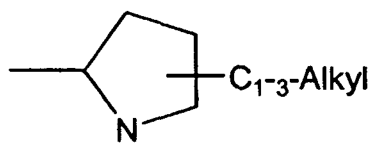 , 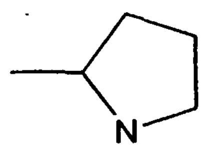 ,

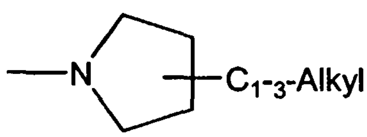 und 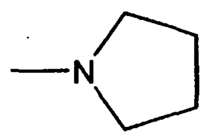 ,

oder für einen cycloaliphatischen Rest

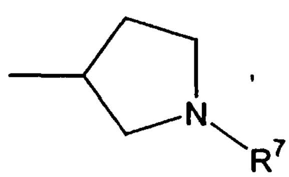 ,

wobei der Rest $R^7$ für einen über eine Methylen-Gruppe gebundenen Phenyl- oder Benzofuranyl-Rest steht, der einfach oder mehrfach, gleich oder verschieden, mit einem Substituenten ausgewählt aus der Gruppe bestehend aus F, Cl, Br, $CF_3$, $CHF_2$, $CH_2F$, Phenoxy, Benzyloxy, Phenyl, $C_{1-4}$-Alkoxy oder $C_{1-4}$ Alkylthio substituiert sein kann,
oder $R^4$ für einen ggf. über eine Methylen-Gruppe gebundenen Phenyl Rest steht, der einfach oder mehrfach, gleich oder verschieden, mit einem Substituenten ausgewählt aus der Gruppe bestehend aus Di-($C_{1-3}$)-Alkylamino. $C_{1-3}$-Methoxy und Morpholinyl substituiert sein kann, und $R^1$ bis $R^3$, $R^5$ und $R^6$ die vorstehend angegebene Bedeutung haben,

ggf. in Form ihrer reinen Stereoisomeren, insbesondere Enantiomeren oder Diastereomeren, ihrer Racemate oder in Form von Mischungen der Stereoisomeren, insbesondere der Enantiomeren und/oder Diastereomeren, in einem beliebigen Mischungsverhältnis, oder ggf. in Form entsprechender Salze oder jeweils in Form entsprechender Solvate.

Bevorzugte substituierte 4,5,6,7-Tetrahydro-benzothiazol-2-ylamin-Verbindungen der allgemeinen Formel I rind ferner solche, in denen $R^5$ und $R^6$ gemeinsam mit dem sie verbrückenden Stickstoffatom als Ringglied einen gesättigten, ungesättigten oder aromatischen, ggf. wenigstens einfach substituierten, ggf. wenigstens ein weiteres Heteroatom als Ringglied aufweisenden 5-, 6- oder 7-gliedrigen heterocyclischen Rest bilden,
vorzugsweise gemeinsam mit dem sie verbrückenden Stickstoffatom als Ringglied einen Rest ausgewählt aus der Gruppe

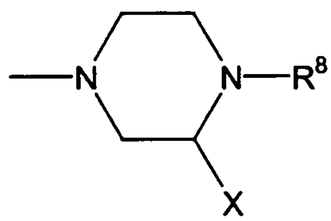

,

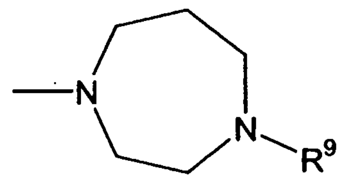

und

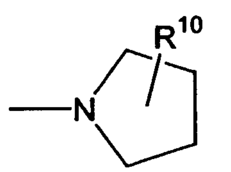

bilden, wobei
X für Wasserstoff oder einen $C_{1-3}$-Alkyl-Rest, vorzugsweise für Wasserstoff oder einen Methylrest, steht,
$R^8$, $R^9$, $R^{10}$ jeweils für einen linearen oder verzweigten, gesättigten oder ungesättigten, ggf. wenigstens einfach substituierten aliphatischen $C_{1-6}$ Rest, für einen ggf. über eine ggf. wenigstens einfach substituierte, ggf. wenigstens ein Heteroatom oder ggf. wenigstens eine Carbonyl-(C=O)-Gruppe als Kettenglied aufweisende $C_{1-6}$-Alkylen-, $C_{2-6}$-Alkenylen- oder $C_{2-6}$-Alkinylen-Gruppe gebundenen, gesättigten oder ungesättigten, ggf. wenigstens einfach substituierten, ggf. wenigstens ein Heteroatom als Ringglied enthaltenden 5-, 6- oder 7-gliedrigen cycloaliphatischen Rest, der mit einem ggf. wenigstens einfach substituierten, mono- oder polyzyklischen Ringsystem kondensiert sein kann, oder für einen ggf. über eine ggf. wenigstens einfach substituierte, ggf. wenigstens ein Heteroatom als Kettenglied aufweisende $C_{1-6}$-Alkylen-, $C_{2-6}$-Alkenylen- oder $C_{2-6}$-Alkinylen-Gruppe gebundenen, ggf. wenigstens einfach substituierten 5- oder 6-gliedrigen Aryl- oder Heteroaryl-Rest, der mit einem ggf. wenigstens einfach substituierten, mono- oder polyzyklischen Ringsystem kondensiert sein kann,
vorzugsweise
$R^8$ für einen ggf. mit einer Di-($C_{1-3}$-Alkyl)-amino-Gruppe substituierten $C_{1-3}$-Alkyl-Rest, einen ggf. wenigstens einfach substituierten Phenyl-Rest, einen ggf. wenigstens einfach substituierten Naphtyhl-Rest, einen ggf. wenigstens einfach substituierten Pyridinyl-Rest, einen ggf. wenigstens einfach substituierten Furanyl-Rest, einen ggf. wenigstens einfach substituierten Thiohenyl-Rest, einen ggf. wenigstens einfach substituierten Pyrroldinyl-Rest, einen ggf. wenigstens einfach substituierten Benzo[1,3]-dioxolyl-Rest oder für einen ggf. wenigstens einfach substituierten Benzofuranyl-Rest steht, wobei die zyklischen Reste jeweils über eine ggf. eine Carbonyl-(C=O)-Gruppe als Kettenglied aufweisende $C_{1-3}$-Alkylen- oder $C_{2-3}$-Alkenylen-Gruppe gebunden und/oder einfach oder mehrfach, gleich oder verschieden, mit einem Substituenten ausgewählt aus der Gruppe bestehend aus - (C=O)-$C_{1-3}$-Alkyl, $C_{1-3}$-Alkoxy, $C_{1-3}$-Alkyl, F, Cl, Br, -CN, $CF_3$, $CF_2H$ und $CFH_2$ substituiert sein können,
$R^9$ für einen linearen oder verzweigten $C_{1-3}$-Alkyl-Rest steht,
und
$R^{10}$ für einen über eine $C_{1-2}$-Alkylen-Gruppe gebundenen Pyrrolidinyl-Rest steht, und jeweils die übrigen Reste $R^1$ bis $R^4$ die vorstehend genannte Bedeutung haben, ggf. in Form ihrer reinen Stereoisomeren, insbesondere Enantiomeren oder Diastereomeren, ihrer Racemate oder in Form von Mischungen der Stereoisomeren, insbesondere der Enantiomeren und/oder Diastereomeren, in einem beliebigen Mischungsverhältnis, oder ggf. in Form entsprechender Salze oder jeweils in Form entsprechender Solvate.

Besonders bevorzugt sind substituierte 4,5,6,7-Tetrahydro-benzothiazol-2-ylamin-Verbindungen der allgemeinen Formel I

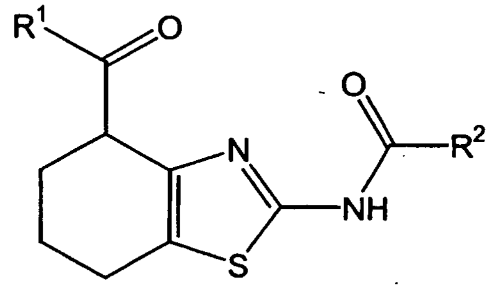

I

worin

$R^1$ für eine Gruppe $-NR^3R^4$ oder für eine Gruppe $-NR^5R^6$ steht,

$R^2$ für einen linearen oder verzweigten $C_{1-5}$-Alkyl-Rest steht, für einen linearen oder verzweigten $C_{2-5}$-Alkenyl-Rest steht, für einen ggf. über eine $-(CH_2)$-Gruppe gebundenen Cyclohexyl-Rest steht, für einen gegebenenfalls über eine $-(CH_2)$- oder -(CH=CH)-Gruppe gebundenen 1- oder 2-Naphthyl-Rest steht, für einen gegebenenfalls über eine $-(CH_2)$- oder -(CH=CH)-Gruppe gebundenen 2- oder 3-Furanyl-Rest steht, für einen gegebenenfalls über eine $-(CH_2)$- oder -(CH=CH)-Gruppe gebundenen 2- oder 3-Thienyl-Rest steht, oder für einen gegebenenfalls über eine $-(CH_2)$-, $-(CH_2)_2$-, $-C(H)(CH_3)$- oder $-(CH_2)$-O-Gruppe gebundenen, unsubstituierten oder wenigstens einfach substituierten Phenyl-Rest steht, wobei dessen Substituenten unabhängig voneinander vorzugsweise ausgewählt werden können aus der Gruppe bestehend aus -F, -Cl, -Br, $-OCH_3$, $-OC_2H_5$, $CH_3$ und $C_2H_5$,

$R^3$ für einen Wasserstoff- oder Methyl-Rest steht,

$R^4$ für einen linearen oder verzweigten, ggf. wenigstens einfach substituierten $C_{1-3}$ Alkyl-Rest, für einen über eine $C_{1-3}$-Alkylen-Gruppe gebundenen cycloaliphatischen Rest ausgewählt aus der Gruppe bestehend aus

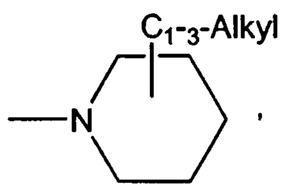 , 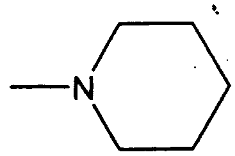 , 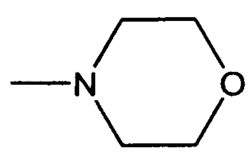 ,

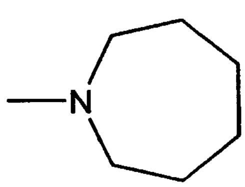 , 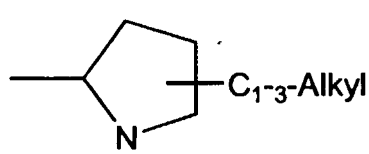 , 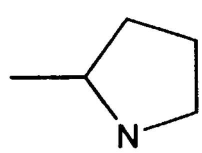 ,

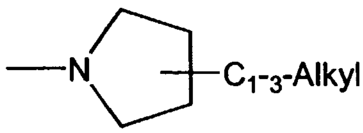

und

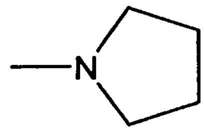

,

oder für einen cycloaliphatischen Rest

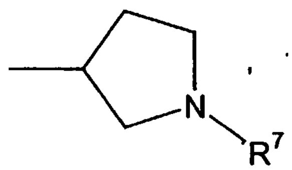

,

steht, wobei der Rest $R^7$ für einen über eine Methylen-Gruppe gebundenen Phenyl- oder Benzofuranyl-Rest steht, der einfach oder mehrfach, gleich oder verschieden, mit einem Substituenten ausgewählt aus der Gruppe bestehend aus F, Cl, Br, $CF_3$, $CHF_2$, $CH_2F$, Phenoxy, Benzyloxy, Phenyl, $C_{1-4}$-Alkoxy oder $C_{1-4}$ Alkylthio substituiert sein kann,
oder der Rest $R^4$ für ggf. über eine Methylen-Gruppe gebundenen Phenyl Rest steht, der einfach oder mehrfach, gleich oder verschieden, mit einem Substituenten ausgewählt aus der Gruppe bestehend aus Di-($C_{1-3}$)-Alkylamino. $C_{1-3}$-Methoxy und Morpholinyl substituiert sein kann,
$R^5$ und $R^6$ gemeinsam mit dem sie verbrückenden Stickstoffatom einen Rest ausgewählt aus der Gruppe

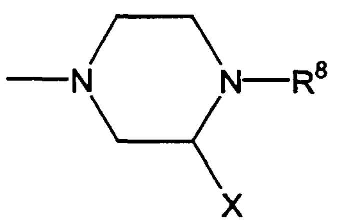

,

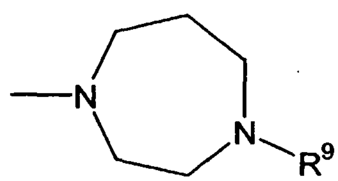

und

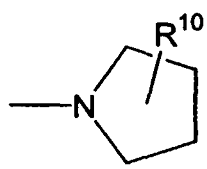

bilden, wobei
X für einen Wasserstoff-Rest oder eine Methyl-Gruppe steht,
$R^8$ für einen ggf. mit einer Dimethylamino-Gruppe substituierten, linearen oder verzweigten $C_{1-3}$-Alkyl-Rest steht, einen ggf. wenigstens einfach substituierten Phenyl-Rest, einen ggf. wenigstens einfach substituierten Naphthyl-Rest, einen ggf. wenigstens einfach substituierten Pyridinyl-Rest, einen ggf. wenigstens einfach substituierten Furanyl-Rest, einen ggf. wenigstens einfach substituierten Thiohenyl-Rest, einen ggf. wenigstens einfach substituierten Pyrroldinyl-Rest, einen ggf. wenigstens einfach substituierten Benzo[1,3]-dioxolyl-Rest oder für einen ggf. wenigstens einfach substituierten Benzofuranyl-Rest steht, wobei die zyklischen Reste jeweils über eine ggf. eine Carbonyl-(C=O)-Gruppe als Kettenglied aufweisende $C_{1-3}$-Alkylen- oder $C_{2-3}$-Alkenylen-Gruppe gebunden und/oder einfach oder mehrfach, gleich oder verschieden, mit einem Substituenten ausgewählt aus der Gruppe bestehend aus -(C=O)-$C_{1-3}$-Alkyl, $C_{1-3}$-Alkoxy, F, Cl, Br, -CN, $CF_3$, $CF_2H$ und $CFH_2$ substituiert sein können,

$R^9$ für einen Methyl- oder Ethyl-Rest steht, und
$R^{10}$ für einen über eine -($CH_2$)-Gruppe gebundenen Pyrrolidinyl-Rest steht, ggf. in Form ihrer reinen Stereoisomeren, insbesondere Enantiomeren oder Diastereomeren, ihrer Racemate oder in Form von Mischungen der Stereoisomeren, insbesondere der Enantiomeren und/oder Diastereomeren, in einem beliebigen Mischungsverhältnis, oder jeweils ggf. in Form entsprechender Salze oder jeweils ggf. in Form entsprechender Solvate.

Ein Gegenstand der vorliegenden Erfindung sind ebenfalls substituierte 4,5,6,7-Tetrahydro-benzothiazol-2-ylamin-Verbindungen der allgemeinen Formel I, worin

$R^1$ für eine Gruppe $-NR^3R^4$ oder für eine Gruppe $-NR^5R^6$ steht,

$R^2$ für einen linearen oder verzweigten, gesättigten oder ungesättigten, ggf. substituierten $C_{1-10}$ aliphatischen Rest;
für einen ungesättigten oder gesättigten, ggf. substituierten 3-, 4-, 5-, 6-, 7-, 8- oder 9-gliedrigen cycloaliphatischen Rest, der über eine lineare oder verzweigte, ggf. 1 oder 2 Heteroatom(e) als Kettenglied(er) aufweisende, ggf. substituierte $C_{1-5}$-Alkylen-, $C_{2-5}$-Alkenylen- oder $C_{2-5}$-Alkinylen-Gruppe gebunden sein kann,
oder für einen ggf. substituierten 5- bis 14-gliedrigen Aryl- oder Heteroaryl- Rest, der über eine lineare oder verzweigte, ggf. 1 oder 2 Heteroatom(e) als Kettenglied(er) aufweisende, ggf. substituierte $C_{1-5}$-Alkylen-, $C_{2-5}$-Alkenylen- oder $C_{2-5}$-Alkinylen-Gruppe gebunden sein kann, steht;

$R^3$ für einen Wasserstoff-Rest;
für einen linearen oder verzweigten, gesättigten oder ungesättigten, ggf. substituierten $C_{1-10}$ aliphatischen Rest;
für einen ungesättigten oder gesättigten, ggf. substituierten 3-, 4-, 5-, 6-, 7-, 8- oder 9-gliedrigen cycloaliphatischen Rest, der über eine lineare oder verzweigte, ggf. 1 oder 2 Heteroatom(e) als Kettenglied(er) aufweisende, ggf. substituierte $C_{1-5}$-Alkylen-, $C_{2-5}$-Alkenylen- oder $C_{2-5}$-Alkinylen-Gruppe gebunden sein kann,
oder für einen ggf. substituierten 5- bis 14-gliedrigen Aryl- oder Heteroaryl- Rest, der über eine lineare oder verzweigte, ggf. 1 oder 2 Heteroatom(e) als Kettenglied(er) aufweisende, ggf. substituierte $C_{1-5}$-Alkylen-, $C_{2-5}$-Alkenylen- oder $C_{2-5}$-Alkinylen-Gruppe gebunden sein kann, steht;

$R^4$ für einen Wasserstoff-Rest;
für einen linearen oder verzweigten, gesättigten oder ungesättigten, ggf. substituierten $C_{1-10}$ aliphatischen Rest;
für einen ungesättigten oder gesättigten, ggf. substituierten 3-, 4-, 5-, 6-, 7-, 8- oder 9-gliedrigen cycloaliphatischen Rest, der über eine lineare oder verzweigte, ggf. 1 oder 2 Heteroatom(e) als Kettenglied(er) aufweisende, ggf. substituierte $C_{1-5}$-Alkylen-, $C_{2-5}$-Alkenylen- oder $C_{2-5}$-Alkinylen-Gruppe gebunden sein kann,
oder für einen ggf. substituierten 5- bis 14-gliedrigen Aryl- oder Heteroaryl- Rest, der über eine lineare oder verzweigte, ggf. 1 oder 2 Heteroatom(e) als Kettenglied(er) aufweisende, ggf. substituierte $C_{1-5}$-Alkylen-, $C_{2-5}$-Alkenylen- oder $C_{2-5}$-Alkinylen-Gruppe gebunden sein kann, steht;

$R^5$ und $R^6$ zusammen mit dem sie verbindenden Stickstoffatom als Ringglied einen gesättigten oder ungesättigten, ggf. substituierten 4-, 5-, 6-, 7-, 8- oder 9-gliedrigen heterocycloaliphatischen Rest bilden, wobei jeweils der heterocycloaliphatische Rest mit einem Rest $R^8$ und ggf. einem Rest X oder einem Rest $R^9$ oder einem Rest $R^{10}$ substituiert sein und/oder jeweils weitere 1, 2 oder 3 Heteroatom(e) unabhängig voneinander ausgewählt aus der X Gruppe bestehend aus Sauerstoff, Stickstoff und Schwefel als Ringglied(er) aufweisen kann; für einen linearen oder verzweigten, gesättigten oder ungesättigten $C_{1-10}$ aliphatischen Rest steht;

$R^8$, $R^9$ und $R^{10}$, unabhängig voneinander, jeweils
für einen linearen oder verzweigten, gesättigten oder ungesättigten, ggf. substituierten $C_{1-10}$ aliphatischen Rest;
für einen ungesättigten oder gesättigten, ggf. substituierten 3-, 4-, 5-, 6-, 7-, 8- oder 9-gliedrigen cycloaliphatischen Rest, der über eine lineare oder verzweigte, ggf. 1 oder 2 Heteroatom(e) als Kettenglied(er) und/oder ggf. eine Carbonyl-(C=O)-Gruppe als Kettenglied aufweisende, ggf. substituierte $C_{1-5}$- Alkylen-, $C_{2-5}$-Alkenylen- oder $C_{2-5}$-Alkinylen-Gruppe gebunden und/oder mit einem gesättigten, ungesättigten oder aromatischen, ggf. substituierten mono- oder polyzyklischen Ringsystem kondensiert sein kann,
oder für einen ggf. substituierten 5- bis 14-gliedrigen Aryl- oder Heteroaryl- Rest, der über eine lineare oder verzweigte, ggf. 1 oder 2 Heteroatom(e) als Kettenglied(er) und/oder ggf. eine Carbonyl-(C=O)-Gruppe als Kettenglied aufweisende, ggf. substituierte $C_{1-5}$-Alkylen-, $C_{2-5}$-Alkenylen- oder $C_{2-5}$- Alkinylen-Gruppe gebunden und/oder mit einem gesättigten oder ungesättigten, ggf. substituierten mono- oder polyzyklischen Ringsystem kondensiert sein kann, steht;

wobei
die vorstehend genannten $C_{1-10}$ aliphatischen Reste jeweils mit 1, 2, 3, 4 oder 5 Substituenten unabhängig voneinander ausgewählt aus der Gruppe bestehend aus F, Cl, Br, -CN, $-NO_2$, Hydroxy, $C_{1-6}$-Alkoxy, $-NH_2$, -NH-($C_{1-3}$-Alkyl), -N ($C_{1-3}$-Alkyl)($C_{1-3}$-Alkyl), -NH-Phenyl, -N($C_{1-3}$-Alkyl)-Phenyl und -N(Phenyl)$_2$ substituiert sein können; die vorstehend genannten cycloaliphatischen Reste jeweils mit 1, 2, 3, 4 oder 5 Substituenten unabhängig voneinander ausgewählt aus der Gruppe bestehend aus F, Cl, Br, -CN, $-CF_3$, $-CHF_2$, $-CH_2F$, Hydroxy, -$C_{1-6}$-Alkoxy, $-NH_2$, -NH-($C_{1-3}$-Alkyl), - N ($C_{1-3}$-Alkyl)($C_{1-3}$-Alkyl), -NH-Phenyl, -N($C_{1-3}$-Alkyl)-Phenyl, -N(Phenyl)$_2$, SH, -$C_{1-6}$-Alkylthio, -$C_{1-6}$-Alkyl, -($CH_2$)-Benzo [b]furanyl, Phenoxy, Benzyloxy, Phenyl und Benzyl substituiert sein können, wobei jeweils der zyklische Teil der Reste Phenoxy, Benzyloxy, Phenyl, -($CH_2$)-Benzo[b]furanyl und Benzyl mit 1, 2, 3, 4 oder 5 Substituenten unabhängig voneinander ausgewählt aus der Gruppe bestehend aus F, Cl, Br, -$C_{1-4}$-Alkyl, $-CF_3$, $-CHF_2$, $-CH_2F$, -$C_{1-4}$-Alkoxy, -$C_{1-4}$-Alkylthio, Phenoxy, Phenyl, und Benzyloxy substituiert sein kann
und die vorstehend genannten cycloaliphatischen Reste jeweils 1, 2 oder 3 Heteroatom(e) unabhängig voneinander ausgewählt aus der Gruppe bestehend aus Sauerstoff, Stickstoff und Schwefel aufweisen können;
die vorstehend genannten $C_{1-5}$-Alkylen-, $C_{2-5}$-Alkenylen- oder $C_{2-5}$-Alkinylen-Gruppen jeweils mit 1, 2, 3, 4 oder 5 Substituenten unabhängig voneinander ausgewählt aus der Gruppe bestehend aus F, Cl, Br, -CN, $-NO_2$, Hydroxy, $C_{1-6}$-Alkoxy, $-NH_2$, -NH-($C_{1-3}$-Alkyl), -N($C_{1-3}$-Alkyl)($C_{1-3}$-Alkyl), -NH-Phenyl, -N($C_{1-3}$-Alkyl)-Phenyl, N(Phenyl)$_2$ und Phenyl substituiert sein können und
die vorstehend genannten $C_{1-5}$-Alkylen-, $C_{2-5}$-Alkenylen- oder $C_{2-5}$-Alkinylen-Gruppen jeweils 1 oder 2 Heteroatom(e) unabhängig voneinander ausgewählt aus der Gruppe bestehend aus Sauerstoff, Stickstoff und Schwefel aufweisen können;
die vorstehend genannten Aryl- oder Heteroaryl-Reste jeweils mit 1, 2, 3, 4 oder 5 Substituenten unabhängig voneinander ausgewählt aus der Gruppe bestehend aus F, Cl, Br, -CN, $-CF_3$, $-CHF_2$, $-CH_2F$, Hydroxy, -$C_{1-6}$-Alkoxy, $-O-CF_3$, $-S-CF_3$, SH, -$C_{1-6}$-Alkylthio, -$C_{1-6}$-Alkyl, $-NH_2$, -NH-($C_{1-3}$-Alkyl), -N($C_{1-3}$-Alkyl)($C_{1-3}$-Alkyl), -NH-Phenyl, - N($C_{1-3}$-Alkyl)-Phenyl, N(Phenyl)$_2$, Phenoxy, Benzyloxy, Phenyl, Benzyl und Morpholinyl substituiert sein können, wobei jeweils der zyklische Teil der Reste Phenoxy, Benzyloxy, Phenyl und Benzyl mit 1, 2, 3, 4 oder 5 Substituenten unabhängig voneinander ausgewählt aus der Gruppe bestehend aus F, Cl, Br, Hydroxy, $-CF_3$, $-SF_5$, -CN, $-NO_2$, -$C_{1-6}$-Alkoxy, $-O-CF_3$, $-S-CF_3$, Phenyl und Benzyloxy substituiert sein kann,
und die vorstehend genannten Heteroaryl-Reste jeweils 1, 2, 3, 4 oder 5 Heteroatom(e) unabhängig voneinander ausgewählt aus der Gruppe bestehend aus Sauerstoff, Stickstoff und Schwefel als Ringglied(er) aufweisen können;
und die Ringe der vorstehend genannten mono- oder polyzyklischen Ringsysteme ggf. jeweils mit 1, 2, 3, 4 oder 5 Substituenten unabhängig voneinander ausgewählt aus der Gruppe bestehend aus F, Cl, Br, -CN, $-CF_3$, $-CHF_2$, $-CH_2F$, Hydroxy, -$C_{1-6}$-Alkoxy, $-O-CF_3$, $-S-CF_3$, SH, -C1$_{-6}$-Alkylthio, -$C_{1-6}$-Alkyl, $-NH_2$, -NH-($C_{1-3}$-Alkyl), -N($C_{1-3}$-Alkyl)($C_{1-3}$-Alkyl), -NH-Phenyl, -N($C_{1-3}$-Alkyl)-Phenyl, N(Phenyl)$_2$, Phenoxy, Benzyloxy, Phenyl, Benzyl und Morpholinyl substituiert sein können, wobei jeweils der zyklische Teil der Reste Phenoxy, Benzyloxy, Phenyl und Benzyl mit 1, 2, 3, 4 oder 5 Substituenten unabhängig voneinander ausgewählt aus der Gruppe bestehend aus F, Cl, Br, Hydroxy, $-CF_3$, $-SF_5$, -CN, $-NO_2$, -$C_{1-6}$-Alkoxy, $-O-CF_3$, $-S-CF_3$, Phenyl und Benzyloxy substituiert sein kann,
und die Ringe der vorstehend genannten mono- oder polyzyklischen Ringsysteme jeweils 5-, 6- oder 7-gliedrig sind und jeweils 1, 2, 3, 4 oder 5 Heteroatom(e) als Ringglied(er) aufweisen können, die unabhängig voneinander aus der Gruppe bestehend aus Sauerstoff, Stickstoff und Schwefel ausgewählt sind;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls bevorzugt sind substituierte 4,5,6,7-Tetrahydro-benzothiazol-2-ylamin-Verbindungen der allgemeinen Formel I, in denen $R^2$
für einen Alkyl-Rest ausgewählt aus der Gruppe bestehend aus Methyl, Ethyl, n-Propyl, iso-Propyl, n-Butyl, sec-Butyl, iso-Butyl, tert-Butyl, n-Pentyl, sec-Pentyl, (1,1)-Dimethylpropyl und n-Hexyl steht, wobei der Alkyl-Rest jeweils mit 1, 2, 3, 4 oder 5 Substituenten unabhängig voneinander ausgewählt aus der Gruppe bestehend aus F, Cl, Br, -CN, $-NO_2$, Hydroxy, $C_{1-6}$-Alkoxy, $-NH_2$, -NH-($C_{1-3}$-Alkyl), -N($C_{1-3}$-Alkyl)($C_{1-3}$-Alkyl), -NH-Phenyl, -N($C_{1-3}$-Alkyl)-Phenyl und -N(Phenyl)$_2$ substituiert sein kann;
für einen Alkenyl-Rest ausgewählt aus der Gruppe bestehend aus Vinyl, 1-Propenyl, 2-Propenyl, 1-Butenyl, 2-Butenyl,

3-Butenyl, 1-Pentenyl, 2-Pentenyl und Pent-1,3-dienyl steht, wobei der Alkenyl-Rest jeweils mit 1, 2, 3, 4 oder 5 Substituenten unabhängig voneinander ausgewählt aus der Gruppe bestehend aus F, Cl, Br, -CN, - $NO_2$, Hydroxy, $C_{1-6}$-Alkoxy, $-NH_2$, -NH-($C_{1-3}$-Alkyl), -N($C_{1-3}$-Alkyl)($C_{1-3}$-Alkyl), -NH-Phenyl, -N($C_{1-3}$-Alkyl)-Phenyl und -N$(Phenyl)_2$ substituiert sein kann;

für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl steht, wobei der (hetero)cycloaliphatische Rest jeweils über eine lineare oder verzweigte, unsubstituierte $C_{1-5}$-Alkylen-Gruppe gebunden und/oder mit 1, 2, 3, 4 oder 5 Substituenten unabhängig voneinander ausgewählt aus der Gruppe bestehend aus F, Cl, Br, -CN, $-CF_3$, $-CHF_2$, $-CH_2F$, Hydroxy, -$C_{1-6}$-Alkoxy, $-NH_2$, -NH-($C_{1-3}$-Alkyl), - N($C_{1-3}$-Alkyl)($C_{1-3}$-Alkyl), -NH-Phenyl, -N($C_{1-3}$-Alkyl)-Phenyl, -N$(Phenyl)_2$, SH, -$C_{1-6}$-Alkylthio, -$C_{1-6}$-Alkyl, -$(CH_2)$-Benzo[b]furanyl, Phenoxy, Benzyloxy, Phenyl und Benzyl substituiert sein kann, wobei jeweils der zyklische Teil der Reste Phenoxy, Benzyloxy, Phenyl, -$(CH_2)$-Benzo[b]furanyl und Benzyl mit 1, 2, 3, 4 oder 5 Substituenten unabhängig voneinander ausgewählt aus der Gruppe bestehend aus F, Cl, Br, -$C_{1-4}$-Alkyl, $-CF_3$, $-CHF_2$, $-CH_2F$, -$C_{1-4}$-Alkoxy, -$C_{1-4}$-Alkylthio, Phenoxy, Phenyl, und Benzyloxy substituiert sein kann;

oder für einen Rest ausgewählt aus der Gruppe bestehend aus Phenyl, Naphthyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl und Isochinolinyl steht, wobei der Rest jeweils über eine lineare oder verzweigte, unsubstituierte, ggf. ein Sauerstoffatom als Kettenglied aufweisende $C_{1-5}$-Alkylen- oder $C_{2-5}$-Alkenylen-Gruppe gebunden und/oder mit 1, 2, 3, 4 oder 5 Substituenten unabhängig voneinander ausgewählt aus der Gruppe bestehend aus F, Cl, Br, -CN, $-CF_3$, $-CHF_2$, $-CH_2F$, Hydroxy, -$C_{1-6}$-Alkoxy, $-O-CF_3$, $-S-CF_3$, SH, -$C_{1-6}$-Alkylthio, -$C_{1-6}$-Alkyl, $-NH_2$, -NH-($C_{1-3}$-Alkyl), -N($C_{1-3}$-Alkyl)($C_{1-3}$-Alkyl), -NH-Phenyl, - N($C_{1-3}$-Alkyl)-Phenyl, N$(Phenyl)_2$, Phenoxy, Benzyloxy, Phenyl, Benzyl und Morpholinyl substituiert sein kann, wobei jeweils der zyklische Teil der Reste Phenoxy, Benzyloxy, Phenyl und Benzyl mit 1, 2, 3, 4 oder 5 Substituenten unabhängig voneinander ausgewählt aus der Gruppe bestehend aus F, Cl, Br, Hydroxy, $-CF_3$, $-SF_5$, -CN, $-NO_2$, -$C_{1-6}$-Alkoxy, $-O-CF_3$, $-S-CF_3$, Phenyl und Benzyloxy substituiert sein kann;

bevorzugt $R^2$

für einen Alkyl-Rest ausgewählt aus der Gruppe bestehend aus Methyl, Ethyl, n-Propyl, iso-Propyl, n-Butyl, sec-Butyl, iso-Butyl, tert-Butyl, n-Pentyl, sec-Pentyl, (1,1)-Dimethylpropyl und n-Hexyl steht;

für einen Alkenyl-Rest ausgewählt aus der Gruppe bestehend aus Vinyl, 1-Propenyl, 2-Propenyl, 1-Butenyl, 2-Butenyl, 3-Butenyl, 1-Pentenyl, 2-Pentenyl und Pent-1,3-dienyl steht;

für einen cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl und Cycloheptenyl steht, der über eine $-(CH_2)-$, $-(CH_2)_2-$, $-CH(CH_3)-$oder $-(CH_2)_3$-Gruppe gebunden und/oder mit 1, 2, 3, 4 oder 5 Substituenten unabhängig voneinander ausgewählt aus der Gruppe bestehend aus F, Cl, Br, -CN, - $CF_3$, $-CHF_2$, $-CH_2F$, Hydroxy, $-O-CH_3$, $-O-C_2H_5$, $-O-C_3H_7$, $-NH_2$, $-NH-CH_3$, $-NH-C_2H_5$, - $N-(CH_3)_2$, $-N(C_2H_5)_2$, $-N(CH_3)(C_2H_5)$, -NH-Phenyl, $-N(CH_3)$-Phenyl, $-N(C_2H_5)$-Phenyl, - N$(Phenyl)_2$, SH, $-S-CH_3$, $-S-C_2H_5$, $-S-C_3H_7$, $-S-C(CH_3)_3$, Methyl, Ethyl, n-Propyl, Isopropyl, n-Butyl, sec-Butyl und tert-Butyl substituiert sein kann;

oder für einen Rest ausgewählt aus der Gruppe bestehend aus Phenyl, 1-Naphthyl, 2-Naphthyl, 2-Furanyl, 3-Furanyl, 2-Thiophenyl oder 3-Thiophenyl steht, der über eine $-(CH_2)-$, $-(CH_2)_2-$, $-(CH_2)-O-$, $-CH(CH_3)-$, $-(CH=CH)-$ oder $-(CH_2)_3$-Gruppe gebunden und/oder mit 1, 2, 3, 4 oder 5 Substituenten unabhängig voneinander ausgewählt aus der Gruppe bestehend aus F, Cl, Br, -CN, $-CF_3$, $-CHF_2$, $-CH_2F$, Hydroxy, $-O-CH_3$, $-O-C_2H_5$, $-O-C_3H_7$, $-O-CF_3$, $-S-CF_3$, -SH, $-S-CH_3$, $-S-C_2H_5$, $-S-C_3H_7$, $-S-C(CH_3)_3$, Methyl, Ethyl, n-Propyl, Isopropyl, n-Butyl, sec-Butyl, tert-Butyl, $-NH_2$, - $NH-CH_3$, $-NH-C_2H_5$, $-N-(CH_3)_2$, $-N(C_2H_5)_2$, $-N(CH_3)(C_2H_5)$, -NH-Phenyl, $-N(CH_3)$-Phenyl, $-N(C_2H_5)$-Phenyl, -N$(Phenyl)_2$, Phenoxy, Benzyloxy, Phenyl, Benzyl und Morpholinyl substituiert sein kann;

besonders bevorzugt $R^2$

für einen Alkyl-Rest ausgewählt aus der Gruppe bestehend aus n-Propyl, iso-Propyl, n-Butyl, sec-Butyl, iso-Butyl, tert-Butyl, n-Pentyl, sec-Pentyl und (1,1)-Dimethylpropyl steht;

für einen Alkenyl-Rest ausgewählt aus der Gruppe bestehend aus 1-Pentenyl, 2-Pentenyl und Pent-1,3-dienyl steht;

für einen cycloaliphatischen Rest ausgewählt aus der Gruppe bestehend aus Cyclopentyl, Cyclohexyl und Cycloheptyl steht, der über eine $-(CH_2)$-Gruppe gebunden ist;

oder für einen Rest ausgewählt aus der Gruppe bestehend aus Phenyl, 1-Naphthyl, 2-Naphthyl, 2-Furanyl, 3-Furanyl, 2-Thiophenyl oder 3-Thiophenyl steht, der über eine $-(CH_2)-$, $-(CH_2)_2-$, $-(CH_2)-O-$, $-CH(CH_3)-$, $-(CH=CH)-$ oder $-(CH_2)_3$-Gruppe gebunden und/oder mit 1, 2 oder 3 Substituenten unabhängig voneinander ausgewählt aus der Gruppe bestehend aus F, Cl, Br, $-O-CH_3$, $-O-C_2H_5$, Methyl, Ethyl, n-Propyl und Isopropyl substituiert sein kann;

und jeweils die übrigen Reste $R^1$, $R^3$ bis $R^6$, X und $R^8$ bis $R^{10}$ die vorstehend angegebenene Bedeutung haben, ggf. in Form ihrer reinen Stereoisomeren, insbesondere Enantiomeren oder Diastereomeren, ihrer Racemate oder in Form von Mischungen der Stereoisomeren, insbesondere der Enantiomeren und/oder Diastereomeren, in einem beliebigen

Mischungsverhältnis, oder ggf. in Form entsprechender Salze oder jeweils in Form entsprechender Solvate.

Ebenfalls besonders bevorzugt sind substituierte 4,5,6,7-Tetrahydro-benzothiazol-2-ylamin-Verbindungen der allgemeinen Formel, worin

$R^3$ für einen Wasserstoff-Rest steht;

für einen Alkyl-Rest ausgewählt aus der Gruppe bestehend aus Methyl, Ethyl, n-Propyl, iso-Propyl, n-Butyl, sec-Butyl, iso-Butyl, tert-Butyl, n-Pentyl, sec-Pentyl, (1,1)-Dimethylpropyl und n-Hexyl steht, wobei der Alkyl-Rest jeweils mit 1, 2, 3, 4 oder 5 Substituenten unabhängig voneinander ausgewählt aus der Gruppe bestehend aus F, Cl, Br, -CN, -$NO_2$, Hydroxy, $C_{1-6}$-Alkoxy, -$NH_2$, -NH-($C_{1-3}$-Alkyl), -N($C_{1-3}$-Alkyl)($C_{1-3}$-Alkyl), -NH-Phenyl, -N($C_{1-3}$-Alkyl)-Phenyl und -N(Phenyl)$_2$ substituiert sein kann;

für einen Alkenyl-Rest ausgewählt aus der Gruppe bestehend aus Vinyl, 1-Propenyl, 2-Propenyl, 1-Butenyl, 2-Butenyl, 3-Butenyl, 1-Pentenyl, 2-Pentenyl und Pent-1,3-dienyl steht, wobei der Alkenyl-Rest jeweils mit 1, 2, 3, 4 oder 5 Substituenten unabhängig voneinander ausgewählt aus der Gruppe bestehend aus F, Cl, Br, -CN, - $NO_2$, Hydroxy, $C_{1-6}$-Alkoxy, -$NH_2$, -NH-($C_{1-3}$-Alkyl), -N($C_{1-3}$-Alkyl)($C_{1-3}$-Alkyl), -NH-Phenyl, -N($C_{1-3}$-Alkyl)-Phenyl und -N(Phenyl)$_2$ substituiert sein kann;

für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl steht, wobei der (hetero)cycloaliphatische Rest jeweils über eine lineare oder verzweigte, unsubstituierte $C_{1-5}$-Alkylen-Gruppe gebunden und/oder mit 1, 2, 3, 4 oder 5 Substituenten unabhängig voneinander ausgewählt aus der Gruppe bestehend aus F, Cl, Br, -CN, -$CF_3$, -$CHF_2$, -$CH_2F$, Hydroxy, -$C_{1-6}$-Alkoxy, -$NH_2$, -NH-($C_{1-3}$-Alkyl), - N($C_{1-3}$-Alkyl)($C_{1-3}$-Alkyl), -NH-Phenyl, -N($C_{1-3}$-Alkyl)-Phenyl, -N(Phenyl)$_2$, -SH, -$C_{1-6}$-Alkylthio, -$C_{1-6}$-Alkyl, -($CH_2$)-Benzo[b]furanyl, Phenoxy, Benzyloxy, Phenyl und Benzyl substituiert sein kann, wobei jeweils der zyklische Teil der Reste Phenoxy, Benzyloxy, Phenyl, -($CH_2$)-Benzo[b]furanyl und Benzyl mit 1, 2, 3, 4 oder 5 Substituenten unabhängig voneinander ausgewählt aus der Gruppe bestehend aus F, Cl, Br, -$C_{1-4}$-Alkyl, -$CF_3$, -$CHF_2$, -$CH_2F$, -$C_{1-4}$-Alkoxy, -$C_{1-4}$-Alkylthio, Phenoxy, Phenyl, und Benzyloxy substituiert sein kann;

oder für einen Rest ausgewählt aus der Gruppe bestehend aus Phenyl, Naphthyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl und Isochinolinyl steht, wobei der Rest jeweils über eine lineare oder verzweigte, unsubstituierte, ggf. ein Sauerstoffatom als Kettenglied aufweisende $C_{1-5}$-Alkylen- oder $C_{2-5}$-Alkenylen-Gruppe gebunden und/oder mit 1, 2, 3, 4 oder 5 Substituenten unabhängig voneinander ausgewählt aus der Gruppe bestehend aus F, Cl, Br, -CN, -$CF_3$, -$CHF_2$, -$CH_2F$, Hydroxy, -$C_{1-6}$-Alkoxy, -O-$CF_3$, -S-$CF_3$, -SH, -$C_{1-6}$-Alkylthio, -$C_{1-6}$-Alkyl, -$NH_2$, -NH-($C_{1-3}$-Alkyl), -N($C_{1-3}$-Alkyl)($C_{1-3}$-Alkyl), -NH-Phenyl, - N($C_{1-3}$-Alkyl)-Phenyl, N(Phenyl)$_2$, Phenoxy, Benzyloxy, Phenyl, Benzyl und Morpholinyl substituiert sein kann, wobei jeweils der zyklische Teil der Reste Phenoxy, Benzyloxy, Phenyl und Benzyl mit 1, 2, 3, 4 oder 5 Substituenten unabhängig voneinander ausgewählt aus der Gruppe bestehend aus F, Cl, Br, Hydroxy, -$CF_3$, -$SF_5$, -CN, -$NO_2$, -$C_{1-6}$-Alkoxy, -O-$CF_3$, -S-$CF_3$, Phenyl und Benzyloxy substituiert sein kann;

bevorzugt $R^3$

für einen Wasserstoff-Rest steht;

für einen Alkyl-Rest ausgewählt aus der Gruppe bestehend aus Methyl, Ethyl, n-Propyl, n-Butyl und n-Pentyl steht, wobei der Alkyl-Rest jeweils mit 1 oder 2 Substituenten unabhängig voneinander ausgewählt aus der Gruppe bestehend aus $NH_2$, -NH-$CH_3$, -NH-$C_2H_5$, -N-$(CH_3)_2$, -N$(C_2H_5)_2$, -N($CH_3$)($C_2H_5$), -NH-Phenyl, - N($CH_3$)-Phenyl, -N($C_2H_5$)-Phenyl und -N(Phenyl)$_2$ substituiert sein kann;

für einen Rest ausgewählt aus der Gruppe bestehend aus

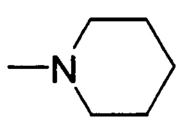 , 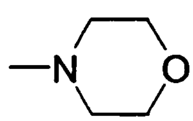 ,

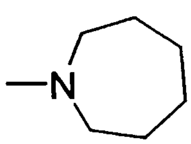 , 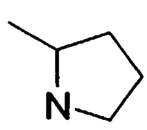 und 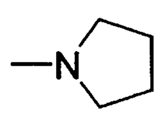 ,

steht, der über eine $-(CH_2)-$, $-(CH_2)_2-$, $-(CH_2)-O-$, $-CH(CH_3)-$, $-(CH=CH)-$ oder $-(CH_2)_3$-Gruppe gebunden und/oder mit 1, 2 oder 3 Substituenten unabhängig voneinander ausgewählt aus der Gruppe bestehend aus Methyl, Ethyl und n-Propyl substituiert sein kann;
für den folgenden Rest,

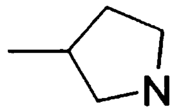 ,

steht, der mit einem Substituenten ausgewählt aus der Gruppe bestehend aus - $(CH_2)$-Benzo[b]furanyl und Benzyl substituiert sein kann, wobei jeweils der zyklische Teil der Reste $-(CH_2)$-Benzo[b]furanyl und Benzyl mit 1, 2, 3, 4 oder 5 Substituenten unabhängig voneinander ausgewählt aus der Gruppe bestehend aus F, Cl, Br, $-C_{1-4}$-Alkyl, $-CF_3$, $-CHF_2$, $-CH_2F$, $-C_{1-4}$-Alkoxy, $-C_{1-4}$-Alkylthio, Phenoxy, Phenyl, und Benzyloxy substituiert sein kann;
oder für einen Rest ausgewählt aus der Gruppe bestehend aus Phenyl und Naphthyl steht, wobei der Rest jeweils eine $-(CH_2)-$, $-(CH_2)_2-$ oder $-(CH_2)_3$-Gruppe gebunden und/oder mit 1, 2 oder 3 Substituenten unabhängig voneinander ausgewählt aus der Gruppe bestehend aus $-C_{1-6}$-Alkoxy, $-C_{1-6}$-Alkyl, $-NH_2$, $-NH-(C_{1-3}$-Alkyl), $-N(C_{1-3}$-Alkyl)$(C_{1-3}$-Alkyl), -NH-Phenyl, $-N(C_{1-3}$-Alkyl)-Phenyl, $N(Phenyl)_2$ und Morpholinyl substituiert sein kann;
besonders bevorzugt $R^3$
für einen Wasserstoff-Rest steht;
für einen Rest ausgewählt aus der Gruppe bestehend aus Methyl, Ethyl, n-Propyl, - $(CH_2)-N(CH_3)_2$, $-(CH_2)-(CH_2)-N(CH_3)_2$, $-(CH_2)-(CH_2)-(CH_2)-N(CH_3)_2$, $-(CH_2)-(CH_2)-N(C_2H_5)_2$, $-(CH_2)-(CH_2)-(CH_2)-N(C_2H_5)_2$, $-(CH_2)-(CH_2)-N(CH_3)$(Phenyl) und $-(CH_2)-(CH_2)-(CH_2)-N(CH_3)$(Phenyl) steht;
für einen Rest ausgewählt aus der Gruppe bestehend aus

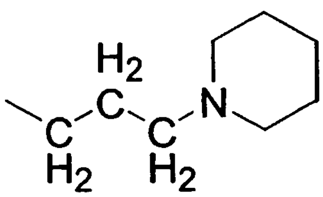 , 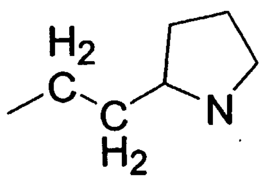 , 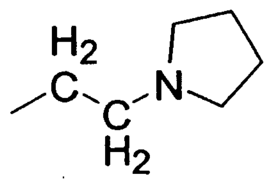 ,

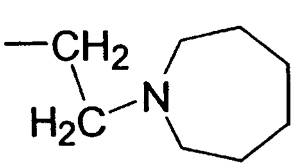 und 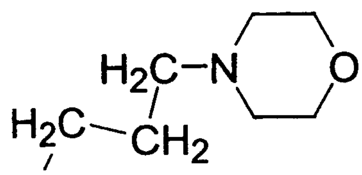 ,

steht, der mit einem Methyl-Rest substituiert sein kann;
für den folgenden Rest,

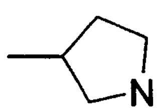 ,

steht, der am Stickstoffatom mit einem Substituenten ausgewählt aus der Gruppe bestehend aus $-(CH_2)$-Benzo[b]furanyl und Benzyl substituiert sein kann, wobei jeweils der zyklische Teil der Reste $-(CH_2)$-Benzo[b]furanyl und Benzyl mit 1, 2, oder 3 Substituenten unabhängig voneinander ausgewählt aus der Gruppe bestehend aus F, Cl, Br, $-O-CH_3$, $-O-C_2H_5$, $-O-C_3H_7$, $-S-CH_3$, $-S-C_2H_5$, $-S-C_3H_7$, $-S-C(CH_3)_3$, Methyl, Ethyl, n-Propyl, Isopropyl, n-Butyl, Phenoxy und Benzyloxy substituiert sein kann;
oder für einen Rest ausgewählt aus der Gruppe bestehend aus Phenyl und Benzyl steht, wobei der zyklische Teil der Reste Phenyl und Benzyl mit 1, 2 oder 3 Substituenten unabhängig voneinander ausgewählt aus der Gruppe bestehend aus - $O-CH_3$, $-O-C_2H_5$, $-O-C_3H_7$, $-N-(CH_3)_2$, $-N(C_2H_5)_2$ und Morpholinyl substituiert sein kann;
ganz besonders bevorzugt
$R^3$ für einen Wasserstoff-Rest
oder für einen Methyl-Rest steht;
und jeweils die übrigen Reste $R^1$, $R^2$, $R^4$ bis $R^6$, X und $R^8$ bis $R^{10}$ die vorstehend angegebenen Bedeutung haben, ggf. in Form ihrer reinen Stereoisomeren, insbesondere Enantiomeren oder Diastereomeren, ihrer Racemate oder in Form von Mischungen der Stereoisomeren, insbesondere der Enantiomeren und/oder Diastereomeren, in einem beliebigen Mischungsverhältnis, oder ggf. in Form entsprechender Salze oder jeweils in Form entsprechender Solvate.

Ebenfalls bevorzugt sind substituierte 4,5,6,7-Tetrahydro-benzothiazol-2-ylamin-Verbindungen der allgemeinen Formel I, worin
$R^4$ für einen Wasserstoff-Rest steht;
für einen Alkyl-Rest ausgewählt aus der Gruppe bestehend aus Methyl, Ethyl, n-Propyl, iso-Propyl, n-Butyl, sec-Butyl, iso-Butyl, tert-Butyl, n-Pentyl, sec-Pentyl, (1,1)-Dimethylpropyl und n-Hexyl steht, wobei der Alkyl-Rest jeweils mit 1, 2, 3, 4 oder 5 Substituenten unabhängig voneinander ausgewählt aus der Gruppe bestehend aus F, Cl, Br, -CN, $-NO_2$, Hydroxy, $C_{1-6}$-Alkoxy, $-NH_2$, -NH-($C_{1-3}$-Alkyl), -N($C_{1-3}$-Alkyl)($C_{1-3}$-Alkyl), -NH-Phenyl, -N($C_{1-3}$-Alkyl)-Phenyl und -N(Phenyl)$_2$ substituiert sein kann;
für einen Alkenyl-Rest ausgewählt aus der Gruppe bestehend aus Vinyl, 1-Propenyl, 2-Propenyl, 1-Butenyl, 2-Butenyl, 3-Butenyl, 1-Pentenyl, 2-Pentenyl und Pent-1,3-dienyl steht, wobei der Alkenyl-Rest jeweils mit 1, 2, 3, 4 oder 5 Substituenten unabhängig voneinander ausgewählt aus der Gruppe bestehend aus F, Cl, Br, -CN, - $NO_2$, Hydroxy, $C_{1-6}$-Alkoxy, $-NH_2$, -NH-($C_{1-3}$-Alkyl), -N($C_{1-3}$-Alkyl)($C_{1-3}$-Alkyl), -NH-Phenyl, -N($C_{1-3}$-Alkyl)-Phenyl und -N(Phenyl)$_2$ substituiert sein kann;
für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl steht, wobei der (hetero)cycloaliphatische Rest jeweils über eine lineare oder verzweigte, unsubstituierte $C_{1-5}$-Alkylen-Gruppe gebunden und/oder mit 1, 2, 3, 4 oder 5 Substituenten unabhängig voneinander ausgewählt aus der Gruppe bestehend aus F, Cl, Br, -CN, $-CF_3$, $-CHF_2$, $-CH_2F$, Hydroxy, -$C_{1-6}$-Alkoxy, $-NH_2$, -NH-($C_{1-3}$-Alkyl), - N($C_{1-3}$-Alkyl)($C_{1-3}$-Alkyl), -NH-Phenyl, -N($C_{1-3}$-Alkyl)-Phenyl, -N(Phenyl)$_2$, -SH, -$C_{1-6}$-Alkylthio, -$C_{1-6}$-Alkyl, -($CH_2$)-Benzo[b]furanyl, Phenoxy, Benzyloxy, Phenyl und Benzyl substituiert sein kann, wobei jeweils der zyklische Teil der Reste Phenoxy, Benzyloxy, Phenyl, -($CH_2$)-Benzo[b]furanyl und Benzyl mit 1, 2, 3, 4 oder 5 Substituenten unabhängig voneinander ausgewählt aus der Gruppe bestehend aus F, Cl, Br, -$C_{1-4}$-Alkyl, $-CF_3$, $-CHF_2$, $-CH_2F$, -$C_{1-4}$-Alkoxy, -$C_{1-4}$-Alkylthio, Phenoxy, Phenyl, und Benzyloxy substituiert sein kann;
oder für einen Rest ausgewählt aus der Gruppe bestehend aus Phenyl, Naphthyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl und Isochinolinyl steht, wobei der Rest jeweils über eine lineare oder verzweigte, unsubstituierte, ggf. ein Sauerstoffatom als Kettenglied aufweisende $C_{1-5}$-Alkylen- oder $C_{2-5}$-Alkenylen-Gruppe gebunden und/oder mit 1, 2, 3, 4 oder 5 Substituenten unabhängig voneinander ausgewählt aus der Gruppe bestehend aus F, Cl, Br, -CN, $-CF_3$, $-CHF_2$, $-CH_2F$, Hydroxy, -$C_{1-6}$-Alkoxy, $-O-CF_3$, $-S-CF_3$, -SH, -$C_{1-6}$-Alkylthio,- $C_{1-6}$-Alkyl, $-NH_2$, -NH-($C_{1-3}$-Alkyl), -N($C_{1-3}$-Alkyl)($C_{1-3}$-Alkyl), -NH-Phenyl, - N($C_{1-3}$-Alkyl)-Phenyl, N(Phenyl)$_2$, Phenoxy, Benzyloxy, Phenyl, Benzyl und Morpholinyl substituiert sein kann, wobei jeweils der zyklische Teil der Reste Phenoxy, Benzyloxy, Phenyl und Benzyl mit 1, 2, 3, 4 oder 5 Substituenten unabhängig voneinander ausgewählt aus der Gruppe bestehend aus F, Cl, Br, Hydroxy, $-CF_3$, $-SF_5$, -CN, $-NO_2$, -$C_{1-6}$-Alkoxy, $-O-CF_3$, $-S-CF_3$, Phenyl und Benzyloxy substituiert sein kann;
bevorzugt $R^4$
für einen Wasserstoff-Rest steht;
für einen Alkyl-Rest ausgewählt aus der Gruppe bestehend aus Methyl, Ethyl, n-Propyl, n-Butyl und n-Pentyl steht, wobei der Alkyl-Rest jeweils mit 1 oder 2 Substituenten unabhängig voneinander ausgewählt aus der Gruppe bestehend aus $NH_2$, $-NH-CH_3$, $-NH-C_2H_5$, $-N-(CH_3)_2$, $-N(C_2H_5)_2$, $-N(CH_3)(C_2H_5)$, -NH-Phenyl, - N($CH_3$)-Phenyl, -N($C_2H_5$)-Phenyl und -N(Phenyl)$_2$ substituiert sein kann,
für einen Rest ausgewählt aus der Gruppe bestehend aus

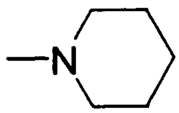, 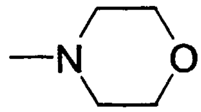,

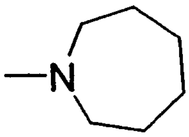, 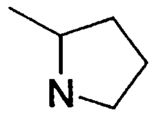 und 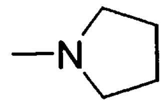,

steht, der über eine $-(CH_2)-$, $-(CH_2)_2-$, $-(CH_2)-O-$, $-CH(CH_3)-$, $-(CH=CH)-$ oder $-(CH_2)_3$-Gruppe gebunden und/oder mit 1, 2 oder 3 Substituenten unabhängig voneinander ausgewählt aus der Gruppe bestehend aus Methyl, Ethyl und n-Propyl substituiert sein kann;
für den folgenden Rest,

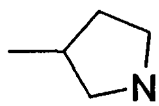,

steht, der mit einem Substituenten ausgewählt aus der Gruppe bestehend aus - $(CH_2)$-Benzo[b]furanyl und Benzyl substituiert sein kann, wobei jeweils der zyklische Teil der Reste $-(CH_2)$-Benzo[b]furanyl und Benzyl mit 1, 2, 3, 4 oder 5 Substituenten unabhängig voneinander ausgewählt aus der Gruppe bestehend aus F, Cl, Br, $-C_{1-4}$-Alkyl, $-CF_3$, $-CHF_2$, $-CH_2F$, $-C_{1-4}$-Alkoxy, $-C_{1-4}$-Alkylthio, Phenoxy, Phenyl, und Benzyloxy substituiert sein kann;
oder für einen Rest ausgewählt aus der Gruppe bestehend aus Phenyl und Naphthyl steht, wobei der Rest jeweils eine $-(CH_2)-$, $-(CH_2)_2-$ oder $-(CH_2)_3$-Gruppe gebunden und/oder mit 1, 2 oder 3 Substituenten unabhängig voneinander ausgewählt aus der Gruppe bestehend aus $-C_{1-6}$-Alkoxy, $-C_{1-6}$-Alkyl, $-NH_2$, $-NH-(C_{1-3}$-Alkyl), $-N(C_{1-3}$-Alkyl)$(C_{1-3}$-Alkyl), -NH-Phenyl, $-N(C_{1-3}$-Alkyl)-Phenyl, $N(Phenyl)_2$ und Morpholinyl substituiert sein kann;
besonders bevorzugt $R^4$
für einen Rest ausgewählt aus der Gruppe bestehend aus Methyl, Ethyl, n-Propyl, - $(CH_2)-N(CH_3)_2$, $-(CH_2)-(CH_2)-N(CH_3)_2$, $-(CH_2)-(CH_2)-(CH_2)-N(CH_3)_2$, $-(CH_2)-(CH_2)-N(C_2H_5)_2$, $-(CH_2)-(CH_2)-(CH_2)-N(C_2H_5)_2$, $-(CH_2)-(CH_2)-N(CH_3)$(Phenyl) und $-(CH_2)-(CH_2)-(CH_2)-N(CH_3)$(Phenyl) steht;
für einen Rest ausgewählt aus der Gruppe bestehend aus

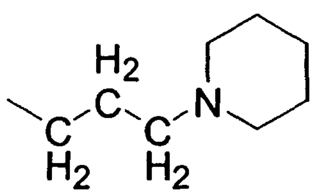, 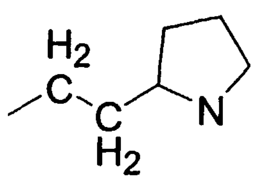, 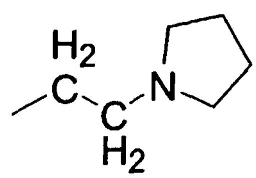,

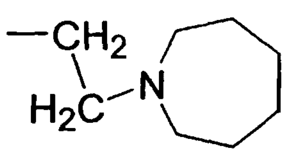 und 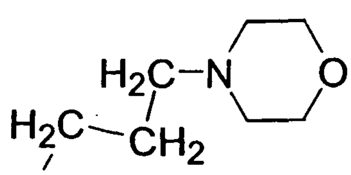,

steht, der mit einem Methyl-Rest substituiert sein kann;
für den folgenden Rest,

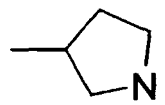 ,

steht, der am Stickstoffatom mit einem Substituenten ausgewählt aus der Gruppe bestehend aus -($CH_2$)-Benzo[b]furanyl und Benzyl substituiert sein kann, wobei jeweils der zyklische Teil der Reste -($CH_2$)-Benzo[b]furanyl und Benzyl mit 1, 2, oder 3 Substituenten unabhängig voneinander ausgewählt aus der Gruppe bestehend aus F, Cl, Br, -O-$CH_3$, -O-$C_2H_5$, -O-$C_3H_7$, -S-$CH_3$, -S-$C_2H_5$, -S-$C_3H_7$, -S-C($CH_3$)$_3$, Methyl, Ethyl, n-Propyl, Isopropyl, n-Butyl, Phenoxy und Benzyloxy substituiert sein kann;
oder für einen Rest ausgewählt aus der Gruppe bestehend aus Phenyl und Benzyl steht, wobei der zyklische Teil der Reste Phenyl und Benzyl mit 1, 2 oder 3 Substituenten unabhängig voneinander ausgewählt aus der Gruppe bestehend aus - O-$CH_3$, -O-$C_2H_5$, -O-$C_3H_7$, -N-($CH_3$)$_2$, -N($C_2H_5$)$_2$ und Morpholinyl substituiert sein kann;
und jeweils die übrigen Reste $R^1$ bis $R^3$, $R^5$ und $R^6$, X und $R^8$ bis $R^{10}$ die vorstehend angegebenen Bedeutung haben, ggf. in Form ihrer reinen Stereoisomeren, insbesondere Enantiomeren oder Diastereomeren, ihrer Racemate oder in Form von Mischungen der Stereoisomeren, insbesondere der Enantiomeren und/oder Diastereomeren, in einem beliebigen Mischungsverhältnis, oder ggf. in Form entsprechender Salze oder jeweils in Form entsprechender Solvate.

Ebenfalls bevorzugt sind substituierte 4,5,6,7-Tetrahydro-benzothiazol-2-ylamin-Verbindungen der allgemeinen Formel I, worin
$R^5$ und $R^6$ zusammen mit dem sie verbindenden Stickstoffatom als Ringglied einen heterocycloaliphatischen Rest bilden ausgewählt aus der Gruppe bestehend aus Piperazinyl, Morpholinyl, Thiomorpholinyl, Pyrrolidinyl, Azepanyl, Diazepanyl und Piperidinyl, wobei die heterocycloaliphatischen Reste mit einem Rest $R^8$ und ggf. einem Rest X oder einem Rest $R^9$ oder einem Rest $R^{10}$ substituiert sein können;
besonders bevorzugt $R^5$ und $R^6$ zusammen mit dem sie verbindenden Stickstoffatom als Ringglied einen der folgenden Reste bilden

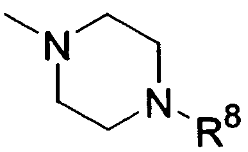 , 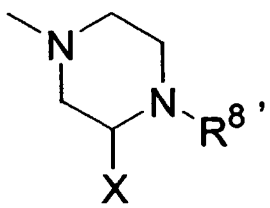 , 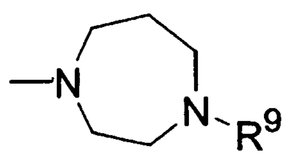 ,

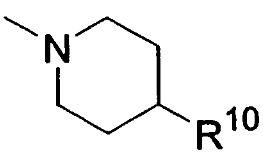 , 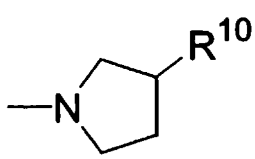 und 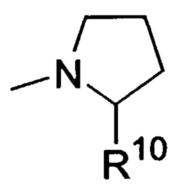 ;

und jeweils die übrigen Reste $R^1$ bis $R^4$, X und $R^8$ bis $R^{10}$ die vorstehend angegebenen Bedeutung haben, ggf. in Form ihrer reinen Stereoisomeren, insbesondere Enantiomeren oder Diastereomeren, ihrer Racemate oder in Form von Mischungen der Stereoisomeren, insbesondere der Enantiomeren und/oder Diastereomeren, in einem beliebigen Mischungsverhältnis, oder ggf. in Form entsprechender Salze oder jeweils in Form entsprechender Solvate.

Weiterhin bevorzugt sind substituierte 4,5,6,7-Tetrahydro-benzothiazol-2-ylamin-Verbindungen der allgemeinen Formel I, worin X
für einen Alkyl-Rest ausgewählt aus der Gruppe bestehend aus Methyl, Ethyl, n-Propyl, iso-Propyl, n-Butyl, sec-Butyl, iso-Butyl, tert-Butyl, n-Pentyl, sec-Pentyl, (1,1)-Dimethylpropyl und n-Hexyl steht;
bevorzugt X für einen Methyl-Rest steht;

und jeweils die übrigen Reste $R^1$ bis $R^6$ und $R^8$ bis $R^{10}$ die vorstehend angegebenen Bedeutung haben, ggf. in Form ihrer reinen Stereoisomeren, insbesondere Enantiomeren oder Diastereomeren, ihrer Racemate oder in Form von Mischungen der Stereoisomeren, insbesondere der Enantiomeren und/oder Diastereomeren, in einem beliebigen Mischungsverhältnis, oder ggf. in Form entsprechender Salze oder jeweils in Form entsprechender Solvate.

Weiterhin bevorzugt sind substituierte 4,5,6,7-Tetrahydro-benzothiazol-2-ylamin-Verbindungen der allgemeinen Formel I, worin

$R^8$, $R^9$ und $R^{10}$, unabhängig voneinander, jeweils für einen Alkyl-Rest ausgewählt aus der Gruppe bestehend aus Methyl, Ethyl, n-Propyl, iso-Propyl, n-Butyl, sec-Butyl, iso-Butyl, tert-Butyl, n-Pentyl, sec-Pentyl, (1,1)-Dimethylpropyl und n-Hexyl stehen, wobei der Alkyl-Rest jeweils mit 1, 2, 3, 4 oder 5 Substituenten unabhängig voneinander ausgewählt aus der Gruppe bestehend aus F, Cl, Br, -CN, $-NO_2$, Hydroxy, $C_{1-6}$-Alkoxy, $-NH_2$, -NH-($C_{1-3}$-Alkyl), -N($C_{1-3}$-Alkyl)($C_{1-3}$-Alkyl), -NH-Phenyl, - N($C_{1-3}$-Alkyl)-Phenyl und $-N(Phenyl)_2$ substituiert sein kann;

für einen Alkenyl-Rest ausgewählt aus der Gruppe bestehend aus Vinyl, 1-Propenyl, 2-Propenyl, 1-Butenyl, 2-Butenyl, 3-Butenyl, 1-Pentenyl, 2-Pentenyl und Pent-1,3-dienyl stehen, wobei der Alkenyl-Rest jeweils mit 1, 2, 3, 4 oder 5 Substituenten unabhängig voneinander ausgewählt aus der Gruppe bestehend aus F, Cl, Br, -CN, - $NO_2$, Hydroxy, $C_{1-6}$-Alkoxy, $-NH_2$, -NH-($C_{1-3}$-Alkyl), -N($C_{1-3}$-Alkyl)($C_{1-3}$-Alkyl), -NH-Phenyl, -N($C_{1-3}$-Alkyl)-Phenyl und $-N(Phenyl)_2$ substituiert sein kann;

für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl stehen, wobei der (hetero)cycloaliphatische Rest jeweils über eine lineare oder verzweigte, ggf. mit eine Carbonyl-(C=O)-Gruppe als Kettenglied aufweisende $C_{1-5}$-AlkylenGruppe gebunden und/oder mit 1, 2, 3, 4 oder 5 Substituenten unabhängig voneinander ausgewählt aus der Gruppe bestehend aus F, Cl, Br, -CN, $-CF_3$, $-CHF_2$, $-CH_2F$, Hydroxy, -$C_{1-6}$-Alkoxy, $-NH_2$, -NH-($C_{1-3}$-Alkyl), -N($C_{1-3}$-Alkyl)($C_{1-3}$-Alkyl), -NH-Phenyl, -N($C_{1-3}$-Alkyl)-Phenyl, -N $(Phenyl)_2$, -SH, -$C_{1-6}$-Alkylthio, -$C_{1-6}$-Alkyl, -($CH_2$)-Benzo[b]furanyl, Phenoxy, Benzyloxy, Phenyl und Benzyl substituiert sein kann, wobei jeweils der zyklische Teil der Reste Phenoxy, Benzyloxy, Phenyl, -($CH_2$)-Benzo[b]furanyl und Benzyl mit 1, 2, 3, 4 oder 5 Substituenten unabhängig voneinander ausgewählt aus der Gruppe bestehend aus F, Cl, Br, -$C_{1-4}$-Alkyl, $-CF_3$, - $CHF_2$, $-CH_2F$, -$C_{1-4}$-Alkoxy, -$C_{1-4}$-Alkylthio, Phenoxy, Phenyl, und Benzyloxy substituiert sein kann;

oder für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo [b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl und Isochinolinyl stehen, wobei der Rest jeweils über eine lineare oder verzweigte, ggf. mit einem Phenyl-Rest substituierte, ggf. eine Carbonyl-(C=O)-Gruppe als Kettenglied aufweisende $C_{1-5}$-Alkylen- oder $C_{2-5}$-Alkenylen-Gruppe gebunden und/oder mit 1, 2, 3, 4 oder 5 Substituenten unabhängig voneinander ausgewählt aus der Gruppe bestehend aus F, Cl, Br, -CN, $-CF_3$, $-CHF_2$, $-CH_2F$, Hydroxy, -$C_{1-6}$-Alkoxy, $-O-CF_3$, $-S-CF_3$, -SH, -$C_{1-6}$-Alkylthio, -$C_{1-6}$-Alkyl, $-NH_2$, -NH-($C_{1-3}$-Alkyl), -N($C_{1-3}$-Alkyl)($C_{1-3}$-Alkyl), -NH-Phenyl, - N($C_{1-3}$-Alkyl)-Phenyl, $N(Phenyl)_2$, Phenoxy, Benzyloxy, Phenyl, Benzyl und Morpholinyl substituiert sein kann, wobei jeweils der zyklische Teil der Reste Phenoxy, Benzyloxy, Phenyl und Benzyl mit 1, 2, 3, 4 oder 5 Substituenten unabhängig voneinander ausgewählt aus der Gruppe bestehend aus F, Cl, Br, Hydroxy, $-CF_3$, $-SF_5$, -CN, $-NO_2$, -$C_{1-6}$-Alkoxy, $-O-CF_3$, $-S-CF_3$, Phenyl und Benzyloxy substituiert sein kann;

bevorzugt

$R^8$ für einen Alkyl-Rest ausgewählt aus der Gruppe bestehend aus Methyl, Ethyl, n-Propyl, Isopropyl, n-Butyl, tert-Butyl und n-Pentyl steht, wobei der Alkyl-Rest jeweils mit 1 oder 2 Substituenten unabhängig voneinander ausgewählt aus der Gruppe bestehend aus $NH_2$, $-NH-CH_3$, $-NH-C_2H_5$, $-N-(CH_3)_2$, $-N(C_2H_5)_2$, $-N(CH_3)(C_2H_5)$, -NH-Phenyl, -N ($CH_3$)-Pheny), -N($C_2H_5$)-Phenyl und $-N(Phenyl)_2$ substituiert sein kann;

für einen cycloaliphatischen Rest ausgewählt aus der Gruppe bestehend aus Pyrrolidinyl und Piperidinyl steht, der über eine -(C=O)- oder -($CH_2$)-(C=O)-Gruppe gebunden und/oder mit 1, 2, 3, 4 oder 5 Substituenten unabhängig voneinander ausgewählt aus der Gruppe bestehend aus F, Cl, Br, -CN, $-CF_3$, $-CHF_2$, $-CH_2F$, Hydroxy, $-O-CH_3$, $-O-C_2H_5$, $-O-C_3H_7$, $-NH_2$, $-NH-CH_3$, $-NH-C_2H_5$, $-N-(CH_3)_2$, $-N(C_2H_5)_2$, $-N(CH_3)(C_2H_5)$, -NH-Phenyl, -N($CH_3$)-Phenyl, -N($C_2H_5$)-Phenyl, -N $(Phenyl)_2$, -SH, $-S-CH_3$, $-S-C_2H_5$, $-S-C_3H_7$, $-S-C(CH_3)_3$, Methyl, Ethyl, n-Propyl, Isopropyl, n-Butyl, sec-Butyl und tert-Butyl substituiert sein kann;

oder für einen Rest ausgewählt aus der Gruppe bestehend aus Phenyl, 1-Naphthyl, 2-Naphthyl, 2-Furanyl, 3-Furanyl, 2-Thiophenyl, 3-Thiophenyl, 1-Pyridinyl, 2-Pyridinyl, 3-Pyridinyl, Benzo[b]furanyl, (1,3)-Benzodioxolyl und (1,4)-Benzodioxanyl steht, der über eine -(C=O)-. -($CH_2$)-, $-(CH_2)_2-$, -CH($CH_3$)-, -(CH=CH)-, -($CH_2$)-(C=O)-, -($CH_2$)-(CH=CH)- oder $-(CH_2)_3$-Gruppe gebunden und/oder mit 1, 2, 3, 4 oder 5 Substituenten unabhängig voneinander ausgewählt aus der Gruppe bestehend aus F, Cl, Br, -CN, $-CF_3$, $-CHF_2$, $-CH_2F$, Hydroxy, $-O-CH_3$, $-O-C_2H_5$, $-O-C_3H_7$, $-O-CF_3$, $-S-CF_3$, -SH, $-S-CH_3$, $-S-C_2H_5$, $-S-C_3H_7$, $-S-C(CH_3)_3$, Methyl, Ethyl, n-Propyl, Isopropyl, n-Butyl, sec-Butyl, tert-Butyl, $-NH_2$, $-NH-CH_3$, $-NH-C_2H_5$, $-N-(CH_3)_2$, $-N(C_2H_5)_2$, - $N(CH_3)(C_2H_5)$, -NH-Phenyl, -N($CH_3$)-Phenyl, -N($C_2H_5$)-Phenyl, $-N(Phenyl)_2$, Phenoxy, Benzyloxy, Phenyl, Benzyl und Morpholinyl substituiert sein kann;

$R^9$ für einen Alkyl-Rest ausgewählt aus der Gruppe bestehend aus Methyl, Ethyl, n-Propyl, iso-Propyl, n-Butyl, sec-Butyl, iso-Butyl, tert-Butyl, n-Pentyl, sec-Pentyl, (1,1)-Dimethylpropyl und n-Hexyl steht
und
$R^{10}$ für einen cycloaliphatischen Rest ausgewählt aus der Gruppe bestehend aus Pyrrolidinyl und Piperidinyl steht, der über eine $-(CH_2)-$, $-(CH_2)_2-$, $-CH(CH_3)-$ oder - $(CH_2)_3$-Gruppe gebunden und/oder mit 1, 2, 3, 4 oder 5 Substituenten unabhängig voneinander ausgewählt aus der Gruppe bestehend aus F, Cl, Br, -CN, $-CF_3$, $-CHF_2$, $-CH_2F$, Hydroxy, $-O-CH_3$, $-O-C_2H_5$, $-O-C_3H_7$, $-NH_2$, $-NH-CH_3$, $-NH-C_2H_5$, $-N-(CH_3)_2$, - $N(C_2H_5)_2$, $-N(CH_3)(C_2H_5)$, -NH-Phenyl, $-N(CH_3)$-Phenyl, $-N(C_2H_5)$-Phenyl, - $N(Phenyl)_2$, -SH, $-S-CH_3$, $-S-C_2H_5$, $-S-C_3H_7$, $-S-C(CH_3)_3$, Methyl, Ethyl, n-Propyl, Isopropyl, n-Butyl, sec-Butyl und tert-Butyl substituiert sein kann;
oder für einen Rest ausgewählt aus der Gruppe bestehend aus Phenyl, 1-Naphthyl und 2-Naphthyl steht, der über eine $-(CH_2)-$, -[(CH)Phenyl]-, $-(CH_2)_2-$, $-(CH_2)_3$-Gruppe gebunden und/oder mit 1, 2, 3, 4 oder 5 Substituenten unabhängig voneinander ausgewählt aus der Gruppe bestehend aus F, Cl, Br, -CN, $-CF_3$, $-CHF_2$, $-CH_2F$, Hydroxy, $-O-CH_3$, $-O-C_2H_5$, $-O-C_3H_7$, $-O-CF_3$, $-S-CF_3$, -SH, $-S-CH_3$, $-S-C_2H_5$, $-S-C_3H_7$, $-S-C(CH_3)_3$, Methyl, Ethyl, n-Propyl, Isopropyl, n-Butyl, sec-Butyl, tert-Butyl, $-NH_2$, - $NH-CH_3$, $-NH-C_2H_5$, $-N-(CH_3)_2$, $-N(C_2H_5)_2$, $-N(CH_3)(C_2H_5)$, -NH-Phenyl, $-N(CH_3)$-Phenyl, $-N(C_2H_5)$-Phenyl, $-N(Phenyl)_2$, Phenoxy, Benzyloxy, Phenyl, Benzyl und Morpholinyl substituiert sein kann;
besonders bevorzugt
$R^8$ für einen Rest ausgewählt aus der Gruppe bestehend aus Methyl, Ethyl, n-Propyl, iso-Propyl und $-(CH_2)-(CH_2)-N(CH_3)_2$,
für einen Pyrrolidinyl-Rest steht, der über eine $-(CH_2)-(C=O)$-Gruppe gebunden ist, oder für einen Rest ausgewählt aus der Gruppe bestehend aus Phenyl, 1-Naphthyl, 2-Naphthyl, 2-Thiophenyl, 3-Thiophenyl, 1-Pyridinyl, 2-Pyridinyl, 3-Pyridinyl, Benzo[b]furanyl, (1,3)-Benzodioxolyl und (1,4)-Benzodioxanyl steht, der über eine - (C=O)-, $-(CH_2)-$, $-(CH_2)_2-$, $-CH(CH_3)-$, -(CH=CH)-, $-(CH_2)-(C=O)-$, $-(CH_2)-(CH=CH)-$ oder $-(CH_2)_3$-Gruppe gebunden und/oder mit 1, 2 oder 3 Substituenten unabhängig voneinander ausgewählt aus der Gruppe bestehend aus F, Cl, Br, -CN, $-CF_3$, $-CHF_2$, $-CH_2F$, $-O-CH_3$ und $-O-C_2H_5$ substituiert sein kann,
$R^9$ für einen Methyl- oder Ethyl-Rest steht
und
$R^{10}$ für einen Pyrrolidinyl-Rest steht, der über eine $-(CH_2)$-Gruppe gebunden ist,
oder für einen Benzyhydryl-Rest steht;
und jeweils die übrigen Reste $R^1$ bis $R^6$ und X die vorstehend angegebenen Bedeutung haben, ggf. in Form ihrer reinen Stereoisomeren, insbesondere Enantiomeren oder Diastereomeren, ihrer Racemate oder in Form von Mischungen der Stereoisomeren, insbesondere der Enantiomeren und/oder Diastereomeren, in einem beliebigen Mischungsverhältnis, oder ggf. in Form entsprechender Salze oder jeweils in Form entsprechender Solvate.

Besonders bevorzugt sind substituierte 4,5,6,7-Tetrahydro-benzothiazol-2-ylamin-Verbindungen der allgemeinen Formel I, worin

$R^1$ für eine Gruppe $-NR^3R^4$ oder für eine Gruppe $-NR^5R^6$ steht;

$R^2$ für einen Alkyl-Rest ausgewählt aus der Gruppe bestehend aus n- Propyl, iso-Propyl, n-Butyl, sec-Butyl, iso-Butyl, tert-Butyl, n-Pentyl, sec- Pentyl und (1,1)-Dimethylpropyl steht;
für einen Alkenyl-Rest ausgewählt aus der Gruppe bestehend aus 1- Pentenyl, 2-Pentenyl und Pent-1,3-dienyl steht;
für einen cycloaliphatischen Rest ausgewählt aus der Gruppe bestehend aus Cyclopentyl, Cyclohexyl und Cycloheptyl steht, der über eine $-(CH_2)$-Gruppe gebunden ist;
oder für einen Rest ausgewählt aus der Gruppe bestehend aus Phenyl, 1-Naphthyl, 2-Naphthyl, 2-Furanyl, 3-Furanyl, 2-Thiophenyl oder 3- Thiophenyl steht, der über eine $-(CH_2)-$, $-(CH_2)_2-$, $-(CH_2)-O-$, $-CH(CH_3)-$, -(CH=CH)- oder $-(CH_2)_3$-Gruppe gebunden und/oder mit 1, 2 oder 3 Substituenten unabhängig voneinander ausgewählt aus der Gruppe bestehend aus F, Cl, Br, $-O-CH_3$, $-O-C_2H_5$, Methyl, Ethyl, n-Propyl und Isopropyl substituiert sein kann;

$R^3$ für einen Wasserstoff-Rest oder für einen Methyl-Rest steht;

$R^4$ für einen Rest ausgewählt aus der Gruppe bestehend aus Methyl, Ethyl, n-Propyl, $-(CH_2)-N(CH_3)_2$, $-(CH_2)-(CH_2)-N(CH_3)_2$, $-(CH_2)-(CH_2)-(CH_2)-N(CH_3)_2$, $-(CH_2)-(CH_2)-N(C_2H_5)_2$, $-(CH_2)-(CH_2)-(CH_2)-N(C_2H_5)_2$, - $(CH_2)-(CH_2)-N(CH_3)$(Phenyl) und $-(CH_2)-(CH_2)-(CH_2)-N(CH_3)$(Phenyl) steht;
für einen Rest ausgewählt aus der Gruppe bestehend aus

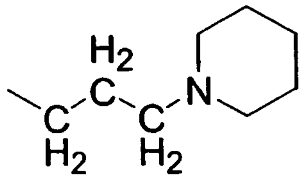 , 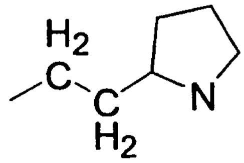 , 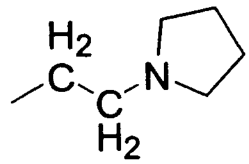 ,

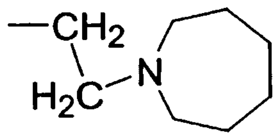 und 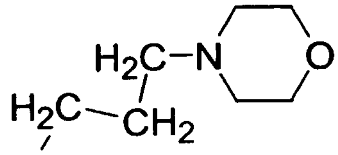 ,

steht, der mit einem Methyl-Rest substituiert sein kann;
für den folgenden Rest,

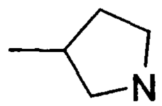 ,

steht, der am Stickstoffatom mit einem Substituenten ausgewählt aus der Gruppe bestehend aus $-(CH_2)$-Benzo[b]furanyl und Benzyl substituiert sein kann, wobei jeweils der zyklische Teil der Reste $-(CH_2)$-Benzo[b]furanyl und Benzyl mit 1, 2, oder 3 Substituenten unabhängig voneinander ausgewählt aus der Gruppe bestehend aus F, Cl, Br, $-O-CH_3$, $-O-C_2H_5$, $-O-C_3H_7$, $-S-CH_3$, $-S-C_2H_5$, $-S-C_3H_7$, $-S-C(CH_3)_3$, Methyl, Ethyl, n-Propyl, Isopropyl, n-Butyl, Phenoxy und Benzyloxy substituiert sein kann;
oder für einen Rest ausgewählt aus der Gruppe bestehend aus Phenyl und Benzyl steht, wobei der zyklische Teil der Reste Phenyl und Benzyl mit 1, 2 oder 3 Substituenten unabhängig voneinander ausgewählt aus der Gruppe bestehend aus $-O-CH_3$, $-O-C_2H_5$, $-O-C_3H_7$, $-N-(CH_3)_2$, $-N(C_2H_5)_2$ und Morpholinyl substituiert sein kann;

$R^5$ und $R^6$ zusammen mit dem sie verbindenden Stickstoffatom als Ringglied einen der folgenden Reste bilden

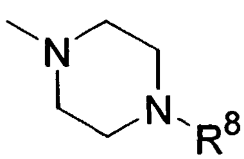 , 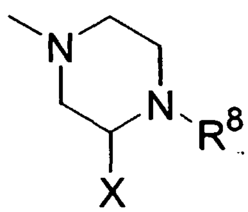 , 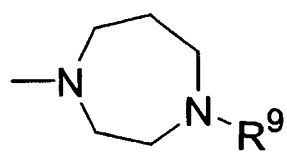 ,

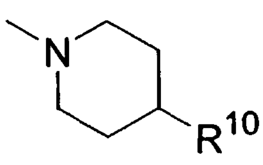 , 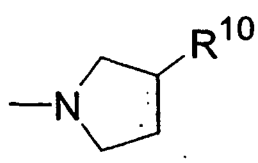 und 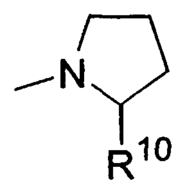 ;

X für einen Methyl-Rest steht,

$R^8$ für einen Rest ausgewählt aus der Gruppe bestehend aus Methyl, Ethyl, n-Propyl, iso-Propyl und $-(CH_2)-(CH_2)-N(CH_3)_2$, für einen Pyrrolidinyl-Rest steht, der über eine $-(CH_2)-(C{=}O)$-Gruppe gebunden ist, oder für einen Rest ausgewählt aus der Gruppe bestehend aus Phenyl, 1-Naphthyl, 2-Naphthyl, 2-Thiophenyl, 3-Thiophenyl, 1-Pyridinyl, 2- Pyridinyl, 3-Pyridinyl, Benzo[b]furanyl, (1,3)-Benzodioxolyl und (1,4)-Benzodioxanyl steht, der über eine -(C=O)-, $-(CH_2)-$, $-(CH_2)_2-$, - $CH(CH_3)$-. -(CH=CH)-, $-(CH_2)-(C{=}O)-$, $-(CH_2)$-(CH=CH)- oder $-(CH_2)_3$- Gruppe gebunden und/oder mit 1, 2 öder 3 Substituenten unabhängig voneinander ausgewählt aus der Gruppe bestehend aus F, Cl, Br, -CN, $-CF_3$, $-CHF_2$, $-CH_2F$, $-O-CH_3$ und $-O-C_2H_5$ substituiert sein kann,

$R^9$ für einen Methyl- oder Ethyl-Rest steht und

$R^{10}$ für einen Pyrrolidinyl-Rest steht, der über eine $-(CH_2)$-Gruppe gebunden ist, oder für einen Benzyhydryl-Rest steht;

jeweils ggf. in Form ihrer reinen Stereoisomeren, insbesondere Enantiomeren oder Diastereomeren, ihrer Racemate oder in Form von Mischungen der Stereoisomeren, insbesondere der Enantiomeren und/oder Diastereomeren, in einem beliebigen Mischungsverhältnis, oder ggf. in Form entsprechender Salze oder jeweils in Form entsprechender Solvate.

Ganz besonders bevorzugt sind substituierte 4,5,6,7-Tetrahydro-benzothiazol-2-ylamin-Verbindungen der allgemeinen Formel I ausgewählt aus der Gruppe bestehend aus.
2-Cyclohexyl-N-{4-[4-(2-dimethylamino-ethyl)-piperazin-1-carbonyl]-4,5,6,7-tetrahydro-benzothiazol-2-yl}-acetamid,
N-[4-(4-Methyl-[1,4]diazepan-1-carbonyl)-4,5,6,7-tetrahydro-benzothiazol-2-yl]-2-phenoxy-acetamid,
2-(2-Phenyl-propionylamino)-4,5,6,7-tetrahydro-benzothiazol-4-carbonsäure (3-dimethylamino-propyl)-amid,
Naphthalin-2-carbonsäure [4-(4-methyl-piperazin-1-carbonyl)-4,5,6,7-tetrahydro-benzothiazol-2-yl]-amid,
N-[4-(2-Pyrrolidin-1-ylmethyl-pyrrolidin-1-carbonyl)-4,5,6,7-tetrahydro-benzothiazol-2-yl]-2-thiophen-2-yl-acetamid,
N-{4-[4-(7-Methoxy-benzo[1,3]dioxol-5-ylmethyl)-piperazin-1-carbonyl]-4,5,6,7-tetrahydro-benzothiazol-2-yl}-2-(2-methoxy-phenyl)-acetamid,
N-{4-[4-(4-Methoxy-phenyl)-3-methyl-piperazin-1-carbonyl]-4,5,6,7-tetrahydro-benzothiazol-2-yl}-benzamid,
Furan-2-carbonsäure-{4-[4-(4-acetyl-phenyl)-piperazin-1-carbonyl]-4,5,6,7-tetrahydro-benzothiazol-2-yl}-amid,
2-[(Furan-2-carbonyl)-amino]-4,5,6,7-tetrahydro-benzothiazol-4-carbonsäure-[1-(2,4,6-trimethoxy-benzyl)-pyrrolidin-3-yl]-amid,
N-[4-(4-Isopropyl-piperazin-1-carbonyl)-4,5,6,7-tetrahydro-benzothiazol-2-yl]-2-phenyl-propionamid,
3-Furan-2-yl-N-[4-(4-thiophen-3-ylmethyl-piperazin-1-carbonyl)-4,5,6,7-tetrahydro-benzothiazol-2-yl]-acrylamid,
2-(4-Methoxy-benzoylamino)-4,5,6,7-tetrahydro-benzothiazol-4-carbonsäure-[1-(2-chlor-6-fluor-benzyl)-pyrrolidin-3-yl]-methyl-amid,
Hexansäure-[4-(4-pyridin-4-yl-piperazin-1-carbonyl)-4,5,6,7-tetrahydro-benzothiazol-2-yl]-amid,
2-(2-Thiophen-2-yl-acetylamino)-4,5,6,7-tetrahydro-benzothiazol-4-carbonsäure- (3-morpholin-4-yl-propyl)-amid,
2-(2-Phenyl-propionylamino)-4,5,6,7-tetrahydro-benzothiazol-4-carbonsäure [1-(4-phenoxy-benzyl)-pyrrolidin-3-yl]-amid,
2-[(Furan-3-carbonyl)-amino]-4,5,6,7-tetrahydro-benzothiazol-4-carbonsäure (2-azepan-1-yl-ethyl)-amid,
Furan-3-carbonsäure [4-(4-benzofuran-2-ylmethyl-piperazin-1-carbonyl)-4,5,6,7-tetrahydro-benzothiazol-2-yl]-amid,
2-Hexanylamino-4,5,6,7-tetrahydro-benzothiazol-4-carbonsäure [3-(2-methylpiperidin-1-yl)-propyl]-amid,
2-(2-Phenyl-propionylamino)-4,5,6,7-tetrahydro-benzothiazol-4-carbonsäure-(2-dimethylamino-ethyl)-amid,
2-Ethoxy-N-[4-(4-phenethyl-piperazin-1-carbonyl)-4,5,6,7-tetrahydro-benzothiazol-2-yl]-benzamid,
2-(4-Fluor-phenoxy)-N-[4-(4-phenyl-piperazin-1-carbonyl)-4,5,6,7-tetrahydro-benzothiazol-2-yl]-acetamid,
2-(2-Phenyl-propionylamino)-4,5,6,7-tetrahydro-benzothiazol-4-carbonsäure [2-(1-methyl-pyrrolidin-2-yl)-ethyl]-amid,
2-(3-Furan-2-yl-acryloylamino)-4,5,6,7-tetrahydro-benzothiazol-4-carbonsäure [3-(2-methyl-piperidin-1-yl)-propyl]-amid,
2-(3,3-Dimethyl-butyrylamino)-4,5,6,7-tetrahydro-benzothiazol-4-carbonsäure-(3-dimethylamino-propyl)-amid,
N-{4-[4-(4-Chlor-benzyl)-piperazin-1-carbonyl]-4,5,6,7-tetrahydro-benzothiazol-2-yl}-4-methoxy-benzamid,
Naphthalin- 2- carbonsäure-[4-(2- pyrrolidin- 1- ylmethyl- pyrrolidin- 1- carbonyl)- 4,5,6,7- tetrahydro- benzothiazol- 2-yl]-amid,
N-[4-(4-Benzofuran-2-ylmethyl-piperazin-1-carbonyl)-4,5,6,7-tetrahydro-benzothiazol-2-yl]-butyramid,
N-{4-[4-(3-Methoxy-phenyl)-piperazin-1-carbonyl]-4,5,6,7-tetrahydro-benzothiazol-2-yl}-benzamid,
2-Butyrylamino-4,5,6,7-tetrahydro-benzothiazol-4-carbonsäure-(1-biphenyl-4-y)methyl-pyrrolidin-3-yl)-methyl-amid.
2-(2-Phenoxy-acetylamino)-4,5,6,7-tetrahydro-benzothiazol-4-carbonsäure (2-dimethylamino-ethyl)-amid,

2-Ethoxy-N-[4-(2-pyrrolidin-1-ylmethyl-pyrrolidin-1-carbonyl)-4,5,6,7-tetrahydro-benzothiazol-2-yl]-benzamid,
Naphthalin-2-carbonsäure {4-[4-(2-oxo-2-pyrrolidin-1-yl-ethyl)-piperazin-1-carbonyl]-4,5,6,7-tetrahydro-benzothiazol-2-yl}-amid,
2-(2-Phenyl-propionylamino)-4,5,6,7-tetrahydro-benzothiazol-4-carbonsäure [1-(2-brom-4,5-dimethoxy-benzyl)-pyrrolidin-3-yl]-amid,
Hexansäure {4-[4-(2,4,6-trimethoxy-benzyl)-piperazin-1-carbonyl]-4,5,6,7-tetrahydro-benzothiazol-2-yl}-amid,
2-(3,5-Dimethyl-benzoylamino)-4,5,6,7-tetrahydro-benzothiazol-4-carbonsäure [1-(2-tert-butylsulfanyl-benzyl)-pyrrolidin-3-yl]-amid,
2-(2-Phenyl-propionylamino)-4,5,6,7-tetrahydro-benzothiazol-4-carbonsäure [1-(2-benzyloxy-benzyl)-pyrrolidin-3-yl]-methyl-amid,
2-Hexa-2,4-dienoylamino-4,5,6,7-tetrahydro-benzothiazol-4-carbonsäure (1-biphenyl-4-ylmethyl-pyrrolidin-3-yl)-methyl-amid,
2-(3-Phenyl-propionylamino)-4,5,6,7-tetrahydro-benzothiazol-4-carbonsäure (2-diethylamino-ethyl)-amid,
2-(2-Thiophen-2-yl-acetylamino)-4,5,6,7-tetrahydro-benzothiazol-4-carbonsäure (2-azepan-1-yl-ethyl)-amid,
2-(2-Thiophen-2-yl-acetylamino)-4,5,6,7-tetrahydro-benzothiazol-4-carbonsäure [1-(2-ethoxy-benzyl)-pyrrolidin-3-yl]-methyl-amid,
2-Hexanylamino-4,5,6,7-tetrahydro-benzothiazol-4-carbonsäure (1-benzofuran-2-ylmethyl-pyrrolidin-3-yl)-methyl-amid,
2-(2-Phenoxy-acetylamino)-4,5,6,7-tetrahydro-benzothiazol-4-carbonsäure [3-(2-methyl-piperidin-1-yl)-propyl]-amid,
2-[(Naphthalin-2-carbonyl)-amino]-4,5,6,7-tetrahydro-benzothiazol-4-carbonsäure (2-pyrrolidin-1-yl-ethyl)-amid,
2-[2-(3,5-Difluor-phenyl)-acetylamino]-4,5,6,7-tetrahydro-benzothiazol-4-carbonsäure [1-(2-ethoxy-benzyl)-pyrrolidin-3-yl]-methyl-amid,
2-[(Naphthalin-2-carbonyl)-amino]-4,5,6,7-tetrahydro-benzothiazol-4-carbonsäure 4-dimethylamino-benzylamid,
2-[2-(4-Fluor-phenoxy)-acetylamino]-4,5,6,7-tetrahydro-benzothiazol-4-carbonsäure [2-(1-methyl-pyrrofidin-2-yl)-ethyl]-amid,
Furan-2-carbonsäure {4-[4-(2-cyano-phenyl)-piperazin-1-carbonyl]-4,5,6,7-tetrahydro-benzothiazol-2-yl}-amid,
2-(3-Phenyl-propionylamino)-4,5,6,7-tetrahydro-benzothiazol-4-carbonsäure (1-benzofuran-2-ylmethyl-pyrrolidin-3-yl)-methyl-amid,
2-Hexa-2,4-dienoylamino-4,5,6,7-tetrahydro-benzothiazol-4-carbonsäure [1-(2,6-dichlor-benzyl)-pyrrolidin-3-yl]-methyl-amid,
2-[(Furan-2-carbonyl)-amino]-4,5,6,7-tetrahydro-benzothiazol-4-carbonsäure [1-(5-brom-2-ethoxy-benzyl)-pyrrolidin-3-yl]-amid,
2-(2-Phenoxy-acetylamino)-4,5,6,7-tetrahydro-benzothiazol-4-carbonsäure [1-(2-ethoxy-benzyl)-pyrrolidin-3-yl]-methyl-amid,
Hexansäure {4-[4-(2-fluor-phenyl)-piperazin-1-carbonyl]-4,5,6,7-tetrahydro-benzothiazol-2-yl}-amid,
Naphthalin-2-carbonsäure {4-[4-(2-fluor-benzyl)-piperazin-1-carbonyl]-4,5,6,7-tetrahydro-benzothiazol-2-yl}-amid,
2-(2-Phenoxy-acetylamino)-4,5,6,7-tetrahydro-benzothiazol-4-carbonsäure methyl-[1-(2-trifluormethyl-benzyl)-pyrrolidin-3-yl]-amid,
2-(3-Phenyl-propionylamino)-4,5,6,7-tetrahydro-benzothiazol-4-carbonsäure (1-biphenyl-4-ylmethyl-pyrrolidin-3-yl)-methyl-amid,
2-[2-(2-Methoxy-phenyl)-acetylamino]-4,5,6,7-tetrahydro-benzothiazol-4-carbonsäure (1-benzofuran-2-ylmethyl-pyrrolidin-3-yl)-methyl-amid,
2-(3,5-Difluor-phenyl)-N-[4-(2-pyrrolidin-1-ylmethyl-pyrrolidin-1-carbonyl)-4,5,6,7-tetrahydro-benzothiazol-2-yl]-acetamid,
2-[(Furan-2-carbonyl)-amino]-4,5,6,7-tetrahydro-benzothiazol-4-carbonsäure 4-dimethylamino-benzylamid,
Hexansäure {4-[4-(3-fluor-4-methoxy-benzyl)-piperazin-1-carbonyl]-4,5,6,7-tetrahydro-benzothiazol-2-yl}-amid,
N-{4-[4-(4-Methoxy-phenyl)-3-methyl-piperazin-1-carbonyl]-4,5,6,7-tetrahydro-benzothiazol-2-yl}-2-phenyl-propionamid,
2-Hexa-2,4-dienoylamino-4,5,6,7-tetrahydro-benzothiazol-4-carbonsäure [1-(2-chlor-6-fluor-benzyl)-pyrrolidin-3-yl]-methyl-amid,
2-[2-(3,5-Difluorphenyl)-acetylamino]-4,5,6,7-tetrahydro-benzothiazol-4-carbonsäure [1-(2,6-dichlor-benzyl)pyrrolidin-3-yl]-methyl-amid,
2-[2-(4-Fluor-phenoxy)-acetylamino]-4,5,6,7-tetrahydro-benzothiazol-4-carbonsäure [1-(2-ethoxy-benzyl)-pyrrolidin-3-yl]-methyl-amid,
Furan-3-carbonsäure [4-(4-pyridin-2-yl-piperazin-1-carbonyl)-4,5,6,7-tetrahydro-benzothiazol-2-yl]-amid,
Hexansäure [4-(4-benzofuran-2-yimethyl-piperazin-1-carbonyl)-4,5,6,7-tetrahydro-benzothiazol-2-yl]-amid,
2-Phenoxy-N-{4-[4-(1-phenyl-ethyl)-piperazin-1-carbonyl]-4,5,6,7-tetrahydro-benzothiazol-2-yl}-acetamid,
2-(2-Cyclohexyl-acetylamino)-4,5,6,7-tetrahydro-benzothiazol-4-carbonsäure [1-(2,6-dichlor-benzyl)-pyrrolidin-3-yl]-methyl-amid,
2-(2-Cyclohexyl-acetyfamino)-4,5,6,7-tetrahydro-benzothiazol-4-carbonsäure [3-(methyl-phenyl-amino)-propyl]-amid,
2-(2-Ethoxy-benzoylamino)-4,5,6,7-tetrahydro-benzothiazol-4-carbonsäure [3-(methyl-phenyl-amino)-propyl]-amid,
3-Phenyl-N-{4-[4-(5-trifluormethyl-pyridin-2-yl)-piperazin-1-carbonyl]-4,5,6,7-tetrahydro-benzothiazol-2-yl}-propionamid,
Furan-3-carbonsäure [4-(4-phenethyl-piperazin-1-carbonyl)-4,5,6,7-tetrahydro-benzothiazol-2-yl]-amid,
3-Phenyl-N-{4-[4-(3-trifluormethyl-phenyl)-piperazin-1-carbonyl]-4,5,6,7-tetrahydro-benzothiazol-2-yl}-propionamid,
2-(3,3-Dimethyl-butyrylamino)-4,5,6,7-tetrahydro-benzothiazol-4-carbonsäure [1-(2-benzyloxy-benzyl)-pyrrolidin-3-yl]-amid,
2-(3,5- Difluor- phenyl)-N- {4-[4-(3- trifluor- methyl- phenyl)-piperazin- 1- carbonyl]- 4,5,6,7- tetrahydro- benzothiazol- 2-yl}-acetamid,
2-Ethoxy-N-{4-[4-(1-phenyl-ethyl)-piperazin-1-carbonyl]-4,5,6,7-tetrahydro-benzothiazol-2-yl}-benzamid,
3-Furan-2-yl-N-{4-[4-(3-trifluormethyl-phenyl)-piperazin-1-carbonyl]-4,5,6,7-tetrahydro-benzothiazol-2-yl}-acrylamid,
N-{4-[4-(2-Cyano-phenyl)-piperazin-1-carbonyl]-4,5,6,7-tetrahydro-benzothiazol-2-yl}-3-phenyl-propionamid,
2-(2-Cyclohexyl-acetylamino)-4,5,6,7-tetrahydro-benzothiazol-4-carbonsäure [1-(2-chlor-6-fluor-benzyl)-pyrrolidin-3-yl]-methyl-amid,
N-{4-[4-(2-Methoxy-naphthalin-1-ylmethyl)-piperazin-1-carbonyl]-4,5,6,7-tetrahydro-benzothiazol-2-yl}-3,3-dimethyl-butyramid,
2-(3,3-Dimethyl-butyrylamino)-4,5,6,7-tetrahydro-benzothiazol-4-carbonsäure (2,5-diethoxy-4-morpholin-4-yl-phenyl)-amid,
N-{4-[4-(2-Chlor-phenyl)-piperazin-1-carbonyl]-4,5,6,7-tetrahydro-benzothiazol-2-yl}-2-(4-fluor-phenyl)-acetamid,
N-[4-(4-Benzyl-piperazin-1-carbonyl)-4,5,6,7-tetrahydro-benzothiazol-2-yl]-3,3-dimethyl-butyramid,
N-[4-(4-Benzhydryl-piperazin-1-carbonyl)-4,5,6,7-tetrahydro-benzothiazol-2-yl]-2-(3,5-difluor-phenyl)-acetamid,
N-[4-(4-Benzhydryl-piperazin-1-carbonyl)-4,5,6,7-tetrahydro-benzothiazol-2-yl]-2-thiophen-2-yl-acetamid,
N-{4-[4-(2-Chlor-phenyl)-piperazin-1-carbonyl]-4,5,6,7-tetrahydro-benzothiazol-2-yl}-2-ethoxy-benzamid,
3,3-Dimethyl-N-{4-[4-(5-trifluormethyl-pyridin-2-yl)-piperazin-1-carbonyl]-4,5,6,7-tetrahydro-benzothiazol-2-yl}-butyramid,
3,3-Dimethyl-N-{4-[4-(3-phenyl-allyl)-piperazin-1-carbonyl]-4,5,6,7-tetrahydro-benzothiazol-2-yl}-butyramid
N-[4-(4-Benzhydryl-piperazin-1-carbonyl)-4,5,6,7-tetrahydro-benzothiazol-2-yl]-2-cyclohexyl-acetamid und
Füran-2-carbonsäure-[4-(4-phenyl-piperazin-1-carbonyl)-4,5,6,7-tetrahydro-benzothiazol-2-yl]-amid.
ggf. in Form ihrer reinen Stereoisomeren, insbesondere Enantiomeren oder Diastereomeren, ihrer Racemate oder in Form von Mischungen der Stereoisomeren, insbesondere der Enantiomeren und/oder Diastereomeren, in einem beliebigen Mischungsverhältnis, oder jeweils ggf. in Form entsprechender Salze oder jeweils ggf. in Form entsprechender Solvate.

Ein weiterer Gegenstand der vorliegenden Erfindung ist ein Verfahren zur Herstellung der erfindungsgemäßen substituierten 4,5,6,7-Tetrahydrobenzothiazol-2-yl-amin-Verbindungen der vorstehend angegebenen allgemeinen Formel I, gemäß dem wenigstens eine Verbindung der Formel II

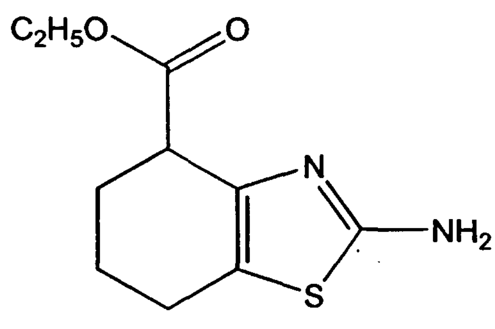

II,

ggf. in Form eines Salzes, durch Umsetzung mit wenigstens einem Acylierungsreagenz der allgemeinen Formel III,

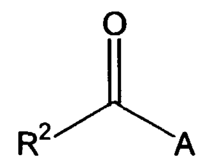

III

worin $R^2$ die vorstehend genannte Bedeutung hat und A für eine vom Acyl-Rest $R^2$-(C=O)- abspaltbare Gruppe, vorzugsweise für -OH, -Cl oder-O-(C=O)-$R^2$ steht,
in eine Verbindung der allgemeinen Formel IV,

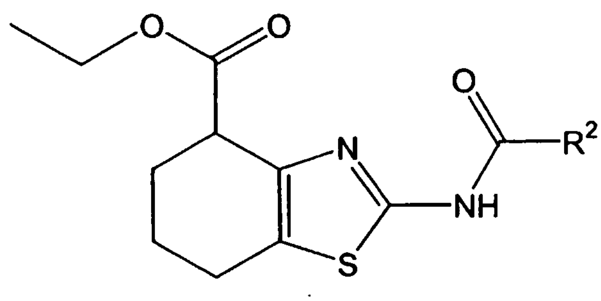

IV

überführt wird, welche ggf. nach üblichen Methoden gereinigt und/oder ggf. isoliert wird, und ggf. durch Spaltung des Ethylesters in eine Verbindung der allgemeinen Formel V

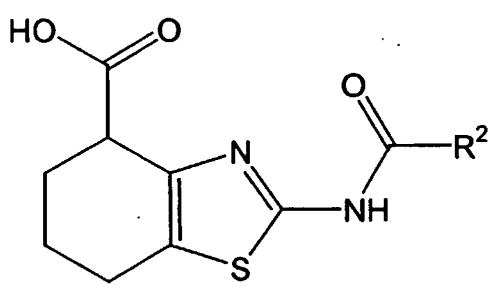

V

überführt wird, welche ggf. nach üblichen Methoden gereinigt und/oder ggf. isoliert wird, und wenigstens eine Verbindung der allgemeinen Formel IV und/oder wenigstens eine Verbindung der allgemeinen Formel V durch Umsetzung mit wenigstens einer Verbindung der allgemeinen Formel $R^1$-H, worin $R^1$ die vorstehend genannte Bedeutung hat, in eine erfindungsgemäße Verbindung der vorstehend allgemeinen Formel I überführt wird, welche ggf. nach üblichen Methoden gereinigt und/oder ggf. isoliert wird.

Die Synthese der Verbindung der vorstehend angegebenen Formel II, ggf. in Form eines entsprechenden Salzes, kann nach üblichen, dem Fachmann bekannten Methoden erfolgen, beispielsweise durch Umsetzung von 2-Oxo-cyclohexancarbonsäureethylester mit Halogen, vorzugsweise Brom oder Chlor, unter üblichen, dem Fachmann bekannten Bedingungen zu einem entsprechend substituierten 3-Halogen-2-oxo-cyclohexancarbonsäureethylester.

Vorzugsweise erfolgt die Umsetzung in einem geeigneten Lösungsmittel wie beispielsweise Chloroform bei einer Temperatur ≤ 30 °C, ggf. unter Inertgasatmosphäre wie Stickstoff.

Der so erhaltene 3-Halogen-2-oxo-cyclohexancarbonsäureethylester, vorzugsweise 3-Brom- oder 3-Chlor-2-oxo-cyclohexancarbonsäureethylester, kann anschließend mit Thioharnstoff nach üblichen, dem Fachmann bekannten Verfahren zu 2-Amino-4,5,6,7-tetrahydro-benzothiazol-4-carbonsäureethylester der allgemeinen Formel II, ggf. in Form eines Salzes, vorzugsweise des Hydrobromids bzw. Hydrochlorid, umgesetzt werden. Vorzugsweise erfolgt die Umsetzung in einem geeigneten Lösungsmittel wie beispielsweise Ethanol bei einer Temperatur ≤ 30 °C, ggf. unter Inertgasatmosphäre wie Stickstoff.

Die Umsetzung des 2-Amino-4,5,6,7-tetrahydro-benzothiazol-4-carbonsäureethylesters der allgemeinen Formel II bzw. eines entsprechenden Salzes mit einem Acylierungsreagenz der allgemeinen Formel III kann unter üblichen dem Fachmann bekannten Bedingungen erfolgen.

Vorzugsweise kann der 2-Amino-4,5,6,7-tetrahydro-benzothiazol-4-carbonsäureethylester durch Acylierung mit einer entsprechenden Carbonsäure in Gegenwart wasserentziehender Mittel wie beispielsweise Natrium- oder Magnesiumsulfat, Phosphoroxid oder in Gegenwart von Kopplungsreagenzien wie beispielsweise 1,1'-Carbonyldiimidazol oder Dicyclohexylcarbodiimid in die entsprechende Verbindung der allgemeinen Formel IV überführt werden.

Ebenfalls bevorzugt kann die Acylierung des 2-Amino-4,5,6,7-tetrahydro-benzothiazol-4-carbonsäureethylesters mit einem entsprechenden Carbonsäurechlorid oder einem entsprechenden Carbonsäureanhydrid vorzugsweise in Gegenwart wenigstens einer Base erfolgen. Als Basen sind übliche anorganische Basen und/oder organische Base wie beispielsweise Diisopropylethylamin, Triethylamin oder Diethylamin geeignet. Die Acylierung mit einem entsprechenden Carbonsäurechlorid erfolgt bevorzugt in einem geeigneten Lösungsmittel wie beispielsweise Methylenchlorid bei einer Temperatur ≤ 30 °C.

Die 2-[(Acyl)amino]-4,5,6,7-tetrahydro-benzothiazol-4-carbonsäureethylester der allgemeinen Formel IV können unter Amidbildung mit entsprechend substituierten primären oder sekundären Aminen der allgemeinen Formel $R^1$-H, worin $R^1$ die vorstehend genannte Bedeutung hat, unter üblichen, dem Fachmann bekannten Bedingungen direkt zu den entsprechenden erfindungsgemäßen substituierten 4,5,6,7-Tetrahydro-benzothiazol-2-yl-amin-Verbindungen der allgemeinen Formel I umgesetzt werden.

Alternativ - und dies ist die bevorzugte Variante des erfindungsgemäßen Verfahrens - kann der entsprechend substituierte 2-[(Acyl)amino]-4,5,6,7-tetrahydro-benzothiazol-4-carbonsäureethylester zunächst einer Esterspaltung unter üblichen, dem Fachmann bekannten Bedingungen unterworfen werden. Vorzugsweise kann die Esterspaltung in Gegenwart wässriger anorganischer Base wie beispielsweise Natronlauge (NaOH), Kalilauge (KOH) oder Lithiumhydroxid (LiOH) bei einer Temperatur ≤ 30 °C, ggf. unter Inertgasatomosphäre erfolgen, wobei die entsprechende 2-[(Acyl)amino]-4,5,6,7-tetrahydro-benzothiazol-4-carbonsäure-Verbindung der allgemeinen Formel V erhalten wird.

Die so erhaltene 2-[(Acyl)amino]-4,5,6,7-tetrahydro-benzothiazol-4-carbonsäure-Verbindung der allgemeinen Formel V kann anschließend unter üblichen, dem Fachmann bekannten Bedingungen, beispielsweise in Gegenwart wasserentziehender Mittel wie Natrium- oder Magnesiumsulfat, Phosphoroxid oder in Gegenwart von Kopplungsreagenzien wie beispielsweise 1,1'-Carbonyldiimidazol oder Dicyclohexylcarbodiimid unter Amidbildung mit entsprechend substituierten primären oder sekundären Aminen der allgemeinen Formel $R^1$-H, worin $R^1$ die vorstehend angegebene Bedeutung hat, zu einer erfindungsgemäßen substituierten 4,5,6,7-Tetrahydro-benzothiazol-2-yl-amin-Verbindung der allgemeinen Formel I umgesetzt werden. Vorzugsweise erfolgt die Umsetzung in einem geeigneten Lösungsmittel wie beispielsweise Methylenchlorid bei einer Temperatur ≤ 30 °C.

Die in den vorstehend genannten Umsetzungen zum Einsatz kommenden Edukte, Reagenzien und Lösungsmittel sind jeweils käuflich am Markt erhältlich bzw. können nach üblichen, dem Fachmann bekannten Verfahren hergestellt werden.

Die jeweiligen Zwischenprodukte der vorstehend beschriebenen Umsetzungen sowie die erfindungsgemäßen substituierten 4,5,6,7-Tetrahydro-benzothiazol-2-ylamin-Verbindungen der vorstehenden allgemeinen Formel I selbst können - sofern erforderlich oder gewünscht - jeweils nach üblichen, dem Fachmann geläufigen Verfahren gereinigt und/oder isoliert werden, beispielsweise durch chromatographische Verfahren, Umkristallisation, Extraktion, Waschen oder Kombinationen aus wenigstens zwei dieser Verfahren.

Sofern die erfindungsgemäßen substituierten 4,5,6,7-Tetrahydro-benzothiazol-2-ylamin-Verbindungen der vorstehenden allgemeinen Formel I nach ihrer Herstellung in Form einer Mischung ihrer Stereoisomeren, beispielsweise in Form ihrer Racemate oder anderer Mischungen ihrer verschiedenen Enantiomeren und/oder Diastereomeren erhalten werden, können diese nach üblichen, dem Fachmann bekannten Verfahren getrennt und ggf. isoliert werden. Beispielhaft seien chromatographische Trennverfahren, insbesondere Flüssigkeits-chromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomere, z.B. mittels HPLC an chiraler Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Die erfindungsgemäßen substituierten 4,5,6,7-Tetrahydro-benzothiazol-2-ylamin-Verbindungen der vorstehenden allgemeinen Formeln I sowie ggf. jeweils entsprechende Stereoisomere können ggf. nach üblichen, dem Fachmann bekannten Verfahren auch in Form ihrer Salze, vorzugsweise ihrer physiologisch verträglichen Salze, erhalten werden, wobei das erfindungsgemäße Arzneimittel eines oder mehrere physiologisch verträgliche Salze einer oder mehrerer dieser Verbindungen aufweisen kann.

Die physiologisch verträglichen Salze der erfindungsgemäßen substituierten 4,5,6,7-Tetrahydro-benzothiazol-2-ylamin-Verbindungen der vorstehenden allgemeinen Formel I können beispielsweise durch Umsetzung mit einer oder mehreren anorganischen oder organischen Säuren, vorzugsweise ausgewählt aus der Gruppe bestehend aus Perchlorsäure, Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, Sacharinsäure, Cyclohexansulfamidsäure, Aspartam, Monomethylsebacinsäure, 5-Oxo-prolin, Hexan-1-sulfonsäure, Nicotinsäure, 2-Aminobenzoesäure, 3-Aminobenzoesäure oder 4-Aminobenzoesäure, 2,4,6-Trimethylbenzoesäure, $\alpha$-Liponsäure, Acetylglycin, Hippursäure, Phosphorsäure, Maleinsäure, Malonsäure und Asparaginsäure, erhalten werden.

Die erfindungsgemäßen substituierten 4,5,6,7-Tetrahydro-benzothiazol-2-ylamin-Verbindungen der vorstehenden allgemeinen Formel I sowie ggf. entsprechende Stereoisomere und jeweils deren physiologisch verträgliche Salze können ggf. nach üblichen, dem Fachmann bekannten Verfahren in Form ihrer Solvate, insbesondere Hydrate erhalten werden.

Es wurde nun überraschend gefunden, daß diese erfindungsgemäßen substituierten 4,5,6,7-Tetrahydro-benzothiazol-2-ylamin-Verbindungen eine Affinität zu 5HT und Noradrenalin-Rezeptoren aufweisen und zur Inhibierung des Noradrenalin-Uptakes sowie zur Inhibierung des 5-Hydroxytryptamin-(5-HT)-Uptakes führen.

Die erfindungsgemäßen substituierten 4,5,6,7-Tetrahydro-benzothiazol-2-ylamin-Verbindungen der vorstehenden allgemeinen Formel I und ggf. entsprechende Stereoisomere sowie jeweils die entsprechenden Salze und Solvate sind toxikologisch unbedenklich und eignen sich daher als pharmazeutische Wirkstoffe in Arzneimitteln.

Ein weiterer Gegenstand der vorliegenden Erfindung ist daher ein Arzneimittel enthaltend wenigstens eine substituierte 4,5,6,7-Tetrahydro-benzothiazol-2-ylamin-Verbindung der allgemeinen Formel I, ggf. in Form ihres reinen Stereoisomeren, insbesondere Enantiomeren oder Diastereomeren, ihres Racemates oder in Form von Mischungen der Stereoisomeren, insbesondere der Enantiomeren und/oder Diastereomeren, in einem beliebigen Mischungsverhältnis, oder ggf. in Form eines entsprechenden Salzes oder jeweils in Form eines entsprechendes Solvates.

Bevorzugt eignet sich das erfindungsgemäße Arzneimitttel zur Regulation der Noradrenalin-Wiederaufnahme (Noradrenalin-Uptake), zur Regulation der 5-Hydroxy-Tryptophan-Wiederaufnahme (5-HT-Uptake), zur Behandlung von Akohol- und/oder Drogen- und/oder Medikamentenmißbrauch, zur Prophylaxe und/oder Behandlung von Akohol- und/oder Drogen- und/oder Medikamentenabhängigkeit, zur Prophylaxe und/oder Behandlung von Entzündungen, zur Prophylaxe und/oder Behandlung von Depressionen, zur Prophylaxe und/oder Behandlung von Antriebslosigkeit, zur Prophylaxe und/oder Behandlung von Störungen der Nahrungsmittelaufnahme, vorzugsweise ausgewählt aus der Gruppe bestehend aus Bulimie, Anorexie, Fettsucht und Kachexie, zur Prophylaxe und/oder Behandlung von Katalepsie, zur Vigilanzsteigerung, zur Libidosteigerung oder zur Anxiolyse. Darüber hinaus zeigen die erfindungsgemäßen Verbindungen auch eine ausgeprägte analgetische Wirkung, so daß sich die erfindungsgemäßen Arzneimittel insbesondere auch zur gleichzeitigen Behandlung von Schmerzen und Depressionen hervorragend eignen.

Ein weiterer Gegenstand der vorliegenden Erfindung ist die Verwendung wenigstens einer substituierten 4,5,6,7-Tetrahydro-benzothiazol-2-ylamin-Verbindung der allgemeinen Formel I, ggf. in Form ihres reinen Stereoisomeren, insbesondere Enantiomeren oder Diastereomeren, ihres Racemates oder in Form von Mischungen der Stereoisomeren, insbesondere der Enantiomeren und/oder Diastereomeren, in einem beliebigen Mischungsverhältnis, oder ggf. in Form eines entsprechenden Salzes oder jeweils in Form eines entsprechendes Solvates, zur Herstellung eines Arzneimittels zur Regulation der Noradrenalin-Wiederaufnahme (Noradrenalin-Uptake), zur Regulation der 5-Hydroxy-Tryptophan-Wiederaufnahme (5-HT-Uptake), zur Behandlung von Akoholmißbrauch und/oder Drogenmißbrauch und/oder Medikamentenmißbrauch, zur Prophylaxe und/oder Behandlung von Akoholmißbrauch und/oder Drogenmißbrauch und/oder Medikamentenabhängigkeit, zur Prophylaxe und/oder Behandlung von Entzündungen, zur Prophylaxe und/oder Behandlung von Depressionen, zur Behandlung von Schmerzen, zur Behandlung von Antriebslosigkeit, zur Prophylaxe und/oder Behandlung von Störungen der Nahrungsmittelaufnahme, ausgewählt aus der Gruppe bestehend aus Bulimie, Anorexie, Fettsucht und Kachexie, zur Prophylaxe und/oder Behandlung von Katalepsie, zur Vigilanzsteigerung, zur Libidosteigerung oder zur Anxiolyse. Besonders bevorzugt ist die Verwendung wenigstens einer substituierten 4,5,6,7-Tetrahydro-benzothiazol-2-ylamin-Verbindung der allgemeinen Formel I, ggf. in Form ihres reinen Stereoisomeren, insbesondere Enantiomeren oder Diastereomeren, ihres Racemates oder in Form von Mischungen der Stereoisomeren, insbesondere der Enantiomeren und/oder Diastereomeren, in einem beliebigen Mischungsverhältnis, oder ggf. in Form eines entsprechenden Salzes oder jeweils in Form eines entsprechendes Solvates, zur Herstellung eines Arzneimittels zur gleichzeitigen Behandlung von Schmerzen und Depressionen.

Das erfindungsgemäße Arzneimittel kann als flüssige, halbfeste oder feste Arzneiform, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form

von Pellets oder Granulaten, ggf. zu Tabletten verpreßt, in Kapseln abgefüllt oder in einer Flüssigkeit suspendiert, vorliegen und als solche auch verabreicht werden.

Neben einer oder mehreren der in dem erfindungsgemäßen Arzneimittel zum Einsatz kommenden substituierten 4,5,6,7-Tetrahydro-benzothiazol-2-ylamin-Verbindungen der vorstehenden allgemeinen Formel I, ggf. in Form ihres reinen Stereoisomeren, insbesondere Enantiomeren oder Diastereomeren, ihres Racemates oder in Form von Mischungen der Stereoisomeren, insbesondere der Enantiomeren oder Diastereomeren, in einem beliebigen Mischungsverhältnis, oder ggf. in Form eines entsprechenden Salzes oder jeweils in Form eines entsprechendes Solvates, enthält das erfindungsgemäße Arzneimittel üblicherweise weitere physiologisch verträgliche pharmazeutische Hilfsstoffe, die bevorzugt ausgewählt werden können aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays.

Die in dem erfindungsgemäßen Arzneimittel zum Einsatz kommenden substituierten 4,5,6,7-Tetrahydro-benzothiazol-2-ylamin-Verbindungen in einem Depot in gelöster Form oder in einem Pflaster, gegebenenfalls unter Zusatz von die Hautpenetration fördernden Mitteln, sind geeignete perkutane Applikationszubereitungen.
Oral oder perkutan anwendbare Zubereitungsformen können die jeweiligen substituierten 4,5,6,7-Tetrahydro-benzothiazol-2-ylamin-Verbindungen auch verzögert freisetzen.

Die an den Patienten zu verabreichende Menge der jeweiligen substituierten 4,5,6,7-Tetrahydro-benzothiazol-2-ylamin-Verbindung kann variieren und ist beispielsweise abhängig vom Gewicht oder Alter des Patienten sowie von der Applikationsart, der Indikation und dem Schweregrad der Erkrankung. Üblicherweise werden 0.005 bis 500 mg/kg, vorzugsweise 0.05 bis 50 mg/kg Körpergewicht des Patienten wenigstens einer substituierten 4,5,6,7-Tetrahydro-benzothiazol-2-ylamin-Verbindung appliziert.

Im folgenden werden die pharmakologischen Methoden zur Bestimmung der analgetischen Wirksamkeit sowie zur Bestimmung der Noradrenalin- bzw. 5-HT-Wiederaufnahmehemmung durch die erfindungsgemäßen substituierten 4,5,6,7-Tetrahydro-benzothiazol-2-ylamin-Verbindungen beschrieben.

**Pharmakologische Methoden:**

**I. Analgesieprüfung im Writhing-Test an der Maus**

Die Untersuchung auf analgetische Wirksamkeit wird im Phenylchinon-induzierten Writhing an der Maus, modifiziert wie in der Veröffentlichung von I.C. Hendershot und J. Forsaith (1959) J. Pharmacol. Exp. Ther. 125, 237-240 beschrieben durchgeführt. Die entsprechende Beschreibung wird hiermit als Referenz eingeführt und gilt als Teil der vorliegenden Offenbarung.
Dazu werden männliche NMRI-Mäuse im Gewicht von 25 bis 30 g verwendet. Gruppen von 10 Tieren pro Substanzdosis erhalten 10 Minuten nach intravenöser Gabe der Prüfsubstanzen 0,3 ml/Maus einer 0,02%igen wäßrigen Lösung von Phenylchinon (Phenylbenzochinon, Fa. Sigma, Deisenhofen; Herstellung der Lösung unter Zusatz von 5 % Ethanol und Aufbewahrung im Wasserbad bei 45°C) intraperitoneal appliziert. Die Tiere werden einzeln in Beobachtungskäfige gesetzt. Mittels eines Drucktastenzähler wird die Anzahl der schmerzinduzierten Streckbewegungen (sogenannte Writhingreaktionen = Durchdrücken des Körpers mit Abstrecken der Hinterextremitäten) 5 bis 20 Minuten nach der Phenylchinon-Gabe ausgezählt. Als Kontrolle werden Tiere mitgeführt, die nur physiologische Kochsalzlösung erhalten. Alle Substanzen werden in der Standarddosierung von 10 mg/kg getestet. Die prozentuale Hemmung (%Hemmung) der Writhingreaktion durch eine Substanz wird nach folgender Formel berechnet:

$$\%\ \text{Hemmung} = 100 - \frac{\text{Writhingreaktionen der behandelten Tiere}}{\text{Writhingreaktionen der Kontrolltiere}} * 100$$

Für einige Substanzen werden aus der dosisabhängigen Abnahme der Writhingreaktionen im Vergleich zu parallel untersuchten Phenylchinon-Kontrollgruppen mittels Regressionsanalyse (Auswerteprogramm Martens EDV Service, Eckental) die $ED_{50}$-Werte mit 95 % Vertrauensbereich der Writhingreaktion berechnet.

**II. Methode zur Bestimmung der Noradrenalin- und der 5HT-Uptake-Inhibierung:**

Für in vitro Studien wurden Synaptosomen aus Rattenhirnarealen frisch isoliert, wie in der Veröffentlichung "The isolation of nerve endings from brain" von E.G. Gray und V.P. Whittaker, J. Anatomy 96, Seiten 79-88, 1962, beschrieben. Die entsprechende Literaturbeschreibung wird hiermit als Referenz eingeführt und gilt als Teil der Offenbarung.
Das Gewebe (Hypothalamus für die Bestimmung der Noradrenalin-Uptake-Inhibierung und Mark und Pons für die Bestimmung der 5HT-Uptake-Inhibierung) wurde in eisgekühlter 0,32 M Sucrose (100 mg Gewebe / 1 mL) in einem Glas-Homogenisierer mit Teflonstößel homogenisiert, indem fünf volle Auf-und Abschläge bei 840 Umdrehungen /Minute benutzt wurden.
Das Homogenat wurde bei 4˚C für 10 Minuten bei 1000 g zentrifugiert. Nach anschließender Zentrifugierung bei 17000 g für 55 Minuten erhält man die Synaptosomen ($P_2$-Fraktion), die in 0,32 M Glucose (0,5 mU 100 mg des ursprünglichen Gewichts) noch einmal suspendiert wurden.
Der jeweilige Uptake wurde in einer 96-well Mikrotiterplatte gemessen. Das Volumen betrug 250 µl und die Inkubation erfolgte bei Raumtemperatur (ca. 20-25 ˚C) untere $O_2$ Atmosphäre.
Die Inkubationszeit betrug 7,5 Minuten für [$^3$H]-NA und 5 Minuten für [$^3$H]-5-HT. Anschließend wurden die 96 Proben durch eine Unifilter GF/B® Mikrotiterplatte (Packard) filtriert und mit 200 mL inkubierten Puffer mit Hilfe eines "Brabdel Cell-Harvester MPXRI-96T" gewaschen. Die Unifilter GF/B Platte wurde bein 55˚C 1 h getrocknet. Im Anschluß wurde die Platte mit einem Back seal® (Packard) verschlossen und 35 µl Szintilationsflüssigkeit pro Well (Ultima Gold®, Packard) versetzt. Nach dem Verschließen mit einem top seal® (Packard) wurde, nach der Einstellung des Gleichgewichts (etwa 5 h), die Radioaktivität in einem "Trilux 1450 Microbeta" (Wallac) bestimmt.
Folgende Kenndaten wurden für den NA-Transporter ermittelt:

$$\text{NA-Uptake : Km} = 0{,}32 \pm 0{,}11\ \mu M$$

Die Menge des bei der vorstehenden Bestimmung eingesetzten Proteins entsprach den aus der Literatur bekannten Werten, wie z.B. in "Protein measurement with the folin phenol reagent", Lowry et al., J. Biol. Chem., 193, 265-275, 1951 beschrieben.
Eine detaillierte Methodenbeschreibung kann auch der Literatur, beispielsweise aus M.Ch. Frink, H.-H. Hennies, W. Engelberger, M. Haurand und B. Witffert (1996) Arzneim.-Forsch./Drug Res. 46 (III), 11, 1029-1036, entnommen werden. Die entsprechenden Literaturbeschreibungen werden hiermit als Referenz eingeführt und gelten als Teil der Offenbarung.
Im folgenden wird die Erfindung anhand von Beispielen erläutert. Diese Erläuterungen sind lediglich beispielhaft und schränken den allgemeinen Erfindungsgedanken nicht ein.

**Beispiele:**

**Allgemeine Vorschrift zur Herstellung der erfindungsgemäßen substituierten 4,5,6,7-Tetrahydrobenzothiazol-2-ylamin-Verbindungen der allgemeinen Formel I:**

**1. Stufe:**

Zu einer Lösung von 2-Oxo-cyclohexancarbonsäureethylester (50 g, 0,29 mol) in $CHCl_3$ (150 ml) wurde bei 0˚C unter Stickstoff als Inertgas langsam tropfenweise Brom (47 g, 0,29 mol) über 45 Minuten zugetropft und für 6 Stunden bei 0˚C und anschließend 16 Stunden bei Raumtemperatur gerührt. Anschließend wurde 2 Stunden Luft in die Reaktionslösung eingeleitet. Die organische Phase wurde mit wässriger gesättigter $NaHCO_3$-Lösung (2 x 15 ml) und wässriger gesättigter NaCl-Lösung (3 x 20 ml) gewaschen. Nach Trocknung der organischen Phase mit $Na_2SO_4$ und Filtration wurde das Lösungsmittel entfernt. Man erhielt das Produkt 3-Brom-2-oxo-cyclohexancarbonsäureethylester mit einer Ausbeute von 72.6 g (entsprechend 99 % der Theorie).

**2. Stufe:**

Zu 3-Brom-2-oxo-cyclohexancarbonsäureethylester (50 g, 0,20 mol) und Thioharnstoff (15,3 g, 0,20 mol) wurde Ethanol (50 ml) gegeben und die Reaktionsmischung für 24 Stunden bei Raumtemperatur unter Stickstoff als Inertgas gerührt. Nach Entfernung des Lösungsmittels wurde Ether (100 ml) zum Rückstand gegeben. Nach Rühren für 2 Stunden bei Raumtemperatur wurde die Suspension abfiltriert und der Feststoff im Vakuum getrocknet. Man erhielt das Produkt 2-Amino-4,5,6,7-tetrahydro-benzothiazol-4-carbonsäureethylester Hydrobromid mit einer Ausbeute von 58.6 g (entsprechend 95 % der Theorie).

**3. Stufe:**

Zu einer Lösung von 2-Amino-4,5,6,7-tetrahydro-benzothiazol-4-carbonsäureethylester Hydrobromid (5.0 g, 16,3 mmol) in Pyridin (50 ml) wurde bei 0˚C tropfenweise das jeweilige Carbonsäurechlorid (16,1 mmol) gelöst in $CH_2Cl_2$ (50 ml) über 30 min langsam zugegeben. Nach Rühren für 1 Stunde bei 0˚C und anschließend über Nacht bei Raumtemperatur gab man $CH_2Cl_2$ (300 ml) und 3 M HCl (300 ml) hinzu. Die organische Phase wurde abgetrennt und die wässrige Phase mit $CH_2Cl_2$ (300 ml) extrahiert. Die vereinigten organischen wurden über $Na_2SO_4$ getrocknet. Nach Filtration und Entfernung des Lösungsmittels erhielt man den jeweiligen 2-[(Acyl)-amino]-4,5,6,7-tetrahydro-benzothiazol-4-carbonsäureethylester.

**4. Stufe:**

Zu dem jeweiligen 2-[(Acyl)-amino]-4,5,6,7-tetrahydro-benzothiazol-4-carbonsäureethylester (16 mmol) und NaOH (163 mmol) wurde Wasser (20 ml) und Methanol (80 ml) zugegeben und bei Raumtemperatur unter Stickstoff als Inertgas bis zur vollständigen Umsetzung gerührt. Nach Entfernung des Methanol wurde 1 M HCl (250 ml) zugegeben und mit $CH_2Cl_2$ (3 x 20 ml) extrahiert. Nach Trocknung der vereinigten organischen Phasen über $Na_2SO_4$ und Filtration erhielt man die jeweilige 2-[(Acyl)amino]-4,5,6,7-tetrahydro-benzothiazol-4-carbonsäure.

**5. Stufe:**

Zu der Lösung der jeweiligen 2-[(Acyl)amino]-4,5,6,7-tetrahydro-benzothiazol-4-carbonsäure (100 μmol) in $CH_2Cl_2$ (2 ml) wurde 1,1'-Carbonyldiimidazol (CDI, 110 μmol) zugegeben und für 2 Stunden bei Raumtemperatur gerührt. Nach Abkühlung der Suspension auf 0˚C wurde das jeweilige Amin (100 μmol) gelöst in $CH_2Cl_2$ (2 ml) zugegeben. Nach Rühren für 30 min bei 0˚C und über Nacht bei Raumtemperatur gab man Wasser (1 ml) hinzu. Nach Filtration, Separation der organischen Phase und Trocknung wurde das Lösungsmittel entfernt und der so erhaltene Rückstand mittels präparativer HPLC aufgereinigt, wobei die jeweilige erfindungsgemäße substituierte 4,5,6,7-Tetrahydro-benzothiazol-2-ylamin-Verbindung erhalten wurde.

**Beispiel 24:** 2-(3,3-Dimethyl-butyrylamino)-4,5,6,7-tetrahydro-benzothiazol-4-carbonsäure (3-dimethylamino-propyl)-amid

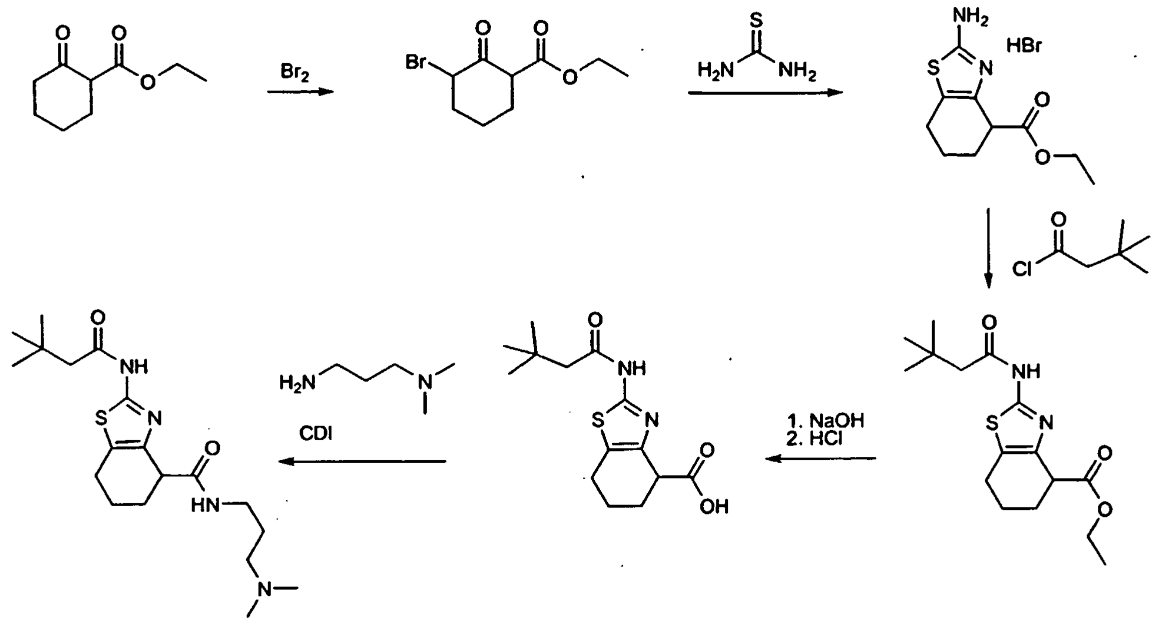


**Stufen 1 und 2:**

Die Herstellung von 2-Amino-4,5,6,7-tetrahydro-benzothiazol-4-carbonsäureethylester Hydrobromid erfolgte gemäß der vorstehend beschriebenen allgemeinen Vorschrift.

**Stufe 3:**

Zu einer Lösung von 2-Amino-4,5,6,7-tetrahydro-benzothiazol-4-carbonsäureethyl-ester Hydrobromid (0.92 g, 0.3 mol) in Acetonitril (25 ml) wurde Triethylamin ($Et_3N$, 0.41 g, 0.4 mol) zugegeben. Nach Abkühlung auf 0˚C wurde tert-Butylacetylchlorid (0.55 g, 0.4 mol) langsam zugegeben und anschließend über Nacht bei Raumtemperatur gerührt. Nach Entfernung des Lösungsmittels und Aufreinigung durch Säulechromatographie erhielt man 2-(3,3-Dimethyl-butyrylamino)-4,5,6,7-tetrahydro-benzothiazol-4-carbonsäureethylester mit einer Ausbeute von 185 mg (entsprechend 19 % der Theorie).

**Stufe 4:**

Zu 2-(3,3-Dimethyl-butyrylamino)-4,5,6,7-tetrahydro-benzothiazol-4-carbonsäureethylester (185 mg, 0.57 mmol) und NaOH (27 mg, 0.675 mmol) wurde Wasser (2 ml) und Methanol (8 ml) zugegeben und bei Raumtemperatur unter Stickstoff als Inertgas gerührt. Zur vollständigen Umsetzung wurde erneut NaOH (200 mg, 5 mmol) zugegeben und für weitere 24 Stunden bei Raumtemperatur gerührt. Nach Entfernung des MeOH wurde 2M HCl (10 ml) zugegeben und mit $CH_2Cl_2$ (3 x 20 ml) extrahiert. Nach Trocknung der vereinigten organischen Phasen über $Na_2SO_4$ und Filtration erhielt man das Produkt 2-(3,3-Dimethyl-butyrylamino)-4,5,6,7-tetrahydro-benzothiazol-4-carbonsäure mit einer Ausbeute von 144 mg (entsprechend 85 % der Theorie).

**Stufe 5:**

Zu einer Lösung von 2-(3,3-Dimethyl-butyrylamino)-4,5,6,7-tetrahydro-benzothiazol-4-carbonsäure (144 mg, 0.486 mmol) in $CH_2Cl_2$ (10 ml) wurde 1,1'-Carbonyldiimidazol (CDI, 87 mg, 0.537 mmol) zugegeben und für 2 Stunden bei Raumtemperatur gerührt. Nach Abkühlung der Suspension auf 0˚C wurde N,N-Dimethyl-1,3-propandiamin (51 mg, 0.50 mmol) gelöst in $CH_2Cl_2$ (10 ml) tropfenweise über 10 Minuten zugegeben. Nach Rühren für 1 Stunde bei 0 ˚C und über Nacht bei Raumtemperatur gab man wenige Tropfen Wasser hinzu und extrahierte mit $CH_2Cl_2$ (3 x 10 ml). Nach Trocknung der organischen Phase über $Na_2SO_4$ und Filtration wurde das Lösungsmittel entfernt und der Rückstand über Säulenchromathographie gereinigt. Man erhielt das Produkt 2-(3,3-Dimethyl-butyrylamino)-4,5,6,7-tetrahydro-benzothiazol-4-carbonsäure-(3-dimethylamino-propyl)-amid mit einer Ausbeute von 125 mg (entsprechend 68 % der Theorie).

**Beispiel 89:** Furan-2-carbonsäure-[4-(4-phenyl-piperazin-1-carbonyl)-4,5,6,7-tetrahydro-benzothiazol-2-yl]-amid

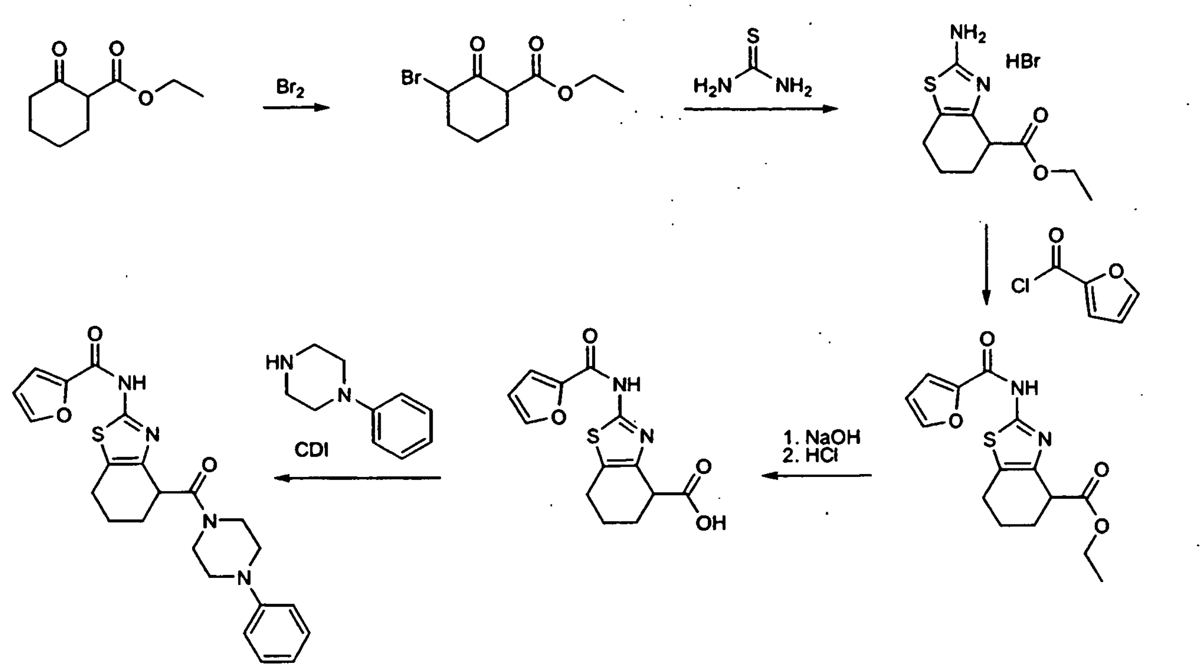


**Stufen 1 und 2:**

Die Herstellung von 2-Amino-4,5,6,7-tetrahydro-benzothiazol-4-carbonsäureethylester Hydrobromid erfolgte gemäß der vorstehend beschriebenen allgemeinen Vorschrift.

**Stufe 3:**

Zu einer Lösung von 2-Amino-4,5,6,7-tetrahydro-benzothiazol-4-carbonsäureethylester Hydrobromid (5.0 g, 16.3 mmol) in Pyridin (50 ml) wurde bei 0°C tropfenweise 2-Furanoylchlorid (2.1 g, 16.1 mmol) gelöst in $CH_2Cl_2$ (50 ml) über 30 Minuten langsam zugegeben. Nach Rühren für 1 Stunde bei 0°C und anschließend über Nacht bei Raumtemperatur gab man $CH_2Cl_2$ (300 ml) und 3 M HCl (300 ml) hinzu. Die organische Phase wurde abgetrennt und die wässrige Phase mit $CH_2Cl_2$ (300 ml) extrahiert. Die vereinigten organischen wurden über $Na_2SO_4$ getrocknet. Nach Filtration und Entfernung des Lösungsmittels erhielt man das Rohprodukt 2-[(Furan-2-carbonyl)-amino]-4,5,6,7-tetrahydro-benzothiazol-4-carbonsäureethylester mit einer Ausbeute von 5.3 g.

**Stufe 4:**

Zu 2-[(Furan-2-carbonyl)-amino]-4,5,6,7-tetrahydro-benzothiazol-4-carbonsäureethyl-ester (5.3 g, 16 mmol) und NaOH (6.5 g, 163 mmol) wurde Wasser (20 ml) und Methanol (80 ml) zugegeben und bei Raumtemperatur unter Stickstoff als Inertgas bis zur vollständigen Umsetzung gerührt. Nach Entfernung des Methanol wurde 1 M HCl (250 ml) zugegeben und mit $CH_2Cl_2$ (3 x 20 ml) extrahiert. Nach Trocknung der vereinigten organischen Phasen über $Na_2SO_4$ und Filtration erhielt man das Produkt 2-[(Furan-2-carbonyl)-amino]-4,5,6,7-tetrahydro-benzothiazol-4-carbonsäure ausgehend von 2-Amino-4,5,6,7-tetrahydro-benzothiazol-4-carbonsäureethylester Hydrobromid mit einer Ausbeute von 3.8 g (entsprechend 81 % der Theorie).

**Stufe 5:**

Zu einer Lösung von 2-[(Furan-2-carbonyl)-amino]-4,5,6,7-tetrahydro-benzothiazol-4-carbonsäure (0.5 g, 1.7 mmol) in $CH_2Cl_2$ (10 ml) wurde 1,1'-Carbonyldümidazol (CDI, 0.31 g, 1.9 mmol) zugegeben und für 2 Stunden bei Raumtemperatur gerührt. Nach Abkühlung der Suspension auf 0°C wurde N-Phenylpiperazin (0.28 g, 1.73 mmol) gelöst in $CH_2Cl_2$ (10 ml) tropfenweise über 5 Minuten zugegeben. Nach Rühren für 30 min bei 0°C und über Nacht bei Raumtemperatur gab man wenige Tropfen Wasser hinzu. Nach Trocknung über $Na_2SO_4$ und Filtration wurde das Lösungsmittel entfernt und der Rückstand über Säulenchromathographie gereinigt. Man erhielt das Produkt Furan-2-carbonsäure [4-(4-phenyl-piperazin-1-carbonyl)-4,5,6,7-tetrahydro-benzothiazol-2-yl]-amid mit einer Ausbeute von 514 mg (entsprechend 69 % der Theorie).

**Abkürzungen:**

| | |
|---|---|
| aq. | wäßrig |
| DCM | Dichlormethan |
| DIPEA | Diisopropylethylamin |
| DMF | Dimethylformamid |
| EDCI | N'-(3-Dimethylaminopropyl)-N-ethylcarbodiimid |
| EtOAc | Ethylacetat |
| EtOH | Ethanol |
| ges. | gesättigt |
| HOAt | 1-Hydroxy-7-azabenzotriazol |
| MeOH | Methanol |
| RT | Raumtemperatur |

**Beispiel 67:** 2-(2-Cyclohexyl-acetylamino)-4,5,6,7-tetrahydro-benzothiazol-4-carbonsäure [1-(2,6-dichlor-benzyl)-pyrrolidin-3-yl]-methyl-amid

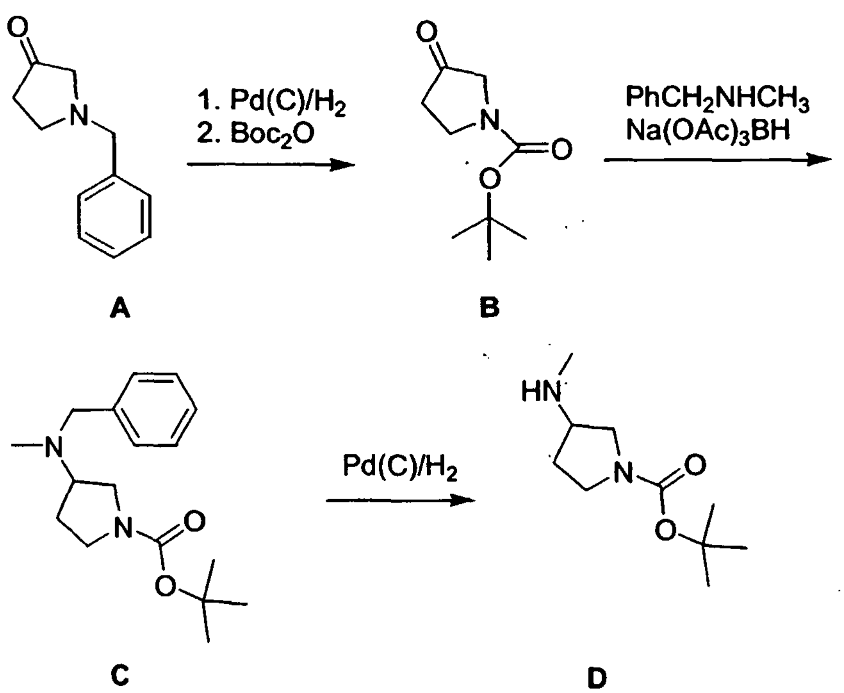


A B C D

**Stufe 1:**

Eine Lösung von 1-(Phenylmethyl)-3-pyrrolidinon (A) (7.18 g, 41 mmol) und Di-tert-butyl-pyrocarbonat (BOC-Anhydrid) (8.94 g, 41 mmol) in absolutiertem EtOH (100 mL) wurde mit Stickstoff gespült. Eine Suspension von Palladium auf Kohle (10 %, 4.36 g, 4.1 mmol) in absolutiertem EtOH (50 mL) wurde hinzu gegeben. Die Reaktionsmischung wurde einer Wasserstoffatomsphäre in einem Ballon (1 atm) ausgesetzt und über Nacht bei RT gerührt. Nach Filtration über Celite und Entfernen des Lösungsmittels im Vakuum wurde das Rohprodukt erhalten, das säulenchromatographisch

($SiO_2$, EtOAc/Heptan 1:2) gereinigt wurde. Es wurden 5.98 g (entsprechend 79 % der Theorie) des gewünschten Produkts B erhalten.

**Stufe 2:**

Die Verbindung B (5.95 g, 32 mmol) wurde in einer Stickstoffatmosphäre in DCM (100 mL) gelöst und N-Methylbenzyl-amin (4.28 g, 35 mmol) wurde hinzu gegeben. Natriumtriacetoxyborhydrid (10.2 g, 48 mmol) wurde langsam bei 0 ˚C hinzu gegeben und die Reaktionsmischung wurde 2 Stunden bei RT gerührt. Anschließend wurden DCM (250 mL) und ges. aq. $NaHCO_3$-Lösung (250 mL) hinzu gefügt. Die Phasen wurden getrennt und die wäßrige Phase wurde mit DCM (250 mL) ausgeschüttelt. Die vereinigten organischen Phasen wurden über $Na_2SO_4$ getrocknet und das Lösungsmittel wurde im Vakuum entfernt. Es wurden 9.72 g (100 % der Theorie) des gewünschten Produkts C erhalten.

**Stufe 3:**

Eine Lösung von Verbindung C (9.7 g, 32 mmol) in 150 mL absolutiertem EtOH wurde mit Stickstoff gespült. Es wurde eine Suspension von Palladium auf Kohle (10 %, 3.42 g, 3.2 mmol) in absolutiertem EtOH (50 mL) hinzu gegeben. Die Reaktionsmischung wurde einer Wasserstoffatomsphäre in einem Ballon (1 atm) ausgesetzt und über Nacht bei RT gerührt. Nach Filtration über Celite und Entfernen des Lösungsmittels im Vakuum wurden 6.74 g (entsprechend 100 % der Theorie) des gewünschten Produkts D erhalten.

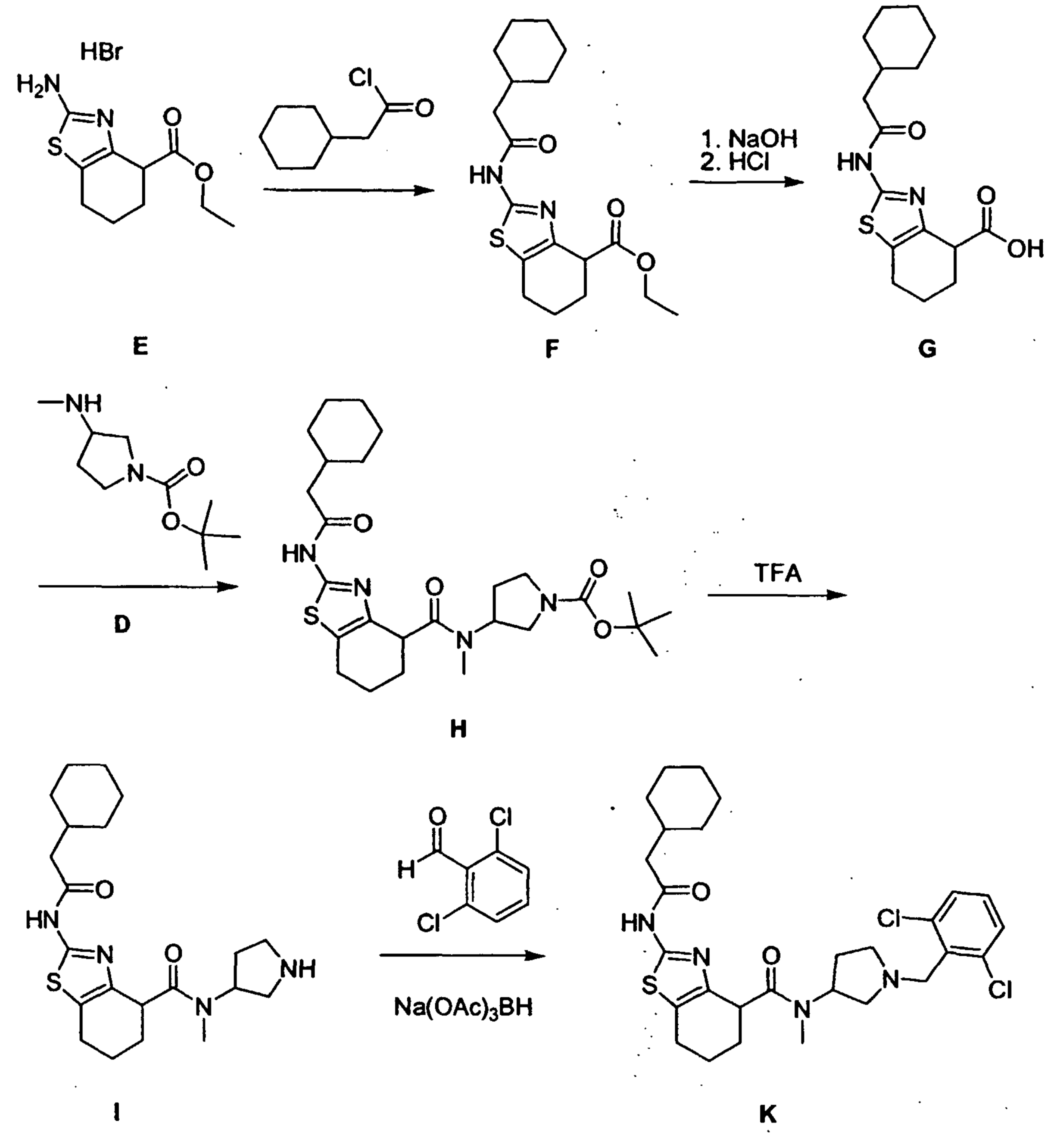


E F G

D

H

I K

### Stufe 1:

Zu einer Lösung von Cyclohexylessigsäure (2.31 g, 16.24 mmol) in DCM (10 mL) wurde Oxalylchlorid (3.10 g, 24.42 mmol) gegeben. Die Reaktionsmischung wurde zwei Stunden bei RT unter einer Stickstoffatmosphäre gerührt. Das Lösungsmittel wurde im Vakuum entfernt, Toluol (50 mL) wurde hinzu gegeben und das Lösungsmittel wurde erneut entfernt. Der Rückstand wurde in DCM (50 mL) gelöst und die resultierende Lösung wurde langsam zu einer Lösung von 2-Amino-4,5,6,7-tetrahydro-benzothiazol-4-carbonsäureethylester Hydrobromid **(E)** (5.0 g, 16.3 mmol) in Pyridin (25 mL) unter einer Stickstoffatmosphäre getropft. Die Reaktionsmischung wurde eine Stunde bei 0 °C und anschließend über Nacht bei RT gerührt. DCM (300 mL) und Salzsäure-Lösung (3 N in Wasser) wurden hinzu gegeben, die Phasen wurden getrennt und die wäßrige Phase wurde mit DCM (300 mL) ausgeschüttelt. Die vereinigten organischen Phasen wurden über $Na_2SO_4$ getrocknet und das Lösungsmittel wurde im Vakuum entfernt. Der Rückstand ergab nach säulenchromatographischer Reinigung ($SiO_2$, EtOAc/Heptan 1:2) 4.9 g (86 % der Theorie) des gewünschten Produkts 2-(2-Cyclohexyl-acetylamino)-4,5,6,7-tetrahydro-benzothiazol-4-carbonsäureethylester **(F).**

### Stufe 2:

Zu 2-(2-Cyclohexyl-acetylamino)-4,5,6,7-tetrahydro-benzothiazol-4-carbonsäureethylester **(F)** (4.9 g, 14 mmol) und NaOH (5.6 g, 0.14 mol) wurde Wasser (20 mL) und MeOH (80 mL) gegeben und bei RT unter Stickstoff als

Inertgas bis zur vollständigen Umsetzung gerührt. Nach Entfernen des MeOH wurde 2 N Salzsäure-Lösung in Wasser (250 mL) und DCM (250 mL) hinzu gegeben. Die Phasen wurden getrennt und die wäßrige Phase wurde mehrfach mit DCM extrahiert. Nach Trocknen der vereinigten organischen Phasen über $Na_2SO_4$ und Filtration erhielt man das Produkt 2-(2-Cyclohexyl-acetylamino)-4,5,6,7-tetrahydro-benzothiazol-4-carbonsäure (G) in einer Ausbeute von 3.9 g (entsprechend 87 % der Theorie).

**Stufe 3:**

Zu einer Lösung des Amins **D** (678 mg, 3.4 mmol) in DCM (15 mL) wurde die Carbonsäure 2-(2-Cyclohexyl-acetylamino)-4,5,6,7-tetrahydro-benzothiazol-4-carbonsäure **(G)** (1201 mg, 3.7 mmol) gegeben. Bei 0 °C wurden EDCI (713 mg, 3.7 mmol) und HOAt (69 mg, 0.5 mmol) hinzu gegeben. Die Reaktionsmischung wurde eine Stunde bei 0 °C und anschließend bei RT über Nacht gerührt. Das Lösungsmittel wurde im Vakuum entfernt und nach säulenchromatographischer Reinigung des Rückstand ($SiO_2$, DCM/MeOH 97:3 → 95:5) wurden 1.59 g des gewünschten Produkts **H** erhalten (93 % der Theorie).

**Stufe 4:**

Zu einer Lösung von **H** (836 mg, 1.66 mmol) in DCM (20 mL) wurde bei RT langsam Trifluoressigsäure (8.0 mL, 108 mmol) gegeben. Nach einer Stunde wurde das Lösungsmittel im Vakuum entfernt. Der Rückstand wurde dreimal in Toluol aufgenommen und jeweils das Lösungsmittel im Vakuum wieder entfernt. Die so erhaltene Verbindung 2-(2-Cyclohexyl-acetylamino)-4,5,6,7-tetrahydro-benzothiazol-4-carbonsäure-(1-pyrrolidin-3-yl)-methyl-amid **(I)** wurde ohne weitere Aufarbeitung in Stufe 5 eingesetzt.

**Stufe 5:**

Verbindung 2-(2-Cyclohexyl-acetylamino)-4,5,6,7-tetrahydro-benzothiazol-4-carbonsäure-(1-pyrrolidin-3-yl)-methyl-amid **(I)** (1.66 mmol) wurde in DCM (15 mL) unter einer Stickstoffatmosphäre gelöst. Es wurden 2,6-Dichlorbenzaldehyd (320 mg, 1.82 mmol) und Natriumtriacetoxyborhydrid (530 mg, 2.48 mmol) hinzu gegeben. Die Reaktionsmischung wurde über Nacht bei RT unter einer Stickstoffatmosphäre gerührt. Nach Entfernen des Lösungsmittels im Vakuum und säulenchromatographischer Reinigung ($SiO_2$, DCM/MeOH 95:5) wurden 623 mg (1.11 mmol, 67 % der Theorie) des gewünschten Produkts 2-(2-Cyclohexyl-acetylamino)-4,5,6,7-tetrahydro-benzothiazol-4-carbonsäure [1-(2,6-dichlor-benzyl)-pyrrolidin-3-yl]-methyl-amid **(K)** erhalten.

**Beispiel 9:** 2-[(Furan-2-carbonyl)-amino]-4,5,6,7-tetrahydro-benzothiazol-4-carbonsäure-[1-(2,4,6-trimethoxy-benzyl)-pyrrolidin-3-yl]-amid

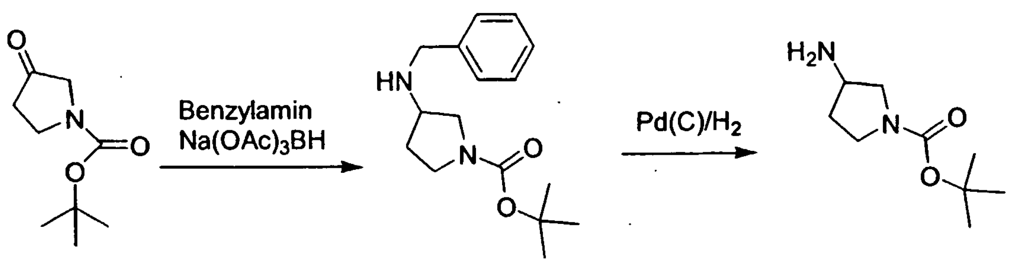


**B** **L** **M**

**Stufe 1:**

Die Verbindung **B** (1.5 g, 8.1 mmol) wurde in einer Stickstoffatmosphäre in DCM (25 mL) gelöst und Benzylamin (955 mg, 8.91 mmol) wurde hinzu gegeben. Natriumtriacetoxyborhydrid (2.58 g, 12.17 mmol) wurde langsam bei 0 °C hinzu gegeben und die Reaktionsmischung wurde 30 Minuten bei RT gerührt. Anschließend wurden DCM (250 mL) und ges. aq. $NaHCO_3$-Lösung (250 mL) hinzu gefügt. Die Phasen wurden getrennt und die wäßrige Phase wurde mit DCM (250 mL) ausgeschüttelt. Die vereinigten organischen Phasen wurden über $Na_2SO_4$ getrocknet und das Lösungs-

mittel wurde im Vakuum entfernt. Es wurden 1.8 g (80 % der Theorie) des gewünschten Produkts **L** erhalten.

**Stufe 2:**

Über eine Lösung von Verbindung **L** (1.8 g, 6.5 mmol) in 50 mL absolutiertem EtOH wurde 20 Minuten Stickstoff geleitet, Palladium auf Kohle (10 %, 347 mg, 0.33 mmol) wurde hinzu gegeben und es wurde für weitere 10 Minuten Stickstoff über die Reaktionsmischung geleitet. Die Reaktionsmischung wurde einer Wasserstoffatomsphäre in einem Ballon (1 atm) ausgesetzt und über Nacht bei RT gerührt. Es wurde 30 Minuten Stickstoff über die Reaktionsmischung geleitet. Nach Filtration über Celite, Waschen des Filterkuchens mit DCM und Entfernen des Lösungsmittels im Vakuum wurden 1.45 g (entsprechend 100 % der Theorie) des gewünschten Produkts **M** erhalten, die ohne weitere Aufreinigung in der nächsten Reaktion eingesetzt wurden.

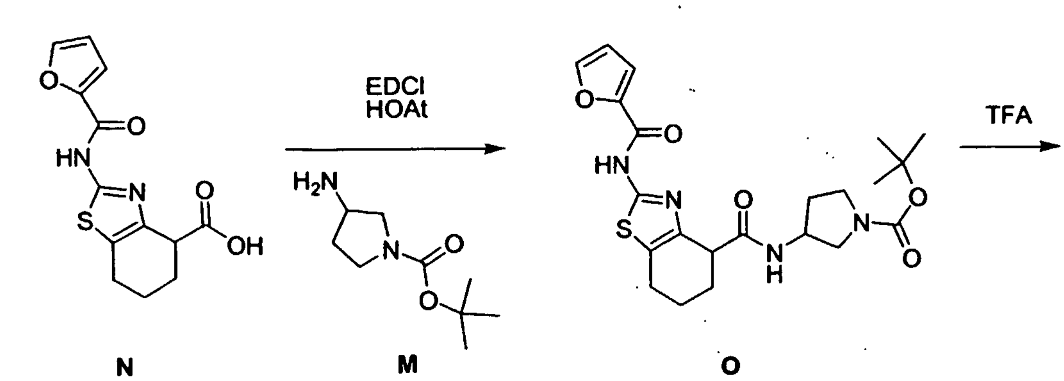


**N** **M** **O**

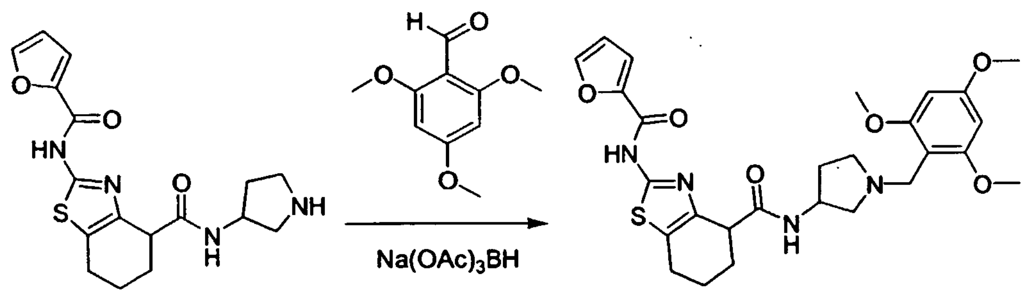


**P** **Q**

Die Verbindung 2-[(Furan-2-carbonyl)-amino]-4,5,6,7-tetrahydro-benzothiazol-4-carbonsäure **(N)** wurde wie in der Synthese von Beispiel 89, Stufe 4, beschrieben erhalten.

**Stufe 1:**

Zu einer Lösung des Amins **M** (727 mg, 3.90 mmol) in getrocknetem DCM (5 mL) und getrocknetem DMF (5 mL) wurde die Carbonsäure 2-[(Furan-2-carbonyl)-amino]-4,5,6,7-tetrahydro-benzothiazol-4-carbonsäure **(N)** (1.14 g, 3.9 mmol) unter Stickstoff als Inertgas gegeben. Bei 0 °C wurden EDCI (822 mg, 4.29 mmol) und HOAt (53 mg, 0.38 mmol) hinzu gegeben. Die Reaktionsmischung wurde 30 Minuten bei 0 °C und anschließend bei RT über Nacht gerührt. Das Lösungsmittel wurde im Vakuum entfernt. Der Rückstand wurde mehrfach in Toluol aufgenommen und das Lösungsmittel im Vakuum wieder entfernt. Nach säulenchromatographischer Reinigung des Rückstand ($SiO_2$, DCM/MeOH 95:5) wurden 550 mg des gewünschten Produkts **O** erhalten (37 % der Theorie).

**Stufe 2:**

Zu einer Lösung von **O** (311 mg, 0.68 mmol) in DCM (5 mL) wurde unter Stickstoff als Inertgas bei RT langsam Trifluoressigsäure (5.0 mL, 67 mmol) gegeben. Nach einer Stunde wurde das Lösungsmittel im Vakuum entfernt. Der Rückstand wurde dreimal in Toluol aufgenommen und jeweils das Lösungsmittel im Vakuum wieder entfernt. Die so

erhaltene Verbindung 2-[(Furan-2-carbonyl)-amino]-4,5,6,7-tetrahydro-benzothiazol-4-carbonsäure-(1-pyrrolidin-3-yl)-amid **(P)** wurde ohne weitere Aufarbeitung in Stufe 3 eingesetzt.

**Stufe 3:**

Verbindung 2-[(Furan-2-carbonyl)-amino]-4,5,6,7-tetrahydro-benzothiazol-4-carbonsäure-(1-pyrrolidin-3-yl)-amid **(P)** (0.67 mmol) wurde unter einer Stickstoffatmosphäre in DCM (40 mL) gelöst . Es wurden 2,4,6-Trimethoxybenzaldehyd (159 mg, 0.81 mmol) und Natriumtriacetoxyborhydrid (215 mg, 1.01 mmol) hinzu gegeben. Die Reaktionsmischung wurde über Nacht bei RT unter einer Stickstoffatmosphäre gerührt. Nach Filtration, Entfernen des Lösungsmittels im Vakuum und säulenchromatographischer Reinigung ($SiO_2$, DCM/MeOH 95:5) wurden 233 mg (61 % der Theorie) des gewünschten Produkts 2-[(Furan-2-carbonyl)-amino]-4,5,6,7-tetrahydro-benzothiazol-4-carbonsäure-[1-(2,4,6-trimethoxy-benzyl)-pyrrolidin-3-yl]-amid **(Q)** erhalten.

**Beispiel 15:** 2-(2-Phenyl-propionylamino)-4,5,6,7-tetrahydro-benzothiazol-4-carbonsäure [1-(4-phenoxy-benzyl)-pyrrolidin-3-yl]-amid

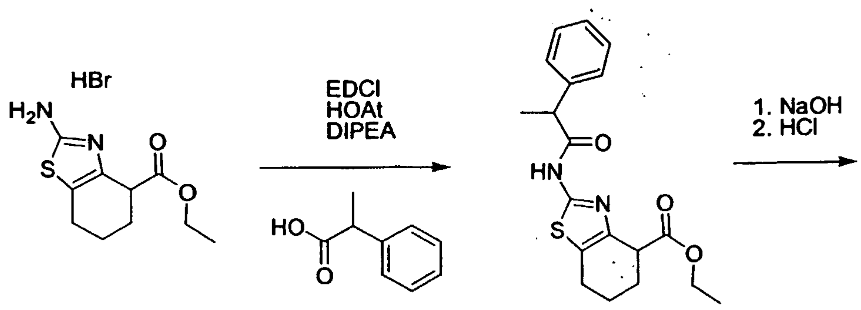


E R

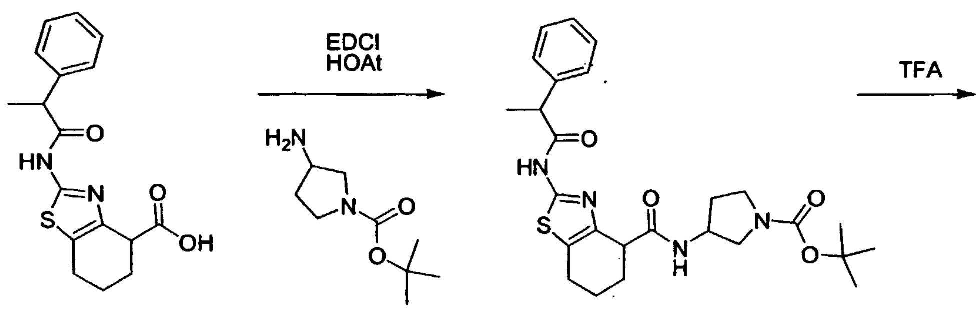


S M T

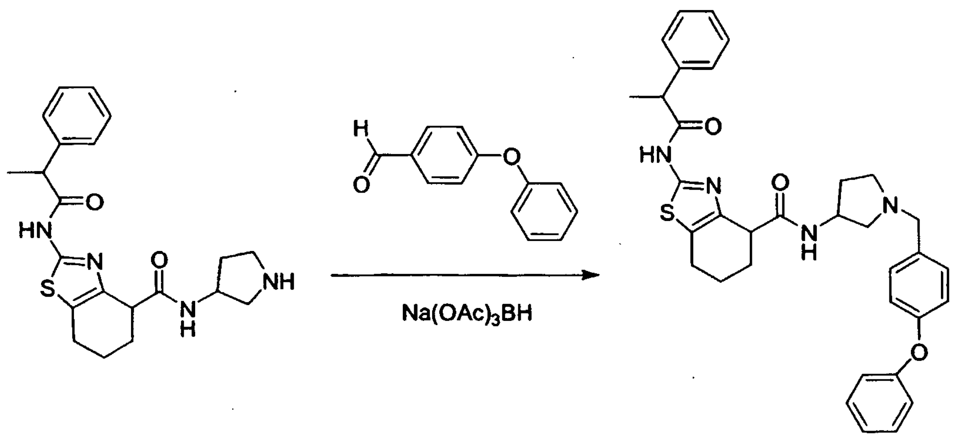


U V

**Stufe 1:**

2-Amino-4,5,6,7-tetrahydro-benzothiazol-4-carbonsäureethylester Hydrobromid **(E)** (5.0 g, 16.3 mmol), 2-Phenylpropionsäure (2.44 g, 16.25 mmol) und DIPEA (2.31 g, 17.87 mmol) wurden mit DCM (100 mL) versetzt. Die Reaktionsmischung wurde auf 0 °C abgekühlt und HOAt (0.22 g, 17.87 mmol) und EDCI (3.43 g, 17.91 mmol) wurden hinzu gegeben. Die resultierende Reaktionsmischung wurde 30 Minuten bei 0 °C und anschließend bei RT über Nacht unter einer Stickstoffatmosphäre gerührt. Eine 0.5 N Salzsäure-Lösung in Wasser (100 mL) wurde hinzu gegeben und die wäßrige Phase wurde mit DCM (100 mL) extrahiert. Nach Trocknen der vereinigten organischen Phasen über $Na_2SO_4$ und Entfernen des Lösungsmittels im Vakuum wurden nach säulenchromatographischer Reinigung des Rückstands ($SiO_2$, EtOAc/Heptan 1:1) 4.95 g des gewünschten Produkts 2-(2-Phenyl-propionylamino)-4,5,6,7-tetrahydro-benzothiazol-4-carbonsäure-ethylester **(R)** (85 % der Theorie) erhalten.

**Stufe 2:**

Zu 2-(2-Phenyl-propionylamino)-4,5,6,7-tetrahydro-benzothiazol-4-carbonsäure-ethylester **(R)** (4.95 g, 13.8

mmol) und NaOH (5.52 g, 0.138 mol) wurde Wasser (20 mL) und MeOH (80 mL) gegeben und bei RT unter Stickstoff als Inertgas bis zur vollständigen Umsetzung gerührt. Nach Entfernen des MeOH wurde 2 N Salzsäure-Lösung in Wasser (250 mL) und DCM (250 mL) hinzu gegeben. Die Phasen wurden getrennt und die wäßrige Phase wurde mehrfach mit DCM (insgesamt 200 mL) extrahiert. Nach Trocknen der vereinigten organischen Phasen über $Na_2SO_4$ und Filtration erhielt man das Produkt 2-(2-Phenyl-propionylamino)-4,5,6,7-tetrahydro-benzothiazol-4-carbonsäure **(S)** mit einer Ausbeute von 4.6 g (entsprechend 100 % der Theorie).

**Stufe 3:**

Zu einer Lösung des Amins **M** (603 mg, 3.2 mmol) in getrocknetem DCM (15 mL) wurde die Carbonsäure 2-(2-Phenyl-propionylamino)-4,5,6,7-tetrahydro-benzothiazol-4-carbonsäure (S) (1173 mg, 3.5 mmol) unter Stickstoff als Inertgas gegeben. Bei 0 °C wurden EDCI (682 mg, 3.56 mmol) und HOAt (66 mg, 0.5 mmol) hinzu gegeben. Die Reaktionsmischung wurde eine Stunde bei 0 °C und anschließend bei RT über Nacht gerührt. Das Lösungsmittel wurde im Vakuum entfernt und nach säulenchromatographischer Reinigung des Rückstand ($SiO_2$, DCM/MeOH 98:2 und Aluminiumoxid, EtOAc/Heptan 1:2) wurden 520 mg des gewünschten Produkts T erhalten (32 % der Theorie).

**Stufe 4:**

Zu einer Lösung von **T** (516 mg, 1.03 mmol) in DCM (15 mL) wurde unter Stickstoff als Inertgas bei RT langsam Trifluoressigsäure (5.0 mL, 67 mmol) gegeben. Nach zwei Stunden wurde das Lösungsmittel im Vakuum entfernt. Der Rückstand wurde dreimal in Toluol aufgenommen und jeweils das Lösungsmittel im Vakuum wieder entfernt. Nach säulenchromatographischer Reinigung des Rückstands ($SiO_2$, DCM/MeOH 95:5 → 9:1, jeweils mit 10 Vol.-% Triethylamin) wurden 268 mg (65 % der Theorie) des gewünschten Produkts 2-(2-Phenyl-propionylamino)-4,5,6,7-tetrahydrobenzothiazol-4-carbonsäure-(1-pyrrolidin-3-yl)-amid **(U)** erhalten.

**Stufe 5:**

Verbindung 2-(2-Phenyl-propionylamino)-4,5;6,7-tetrahydro-benzothiazol-4-carbonsäure-(1-pyrrolidin-3-yl)-amid **(U)** (268 mg, 0.67 mmol) wurde unter einer Stickstoffatmosphäre in DCM (10 mL) gelöst. Es wurden 4-Phenoxybenzaldehyd (157 mg, 0.79 mmol) und Natriumtriacetoxyborhydrid (214 mg, 1.01 mmol) hinzu gegeben. Die Reaktionsmischung wurde über Nacht bei RT unter einer Stickstoffatmosphäre gerührt. Nach Filtration, Entfernen des Lösungsmittels im Vakuum und säulenchromatographischer Reinigung ($SiO_2$, DCM/MeOH 95:5 mit 10 Vol.-% Triethylamin und anschließend $SiO_2$, DCM/MeOH 96:4) wurden 210 mg (54 % der Theorie) des gewünschten Produkts 2-(2-Phenylpropionylamino)-4,5,6,7-tetrahydro-benzothiazol-4-carbonsäure [1-(4-phenoxy-benzyl)-pyrrolidin-3-yl]-amid **(V)** erhalten.

Die folgenden beispielgemäßen substituierten 4,5,6,7-Tetrahydro-benzothiazol-2-yl-amin-Verbindungen wurden entsprechend nach der vorstehend beschriebenen allgemeinen Herstellungsvorschrift erhalten:

[1] 2-Cyclohexyl-N-{4-[4-(2-dimethylamino-ethyl)-piperazin-1-carbonyl]-4,5,6,7-tetrahydro-benzothiazol-2-yl}-acetamid,

[2] N-[4-(4-Methyl-[1,4]diazepan-1-carbonyl)-4,5,6,7-tetrahydro-benzothiazol-2-yl]-2-phenoxy-acetamid,

[3] 2-(2-Phenyl-propionylamino)-4,5,6,7-tetrahydro-benzothiazol-4-carbonsäure (3-dimethylamino-propyl)-amid,

[4] Naphthalin-2-carbonsäure [4-(4-methyl-piperazin-1-carbonyl)-4,5,6,7-tetrahydro-benzothiazol-2-yl]-amid,

[5] N-[4-(2-Pyrrolidin-1-ylmethyl-pyrrolidin-1-carbonyl)-4,5,6,7-tetrahydro-benzothiazol-2-yl]-2-thiophen-2-yl-acetamid

[6] N-{4-[4-(7-Methoxy-benzo[1,3]dioxol-5-ylmethyl)-piperazin-1-carbonyl]-4,5,6,7-tetrahydro-benzothiazol-2-yl}-2-(2-methoxy-phenyl)-acetamid,

[7] N-{4-[4-(4-Methoxy-phenyl)-3-methyl-piperazin-1-carbonyl]-4,5,6,7-tetrahydro-benzothiazol-2-yl}-benzamid,

[8] Furan-2-carbonsäure-{4-[4-(4-acetyl-phenyl)-piperazin-1-carbonyl]-4,5,6,7-tetrahydro-benzothiazol-2-yl}-amid,

[9] 2-[(Furan-2-carbonyl)-amino]-4,5,6,7-tetrahydro-benzothiazol-4-carbonsäure-[1-(2,4,6-trimethoxy-benzyl)-pyrrolidin-3-yl]-amid,

[10] N-[4-(4-Isopropyl-piperazin-1-carbonyl)-4,5,6,7-tetrahydro-benzothiazol-2-yl]-2-phenyl-propionamid,

[11] 3-Furan-2-yl-N-[4-(4-thiophen-3-ylmethyl-piperazin-1-carbonyl)-4,5,6,7-tetrahydro-benzothiazol-2-yl]-acrylamid,

[12] 2-(4-Methoxy-benzoylamino)-4,5,6,7-tetrahydro-benzothiazol-4-carbonsäure-[1-(2-chlor-6-fluor-benzyl)-pyrrolidin-3-yl]-methyl-amid,

[13] Hexansäure-[4-(4-pyridin-4-yl-piperazin-1-carbonyl)-4,5,6,7-tetrahydro-benzothiazol-2-yl]-amid,

[14] 2-(2-Thiophen-2-yl-acetylamino)-4,5,6,7-tetrahydro-benzothiazol-4-carbonsäure- (3-morpholin-4-yl-propyl)-amid,

[15] 2-(2-Phenyl-propionylamino)-4,5,6,7-tetrahydro-benzothiazol-4-carbonsäure[1-(4-phenoxy-benzyl)-pyrrolidin-3-yl]-amid,

[16] 2-[(Furan-3-carbonyl)-amino]-4,5,6,7-tetrahydro-benzothiazol-4-carbonsäure (2-azepan-1-yl-ethyl)-amid,

[17] Furan-3-carbonsäure [4-(4-benzofuran-2-ylmethyl-piperazin-1-carbonyl)-4,5,6,7-tetrahydro-benzothiazol-2-yl]-amid,

[18] 2-Hexanylamino-4,5,6,7-tetrahydro-benzothiazol-4-carbonsäure [3-(2-methylpiperidin-1-yl)-propyl]-amid,

[19] 2-(2-Phenyl-propionylamino)-4,5,6,7-tetrahydro-benzothiazol-4-carbonsäure-(2-dimethylamino-ethyl)-amid,

[20] 2-Ethoxy-N-[4-(4-phenethyl-piperazin-1-carbonyl)-4,5,6,7-tetrahydro-benzothiazol-2-yl]-benzamid,

[21] 2-(4-Fluor-phenoxy)-N-[4-(4-phenyl-piperazin-l-carbonyl)-4,5,6,7-tetrahydro-benzothiazol-2-yl]-acetamid,

[22] 2-(2-Phenyl-propionylamino)-4,5,6,7-tetrahydro-benzothiazol-4-carbonsäure [2-(1-methyl-pyrrolidin-2-yl)-ethyl]-amid,

[23] 2-(3-Furan-2-yl-acryloylamino)-4,5,6,7-tetrahydro-benzothiazol-4-carbonsäure [3-(2-methyl-piperidin-1-yl)-propyl]-amid,

[24] 2-(3,3-Dimethyl-butyrylamino)-4,5,6,7-tetrahydro-benzothiazol-4-carbonsäure-(3-dimethylamino-propyl)-amid,

[25] N-{4-[4-(4-Chlor-benzyl)-piperazin-1-carbonyl]-4,5,6,7-tetrahydro-benzothiazol-2-yl}-4-methoxy-benzamid,

[26] Naphthalin-2-carbonsäure-[4-(2-pyrrolidin-1-ylmethyl-pyrrolidin-1-carbonyl)-4,5,6,7-tetrahydro-benzothiazol-2-yl]-amid,

[27] N-[4-(4-Benzofuran-2-ylmethyl-piperazin-1-carbonyl)-4,5,6,7-tetrahydro-benzothiazol-2-yl]-butyramid,

[28] N-{4-[4-(3-Methoxy-phenyl)-piperazin-1-carbonyl]-4,5,6,7-tetrahydro-benzothiazol-2-yl}-benzamid,

[29] 2-Butyrylamino-4,5,6,7-tetrahydro-benzothiazol-4-carbonsäure-(1-biphenyl-4-ylmethyl-pyrrolidin-3-yl)-methyl-amid,

[30] 2-(2-Phenoxy-acetylamino)-4,5,6,7-tetrahydro-benzothiazol-4-carbonsäure (2-dimethylamino-ethyl)-amid,

[31] 2-Ethoxy-N-[4-(2-pyrrolidin-1-ylmethyl-pyrrolidin-1-carbonyl)-4,5,6,7-tetrahydro-benzothiazol-2-yl]-benzamid,

[32] Naphthalin-2-carbonsäure {4-[4-(2-oxo-2-pyrrolidin-1-yl-ethyl)-piperazin-1-carbonyl]-4,5,6,7-tetrahydro-benzothiazol-2-yl}-amid,

[33] 2-(2-Phenyl-propionylamino)-4,5,6,7-tetrahydro-benzothiazol-4-carbonsäure [1-(2-brom-4,5-dimethoxy-benzyl)-pyrrolidin-3-yl]-amid,

[34] Hexansäure {4-[4-(2,4,6-trimethoxy-benzyl)-piperazin-1-carbonyl]-4,5,6,7-tetrahydro-benzothiazol-2-yl}-amid,

[35] 2-(3,5-Dimethyl-benzoylamino)-4,5,6,7-tetrahydro-benzothiazol-4-carbonsäure [1-(2-tert-butylsulfanyl-benzyl)-pyrrolidin-3-yl]-amid,

[36] 2-(2-Phenyl-propionylamino)-4,5,6,7-tetrahydro-benzothiazol-4-carbonsäure [1-(2-benzyloxy-benzyl)-pyrrolidin-3-yl]-methyl-amid,

[37] 2-Hexa-2,4-dienoylamino-4,5,6,7-tetrahydro-benzothiazol-4-carbonsäure (1-biphenyl-4-ylmethyl-pyrrolidin-3-yl)-methyl-amid,

[38] 2-(3-Phenyl-propionylamino)-4,5,6,7-tetrahydro-benzothiazol-4-carbonsäure (2-diethylamino-ethyl)-amid,

[39] 2-(2-Thiophen-2-yl-acetylamino)-4,5,6,7-tetrahydro-benzothiazol-4-carbonsäure (2-azepan-1-yl-ethyl)-amid,

[40] 2-(2-Thiophen-2-yl-acetylamino)-4,5,6,7-tetrahydro-benzothiazol-4-carbonsäure [1-(2-ethoxy-benzyl)-pyrrolidin-3-yl]-methyl-amid,

[41] 2-Hexanylamino-4,5,6,7-tetrahydro-benzothiazol-4-carbonsäure (1-benzofuran-2-ylmethyl-pyrrolidin-3-yl)-methyl-amid,

[42] 2-(2-Phenoxy-acetylamino)-4,5,6,7-tetrahydro-benzothiazol-4-carbonsäure [3-(2-methyl-piperidin-1-yl)-propyl]-amid,

[43] 2-[(Naphthalin-2-carbonyl)-amino]-4,5,6,7-tetrahydro-benzothiazol-4-carbonsäure (2-pyrrolidin-1-yl-ethyl)-amid,

[44] 2-[2-(3,5-Difluor-phenyl)-acetylamino]-4,5,6,7-tetrahydro-benzothiazol-4-carbonsäure [1-(2-ethoxy-benzyl)-pyrrolidin-3-yl]-methyl-amid,

[45] 2-[(Naphthalin-2-carbonyl)-amino]-4,5,6,7-tetrahydro-benzothiazol-4-carbonsäure 4-dimethylamino-benzylamid,

[46] 2-[2-(4-Fluor-phenoxy)-acetylamino]-4,5,6,7-tetrahydro-benzothiazol-4-carbonsäure [2-(1-methyl-pyrrolidin-2-yl)-ethyl]-amid,

[47] Furan-2-carbonsäure {4-[4-(2-cyano-phenyl)-piperazin-1-carbonyl]-4,5,6,7-tetrahydro-benzothiazol-2-yl}-amid,

[48] 2-(3-Phenyl-propionylamino)-4,5,6,7-tetrahydro-benzothiazol-4-carbonsäure (1-benzofuran-2-ylmethyl-pyrrolidin-3-yl)-methyl-amid,

[49] 2-Hexa-2,4-dienoylamino-4,5,6,7-tetrahydro-benzothiazol-4-carbonsäure [1-(2,6-dichlor-benzyl)-pyrrolidin-3-yl]-methyl-amid,

[50] 2-[(Furan-2-carbonyl)-amino]-4,5,6,7-tetrahydro-benzothiazol-4-carbonsäure [1-(5-brom-2-ethoxy-benzyl)-pyrrolidin-3-yl]-amid,

[51] 2-(2-Phenoxy-acetylamino)-4,5,6,7-tetrahydro-benzothiazol-4-carbonsäure [1-(2-ethoxy-benzyl)-pyrrolidin-3-yl]-methyl-amid,

[52] Hexansäure {4-[4-(2-fluor-phenyl)-piperazin-1-carbonyl]-4,5,6,7-tetrahydro-benzothiazol-2-yl}-amid,

[53] Naphthalin-2-carbonsäure {4-[4-(2-fluor-benzyl)-piperazin-1-carbonyl]-4,5,6,7-tetrahydro-benzothiazol-2-yl}-amid,

[54] 2-(2-Phenoxy-acetylamino)-4,5,6,7-tetrahydro-benzothiazol-4-carbonsäure methyl-[1-(2-trifluormethyl-benzyl)-pyrrolidin-3-yl]-amid,

[55] 2-(3-Phenyl-propionylamino)-4,5,6,7-tetrahydro-benzothiazol-4-carbonsäure (1-biphenyl-4-ylmethyl-pyrrolidin-3-yl)-methyl-amid,

[56] 2-[2-(2-Methoxy-phenyl)-acetylamino]-4,5,6,7-tetrahydro-benzothiazol-4-carbonsäure (1-benzofuran-2-ylmethyl-pyrrolidin-3-yl)-methyl-amid,

[57] 2-(3,5-Difluor-phenyl)-N-[4-(2-pyrrolidin-1-ylmethyl-pyrrolidin-1-carbonyl)-4,5,6,7-tetrahydro-benzothiazol-2-yl]-acetamid,

[58] 2-[(Furan-2-carbonyl)-amino]-4,5,6,7-tetrahydro-benzothiazol-4-carbonsäure 4-dimethylamino-benzylamid,

[59] Hexansäure {4-[4-(3-fluor-4-methoxy-benzyl)-piperazin-1-carbonyl]-4,5,6,7-tetrahydro-benzothiazol-2-yl}-amid,

[60] N-{4-[4-(4-Methoxy-phenyl)-3-methyl-piperazin-1-carbonyl]-4,5,6,7-tetrahydro-benzothiazol-2-yl}-2-phenyl-propionamid,

[61] 2-Hexa-2,4-dienoylamino-4,5,6,7-tetrahydro-benzothiazol-4-carbonsäure [1-(2-chlor-6-fluor-benzyl)-pyrrolidin-3-yl]-methyl-amid,

[62] 2-[2-(3,5-Difluor-phenyl)-acetylamino]-4,5,6,7-tetrahydro-benzothiazol-4-carbonsäure [1-(2,6-dichlor-benzyl)-pyrrolidin-3-yl]-methyl-amid,

[63] 2-[2-(4-Fluor-phenoxy)-acetylamino]-4,5,6,7-tetrahydro-benzothiazol-4-carbonsäure [1-(2-ethoxy-benzyl)-pyrrolidin-3-yl]-methyl-amid,

[64] Furan-3-carbonsäure [4-(4-pyridin-2-yl-piperazin-1-carbonyl)-4,5,6,7-tetrahydro-benzothiazol-2-yl]-amid,

[65] Hexansäure [4-(4-benzofuran-2-ylmethyl-piperazin-1-carbonyl)-4,5,6,7-tetrahydro-benzothiazol-2-yl]-amid,

[66] 2-Phenoxy-N-{4-[4-(1-phenyl-ethyl)-piperazin-1-carbonyl]-4,5,6,7-tetrahydro-benzothiazol-2-yl}-acetamid,

[67] 2-(2-Cyclohexyl-acetylamino)-4,5,6,7-tetrahydro-benzothiazol-4-carbonsäure [1-(2,6-dichlor-benzyl)-pyrrolidin-3-yl]-methyl-amid,

[68] 2-(2-Cyclohexyl-acetylamino)-4,5,6,7-tetrahydro-benzothiazol-4-carbonsäure [3-(methyl-phenyl-amino)-propyl]-amid,

[69] 2-(2-Ethoxy-benzoylamino)-4,5,6,7-tetrahydro-benzothiazol-4-carbonsäure [3-(methyl-phenyl-amino)-propyl]-amid,

[70] 3-Phenyl-N-{4-[4-(5-trifluormethyl-pyridin-2-yl)piperazin-1-carbonyl]-4,5,6,7-tetrahydro-benzothiazol-2-yl}-propionamid,

[71] Furan-3-carbonsäure [4-(4-phenethyl-piperazin-1-carbonyl)-4,5,6,7-tetrahydro-benzothiazol-2-yl]-amid,

[72]3-Phenyl-N-{4-[4-(3-trifluormethyl-phenyl)-piperazin-1-carbonyl]-4,5,6,7-tetrahydro-benzothiazol-2-yl}-propionamid,

[73] 2-(3,3-Dimethyl-butyrylamino)-4,5,6,7-tetrahydro-benzothiazol-4-carbonsäure [1-(2-benzyloxy-benzyl)-pyrrolidin-3-yl]-amid,

[74] 2-(3,5-Difluor-phenyl)-N-{4-[4-(3-trifluor-methyl-phenyl)-piperazin-1-carbonyl]-4,5,6,7-tetrahydro-benzothiazol-2-yl}-acetamid,

[75] 2-Ethoxy-N-{4-[4-(1-phenyl-ethyl)-piperazin-1-carbonyl]-4,5,6,7-tetrahydro-benzothiazol-2-yl}-benzamid,

[76] 3-Furan-2-yl-N-{4-[4-(3-trifluormethyl-phenyl)-piperazin-1-carbonyl]-4,5,6,7-tetrahydro-benzothiazol-2-yl}-acrylamid,

[77] N-{4-[4-(2-Cyano-phenyl)-piperazin-1-carbonyl]-4,5,6,7-tetrahydro-benzothiazol-2-yl}-3-phenyl-propionamid,

[78] 2-(2-Cyclohexyl-acetylamino)-4,5,6,7-tetrahydro-benzothiazol-4-carbonsäure [1-(2-chlor-6-fluor-benzyl)-pyrrolidin-3-yl]-methyl-amid,

[79] N-{4-[4-(2-Methoxy-naphthalin-1-ylmethyl)-piperazin-1-carbonyl]-4,5,6,7-tetrahydro-benzothiazol-2-yl}-3,3-dimethyl-butyramid,

[80] 2-(3,3-Dimethyl-butyrylamino)-4,5,6,7-tetrahydro-benzothiazol-4-carbonsäure (2,5-diethoxy-4-morpholin-4-yl-phenyl)-amid,

[81] N-{4-[4-(2-Chlor-phenyl)-piperazin-1-carbonyl]-4,5,6,7-tetrahydro-benzothiazol-2-yl}-2-(4-fluor-phenyl)-acetamid,

[82] N-[4-(4-Benzyl-piperazin-1-carbonyl)-4,5,6,7-tetrahydro-benzothiazol-2-yl]-3,3-dimethyl-butyramid,

[83] N-[4-(4-Benzhydryl-piperazin-1-carbonyl)-4,5,6,7-tetrahydro-benzothiazol-2-yl]-2-(3,5-difluor-phenyl)-acetamid,

[84] N-[4-(4-Benzhydryl-piperazin-1-carbonyl)-4,5,6,7-tetrahydro-benzothiazol-2-yl]-2-thiophen-2-yl-acetamid,

[85] N-{4-[4-(2-Chlor-phenyl)-piperazin-1-carbonyl]-4,5,6,7-tetrahydro-benzothiazol-2-yl}-2-ethoxy-benzamid,

[86] 3,3-Dimethyl-N-{4-[4-(5-trifluormethyl-pyridin-2-yl)-piperazin-1-carbonyl]-4,5,6,7-tetrahydro-benzothiazol-2-yl}-butyramid,

[87] 3,3-Dimethyl-N-{4-[4-(3-phenyl-allyl)-piperazin-1-carbonyl]-4,5,6,7-tetrahydro-benzothiazol-2-yl}-butyramid,

[88] N-[4-(4-Benzhydryl-piperazin-1-carbonyl)-4,5,6,7-tetrahydro-benzothiazol-2-yl]-2-cyclohexyl-acetamid,

[89] Furan-2-carbonsäure-[4-(4-phenyl-piperazin-1-carbonyl)-4,5,6,7-tetrahydro-benzothiazol-2-yl]-amid.

In der folgenden Tabelle I sind die jeweils zur Herstellung der erfindungsgemäßen Verbindungen gemäß den Beispielen 1-88 eingesetzten Carbonsäurechloride und Amine wiedergegeben.

**Tabelle I:**

| Beispiel: | Carbonsäurechloride | Amine |
|---|---|---|
| 1 | 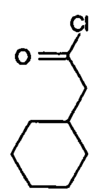 | 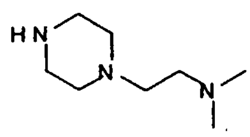 |
| 2 | 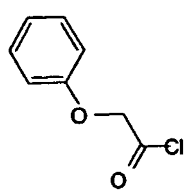 | 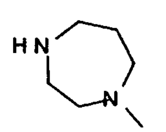 |

(fortgesetzt)

| Beispiel: | Carbonsäurechloride | Amine |
|---|---|---|
| 3 | 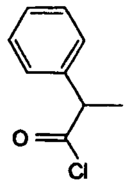 | 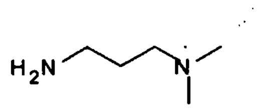 |
| 4 | 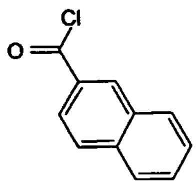 | 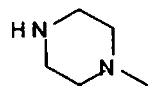 |
| 5 | 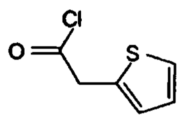 | 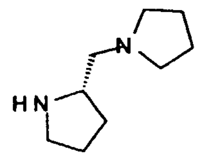 |
| 6 | 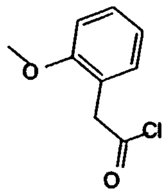 | 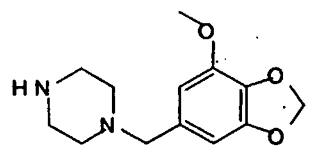 |
| 7 | 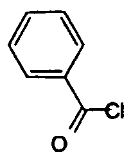 | 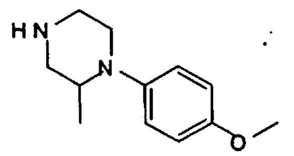 |
| 8 | 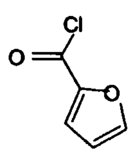 | 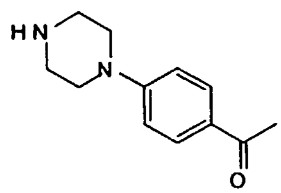 |
| 9 | 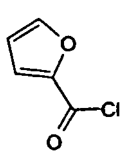 | 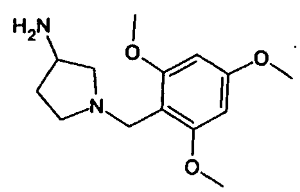 |
| 10 | 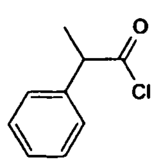 | 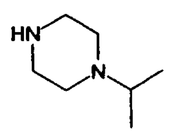 |

(fortgesetzt)

| Beispiel: | Carbonsäurechloride | Amine |
|---|---|---|
| 11 | 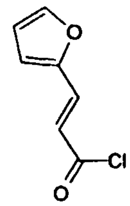 | 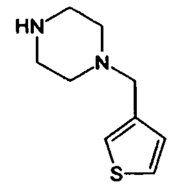 |
| 12 | 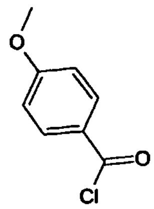 | 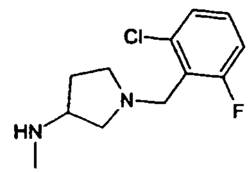 |
| 13 | 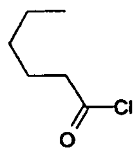 | 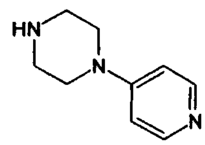 |
| 14 | 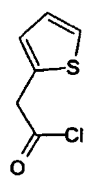 | 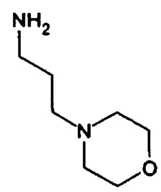 |
| 15 | 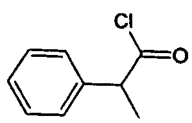 | 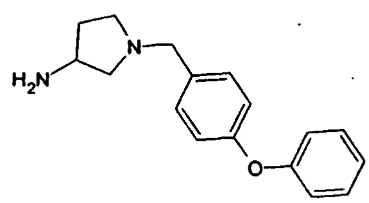 |
| 16 | 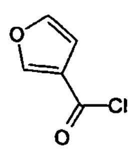 | 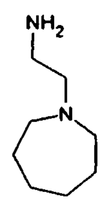 |
| 17 | 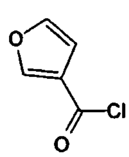 | 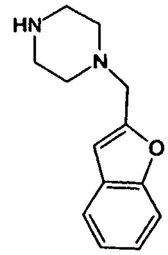 |

(fortgesetzt)

| Beispiel: | Carbonsäurechloride | Amine |
|---|---|---|
| 18 | 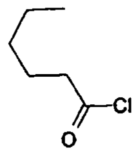 | 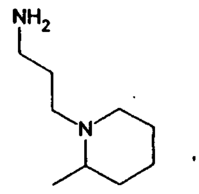 |
| 19 | 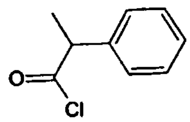 | 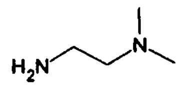 |
| 20 | 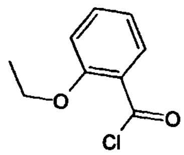 | 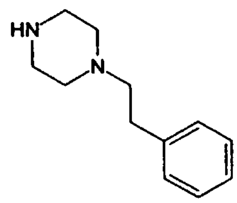 |
| 21 | 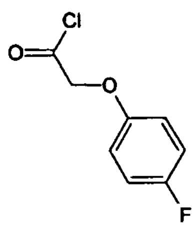 | 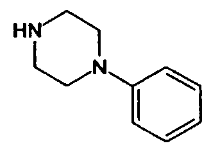 |
| 22 | 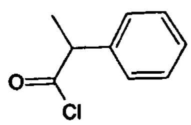 | 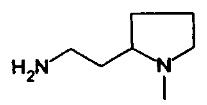 |
| 23 | 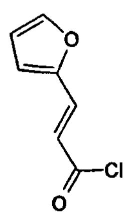 | 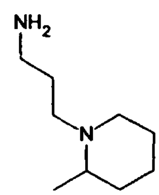 |
| 24 | 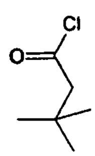 | 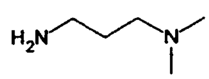 |

(fortgesetzt)

| Beispiel: | Carbonsäurechloride | Amine |
|---|---|---|
| 25 | 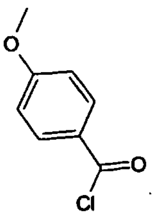 | 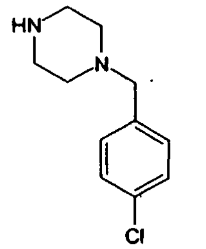 |
| 26 | 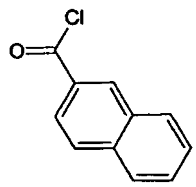 | 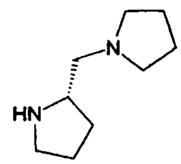 |
| 27 | 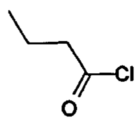 | 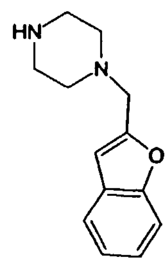 |
| 28 | 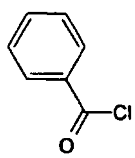 | 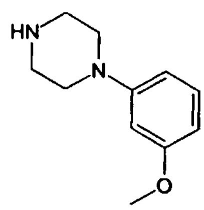 |
| 29 | 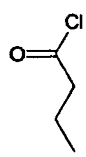 | 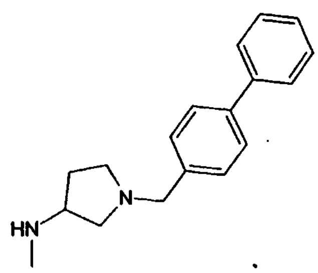 |
| 30 | 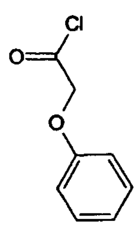 | 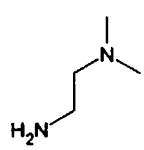 |

(fortgesetzt)

| Beispiel: | Carbonsäurechloride | Amine |
|---|---|---|
| 31 | 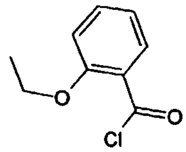 | 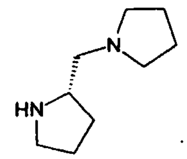 |
| 32 | 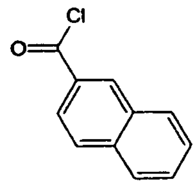 | 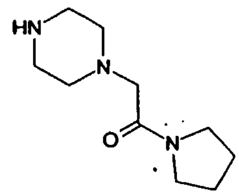 |
| 33 | 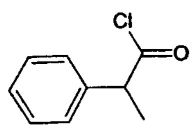 | 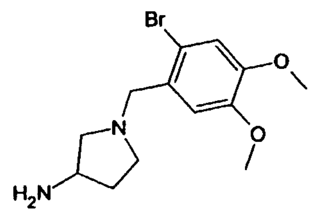 |
| 34 | 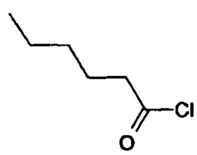 | 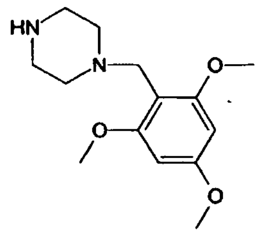 |
| 35 | 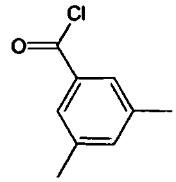 | 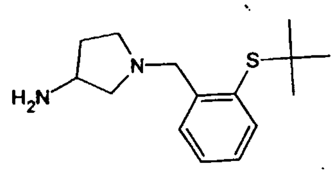 |
| 36 | 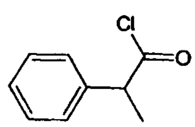 | 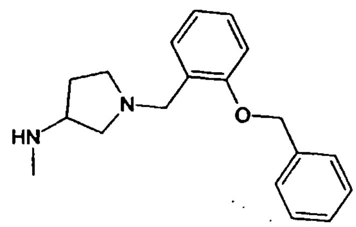 |

(fortgesetzt)

| Beispiel: | Carbonsäurechloride | Amine |
|---|---|---|
| 37 | 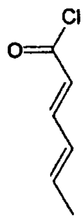 | 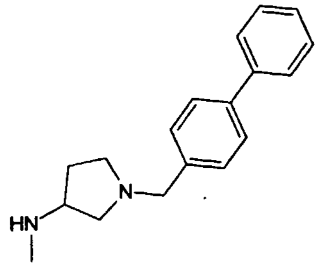 |
| 38 | 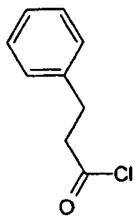 | 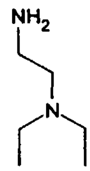 |
| 39 | 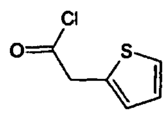 | 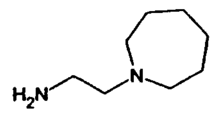 |
| 40 | 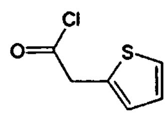 | 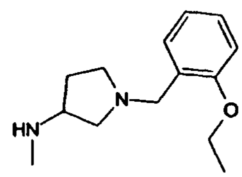 |
| 41 | 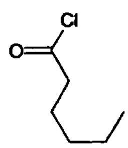 | 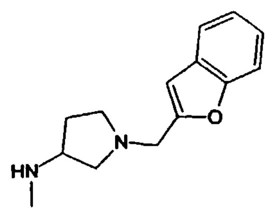 |
| 42 | 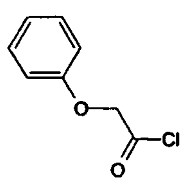 | 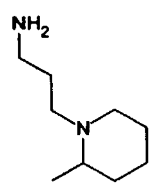 |
| 43 | 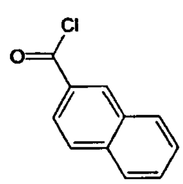 | 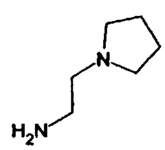 |

(fortgesetzt)

| Beispiel: | Carbonsäurechloride | Amine |
|---|---|---|
| 44 | 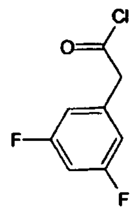 | 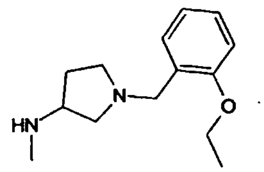 |
| 45 | 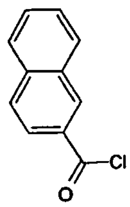 | 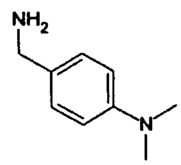 |
| 46 | 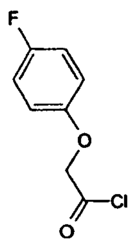 | 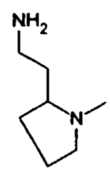 |
| 47 | 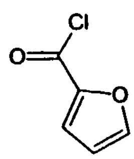 | 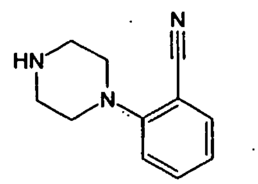 |
| 48 | 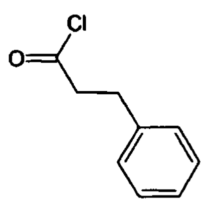 | 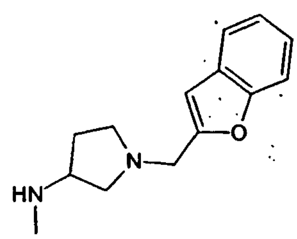 |
| 49 | 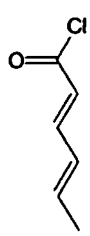 | 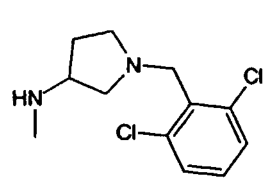 |

(fortgesetzt)

| Beispiel: | Carbonsäurechloride | Amine |
|---|---|---|
| 50 | 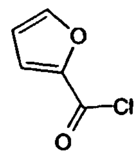 | 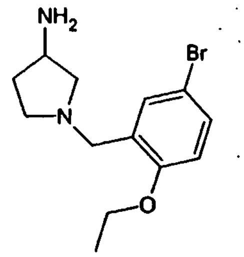 |
| 51 | 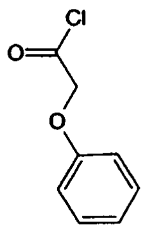 | 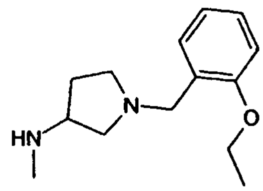 |
| 52 | 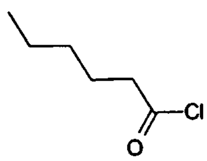 | 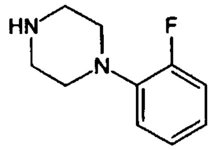 |
| 53 | 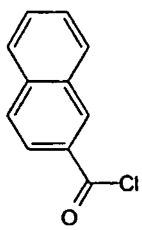 | 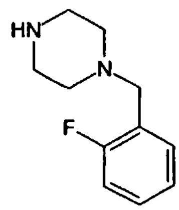 |
| 54 | 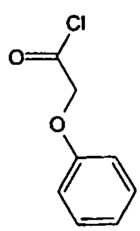 | 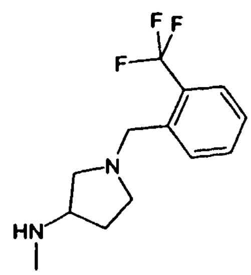 |
| 55 | 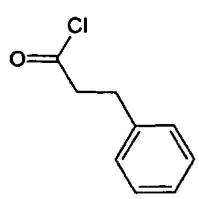 | 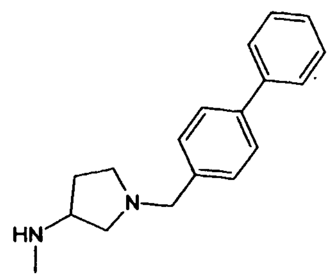 |

(fortgesetzt)

| Beispiel: | Carbonsäurechloride | Amine |
|---|---|---|
| 56 | 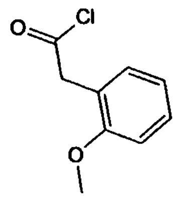 | 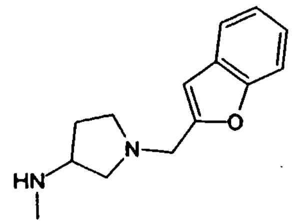 |
| 57 | 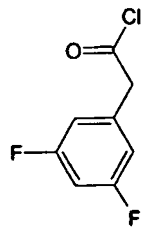 | 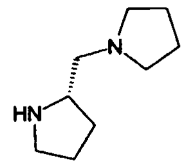 |
| 58 | 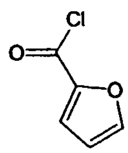 | 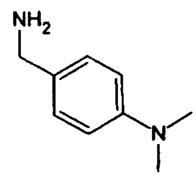 |
| 59 | 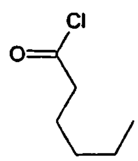 | 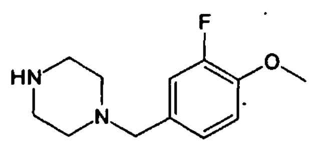 |
| 60 | 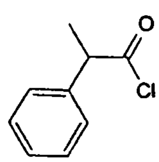 | 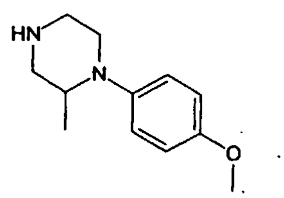 |
| 61 | 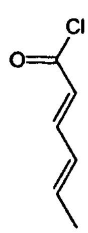 | 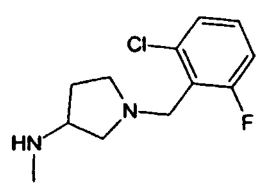 |

(fortgesetzt)

| Beispiel: | Carbonsäurechloride | Amine |
|---|---|---|
| 62 | 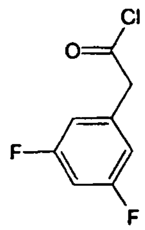 | 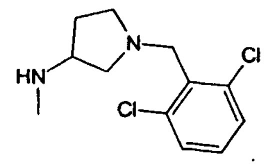 |
| 63 | 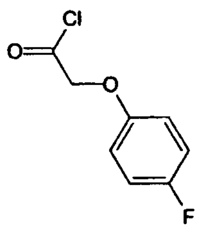 | 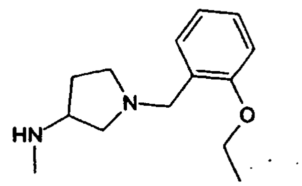 |
| 64 | 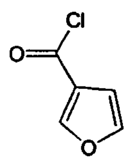 | 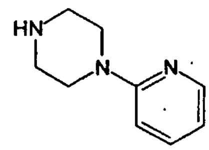 |
| 65 | 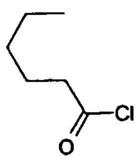 | 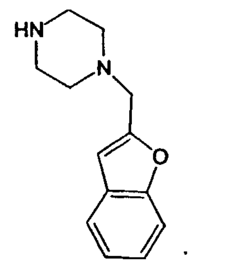 |
| 66 | 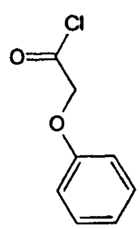 | 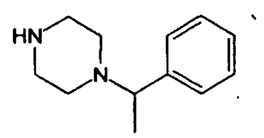 |
| 67 | 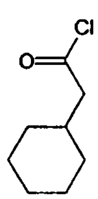 | 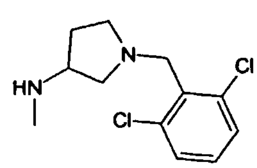 |

(fortgesetzt)

| Beispiel: | Carbonsäurechloride | Amine |
|---|---|---|
| 68 | 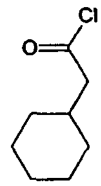 | 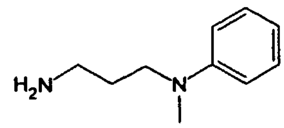 |
| 69 | 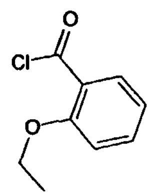 | 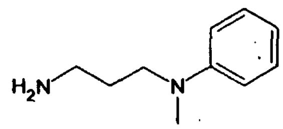 |
| 70 | 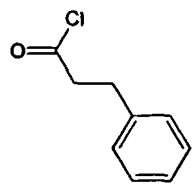 | 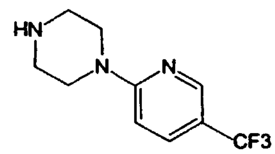 |
| 71 | 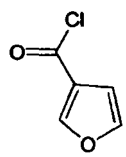 | 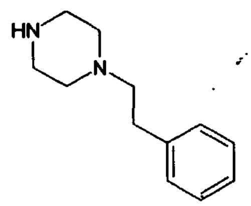 |
| 72 | 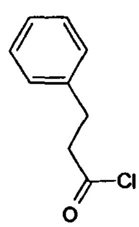 | 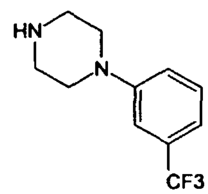 |
| 73 | 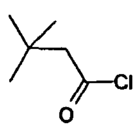 | 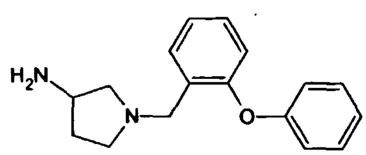 |
| 74 | 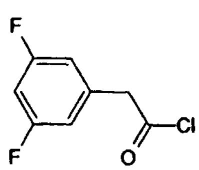 | 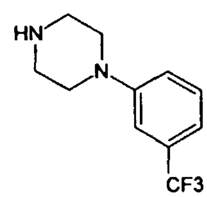 |

(fortgesetzt)

| Beispiel: | Carbonsäurechloride | Amine |
|---|---|---|
| 75 | 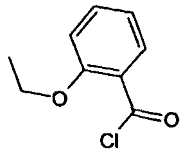 | 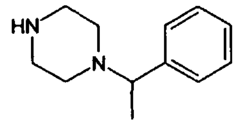 |
| 76 | 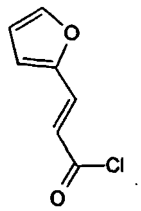 | 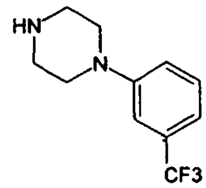 |
| 77 | 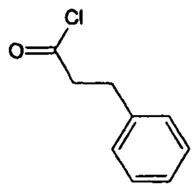 | 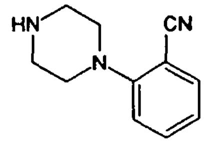 |
| 78 | 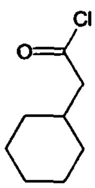 | 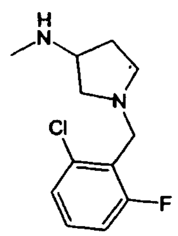 |
| 79 | 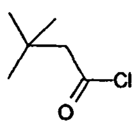 | 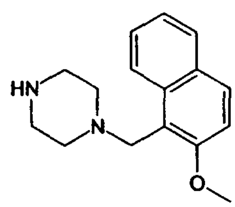 |
| 80 | 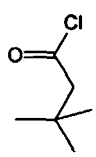 | 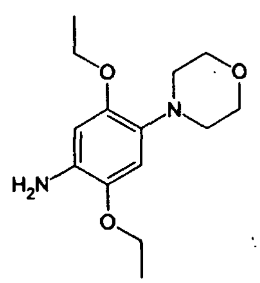 |

(fortgesetzt)

| Beispiel: | Carbonsäurechloride | Amine |
|---|---|---|
| 81 | 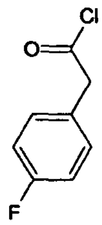 | 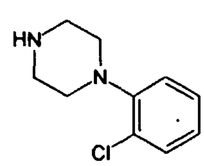 |
| 82 | 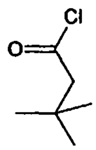 | 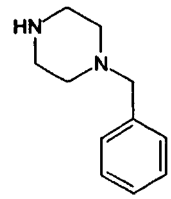 |
| 83 | 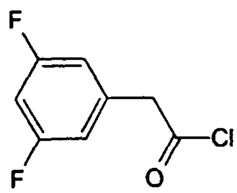 | 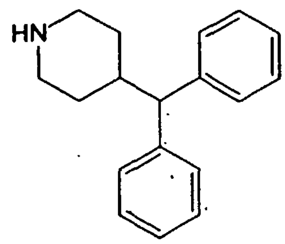 |
| 84 | 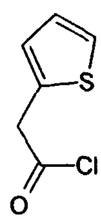 | 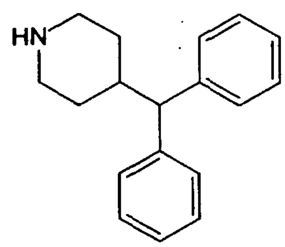 |
| 85 | 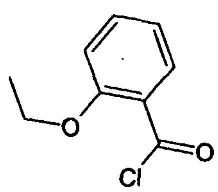 | 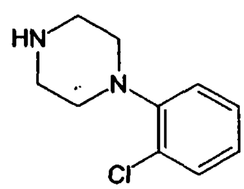 |
| 86 | 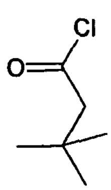 | 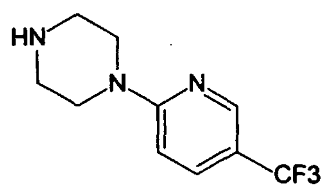 |

(fortgesetzt)

| Beispiel: | Carbonsäurechloride | Amine |
|---|---|---|
| 87 | 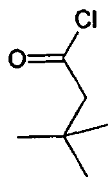 | 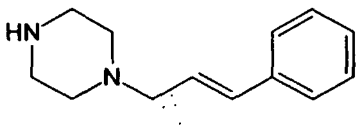 |
| 88 | 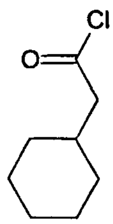 | 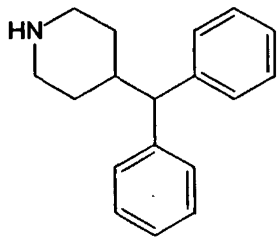 |

**Pharmakologische Daten:**

**I. Analgesieprüfung im Writhing-Test an der Maus**

Die analgetische Wirkung der erfindungsgemäßen substituierten 4,5,6,7-Tetrahydro-benzothiazol-2-ylamin-Verbindungen wurde wie vorstehend beschrieben bestimmt. In der folgenden Tabelle II sind die entsprechenden Daten für die erfindungsgemäße Verbindung gemäß Beispiel 89 wiedergegeben:

**Tabelle II:**

| Verbindung gemäß Beispiel | %Hemmung der Writhingreaktion bei 10 mg/kg intravenös |
|---|---|
| 89 | 41 |

Die erfindungsgemäßen Verbindungen zeigen eine gute bis sehr gute analgetische Wirksamkeit.

**II. Bestimmung der Noradrenalin- und Serotonin-Wiederaufnahmehemmung (NA- und 5HT-Uptake-Hemmung)**

Die Noradrenalin-Uptake-Hemmung und die 5-HT-Uptake-Hemmung durch die erfindungsgemäßen substituierten 4,5,6,7-Tetrahydro-benzothiazol-2-ylamin-Verbindungen der allgemeinen Formel I wurde wie vorstehend beschrieben bestimmt. Die erfindungsgemäßen Verbindungen zeigen eine gute bis sehr gute Hemmung der NA- bzw. 5-HT-Wiederaufnahme.

Die Werte einiger erfindungsgemäßer Verbindungen sind in der nachfolgenden Tabelle III wiedergegeben:

**Tabelle III:**

| Verbindung gemäß: | 5-HT-Uptake [10 μM], %Hemmung | NA-Uptake [10μM], % Hemmung |
|---|---|---|
| Beispiel 1 | 65 | |
| Beispiel 2 | 58 | |
| Beispiel 3 | 61 | 65 |
| Beispiel 4 | 48 | |
| Beispiel 5 | 41 | |
| Beispiel 6 | 51 | |
| Beispiel 7 | 51 | 48 |

(fortgesetzt)

| Verbindung gemäß: | 5-HT-Uptake [10 µM], %Hemmung | NA-Uptake [10µM], % Hemmung |
|---|---|---|
| Beispiel 8 | 89 | 78 |
| Beispiel 9 | 53 | 98 |
| Beispiel 10 | 42 | 64 |
| Beispiel 11 | | 57 |
| Beispiel 12 | 55 | 48 |
| Beispiel 14 | 50 | 43 |
| Beispiel 15 | | 91 |
| Beispiel 16 | | 88 |
| Beispiel 17 | 72 | 81 |
| Beispiel 18 | 57 | 43 |
| Beispiel 19 | | 71 |
| Beispiel 20 | 64 | 77 |
| Beispiel 21 | 47 | 55 |
| Beispiel 22 | 44 | 60 |
| Beispiel 23 | 57 | |
| Beispiel 24 | 89 | |
| Beispiel 25 | 53 | |
| Beispiel 26 | 80 | |
| Beispiel 27 | 61 | 47 |
| Beispiel 28 | 48 | 60 |
| Beispiel 29 | 55 | 73 |
| Beispiel 30 | 64 | |
| Beispiel 31 | 59 | |
| Beispiel32 | 51 | |
| Beispiel 33 | 63 | 79 |
| Beispiel 34 | 63 | 50 |
| Beispiel 35 | 41 | |
| Beispiel 36 | | 62 |
| Beispiel 37 | 44 | |
| Beispiel 38 | | 42 |
| Beispiel 39 | 49 | 50 |
| Beispiel 40 | 48 | 47 |
| Beispiel 41 | 77 | 76 |
| Beispiel 42 | 42 | |
| Beispiel 43 | 63 | |
| Beispiel 44 | 41 | |
| Beispiel 45 | 42 | |
| Beispiel 46 | 53 | |

(fortgesetzt)

| Verbindung gemäß: | 5-HT-Uptake [10 μM], %Hemmung | NA-Uptake [10μM], % Hemmung |
|---|---|---|
| Beispiel 47 | | 47 |
| Beispiel 48 | 51 | 67 |
| Beispiel 49 | 51 | 44 |
| Beispiel 50 | 72 | 93 |
| Beispiel 51 | 59 | |
| Beispiel 52 | | 72 |
| Beispiel 53 | 42 | |
| Beispiel 54 | | 44 |
| Beispiel 55 | 54 | 44 |
| Beispiel 56 | | 49 |
| Beispiel 57 | 72 | |
| Beispiel 58 | | 72 |
| Beispiel 59 | 46 | |
| Beispiel 60 | | 41 |
| Beispiel 61 | 46 | 45 |
| Beispiel 62 | 53 | |
| Beispiel 64 | 48 | 53 |
| Beispiel 65 | 44 | |
| Beispiel 66 | | 48 |
| Beispiel 67 | 97 | 42 |
| Beispiel 68 | 84 | |
| Beispiel 69 | 80 | |
| Beispiel 70 | 44 | |
| Beispiel 71 | 66 | 90 |
| Beispiel 72 | 84 | 51 |
| Beispiel 73 | 71 | 75 |
| Beispiel 74 | 82 | |
| Beispiel 75 | 59 | 51 |
| Beispiel 76 | 62 | |
| Beispiel 77 | | 44 |
| Beispiel 78 | 54 | 41 |
| Beispiel 79 | | 71 |
| Beispiel 80 | 52 | |
| Beispiel 81 | 50 | 80 |
| Beispiel 82 | 41 | 64 |
| Beispiel 83 | 44 | |
| Beispiel 84 | 53 | |
| Beispiel 85 | 48 | |

(fortgesetzt)

| Verbindung gemäß: | 5-HT-Uptake [10 μM], %Hemmung | NA-Uptake [10μM], % Hemmung |
|---|---|---|
| Beispiel 86 | 54 | |
| Beispiel 87 | 56 | 46 |
| Beispiel 88 | 41 | |
| Beispiel 89 | | 77 |

**Patentansprüche**

**1.** Substituierte 4,5,6,7-Tetrahydro-benzothiazol-2-ylamin-Verbindungen der allgemeinen Formel I,

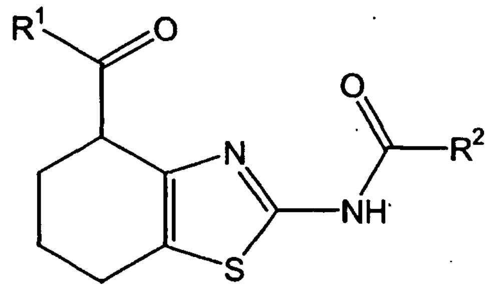

I

worin

$R^1$ für eine Gruppe $-NR^3R^4$ oder für eine Gruppe $-NR^5R^6$ steht,

$R^2$ für einen linearen oder verzweigten, gesättigten oder ungesättigten, ggf. wenigstens einfach substituierten aliphatischen Rest steht, für einen gesättigten oder ungesättigten, ggf. wenigstens einfach substituierten, ggf. wenigstens ein Heteroatom als Ringglied aufweisenden cycloaliphatischen Rest steht, der über eine ggf. wenigstens einfach substituierte, ggf. wenigstens ein Heteroatom als Kettenglied aufweisende Alkylen-, Alkenylen- oder Alkinylen-Gruppe gebunden sein kann, oder für einen ggf. wenigstens einfach substituierten Aryl- oder Heteroaryl-Rest steht, der über eine ggf. wenigstens einfach substituierte, ggf. wenigstens ein Heteroatom als Kettenglied aufweisende Alkylen-, Alkenylen- oder Alkinylen-Gruppe gebunden sein kann,

$R^3$ für einen Wasserstoff-Rest steht, für einen linearen oder verzweigten, gesättigten oder ungesättigten, ggf. wenigstens einfach substituierten aliphatischen Rest steht, für einen gesättigten oder ungesättigten, ggf. wenigstens einfach substituierten, ggf. wenigstens ein Heteroatom als Ringglied aufweisenden cycloaliphatischen Rest steht, der über eine ggf. wenigstens einfach substituierte, ggf. wenigstens ein Heteroatom als Kettenglied aufweisende Alkylen-, Alkenylen- oder Alkinylen-Gruppe gebunden sein kann, oder für einen ggf. wenigstens einfach substituierten Aryl- oder Heteroaryl-Rest steht, der über eine ggf. wenigstens einfach substituierte, ggf. wenigstens ein Heteroatom als Kettenglied aufweisende Alkylen-, Alkenylen- oder Alkinylen-Gruppe gebunden sein kann,

$R^4$ für einen Wasserstoff-Rest steht, für einen linearen oder verzweigten, gesättigten oder ungesättigten, ggf. wenigstens einfach substituierten aliphatischen Rest steht, für einen gesättigten oder ungesättigten, ggf. wenigstens einfach substituierten, ggf. wenigstens ein Heteroatom als Ringglied aufweisenden cycloaliphatischen Rest steht, der über eine ggf. wenigstens einfach substituierte, ggf. wenigstens ein Heteroatom als Kettenglied aufweisende Alkylen-, Alkenylen- oder Alkinylen-Gruppe gebunden sein kann, oder für einen ggf. wenigstens einfach substituierten Aryl- oder Heteroaryl-Rest steht, der über eine ggf. wenigstens einfach substituierte, ggf. wenigstens ein Heteroatom als Kettenglied aufweisende Alkylen-, Alkenylen- oder Alkinylen-Gruppe gebunden sein kann,

$R^5$ und $R^6$ gemeinsam mit dem sie verbrückenden Stickstoffatom als Ringglied einen gesättigten, ungesättigten oder aromatischen, ggf. wenigstens einfach substituierten, ggf. wenigstens ein weiteres Heteroatom als Ringglied aufweisenden heterocyclischen Rest bilden,

ggf. in Form ihrer reinen Stereoisomeren, insbesondere Enantiomeren oder Diastereomeren, ihrer Racemate oder in Form von Mischungen der Stereoisomeren, insbesondere der Enantiomeren und/oder Diastereomeren, in einem beliebigen Mischungsverhältnis, oder jeweils ggf. in Form entsprechender Salze oder jeweils ggf. in Form entsprechender Solvate.

2. Verbindungen gemäß Anspruch 1, **dadurch gekennzeichnet, daß** $R^2$ für einen linearen oder verzweigten, gesättigten oder ungesättigten, ggf. wenigstens einfach substituierten aliphatischen $C_1$-$C_{10}$-Rest steht, für einen gesättigten oder ungesättigten, ggf. wenigstens einfach substituierten, ggf. wenigstens ein Heteroatom als Ringglied aufweisenden drei- bis siebengliedrigen, cycloaliphatischen Rest steht, der über eine ggf. wenigstens einfach substituierte, ggf. wenigstens ein Heteroatom als Kettenglied aufweisende $C_1$-$C_{10}$-Alkylen-, $C_2$-$C_{10}$-Alkenylen- oder $C_2$-$C_{10}$-Alkinylen-Gruppe gebunden sein kann, oder für einen ggf. wenigstens einfach substituierten, fünf- bis zwölfgliedrigen Aryl- oder Heteroaryl-Rest steht, der über eine ggf. wenigstens einfach substituierte, ggf. wenigstens ein Heteroatom als Kettenglied aufweisende $C_1$-$C_{10}$-Alkylen-, $C_2$-$C_{10}$-Alkenylen- oder $C_2$-$C_{10}$-Alkinylen-Gruppe gebunden sein kann,
vorzugsweise für einen linearen oder verzweigten $C_1$-$C_{10}$ Alkyl-Rest steht, für einen linearen oder verzweigten $C_2$-$C_{10}$ Alkenyl-Rest steht, für einen gesättigten oder ungesättigten, ggf. wenigstens einfach substituierten, ggf. wenigstens ein Heteroatom als Ringglied aufweisenden fünf- oder sechs-gliedrigen cycloaliphatischen Rest steht, der über eine ggf. wenigstens einfach substituierte, ggf. wenigstens ein Heteroatom als Kettenglied aufweisende $C_1$-$C_5$-Alkylen- oder $C_2$-$C_5$-Alkenylen-Gruppe gebunden sein kann, oder für einen Phenyl-, 1-Naphthyl-, 2-Naphthyl, 2-Furanyl-, 3-Furanyl-, 2-Thiophenyl- oder 3-Thiophenyl-Rest steht, der jeweils wenigstens einfach substituiert und/oder über eine ggf. wenigstens einfach substituierte, ggf. wenigstens ein Heteroatom als Kettenglied aufweisende $C_1$-$C_5$-Alkylen- oder $C_2$-$C_5$-Alkenylen-Gruppe gebunden sein kann,
besonders bevorzugt für einen linearen oder verzweigten $C_1$-$C_5$ Alkyl-Rest steht, einen linearen oder verzweigten $C_2$-$C_5$ Alkenyl-Rest steht, für einen gesättigten oder ungesättigten, ggf. wenigstens einfach substituierten, ggf. wenigstens ein Heteroatom als Ringglied aufweisenden sechs-gliedrigen cycloaliphatischen Rest steht, der über eine $C_1$-$C_3$-Alkylen-Gruppe gebunden sein kann, oder für einen Phenyl-, 1-Naphthyl-, 2-Naphthyl, 2-Furanyl-, 3-Furanyl-, 2-Thiophenyl- oder 3-Thiophenyl-Rest steht, der jeweils wenigstens einfach substituiert und/oder über eine ggf. wenigstens einfach substituierte, ggf. wenigstens ein Sauerstoffatom als Kettenglied aufweisende $C_1$-$C_5$-Alkylen- oder $C_2$-$C_5$-Alkenylen-Gruppe gebunden sein kann.

3. Verbindungen gemäß Anspruch 1 oder 2, **dadurch gekennzeichnet, daß** der Rest $R^3$ für einen Wasserstoff-Rest steht, für einen linearen oder verzweigten, gesättigten oder ungesättigten, ggf. wenigstens einfach substituierten aliphatischen $C_1$-$C_{10}$-Rest steht, für einen gesättigten oder ungesättigten, ggf. wenigstens einfach substituierten, ggf. wenigstens ein Heteroatom als Ringglied aufweisenden drei- bis siebengliedrigen cycloaliphatischen Rest steht, der über eine ggf. wenigstens einfach substituierte, ggf. wenigstens ein Heteroatom als Kettenglied aufweisende $C_1$-$C_{10}$-Alkylen-, $C_2$-$C_{10}$-Alkenylen- oder $C_2$-$C_{10}$-Alkinylen-Gruppe gebunden sein kann, oder für einen ggf. wenigstens einfach substituierten fünf- bis zwölfgliedrigen Aryl- oder Heteroaryl-Rest steht, der über eine ggf. wenigstens einfach substituierte, ggf. wenigstens ein Heteroatom als Kettenglied aufweisende $C_1$-$C_{10}$-Alkylen-, $C_2$-$C_{10}$-Alkenylen- oder $C_2$-$C_{10}$-Alkinylen-Gruppe gebunden sein kann,
vorzugsweise für einen Wasserstoff-Rest steht, für einen linearen oder verzweigten, ggf. wenigstens einfach substituierten $C_1$-$C_{10}$-Alkyl-Rest steht, für einen gesättigten oder ungesättigten, ggf. wenigstens einfach substituierten, ggf. wenigstens ein Heteroatom als Ringglied aufweisenden fünf-, sechs- oder siebengliedrigen cycloaliphatischen Rest steht, der über eine ggf. wenigstens einfach substituierte, ggf. wenigstens ein Heteroatom als Kettenglied aufweisende $C_1$-$C_{10}$-Alkylen-, $C_2$-$C_{10}$-Alkenylen- oder $C_2$-$C_{10}$-Alkinylen-Gruppe gebunden sein kann, oder für einen ggf. wenigstens einfach substituierten fünfbis zwölfgliedrigen Aryl- oder Heteroaryl-Rest steht, der über eine ggf. wenigstens einfach substituierte, ggf. wenigstens ein Heteroatom als Kettenglied aufweisende $C_1$-$C_{10}$-Alkylen-, $C_2$-$C_{10}$-Alkenylen- oder $C_2$-$C_{10}$-Alkinylen-Gruppe gebunden sein kann,
besonders bevorzugt für einen Wasserstoff-Rest steht, für einen linearen oder verzweigten, ggf. wenigstens einfach substituierten $C_{1-3}$ Alkyl-Rest steht, für einen über eine $C_{1-3}$-Alkylen-Gruppe gebundenen cycloaliphatischen Rest ausgewählt aus der Gruppe bestehend aus

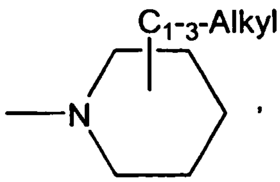, 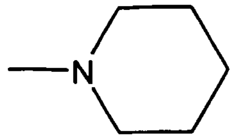, 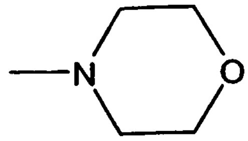,

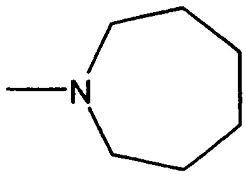, 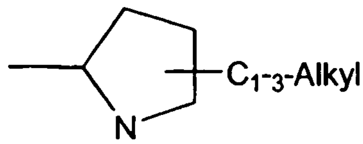, 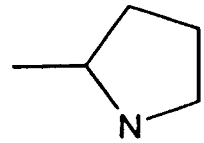,

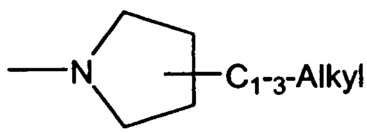 und 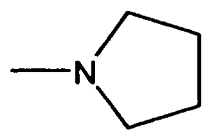,

oder für einen cycloaliphatischen Rest

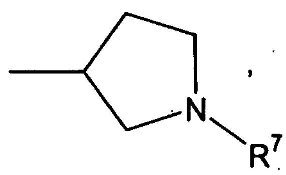,

steht, wobei der Rest $R^7$ für einen über eine Methylen-Gruppe gebundenen Phenyl- oder Benzofuranyl-Rest steht, der einfach oder mehrfach, gleich oder verschieden, mit einem Substituenten ausgewählt aus der Gruppe bestehend aus F, Cl, Br, $CF_3$, $CHF_2$, $CH_2F$, Phenoxy, Benzyloxy, Phenyl, $C_{1-4}$-Alkoxy oder $C_{1-4}$ Alkylthio substituiert sein kann, oder der Rest $R^3$ für einen ggf. über eine Methylen-Gruppe gebundenen Phenyl Rest steht, der einfach oder mehrfach, gleich oder verschieden, mit einem Substituenten ausgewählt aus der Gruppe bestehend aus Di-($C_{1-3}$)-Alkylamino. $C_{1-3}$-Methoxy und Morpholinyl substituiert sein kann.

**4.** Verbindungen gemäß einem der Ansprüche 1 bis 3, **dadurch gekennzeichnet, daß** der Rest $R^4$ für einen Wasserstoff-Rest steht, für einen linearen oder verzweigten, gesättigten oder ungesättigten, ggf. wenigstens einfach substituierten aliphatischen $C_1$-$C_{10}$-Rest steht, für einen gesättigten oder ungesättigten, ggf. wenigstens einfach substituierten, ggf. wenigstens ein Heteroatom als Ringglied aufweisenden drei- bis siebengliedrigen cycloaliphatischen Rest steht, der über eine ggf. wenigstens einfach substituierte, ggf. wenigstens ein Heteroatom als Kettenglied aufweisende $C_1$-$C_{10}$-Alkylen-, $C_2$-$C_{10}$-Alkenylen- oder $C_2$-$C_{10}$-Alkinylen-Gruppe gebunden sein kann, oder für einen ggf. wenigstens einfach substituierten fünf- bis zwölfgliedrigen Aryl- oder Heteroaryl-Rest steht, der über eine ggf. wenigstens einfach substituierte, ggf. wenigstens ein Heteroatom als Kettenglied aufweisende $C_1$-$C_{10}$-Alkylen-, $C_2$-$C_{10}$-Alkenylen- oder $C_2$-$C_{10}$-Alkinylen-Gruppe gebunden sein kann,
vorzugsweise für einen Wasserstoff-Rest steht, für einen linearen oder verzweigten, ggf. wenigstens einfach sub-

stituierten $C_1$-$C_{10}$-Alkyl-Rest steht, für einen gesättigten oder ungesättigten, ggf. wenigstens einfach substituierten, ggf. wenigstens ein Heteroatom als Ringglied aufweisenden fünf-, sechs- oder siebengliedrigen cycloaliphatischen Rest steht, der über eine ggf. wenigstens einfach substituierte, ggf. wenigstens ein Heteroatom als Kettenglied aufweisende $C_1$-$C_{10}$-Alkylen-, $C_2$-$C_{10}$-Alkenylen- oder $C_2$-$C_{10}$-Alkinylen-Gruppe gebunden sein kann, oder für einen ggf. wenigstens einfach substituierten fünfbis zwölfgliedrigen Aryl- oder Heteroaryl-Rest steht, der über eine ggf. wenigstens einfach substituierte, ggf. wenigstens ein Heteroatom als Kettenglied aufweisende $C_1$-$C_{10}$-Alkylen-, $C_2$-$C_{10}$-Alkenylen- oder $C_2$-$C_{10}$-Alkinylen-Gruppe gebunden sein kann,

besonders bevorzugt für einen linearen oder verzweigten, ggf. wenigstens einfach substituierten $C_{1-3}$ Alkyl-Rest steht, für einen über eine $C_{1-3}$-AlkylenGruppe gebundenen cycloaliphatischen Rest ausgewählt aus der Gruppe bestehend aus

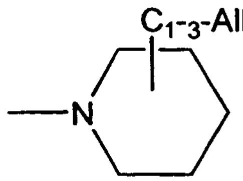 , 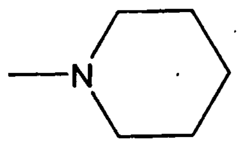 , 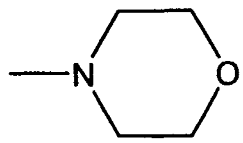 ,

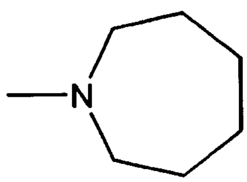 , 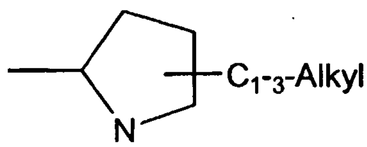 , 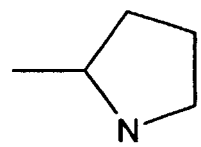 ,

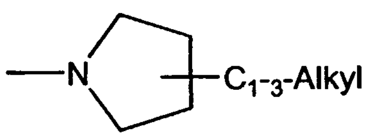 und 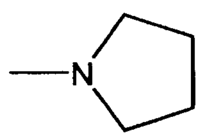 ,

oder für einen cycloaliphatischen Rest

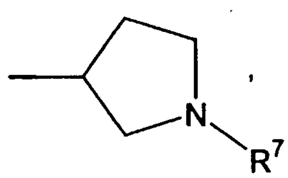 ,

steht, wobei der Rest $R^7$ für einen über eine Methylen-Gruppe gebundenen Phenyl- oder Benzofuranyl-Rest steht, der einfach oder mehrfach, gleich oder verschieden, mit einem Substituenten ausgewählt aus der Gruppe bestehend aus F, Cl, Br, $CF_3$, $CHF_2$, $CH_2F$, Phenoxy, Benzyloxy, Phenyl, $C_{1-4}$-Alkoxy oder $C_{1-4}$ Alkylthio substituiert sein kann, oder der Rest $R^4$ für ggf. über eine Methylen-Gruppe gebundenen Phenyl Rest steht, der einfach oder mehrfach, gleich oder verschieden, mit einem Substituenten ausgewählt aus der Gruppe bestehend aus Di-($C_{1-3}$)-Alkylamino. $C_{1-3}$-Methoxy und Morpholinyl substituiert sein kann.

**5.** Verbindungen gemäß einem der Ansprüche 1 bis 4, **dadurch gekennzeichnet, daß** $R^5$ und $R^6$ gemeinsam mit dem sie verbrückenden Stickstoffatom als Ringglied einen gesättigten, ungesättigten oder aromatischen, ggf. wenigstens einfach substituierten, ggf. wenigstens ein weiteres Heteroatom als Ringglied aufweisenden 5-, 6- oder 7-gliedrigen heterocyclischen Rest bilden,
vorzugsweise gemeinsam mit dem sie verbrückenden Stickstoffatom als Ringglied einen Rest ausgewählt aus der Gruppe

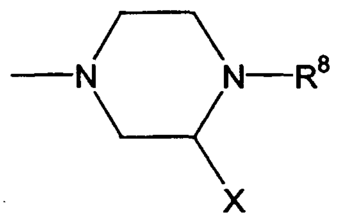
,
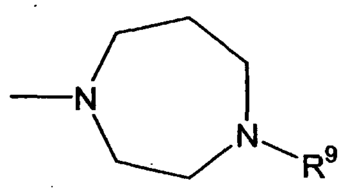

und

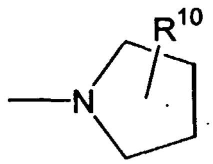

bilden, wobei
X für Wasserstoff oder einen linearen oder ggf. verzweigten $C_{1-3}$-Alkyl-Rest steht, und
$R^8$, $R^9$, $R^{10}$, unabhängig voneinander, jeweils für einen linearen oder verzweigten, gesättigten oder ungesättigten, ggf. wenigstens einfach substituierten aliphatischen $C_{1-6}$ Rest stehen, für einen ggf. über eine ggf. wenigstens einfach substituierte, ggf. wenigstens ein Heteroatom oder ggf. wenigstens eine Carbonyl-(C=O)-Gruppe als Kettenglied aufweisende $C_{1-6}$-Alkylen-, $C_{2-6}$-Alkenylen- oder $C_{2-6}$-Alkinylen-Gruppe gebundenen, gesättigten oder ungesättigten, ggf. wenigstens einfach substituierten, ggf. wenigstens ein Heteroatom als Ringglied enthaltenden 5-, 6- oder 7-gliedrigen cycloaliphatischen Rest stehen, der mit einem ggf. wenigstens einfach substituierten, mono- oder polyzyklischen Ringsystem kondensiert sein kann, oder für einen ggf. über eine ggf. wenigstens einfach substituierte, ggf.
wenigstens ein Heteroatom als Kettenglied aufweisende $C_{1-6}$-Alkylen-, $C_{2-6}$-Alkenylen- oder $C_{2-6}$-Alkinylen--Gruppe gebundenen, ggf. wenigstens einfach substituierten 5- oder 6-gliedrigen Aryl- oder Heteroaryl-Rest stehen, der mit einem ggf. wenigstens einfach substituierten; mono- oder polyzyklischen Ringsystem kondensiert sein kann.

**6.** Verbindungen gemäß Anspruch 5, **dadurch gekennzeichnet, daß**
X für einen Wasserstoff-Rest oder einen Methyl-Rest steht,
$R^8$ für einen ggf. mit einer Di-($C_{1-3}$-Alkyl)-amino-Gruppe substituierten $C_{1-3}$-Alkyl-Rest steht, für einen ggf. wenigstens einfach substituierten Phenyl-Rest, einen ggf. wenigstens einfach substituierten Naphthyl-Rest, einen ggf. wenigstens einfach substituierten Pyridinyl-Rest, einen ggf. wenigstens einfach substituierten Furanyl-Rest, einen ggf. wenigstens einfach substituierten Thiohenyl-Rest, einen ggf. wenigstens einfach substituierten Pyrroldinyl-Rest, einen ggf. wenigstens einfach substituierten Benzo[1,3]-dioxolyl-Rest oder für einen ggf. wenigstens einfach substituierten Benzofuranyl-Rest steht, wobei die zyklischen Reste jeweils über eine ggf. eine Carbonyl-(C=O)-Gruppe als Kettenglied aufweisende $C_{1-3}$-Alkylen- oder $C_{2-3}$-Alkenylen-Gruppe gebunden und/oder einfach oder mehrfach, gleich oder verschieden, mit einem Substituenten ausgewählt aus der Gruppe bestehend aus -(C=O)-$C_{1-3}$-Alkyl, $C_{1-3}$-Alkoxy, F, Cl, Br, -CN, $CF_3$, $CF_2H$ und $CFH_2$ substituiert sein können,
$R^9$ für einen linearen oder ggf. verzweigten $C_{1-3}$-Alkyl-Rest steht,
$R^{10}$ für einen über eine $C_{1-2}$-Alkylen-Gruppe gebundenen Pyrrolidinyl-Rest steht.

**7.** Verbindungen gemäß einem der Ansprüche 1 bis 6, **dadurch gekennzeichnet, daß**
$R^1$ für eine Gruppe $-NR^3R^4$ oder für eine Gruppe $-NR^5R^6$ steht, $R^2$ für einen linearen oder verzweigten $C_{1-5}$-Alkyl-Rest steht, für einen linearen oder verzweigten $C_{2-5}$-Alkenyl-Rest steht, für einen ggf. über eine -($CH_2$)-Gruppe gebundenen Cyclohexyl-Rest steht, für einen gegebenenfalls über eine -($CH_2$)-oder -(CH=CH)-Gruppe gebundenen

1- oder 2-Naphthyl-Rest steht, für einen gegebenenfalls über eine -($CH_2$)- oder -(CH=CH)-Gruppe gebundenen 2- oder 3-Furanyl-Rest steht, für einen gegebenenfalls über eine -($CH_2$)- oder -(CH=CH)-Gruppe gebundenen 2- oder 3-Thienyl-Rest steht, oder für einen gegebenenfalls über eine -($CH_2$)-, -($CH_2$)$_2$-, -C(H)($CH_3$)- oder -($CH_2$)-O-Gruppe gebundenen, unsubstituierten oder wenigstens einfach substituierten Phenyl-Rest steht, wobei dessen Substituenten unabhängig voneinander vorzugsweise ausgewählt werden können aus der Gruppe bestehend aus -F, -Cl, -Br, -$OCH_3$, - $OC_2H_5$, $CH_3$ und $C_2H_5$,

$R^3$ für einen Wasserstoff- oder Methyl-Rest steht;

$R^4$ für einen linearen oder verzweigten, ggf. wenigstens einfach substituierten $C_{1-3}$ Alkyl-Rest, für einen über eine $C_{1-3}$-Alkylen-Gruppe gebundenen cycloaliphatischen Rest ausgewählt aus der Gruppe bestehend aus

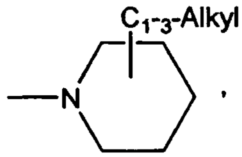 , 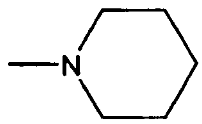 , 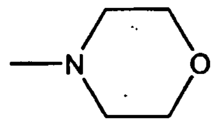 ,

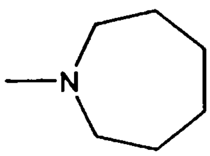 , 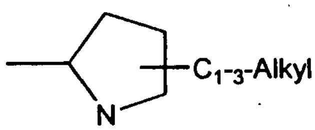 , 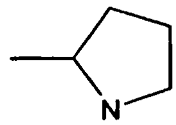 ,

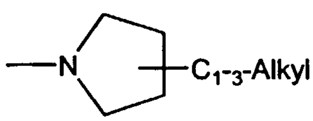 und 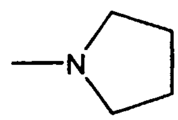 ,

oder für einen cycloaliphatischen Rest

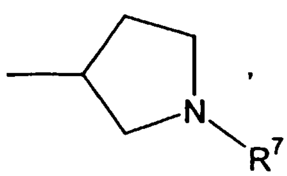 ,

steht, wobei der Rest $R^7$ für einen über eine Methylen-Gruppe gebundenen Phenyl- oder Benzofuranyl-Rest steht, der einfach oder mehrfach, gleich oder verschieden, mit einem Substituenten ausgewählt aus der Gruppe bestehend aus F, Cl, Br, $CF_3$, $CHF_2$, $CH_2F$, Phenoxy, Benzyloxy, Phenyl, $C_{1-4}$-Alkoxy oder $C_{1-4}$ Alkylthio substituiert sein kann, oder der Rest $R^4$ für ggf. über eine Methylen-Gruppe gebundenen Phenyl Rest steht, der einfach oder mehrfach, gleich oder verschieden, mit einem Substituenten ausgewählt aus der Gruppe bestehend aus Di-($C_{1-3}$)-Alkylamino. $C_{1-3}$-Methoxy und Morpholinyl substituiert sein kann,

$R^5$ und $R^6$ gemeinsam mit dem sie verbrückenden Stickstoffatom einen Rest ausgewählt aus der Gruppe

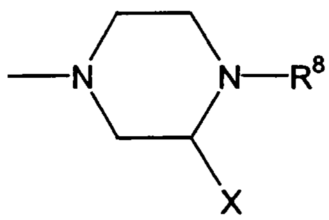,
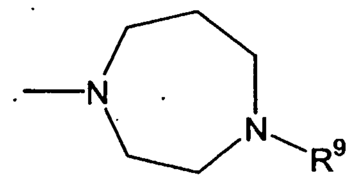

und

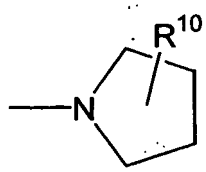

bilden, wobei X für einen Wasserstoff-Rest oder eine Methyl-Gruppe steht,
$R^8$ für einen ggf. mit einer Dimethylamino-Gruppe substituierten, linearen oder verzweigten $C_{1-3}$-Alkyl-Rest steht, einen ggf. wenigstens einfach substituierten Phenyl-Rest, einen ggf. wenigstens einfach substituierten Naphthyl-Rest, einen ggf. wenigstens einfach substituierten Pyridinyl-Rest, einen ggf. wenigstens einfach substituierten Furanyl-Rest, einen ggf. wenigstens einfach substituierten Thiohenyl-Rest, einen ggf. wenigstens einfach substituierten Pyrroldinyl-Rest, einen ggf. wenigstens einfach substituierten Benzo[1,3]-dioxolyl-Rest oder für einen ggf. wenigstens einfach substituierten Benzofuranyl-Rest steht, wobei die zyklischen Reste jeweils über eine ggf. eine Carbonyl-(C=O)-Gruppe als Kettenglied aufweisende $C_{1-3}$-Alkylen- oder $C_{2-3}$-Alkenylen-Gruppe gebunden und/oder einfach oder mehrfach, gleich oder verschieden, mit einem Substituenten ausgewählt aus der Gruppe bestehend aus -(C=O)-$C_{1-3}$-Alkyl, $C_{1-3}$-Alkoxy, F, Cl, Br, -CN, $CF_3$, $CF_2H$ und $CFH_2$ substituiert sein können,
$R^9$ für einen Methyl- oder Ethyl-Rest steht, und
$R^{10}$ für einen über eine -($CH_2$)-Gruppe gebundenen Pyrrolidinyl-Rest steht.

**8.** Verbindungen gemäß einem oder mehreren der Ansprüche 1-7, **dadurch gekennzeichnet, dass**

$R^1$ für eine Gruppe -$NR^3R^4$ oder für eine Gruppe -$NR^5R^6$ steht,
$R^2$ für einen linearen oder verzweigten, gesättigten oder ungesättigten, ggf. substituierten $C_{1-10}$ aliphatischen Rest;
für einen ungesättigten oder gesättigten, ggf. substituierten 3-, 4-, 5-, 6-, 7-, 8- oder 9-gliedrigen cycloaliphatischen Rest, der über eine lineare oder verzweigte, ggf. 1 oder 2 Heteroatom(e) als Kettenglied(er) aufweisende, ggf. substituierte $C_{1-5}$-Alkylen-, $C_{2-5}$-Alkenylen- oder $C_{2-5}$- Alkinylen-Gruppe gebunden sein kann,
oder für einen ggf. substituierten 5- bis 14-gliedrigen Aryl- oder Heteroaryl-Rest, der über eine lineare oder verzweigte, ggf. 1 oder 2 Heteroatom(e) als Kettenglied(er) aufweisende, ggf. substituierte $C_{1-5}$- Alkylen-, $C_{2-5}$-Alkenylen- oder $C_{2-5}$-Alkinylen-Gruppe gebunden sein kann, steht;
$R^3$ für einen Wasserstoff-Rest;
für einen linearen oder verzweigten, gesättigten oder ungesättigten, ggf. substituierten $C_{1-10}$ aliphatischen Rest;
für einen ungesättigten oder gesättigten, ggf. substituierten 3-, 4-, 5-, 6-, 7-, 8- oder 9-gliedrigen cycloaliphatischen Rest, der über eine lineare oder verzweigte, ggf. 1 oder 2 Heteroatom(e) als Kettenglied(er) aufweisende, ggf. substituierte $C_{1-5}$-Alkylen-, $C_{2-5}$-Alkenylen- oder $C_{2-5}$- Alkinylen-Gruppe gebunden sein kann,
oder für einen ggf. substituierten 5- bis 14-gliedrigen Aryl- oder Heteroaryl-Rest, der über eine lineare oder verzweigte, ggf. 1 oder 2 Heteroatom(e) als Kettenglied(er) aufweisende, ggf. substituierte $C_{1-5}$- Alkylen-, $C_{2-5}$-Alkenylen- oder $C_{2-5}$-Alkinylen-Gruppe gebunden sein kann, steht;
$R^4$ für einen Wasserstoff-Rest;
für einen linearen oder verzweigten, gesättigten oder ungesättigten, ggf. substituierten $C_{1-10}$ aliphatischen Rest;
für einen ungesättigten oder gesättigten, ggf. substituierten 3-, 4-, 5-, 6-, 7-, 8- oder 9-gliedrigen cycloaliphatischen Rest, der über eine lineare oder verzweigte, ggf. 1 oder 2 Heteroatom(e) als Kettenglied(er) aufweisende, ggf. substituierte $C_{1-5}$-Alkylen-, $C_{2-5}$-Alkenylen- oder $C_{2-5}$- Alkinylen-Gruppe gebunden sein kann,
oder für einen ggf. substituierten 5- bis 14-gliedrigen Aryl- oder Heteroaryl-Rest, der über eine lineare oder

verzweigte, ggf. 1 oder 2 Heteroatom(e) als Kettenglied(er) aufweisende, ggf. substituierte $C_{1-5}$- Alkylen-, $C_{2-5}$-Alkenylen- oder $C_{2-5}$-Alkinylen-Gruppe gebunden sein kann, steht;
$R^5$ und $R^6$ zusammen mit dem sie verbindenden Stickstoffatom als Ringglied einen gesättigten oder ungesättigten, ggf. substituierten 4-, 5-, 6-, 7-, 8- oder 9-gliedrigen heterocycloaliphatischen Rest bilden, wobei jeweils der heterocycloaliphatische Rest mit einem Rest $R^8$ und ggf. einem Rest X oder einem Rest $R^9$ oder einem Rest $R^{10}$ substituiert sein und/oder jeweils weitere 1, 2 oder 3 Heteroatom(e) unabhängig voneinander ausgewählt aus der Gruppe bestehend aus Sauerstoff, Stickstoff und Schwefel als Ringglied(er) aufweisen kann;
X für einen linearen oder verzweigten, gesättigten oder ungesättigten $C_{1-10}$ aliphatischen Rest steht;
$R^8$, $R^9$ und $R^{10}$, unabhängig voneinander, jeweils
für einen linearen oder verzweigten, gesättigten oder ungesättigten, ggf. substituierten $C_{1-10}$ aliphatischen Rest;
für einen ungesättigten oder gesättigten, ggf. substituierten 3-, 4-, 5-, 6-, 7-, 8- oder 9-gliedrigen cycloaliphatischen Rest, der über eine lineare oder verzweigte, ggf. 1 oder 2 Heteroatom(e) als Kettenglied(er) und/oder ggf. eine Carbonyl-(C=O)-Gruppe als Kettenglied aufweisende, ggf. substituierte $C_{1-5}$-Alkylen-, $C_{2-5}$-Alkenylen- oder $C_{2-5}$- Alkinylen-Gruppe gebunden und/oder mit einem gesättigten, ungesättigten oder aromatischen, ggf. substituierten mono- oder polyzyklischen Ringsystem kondensiert sein kann,
oder für einen ggf. substituierten 5- bis 14-gliedrigen Aryl- oder Heteroaryl-Rest, der über eine lineare oder verzweigte, ggf. 1 oder 2 Heteroatom(e) als Kettenglied(er) und/oder ggf. eine Carbonyl-(C=O)- Gruppe als Kettenglied aufweisende, ggf. substituierte $C_{1-5}$-Alkylen-, $C_{2-5}$-Alkenylen- oder $C_{2-5}$-Alkinylen- Gruppe gebunden und/ oder mit einem gesättigten oder ungesättigten, ggf. substituierten mono- oder polyzyklischen Ringsystem kondensiert sein kann, steht;

wobei
die vorstehend genannten $C_{1-10}$ aliphatischen Reste jeweils mit 1, 2, 3, 4 oder 5 Substituenten unabhängig voneinander ausgewählt aus der Gruppe bestehend aus F, Cl, Br, -CN, $-NO_2$, Hydroxy, $C_{1-6}$-Alkoxy, $-NH_2$, -NH-($C_{1-3}$-Alkyl), -N($C_{1-3}$-Alkyl)($C_{1-3}$-Alkyl), -NH-Phenyl, -N($C_{1-3}$-Alkyl)-Phenyl und - $N(Phenyl)_2$ substituiert sein können;
die vorstehend genannten cycloaliphatischen Reste jeweils mit 1, 2, 3, 4 oder 5 Substituenten unabhängig voneinander ausgewählt aus der Gruppe bestehend aus F, Cl, Br, -CN, $-CF_3$, $-CHF_2$, $-CH_2F$, Hydroxy, -$C_{1-6}$-Alkoxy, - $NH_2$, -NH-($C_{1-3}$-Alkyl), -N($C_{1-3}$-Alkyl)($C_{1-3}$-Alkyl), -NH-Phenyl, -N($C_{1-3}$-Alkyl)-Phenyl, $-N(Phenyl)_2$, -SH, -$C_{1-6}$-Alkylthio, -$C_{1-6}$-Alkyl, -($CH_2$)-Benzo[b]furanyl, Phenoxy, Benzyloxy, Phenyl und Benzyl substituiert sein können, wobei jeweils der zyklische Teil der Reste Phenoxy, Benzyloxy, Phenyl, -($CH_2$)-Benzo[b]furanyl und Benzyl mit 1, 2, 3, 4 oder 5 Substituenten unabhängig voneinander ausgewählt aus der Gruppe bestehend aus F, Cl, Br, -$C_{1-4}$-Alkyl, - $CF_3$, $-CHF_2$, $-CH_2F$, -$C_{1-4}$-Alkoxy, -$C_{1-4}$-Alkylthio, Phenoxy, Phenyl, und Benzyloxy substituiert sein kann
und die vorstehend genannten cycloaliphatischen Reste jeweils 1, 2 oder 3 Heteroatom(e) unabhängig voneinander ausgewählt aus der Gruppe bestehend aus Sauerstoff, Stickstoff und Schwefel aufweisen können;
die vorstehend genannten $C_{1-5}$-Alkylen-, $C_{2-5}$-Alkenylen- oder $C_{2-5}$-Alkinylen-Gruppen jeweils mit 1, 2, 3, 4 oder 5 Substituenten unabhängig voneinander ausgewählt aus der Gruppe bestehend aus F, Cl, Br, -CN, $-NO_2$, Hydroxy, $C_{1-6}$-Alkoxy, $-NH_2$, -NH-($C_{1-3}$-Alkyl), -N($C_{1-3}$-Alkyl)($C_{1-3}$-Alkyl), -NH-Phenyl, -N($C_{1-3}$-Alkyl)-Phenyl, $N(Phenyl)_2$ und Phenyl substituiert sein können und
die vorstehend genannten $C_{1-5}$-Alkylen-, $C_{2-5}$-Alkenylen- oder $C_{2-5}$-Alkinylen-Gruppen jeweils 1 oder 2 Heteroatom (e) unabhängig voneinander ausgewählt aus der Gruppe bestehend aus Sauerstoff, Stickstoff und Schwefel aufweisen können;
die vorstehend genannten Aryl- oder Heteroaryl-Reste jeweils mit 1, 2, 3, 4 oder 5 Substituenten unabhängig voneinander ausgewählt aus der Gruppe bestehend aus F, Cl, Br, -CN, $-CF_3$, $-CHF_2$, $-CH_2F$, Hydroxy, -$C_{1-6}$-Alkoxy, - (C=O)-$CH_3$, -O-$CF_3$, -S-$CF_3$, -SH, -$C_{1-6}$-Alkylthio, -$C_{1-6}$-Alkyl, $-NH_2$, -NH-($C_{1-3}$-Alkyl), -N($C_{1-3}$-Alkyl)($C_{1-3}$-Alkyl), -NH-Phenyl, -N($C_{1-3}$-Alkyl)-Phenyl, $N(Phenyl)_2$, Phenoxy, Benzyloxy, Phenyl, Benzyl und Morpholinyl substituiert sein können, wobei jeweils der zyklische Teil der Reste Phenoxy, Benzyloxy, Phenyl und Benzyl mit 1, 2, 3, 4 oder 5 Substituenten unabhängig voneinander ausgewählt aus der Gruppe bestehend aus F, Cl, Br, Hydroxy, $-CF_3$, $-SF_5$, - CN, $-NO_2$, -$C_{1-6}$-Alkoxy, -O-$CF_3$, -S-$CF_3$, Phenyl und Benzyloxy substituiert sein kann,
und die vorstehend genannten Heteroaryl-Reste jeweils 1, 2, 3, 4 oder 5 Heteroatom(e) unabhängig voneinander ausgewählt aus der Gruppe bestehend aus Sauerstoff, Stickstoff und Schwefel als Ringglied(er) aufweisen können;
und die Ringe der vorstehend genannten mono- oder polyzyklischen Ringsysteme ggf. jeweils mit 1, 2, 3, 4 oder 5 Substituenten unabhängig voneinander ausgewählt aus der Gruppe bestehend aus F, Cl, Br, -CN, $-CF_3$, - $CHF_2$, $-CH_2F$, Hydroxy, -$C_{1-6}$-Alkoxy, -O-$CF_3$, -S-$CF_3$, -SH, -$C_{1-6}$-Alkylthio, -$C_{1-6}$-Alkyl, $-NH_2$, -NH-($C_{1-3}$-Alkyl), -N($C_{1-3}$-Alkyl)($C_{1-3}$-Alkyl), -NH-Phenyl, -N($C_{1-3}$-Alkyl)-Phenyl, $N(Phenyl)_2$, Phenoxy, Benzyloxy, Phenyl, Benzyl und Morpholinyl substituiert sein können, wobei jeweils der zyklische Teil der Reste Phenoxy, Benzyloxy, Phenyl und Benzyl mit 1, 2, 3, 4 oder 5 Substituenten unabhängig voneinander ausgewählt aus der Gruppe bestehend aus F, Cl, Br, Hydroxy, $-CF_3$, $-SF_5$, -CN, $-NO_2$, -$C_{1-6}$-Alkoxy, -O-$CF_3$, -S-$CF_3$, Phenyl und Benzyloxy substituiert sein kann,
und die Ringe der vorstehend genannten mono- oder polyzyklischen Ringsysteme jeweils 5-, 6- oder 7-gliedrig sind

und jeweils 1, 2, 3, 4 oder 5 Heteroatom(e) als Ringglied(er) aufweisen können, die unabhängig voneinander aus der Gruppe bestehend aus Sauerstoff, Stickstoff und Schwefel ausgewählt sind;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

**9.** Verbindungen gemäß einem oder mehreren der Ansprüche 1-8, **dadurch gekennzeichnet, dass** der Rest
$R^2$ für einen Alkyl-Rest ausgewählt aus der Gruppe bestehend aus Methyl, Ethyl, n-Propyl, iso-Propyl, n-Butyl, sec-Butyl, iso-Butyl, tert-Butyl, n-Pentyl, sec-Pentyl, (1,1)-Dimethylpropyl und n-Hexyl steht, wobei der Alkyl-Rest jeweils mit 1, 2, 3, 4 oder 5 Substituenten unabhängig voneinander ausgewählt aus der Gruppe bestehend aus F, Cl, Br, -CN, $-NO_2$, Hydroxy, $C_{1-6}$-Alkoxy, $-NH_2$, -NH-($C_{1-3}$-Alkyl), -N($C_{1-3}$-Alkyl)($C_{1-3}$-Alkyl), -NH-Phenyl, -N($C_{1-3}$-Alkyl)-Phenyl und -N(Phenyl)$_2$ substituiert sein kann;
für einen Alkenyl-Rest ausgewählt aus der Gruppe bestehend aus Vinyl, 1-Propenyl, 2-Propenyl, 1-Butenyl, 2-Butenyl, 3-Butenyl, 1-Pentenyl, 2-Pentenyl und Pent-1,3-dienyl steht, wobei der Alkenyl-Rest jeweils mit 1, 2, 3, 4 oder 5 Substituenten unabhängig voneinander ausgewählt aus der Gruppe bestehend aus F, Cl, Br, -CN, $-NO_2$, Hydroxy, $C_{1-6}$-Alkoxy, $-NH_2$, -NH-($C_{1-3}$-Alkyl), -N($C_{1-3}$-Alkyl)($C_{1-3}$-Alkyl), -NH-Phenyl, -N($C_{1-3}$-Alkyl)-Phenyl und -N (Phenyl)$_2$ substituiert sein kann;
für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl steht,
wobei der (hetero)cycloaliphatische Rest jeweils über eine lineare oder verzweigte, unsubstituierte $C_{1-5}$-Alkylen-Gruppe gebunden und/oder mit 1, 2, 3, 4 oder 5 Substituenten unabhängig voneinander ausgewählt aus der Gruppe bestehend aus F, Cl, Br, -CN, $-CF_3$, $-CHF_2$, $-CH_2F$, Hydroxy, -$C_{1-6}$-Alkoxy, $-NH_2$, - NH-($C_{1-3}$-Alkyl), -N($C_{1-3}$-Alkyl) ($C_{1-3}$-Alkyl), -NH-Phenyl, -N($C_{1-3}$-Alkyl)-Phenyl, - N(Phenyl)$_2$, -SH, -$C_{1-6}$-Alkylthio, -$C_{1-6}$-Alkyl, -($CH_2$)-Benzo[b]furanyl, Phenoxy, Benzyloxy, Phenyl und Benzyl substituiert sein kann, wobei jeweils der zyklische Teil der Reste Phenoxy, Benzyloxy, Phenyl, -($CH_2$)-Benzo[b]furanyl und Benzyl mit 1, 2, 3, 4 oder 5 Substituenten unabhängig voneinander ausgewählt aus der Gruppe bestehend aus F, Cl, Br, -$C_{1-4}$-Alkyl, $-CF_3$, $-CHF_2$, $-CH_2F$, -$C_{1-4}$-Alkoxy, - $C_{1-4}$-Alkylthio, Phenoxy, Phenyl, und Benzyloxy substituiert sein kann;
oder für einen Rest ausgewählt aus der Gruppe bestehend aus Phenyl, Naphthyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl und Isochinolinyl steht, wobei der Rest jeweils über eine lineare oder verzweigte, unsubstituierte, ggf. ein Sauerstoffatom als Kettenglied aufweisende $C_{1-5}$-Alkylen- oder $C_{2-5}$-Alkenylen-Gruppe gebunden und/oder mit 1, 2, 3, 4 oder 5 Substituenten unabhängig voneinander ausgewählt aus der Gruppe bestehend aus F, Cl, Br, -CN, $-CF_3$, $-CHF_2$, $-CH_2F$, Hydroxy, -$C_{1-6}$-Alkoxy, $-O-CF_3$, $-S-CF_3$, - SH, -$C_{1-6}$-Alkylthio, -$C_{1-6}$-Alkyl, $-NH_2$, -NH-($C_{1-3}$-Alkyl), -N($C_{1-3}$-Alkyl) ($C_{1-3}$-Alkyl), - NH-Phenyl, -N($C_{1-3}$-Alkyl)-Phenyl, N(Phenyl)$_2$, Phenoxy, Benzyloxy, Phenyl, Benzyl und Morpholinyl substituiert sein kann, wobei jeweils der zyklische Teil der Reste Phenoxy, Benzyloxy, Phenyl und Benzyl mit 1, 2, 3, 4 oder 5 Substituenten unabhängig voneinander ausgewählt aus der Gruppe bestehend aus F, Cl, Br, Hydroxy, $-CF_3$, $-SF_5$, -CN, $-NO_2$, -$C_{1-6}$-Alkoxy, $-O-CF_3$, $-S-CF_3$, Phenyl und Benzyloxy substituiert sein kann;
bevorzugt $R^2$
für einen Alkyl-Rest ausgewählt aus der Gruppe bestehend aus Methyl, Ethyl, n-Propyl, iso-Propyl, n-Butyl, sec-Butyl, iso-Butyl, tert-Butyl, n-Pentyl, sec-Pentyl, (1,1)-Dimethylpropyl und n-Hexyl steht;
für einen Alkenyl-Rest ausgewählt aus der Gruppe bestehend aus Vinyl, 1-Propenyl, 2-Propenyl, 1-Butenyl, 2-Butenyl, 3-Butenyl, 1-Pentenyl, 2-Pentenyl und Pent-1,3-dienyl steht;
für einen cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl und Cycloheptenyl steht, der über eine $-(CH_2)-$, $-(CH_2)_2-$, $-CH(CH_3)$-oder $-(CH_2)_3$-Gruppe gebunden und/oder mit 1, 2, 3, 4 oder 5 Substituenten unabhängig voneinander ausgewählt aus der Gruppe bestehend aus F, Cl, Br,-CN, $-CF_3$, $-CHF_2$, $-CH_2F$, Hydroxy, $-O-CH_3$, $-O-C_2H_5$, $-O-C_3H_7$, $-NH_2$, $-NH-CH_3$, - $NH-C_2H_5$, $-N-(CH_3)_2$, $-N(C_2H_5)_2$, $-N(CH_3)(C_2H_5)$, -NH-Phenyl, $-N(CH_3)$-Phenyl, - N ($C_2H_5$)-Phenyl, -N(Phenyl)$_2$, -SH, $-S-CH_3$, $-S-C_2H_5$, $-S-C_3H_7$, $-S-C(CH_3)_3$, Methyl, Ethyl, n-Propyl, Isopropyl, n-Butyl, sec-Butyl und tert-Butyl substituiert sein kann;
oder für einen Rest ausgewählt aus der Gruppe bestehend aus Phenyl, 1-Naphthyl, 2-Naphthyl, 2-Furanyl, 3-Furanyl, 2-Thiophenyl oder 3-Thiophenyl steht, der über eine $-(CH_2)-$, $-(CH_2)_2-$, $-(CH_2)-O-$, $-CH(CH_3)-$, $-(CH=CH)-$ oder - $(CH_2)_3$-Gruppe gebunden und/oder mit 1, 2, 3, 4 oder 5 Substituenten unabhängig voneinander ausgewählt aus der Gruppe bestehend aus F, Cl, Br, - CN, $-CF_3$, $-CHF_2$, $-CH_2F$, Hydroxy, $-O-CH_3$, $-O-C_2H_5$, $-O-C_3H_7$, $-O-CF_3$, $-S-CF_3$, - SH, $-S-CH_3$, $-S-C_2H_5$, $-S-C_3H_7$, $-S-C(CH_3)_3$, Methyl, Ethyl, n-Propyl, Isopropyl, n-Butyl, sec-Butyl, tert-Butyl,

$-NH_2$, $-NH-CH_3$, $-NH-C_2H_5$, $-N-(CH_3)_2$, $-N(C_2H_5)_2$, $-N(CH_3)(C_2H_5)$, -NH-Phenyl, $-N(CH_3)$-Phenyl, $-N(C_2H_5)$-Phenyl, $-N(Phenyl)_2$, Phenoxy, Benzyloxy, Phenyl, Benzyl und Morpholinyl substituiert sein kann;
besonders bevorzugt $R^2$
für einen Alkyl-Rest ausgewählt aus der Gruppe bestehend aus n-Propyl, iso-Propyl, n-Butyl, sec-Butyl, iso-Butyl, tert-Butyl, n-Pentyl, sec-Pentyl und (1,1)-Dimethylpropyl steht;
für einen Alkenyl-Rest ausgewählt aus der Gruppe bestehend aus 1-Pentenyl, 2-Pentenyl und Pent-1,3-dienyl steht;
für einen cycloaliphatischen Rest ausgewählt aus der Gruppe bestehend aus Cyclopentyl, Cyclohexyl und Cycloheptyl steht, der über eine $-(CH_2)$-Gruppe gebunden ist;
oder für einen Rest ausgewählt aus der Gruppe bestehend aus Phenyl, 1-Naphthyl, 2-Naphthyl, 2-Furanyl, 3-Furanyl, 2-Thiophenyl oder 3-Thiophenyl steht, der über eine $-(CH_2)-$, $-(CH_2)_2-$, $-(CH_2)-O-$, $-CH(CH_3)-$. -(CH=CH)- oder $-(CH_2)_3$-Gruppe gebunden und/oder mit 1, 2 oder 3 Substituenten unabhängig voneinander ausgewählt aus der Gruppe bestehend aus F, Cl, Br, $-O-CH_3$, $-O-C_2H_5$, Methyl, Ethyl, n-Propyl und Isopropyl substituiert sein kann.

**10.** Verbindungen gemäß einem oder mehreren der Ansprüche 1-9, **dadurch gekennzeichnet, dass** der Rest
$R^3$ für einen Wasserstoff-Rest steht;
für einen Alkyl-Rest ausgewählt aus der Gruppe bestehend aus Methyl, Ethyl, n-Propyl, iso-Propyl, n-Butyl, sec-Butyl, iso-Butyl, tert-Butyl, n-Pentyl, sec-Pentyl, (1,1)-Dimethylpropyl und n-Hexyl steht, wobei der Alkyl-Rest jeweils mit 1, 2, 3, 4 oder 5 Substituenten unabhängig voneinander ausgewählt aus der Gruppe bestehend aus F, Cl, Br, -CN, $-NO_2$, Hydroxy, $C_{1-6}$-Alkoxy, $-NH_2$, $-NH-(C_{1-3}$-Alkyl), $-N(C_{1-3}$-Alkyl)($C_{1-3}$-Alkyl), -NH-Phenyl, $-N(C_{1-3}$-Alkyl)-Phenyl und $-N(Phenyl)_2$ substituiert sein kann;
für einen Alkenyl-Rest ausgewählt aus der Gruppe bestehend aus Vinyl, 1-Propenyl, 2-Propenyl, 1-Butenyl, 2-Butenyl, 3-Butenyl, 1-Pentenyl, 2-Pentenyl und Pent-1,3-dienyl steht, wobei der Alkenyl-Rest jeweils mit 1, 2, 3, 4 oder 5 Substituenten unabhängig voneinander ausgewählt aus der Gruppe bestehend aus F, Cl, Br, -CN, $-NO_2$, Hydroxy, $C_{1-6}$-Alkoxy, $-NH_2$, $-NH-(C_{1-3}$-Alkyl), $-N(C_{1-3}$-Alkyl)($C_{1-3}$-Alkyl), -NH-Phenyl, $-N(C_{1-3}$-Alkyl)-Phenyl und $-N(Phenyl)_2$ substituiert sein kann;
für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl steht, wobei der (hetero)cyctoaliphatische Rest jeweils über eine lineare oder verzweigte, unsubstituierte $C_{1-5}$-Alkylen-Gruppe gebunden und/oder mit 1, 2, 3, 4 oder 5 Substituenten unabhängig voneinander ausgewählt aus der Gruppe bestehend aus F, Cl, Br, -CN, $-CF_3$, $-CHF_2$, $-CH_2F$, Hydroxy, $-C_{1-6}$Alkoxy, $-NH_2$, $-NH-(C_{1-3}$-Alkyl), $-N(C_{1-3}$-Alkyl)($C_{1-3}$-Alkyl), -NH-Phenyl, $-N(C_{1-3}$-Alkyl)-Phenyl, $-N(Phenyl)_2$, -SH, $-C_{1-6}$-Alkylthio, $-C_{1-6}$-Alkyl, $-(CH_2)$-Benzo[b]furanyl, Phenoxy, Benzyloxy, Phenyl und Benzyl substituiert sein kann, wobei jeweils der zyklische Teil der Reste Phenoxy, Benzyloxy, Phenyl, $-(CH_2)$-Benzo[b]furanyl und Benzyl mit 1, 2, 3, 4 oder 5 Substituenten unabhängig voneinander ausgewählt aus der Gruppe bestehend aus F, Cl, Br, $-C_{1-4}$-Alkyl, $-CF_3$, $-CHF_2$, $-CH_2F$, $-C_{1-4}$-Alkoxy, $-C_{1-4}$-Alkylthio, Phenoxy, Phenyl, und Benzyloxy substituiert sein kann;
oder für einen Rest ausgewählt aus der Gruppe bestehend aus Phenyl, Naphthyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl und Isochinolinyl steht, wobei der Rest jeweils über eine lineare oder verzweigte, unsubstituierte, ggf. ein Sauerstoffatom als Kettenglied aufweisende $C_{1-5}$-Alkylen- oder $C_{2-5}$-Alkenylen-Gruppe gebunden und/oder mit 1, 2, 3, 4 oder 5 Substituenten unabhängig voneinander ausgewählt aus der Gruppe bestehend aus F, Cl, Br, -CN, $-CF_3$, $-CHF_2$, $-CH_2F$, Hydroxy, $-C_{1-6}$-Alkoxy, $-O-CF_3$, $-S-CF_3$, - SH, $-C_{1-6}$-Alkylthio, $-C_{1-6}$-Alkyl, $-NH_2$, $-NH-(C_{1-3}$-Alkyl), $-N(C_{1-3}$-Alkyl)($C_{1-3}$-Alkyl), - NH-Phenyl, $-N(C_{1-3}$-Alkyl)-Phenyl, $N(Phenyl)_2$, Phenoxy, Benzyloxy, Phenyl, Benzyl und Morpholinyl substituiert sein kann, wobei jeweils der zyklische Teil der Reste Phenoxy, Benzyloxy, Phenyl und Benzyl mit 1, 2, 3, 4 oder 5 Substituenten unabhängig voneinander ausgewählt aus der Gruppe bestehend aus F, Cl, Br, Hydroxy, $-CF_3$, $-SF_5$, -CN, $-NO_2$, $-C_{1-6}$-Alkoxy, $-O-CF_3$, $-S-CF_3$, Phenyl und Benzyloxy substituiert sein kann;
bevorzugt $R^3$
für einen Wasserstoff-Rest steht;
für einen Alkyl-Rest ausgewählt aus der Gruppe bestehend aus Methyl, Ethyl, n-Propyl, n-Butyl und n-Pentyl steht, wobei der Alkyl-Rest jeweils mit 1 oder 2 Substituenten unabhängig voneinander ausgewählt aus der Gruppe bestehend aus $NH_2$, $-NH-CH_3$, $-NH-C_2H_5$, $-N-(CH_3)_2$, $-N(C_2H_5)_2$, $-N(CH_3)(C_2H_5)$, -NH-Phenyl, $-N(CH_3)$-Phenyl, $-N(C_2H_5)$-Phenyl und $-N(Phenyl)_2$ substituiert sein kann;
für einen Rest ausgewählt aus der Gruppe bestehend aus

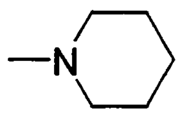 , 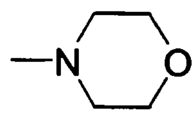 ,

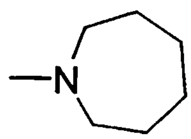 , 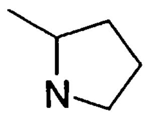 und 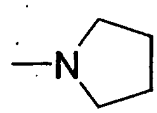 ,

steht, der über eine $-(CH_2)-$, $-(CH_2)_2-$, $-(CH_2)-O-$, $-CH(CH_3)-$, $-(CH=CH)-$ oder-$(CH_2)_3$-Gruppe gebunden und/oder mit 1, 2 oder 3 Substituenten unabhängig voneinander ausgewählt aus der Gruppe bestehend aus Methyl, Ethyl und n-Propyl substituiert sein kann;
für den folgenden Rest,

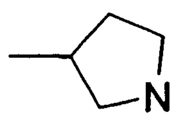 ,

steht, der mit einem Substituenten ausgewählt aus der Gruppe bestehend aus - $(CH_2)$-Benzo[b]furanyl und Benzyl substituiert sein kann, wobei jeweils der zyklische Teil der Reste $-(CH_2)$-Benzo[b]furanyl und Benzyl mit 1, 2, 3, 4 oder 5 Substituenten unabhängig voneinander ausgewählt aus der Gruppe bestehend aus F, Cl, Br, $-C_{1-4}$-Alkyl, $-CF_3$, $-CHF_2$, $-CH_2F$, $-C_{1-4}$-Alkoxy, $-C_{1-4}$-Alkylthio, Phenoxy, Phenyl, und Benzyloxy substituiert sein kann;
oder für einen Rest ausgewählt aus der Gruppe bestehend aus Phenyl und Naphthyl steht, wobei der Rest jeweils eine $-(CH_2)-$, $-(CH_2)_2-$ oder $-(CH_2)_3$-Gruppe gebunden und/oder mit 1, 2 oder 3 Substituenten unabhängig voneinander ausgewählt aus der Gruppe bestehend aus $-C_{1-6}$-Alkoxy, $-C_{1-6}$-Alkyl, $-NH_2$, $-NH-(C_{1-3}$-Alkyl), $-N(C_{1-3}$-Alkyl)($C_{1-3}$-Alkyl), -NH-Phenyl, $-N(C_{1-3}$-Alkyl)-Phenyl, N(Phenyl)$_2$ und Morpholinyl substituiert sein kann;
besonders bevorzugt $R^3$
für einen Wasserstoff-Rest steht;
für einen Rest ausgewählt aus der Gruppe bestehend aus Methyl, Ethyl, n-Propyl, $-(CH_2)-N(CH_3)_2$, $-(CH_2)-(CH_2)-N(CH_3)_2$, $-(CH_2)-(CH_2)-(CH_2)-N(CH_3)_2$, - $(CH_2)-(CH_2)-N(C_2H_5)_2$. $-(CH_2)-(CH_2)-(CH_2)-N(C_2H_5)_2$, $-(CH_2)-(CH_2)-N(CH_3)$(Phenyl) und $-(CH_2)-(CH_2)-(CH_2)-N(CH_3)$(Phenyl) steht;
für einen Rest ausgewählt aus der Gruppe bestehend aus

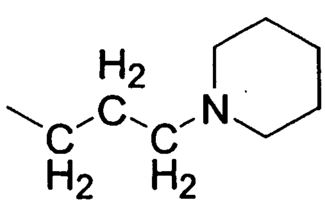 , 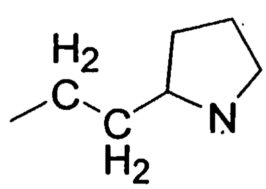 , 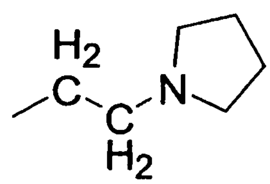 ,

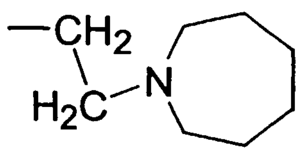 und 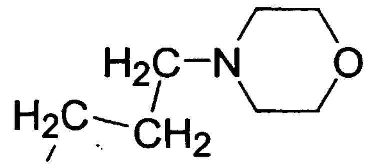 ,

steht, der mit einem Methyl-Rest substituiert sein kann;
für den folgenden Rest,

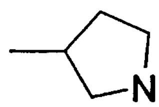 ,

steht, der am Stickstoffatom mit einem Substituenten ausgewählt aus der Gruppe bestehend aus -($CH_2$)-Benzo[b]furanyl und Benzyl substituiert sein kann, wobei jeweils der zyklische Teil der Reste -($CH_2$)-Benzo[b]furanyl und Benzyl mit 1, 2, oder 3 Substituenten unabhängig voneinander ausgewählt aus der Gruppe bestehend aus F, Cl, Br, -$CF_3$, -O-$CH_3$, -O-$C_2H_5$, -O-$C_3H_7$, -S-$CH_3$, -S-$C_2H_5$, -S-$C_3H_7$, -S-C($CH_3$)$_3$, Methyl, Ethyl, n-Propyl, Isopropyl, n-Butyl, Phenoxy und Benzyloxy substituiert sein kann;
oder für einen Rest ausgewählt aus der Gruppe bestehend aus Phenyl und Benzyl steht, wobei der zyklische Teil der Reste Phenyl und Benzyl mit 1, 2 oder 3 Substituenten unabhängig voneinander ausgewählt aus der Gruppe bestehend aus -O-$CH_3$, -O-$C_2H_5$, -O-$C_3H_7$, -N-($CH_3$)$_2$, -N($C_2H_5$)$_2$ und Morpholinyl substituiert sein kann;
ganz besonders bevorzugt
$R^3$ für einen Wasserstoff-Rest
oder für einen Methyl-Rest steht.

**11.** Verbindungen gemäß einem oder mehreren der Ansprüche 1 bis 10, **dadurch gekennzeichnet, dass** der Rest $R^4$ für einen Wasserstoff-Rest steht;
für einen Alkyl-Rest ausgewählt aus der Gruppe bestehend aus Methyl, Ethyl, n-Propyl, iso-Propyl, n-Butyl, sec-Butyl, iso-Butyl; tert-Butyl, n-Pentyl, sec-Pentyl, (1,1)-Dimethylpropyl und n-Hexyl steht, wobei derAlkyl-Rest jeweils mit 1, 2, 3, 4 oder 5 Substituenten unabhängig voneinander ausgewählt aus der Gruppe bestehend aus F, Cl, Br, -CN, -$NO_2$, Hydroxy, $C_{1-6}$-Alkoxy, -$NH_2$, -NH-($C_{1-3}$-Alkyl), -N($C_{1-3}$-Alkyl)($C_{1-3}$-Alkyl), -NH-Phenyl, -N($C_{1-3}$-Alkyl)-Phenyl und -N(Phenyl)$_2$ substituiert sein kann;
für einen Alkenyl-Rest ausgewählt aus der Gruppe bestehend aus Vinyl, 1-Propenyl, 2-Propenyl, 1-Butenyl, 2-Butenyl, 3-Butenyl, 1-Pentenyl, 2-Pentenyl und Pent-1,3-dienyl steht, wobei der Alkenyl-Rest jeweils mit 1, 2, 3, 4 oder 5 Substituenten unabhängig voneinander ausgewählt aus der Gruppe bestehend aus F, Cl, Br, -CN, -$NO_2$, Hydroxy, $C_{1-6}$-Alkoxy,-$NH_2$, -NH-($C_{1-3}$-Alkyl), -N($C_{1-3}$-Alkyl)($C_{1-3}$-Alkyl), -NH-Phenyl, -N($C_{1-3}$-Alkyl)-Phenyl und -N (Phenyl)$_2$ substituiert sein kann;
für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl steht, wobei der (hetero)cycloaliphatische Rest jeweils über eine lineare oder verzweigte, unsubstituierte $C_{1-5}$-Alkylen-Gruppe gebunden und/oder mit 1, 2, 3, 4 oder 5 Substituenten unabhängig voneinander ausgewählt aus der Gruppe bestehend aus F, Cl, Br, -CN, -$CF_3$, -$CHF_2$, -$CH_2F$, Hydroxy, -$C_{1-6}$-Alkoxy, -$NH_2$, - NH-($C_{1-3}$-Alkyl), -N($C_{1-3}$-Alkyl)($C_{1-3}$-Alkyl), -NH-Phenyl, -N($C_{1-3}$-Alkyl)-Phenyl, - N(Phenyl)$_2$, -SH, -$C_{1-6}$-Alkylthio, -$C_{1-6}$-Alkyl, -($CH_2$)-Benzo[b]furanyl, Phenoxy, Benzyloxy, Phenyl und Benzyl substituiert sein kann, wobei jeweils der zyklische Teil der Reste Phenoxy, Benzyloxy, Phenyl, -($CH_2$)-Benzo[b]furanyl und Benzyl mit 1, 2, 3, 4 oder 5 Substituenten unabhängig voneinander ausgewählt aus der Gruppe bestehend aus F, Cl, Br, -$C_{1-4}$-Alkyl, -$CF_3$, -$CHF_2$, -$CH_2F$, -$C_{1-4}$-Alkoxy, - $C_{1-4}$-Alkylthio, Phenoxy, Phenyl, und Benzyloxy substituiert sein kann;
oder für einen Rest ausgewählt aus der Gruppe bestehend aus Phenyl, Naphthyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl und Isochinolinyl steht, wobei der Rest jeweils über eine lineare oder verzweigte, unsubstituierte, ggf. ein Sauerstoffatom als

Kettenglied aufweisende $C_{1-5}$-Alkylen- oder $C_{2-5}$-Alkenylen-Gruppe gebunden und/oder mit 1, 2, 3, 4 oder 5 Substituenten unabhängig voneinander ausgewählt aus der Gruppe bestehend aus F, Cl, Br, -CN, $-CF_3$, $-CHF_2$, $-CH_2F$, Hydroxy, $-C_{1-6}$-Alkoxy, $-O-CF_3$, $-S-CF_3$, - SH, $-C_{1-6}$-Alkylthio, $-C_{1-6}$-Alkyl, $-NH_2$, -NH-($C_{1-3}$-Alkyl), -N($C_{1-3}$-Alkyl)($C_{1-3}$-Alkyl), - NH-Phenyl, -N($C_{1-3}$-Alkyl)-Phenyl, $N(Phenyl)_2$, Phenoxy, Benzyloxy, Phenyl, Benzyl und Morpholinyl substituiert sein kann, wobei jeweils der zyklische Teil der Reste Phenoxy, Benzyloxy, Phenyl und Benzyl mit 1, 2, 3, 4 oder 5 Substituenten unabhängig voneinander ausgewählt aus der Gruppe bestehend aus F, Cl, Br, Hydroxy, $-CF_3$, $-SF_5$, -CN, $-NO_2$, $-C_{1-6}$-Alkoxy, $-O-CF_3$, $-S-CF_3$, Phenyl und Benzyloxy substituiert sein kann:

bevorzugt $R^4$

für einen Wasserstoff-Rest steht;

für einen Alkyl-Rest ausgewählt aus der Gruppe bestehend aus Methyl, Ethyl, n-Propyl, n-Butyl und n-Pentyl steht, wobei der Alkyl-Rest jeweils mit 1 oder 2 Substituenten unabhängig voneinander ausgewählt aus der Gruppe bestehend aus $NH_2$, $-NH-CH_3$, $-NH-C_2H_5$, $-N-(CH_3)_2$, $-N(C_2H_5)_2$, $-N(CH_3)(C_2H_5)$, -NH-Phenyl, $-N(CH_3)$-Phenyl, -N($C_2H_5$)-Phenyl und $-N(Phenyl)_2$ substituiert sein kann,

für einen Rest ausgewählt aus der Gruppe bestehend aus

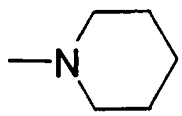 , 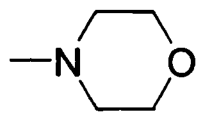 ;

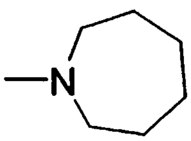 , 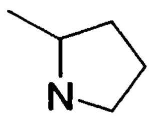 und 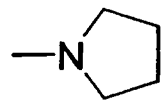 ,

steht, der über eine $-(CH_2)$-. $-(CH_2)_2$-, $-(CH_2)$-O-, $-CH(CH_3)$-, -(CH=CH)- oder - $(CH_2)_3$-Gruppe gebunden und/oder mit 1, 2 oder 3 Substituenten unabhängig voneinander ausgewählt aus der Gruppe bestehend aus Methyl, Ethyl und n-Propyl substituiert sein kann;

für den folgenden Rest,

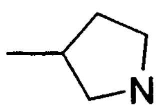 ,

steht, der mit einem Substituenten ausgewählt aus der Gruppe bestehend aus - $(CH_2)$-Benzo[b]furanyl und Benzyl substituiert sein kann, wobei jeweils der zyklische Teil der Reste $-(CH_2)$-Benzo[b]furanyl und Benzyl mit 1, 2, 3, 4 oder 5 Substituenten unabhängig voneinander ausgewählt aus der Gruppe bestehend aus F, Cl, Br, $-C_{1-4}$-Alkyl, $-CF_3$, $-CHF_2$, $-CH_2F$, $-C_{1-4}$-Alkoxy, $-C_{1-4}$-Alkylthio, Phenoxy, Phenyl, und Benzyloxy substituiert sein kann;

oder für einen Rest ausgewählt aus der Gruppe bestehend aus Phenyl und Naphthyl steht, wobei der Rest jeweils eine $-(CH_2)$-, $-(CH_2)_2$- oder $-(CH_2)_3$-Gruppe gebunden und/oder mit 1, 2 oder 3 Substituenten unabhängig voneinander ausgewählt aus der Gruppe bestehend aus $-C_{1-6}$-Alkoxy, $-C_{1-6}$-Alkyl, $-NH_2$, -NH-($C_{1-3}$-Alkyl), -N($C_{1-3}$-Alkyl)($C_{1-3}$-Alkyl), -NH-Phenyl, -N($C_{1-3}$-Alkyl)-Phenyl, $N(Phenyl)_2$ und Morpholinyl substituiert sein kann;

besonders bevorzugt $R^4$

für einen Rest ausgewählt aus der Gruppe bestehend aus Methyl, Ethyl, n-Propyl, $-(CH_2)-N(CH_3)_2$, $-(CH_2)-(CH_2)-N(CH_3)_2$, $-(CH_2)-(CH_2)-(CH_2)-N(CH_3)_2$, - $(CH_2)$-(CH2)-N$(C2H_5)_2$, $-(CH_2)-(CH_2)-(CH_2)-N(C_2H_5)_2$. $-(CH_2)-(CH_2)-N(CH_3)$(Phenyl) und $-(CH_2)-(CH_2)-(CH_2)-N(CH_3)$(Phenyl) steht;

für einen Rest ausgewählt aus der Gruppe bestehend aus

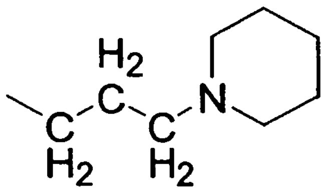 , 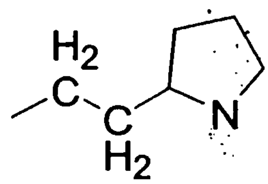 , 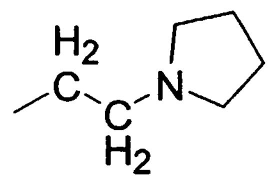 ,

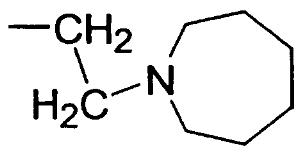 und 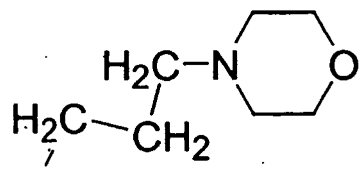 ,

steht, der mit einem Methyl-Rest substituiert sein kann;
für den folgenden Rest,

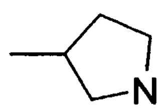 ,

steht, der am Stickstoffatom mit einem Substituenten ausgewählt aus der Gruppe bestehend aus $-(CH_2)$-Benzo[b]furanyl und Benzyl substituiert sein kann, wobei jeweils der zyklische Teil der Reste $-(CH_2)$-Benzo[b]furanyl und Benzyl mit 1, 2, oder 3 Substituenten unabhängig voneinander ausgewählt aus der Gruppe bestehend aus F, Cl, Br, , $-CF_3$, $-O-CH_3$, $-O-C_2H_5$, $-O-C_3H_7$, $-S-CH_3$, $-S-C_2H_5$, $-S-C_3H_7$, $-S-C(CH_3)_3$, Methyl, Ethyl, n-Propyl, Isopropyl, n-Butyl, Phenoxy und Benzyloxy substituiert sein kann;
oder für einen Rest ausgewählt aus der Gruppe bestehend aus Phenyl und Benzyl steht, wobei der zyklische Teil der Reste Phenyl und Benzyl mit 1, 2 oder 3 Substituenten unabhängig voneinander ausgewählt aus der Gruppe bestehend aus $-O-CH_3$, $-O-C_2H_5$, $-O-C_3H_7$, $-N-(CH_3)_2$, $-N(C_2H_5)_2$ und Morpholinyl substituiert sein kann.

**12.** Verbindungen gemäß einem oder mehreren der Ansprüche 1 bis 11, **dadurch gekennzeichnet, dass** die Reste $R^5$ und $R^6$ zusammen mit dem sie verbindenden Stickstoffatom als Ringglied einen heterocycloaliphatischen Rest bilden ausgewählt aus der Gruppe bestehend aus Piperazinyl, Morpholinyl, Thiomorpholinyl, Pyrrolidinyl, Azepanyl, Diazepanyl und Piperidinyl, wobei die heterocycloaliphatischen Reste mit einem Rest $R^8$ und ggf. einem Rest X oder einem Rest $R^9$ oder einem Rest $R^{10}$ substituiert sein können;
besonders bevorzugt $R^5$ und $R^6$ zusammen mit dem sie verbindenden Stickstoffatom als Ringglied einen der folgenden Reste bilden

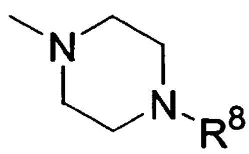 , 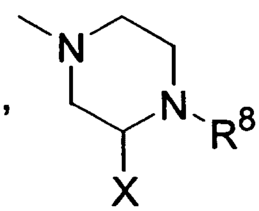 , 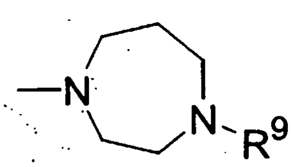 ,

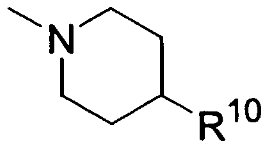, 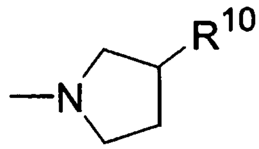 und 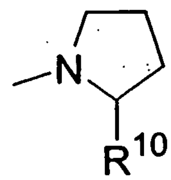.

**13.** Verbindungen gemäß einem oder mehreren der Ansprüche 1 bis 12, **dadurch gekennzeichnet, dass** der Rest X für einen Alkyl-Rest ausgewählt aus der Gruppe bestehend aus Methyl, Ethyl, n-Propyl, iso-Propyl, n-Butyl, sec-Butyl, iso-Butyl, tert-Butyl, n-Pentyl, sec-Pentyl, (1,1)-Dimethylpropyl und n-Hexyl steht;
bevorzugt X für einen Methyl-Rest steht.

**14.** Verbindungen gemäß einem oder mehreren der Ansprüche 1 bis 13, **dadurch gekennzeichnet, dass** die Reste $R^8$, $R^9$ und $R^{10}$, unabhängig voneinander, jeweils für einen Alkyl-Rest ausgewählt aus der Gruppe bestehend aus Methyl, Ethyl, n-Propyl, iso-Propyl, n-Butyl, sec-Butyl, iso-Butyl, tert-Butyl, n-Pentyl, sec-Pentyl, (1,1)-Dimethylpropyl und n-Hexyl stehen, wobei der Alkyl-Rest jeweils mit 1, 2, 3, 4 oder 5 Substituenten unabhängig voneinander ausgewählt aus der Gruppe bestehend aus F, Cl, Br, - CN, $-NO_2$, Hydroxy, $C_{1-6}$-Alkoxy, $-NH_2$, -NH-($C_{1-3}$-Alkyl), -N($C_{1-3}$-Alkyl)($C_{1-3}$-Alkyl), -NH-Phenyl, -N($C_{1-3}$-Alkyl)-Phenyl und $-N(Phenyl)_2$ substituiert sein kann;
für einen Alkenyl-Rest ausgewählt aus der Gruppe bestehend aus Vinyl, 1-Propenyl, 2-Propenyl, 1-Butenyl, 2-Butenyl, 3-Butenyl, 1-Pentenyl, 2-Pentenyl und Pent-1,3-dienyl stehen, wobei der Alkenyl-Rest jeweils mit 1, 2, 3, 4 oder 5 Substituenten unabhängig voneinander ausgewählt aus der Gruppe bestehend aus F, Cl, Br, -CN, $-NO_2$, Hydroxy, $C_{1-6}$-Alkoxy, $-NH_2$, -NH-($C_{1-3}$-Alkyl), -N($C_{1-3}$-Alkyl)($C_{1-3}$-Alkyl), -NH-Phenyl, -N($C_{1-3}$-Alkyl)-Phenyl und -N$(Phenyl)_2$ substituiert sein kann;
für einen (hetero)cycloaliphatischen Rest ausgewählt aus der Gruppe bestehend aus Cyclopropyl, Cyclobutyl, Cyclopentyl, Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl stehen, wobei der (hetero)cycloaliphatische Rest jeweils über eine lineare oder verzweigte, ggf. mit eine Carbonyl-(C=O)-Gruppe als Kettenglied aufweisende $C_{1-5}$-Alkylen-Gruppe gebunden und/oder mit 1, 2, 3, 4 oder 5 Substituenten unabhängig voneinander ausgewählt aus der Gruppe bestehend aus F, Cl, Br,-CN, $-CF_3$, $-CHF_2$, $-CH_2F$, Hydroxy, -$C_{1-6}$-Alkoxy, $-NH_2$, -NH-($C_{1-3}$-Alkyl), -N($C_{1-3}$-Alkyl)($C_{1-3}$-Alkyl), -NH-Phenyl, -N($C_{1-3}$-Alkyl)-Phenyl, $-N(Phenyl)_2$, -SH, -$C_{1-6}$-Alkylthio, -$C_{1-6}$-Alkyl, -($CH_2$)-Benzo[b]furanyl, Phenoxy, Benzyloxy, Phenyl und Benzyl substituiert sein kann, wobei jeweils der zyklische Teil der Reste Phenoxy, Benzyloxy, Phenyl, -($CH_2$)-Benzo[b]furanyl und Benzyl mit 1, 2, 3, 4 oder 5 Substituenten unabhängig voneinander ausgewählt aus der Gruppe bestehend aus F, Cl, Br, -$C_{1-4}$-Alkyl, $-CF_3$, $-CHF_2$, $-CH_2F$, -$C_{1-4}$-Alkoxy, -$C_{1-4}$-Alkylthio, Phenoxy, Phenyl, und Benzyloxy substituiert sein kann;
oder für einen Rest ausgewählt aus der Gruppe bestehend aus Phenyl, Naphthyl, (1,3)-Benzodioxolyl, (1,4)-Benzodioxanyl, Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl und Isochinolinyl stehen, wobei der Rest jeweils über eine lineare oder verzweigte, ggf. mit einem Phenyl-Rest substituierte, ggf. eine Carbonyl-(C=O)-Gruppe als Kettenglied aufweisende $C_{1-5}$-Alkylen- oder $C_{2-5}$-Alkenylen-Gruppe gebunden und/oder mit 1, 2, 3, 4 oder 5 Substituenten unabhängig voneinander ausgewählt aus der Gruppe bestehend aus F, Cl, Br, -CN, $-CF_3$, $-CHF_2$, $-CH_2F$, Hydroxy, -$C_{1-6}$-Alkoxy, $-O-CF_3$, $-S-CF_3$, - (C=O)-$CH_3$, -SH, -$C_{1-6}$-Alkylthio, -$C_{1-6}$-Alkyl, $-NH_2$, -NH-($C_{1-3}$-Alkyl), -N($C_{1-3}$-Alkyl)($C_{1-3}$-Alkyl), -NH-Phenyl, -N($C_{1-3}$-Alkyl)-Phenyl, $N(Phenyl)_2$, Phenoxy, Benzyloxy, Phenyl, Benzyl und Morpholinyl substituiert sein kann, wobei jeweils der zyklische Teil der Reste Phenoxy, Benzyloxy, Phenyl und Benzyl mit 1, 2, 3, 4 oder 5 Substituenten unabhängig voneinander ausgewählt aus der Gruppe bestehend aus F, Cl, Br, Hydroxy, $-CF_3$, $-SF_5$, -CN, $-NO_2$, -$C_{1-6}$-Alkoxy, $-O-CF_3$, - $S-CF_3$, Phenyl und Benzyloxy substituiert sein kann;
bevorzugt
$R^8$ für einen Alkyl-Rest ausgewählt aus der Gruppe bestehend aus Methyl, Ethyl, n-Propyl, Isopropyl, n-Butyl, tert-Butyl und n-Pentyl steht, wobei der Alkyl-Rest jeweils mit 1 oder 2 Substituenten unabhängig voneinander ausgewählt aus der Gruppe bestehend aus $NH_2$, $-NH-CH_3$, $-NH=C_2H_5$, $-N-(CH_3)_2$, $-N(C_2H_5)_2$,$-N(CH_3)(C_2H_5)$, -NH-Phenyl, -N$(CH_3)$-Phenyl, -N$(C_2H_5)$-Phenyl und $-N(Phenyl)_2$ substituiert sein kann;
für einen cycloaliphatischen Rest ausgewählt aus der Gruppe bestehend aus Pyrrolidinyl und Piperidinyl steht, der über eine -(C=O)- oder -($CH_2$)-(C=O)-Gruppe gebunden und/oder mit 1, 2, 3, 4 oder 5 Substituenten unabhängig

voneinander ausgewählt aus der Gruppe bestehend aus F, Cl, Br, -CN, $-CF_3$, - $CHF_2$, $-CH_2F$, Hydroxy, $-O-CH_3$, $-O-C_2H_5$, $-O-C_3H_7$, $-NH_2$, $-NH-CH_3$, $-NH-C_2H_5$, - $N-(CH_3)_2$, $-N(C_2H_5)_2$, $-N(CH_3)(C_2H_5)$, -NH-Phenyl, $-N(CH_3)$-Phenyl, $-N(C_2H_5)$-Phenyl, $-N(Phenyl)_2$, -SH, $-S-CH_3$, $-S-C_2H_5$, $-S-C_3H_7$, $-S-C(CH_3)_3$, Methyl, Ethyl, n-Propyl, Isopropyl, n-Butyl, sec-Butyl und tert-Butyl substituiert sein kann;
oder für einen Rest ausgewählt aus der Gruppe bestehend aus Phenyl, 1-Naphthyl, 2-Naphthyl, 2-Furanyl, 3-Furanyl, 2-Thiophenyl, 3-Thiophenyl, 1-Pyridinyl, 2-Pyridinyl, 3-Pyridinyl, Benzo[b]furanyl, (1,3)-Benzodioxolyl und (1,4)-Benzodioxanyl steht, der über eine -(C=O)-, $-(CH_2)-$, $-(CH_2)_2-$, $-CH(CH_3)-$. - (CH=CH)-, $-(CH_2)$-(C=O)-, $-(CH_2)$-(CH=CH)- oder $-(CH_2)_3$-Gruppe gebunden und/oder mit 1, 2, 3, 4 oder 5 Substituenten unabhängig voneinander ausgewählt aus der Gruppe bestehend aus F, Cl, Br, -CN, $-CF_3$, $-CHF_2$, $-CH_2F$, Hydroxy, - $(C=O)-CH_3$, $-O-CH_3$, $-O-C_2H_5$, $-O-C_3H_7$, $-O-CF_3$, $-S-CF_3$, -SH, $-S-CH_3$, $-S-C_2H_5$, - $S-C_3H_7$, $-S-C(CH_3)_3$, Methyl, Ethyl, n-Propyl, Isopropyl, n-Butyl, sec-Butyl, tert-Butyl, $-NH_2$, $-NH-CH_3$, $-NH-C_2H_5$, $-N-(CH_3)_2$, $-N(C_2H_5)_2$, $-N(CH_3)(C_2H_5)$, -NH-Phenyl, $-N(CH_3)$-Phenyl, $-N(C_2H_5)$-Phenyl, $-N(Phenyl)_2$, Phenoxy, Benzyloxy, Phenyl, Benzyl und Morpholinyl substituiert sein kann;
$R^9$ für einen Alkyl-Rest ausgewählt aus der Gruppe bestehend aus Methyl, Ethyl, n-Propyl, iso-Propyl, n-Butyl, sec-Butyl, iso-Butyl, tert-Butyl, n-Pentyl, sec-Pentyl, (1,1)-Dimethylpropyl und n-Hexyl steht
und
$R^{10}$ für einen cycloaliphatischen Rest ausgewählt aus der Gruppe bestehend aus Pyrrolidinyl und Piperidinyl steht, der über eine $-(CH_2)-$, $-(CH_2)_2-$, $-CH(CH_3)-$ oder $-(CH_2)_3$-Gruppe gebunden und/oder mit 1, 2, 3, 4 oder 5 Substituenten unabhängig voneinander ausgewählt aus der Gruppe bestehend aus F, Cl, Br,-CN, $-CF_3$, $-CHF_2$, $-CH_2F$, Hydroxy, $-O-CH_3$, $-O-C_2H_5$, $-O-C_3H_7$, $-NH_2$, $-NH-CH_3$, - $NH-C_2H_5$, $-N-(CH_3)_2$, $-N(C_2H_5)_2$, $-N(CH_3)(C_2H_5)$, -NH-Phenyl, $-N(CH_3)$-Phenyl, - $N(C_2H_5)$-Phenyl, $-N(Phenyl)_2$, -SH, $-S-CH_3$, $-S-C_2H_5$, $-S-C_3H_7$, $-S-C(CH_3)_3$, Methyl, Ethyl, n-Propyl, Isopropyl, n-Butyl, sec-Butyl und tert-Butyl substituiert sein kann;
oder für einen Rest ausgewählt aus der Gruppe bestehend aus Phenyl, 1-Naphthyl und 2-Naphthyl steht, der über eine $-(CH_2)-$, -[(CH)Phenyl]-, $-(CH_2)_2-$, - $(CH_2)_3$-Gruppe gebunden und/oder mit 1, 2, 3, 4 oder 5 Substituenten unabhängig voneinander ausgewählt aus der Gruppe bestehend aus F, Cl, Br,-CN, $-CF_3$, $-CHF_2$, $-CH_2F$, Hydroxy, $-O-CH_3$, $-O-C_2H_5$, $-O-C_3H_7$, $-O-CF_3$, $-S-CF_3$, Thio, $-S-CH_3$, $-S-C_2H_5$, $-S-C_3H_7$, $-S-C(CH_3)_3$, Methyl, Ethyl, n-Propyl, Isopropyl, n-Butyl, sec-Butyl, tert-Butyl, $-NH_2$, $-NH-CH_3$, $-NH-C_2H_5$, $-N-(CH_3)_2$, $-N(C_2H_5)_2$, - $N(CH_3)(C_2H_5)$, -NH-Phenyl, $-N(CH_3)$-Phenyl, $-N(C_2H_5)$-Phenyl, $-N(Phenyl)_2$, Phenoxy, Benzyloxy, Phenyl, Benzyl und Morpholinyl substituiert sein kann;
besonders bevorzugt
$R^8$ für einen Rest ausgewählt aus der Gruppe bestehend aus Methyl, Ethyl, n-Propyl, iso-Propyl und $-(CH_2)-(CH_2)-N(CH_3)_2$,
für einen Pyrrolidinyl-Rest steht, der über eine $-(CH_2)$-(C=O)-Gruppe gebunden ist,
oder für einen Rest ausgewählt aus der Gruppe bestehend aus Phenyl, 1-Naphthyl, 2-Naphthyl, 2-Thiophenyl, 3-Thiophenyl, 1-Pyridinyl, 2-Pyridinyl, 3-Pyridinyl, Benzo[b]furanyl, (1,3)-Benzodioxolyl und (1,4)-Benzodioxanyl steht, der über eine -(C=O)-, $-(CH_2)-$, $-(CH_2)_2-$, $-CH(CH_3)-$, -(CH=CH)-, $-(CH_2)$-(C=O)-, - $(CH_2)$-(CH=CH)- oder $-(CH_2)_3$-Gruppe gebunden und/oder mit 1, 2 oder 3 Substituenten unabhängig voneinander ausgewählt aus der Gruppe bestehend aus F, Cl, Br, -CN, $-CF_3$, $-CHF_2$, $-CH_2F$, $-(C=O)-CH_3$, $-O-CH_3$ und $-O-C_2H_5$ substituiert sein kann,
$R^9$ für einen Methyl- oder Ethyl-Rest steht
und
$R^{10}$ für einen Pyrrolidinyl-Rest steht, der über eine $-(CH_2)$-Gruppe gebunden ist, oder für einen Benzyhydryl-Rest steht.

**15.** Verbindungen gemäß einem oder mehreren der Ansprüche 1 bis 14, **dadurch gekennzeichnet, dass**

$R^1$ für eine Gruppe $-NR^3R^4$ oder für eine Gruppe $-NR^5R^6$ steht;
$R^2$ für einen Alkyl-Rest ausgewählt aus der Gruppe bestehend aus n- Propyl, iso-Propyl, n-Butyl, sec-Butyl, iso-Butyl, tert-Butyl, n-Pentyl, sec- Pentyl und (1,1)-Dimethylpropyl steht;
für einen Alkenyl-Rest ausgewählt aus der Gruppe bestehend aus 1- Pentenyl, 2-Pentenyl und Pent-1,3-dienyl steht;
für einen cycloaliphatischen Rest ausgewählt aus der Gruppe bestehend aus Cyclopentyl, Cyclohexyl und Cycloheptyl steht, der über eine $-(CH_2)$-Gruppe gebunden ist;
oder für einen Rest ausgewählt aus der Gruppe bestehend aus Phenyl, 1-Naphthyl, 2-Naphthyl, 2-Furanyl, 3-Furanyl, 2-Thiophenyl oder 3- Thiophenyl steht, der über eine $-(CH_2)-$, $-(CH_2)_2-$, $-(CH_2)$-O-, $-CH(CH_3)-$, -(CH=CH)- oder $-(CH_2)_3$-Gruppe gebunden und/oder mit 1, 2 oder 3 Substituenten unabhängig voneinander ausgewählt aus der Gruppe bestehend aus F, Cl, Br, $-O-CH_3$, $-O-C_2H_5$, Methyl, Ethyl, n-Propyl und Isopropyl substituiert sein kann;
$R^3$ für einen Wasserstoff-Rest oder für einen Methyl-Rest steht;

$R^4$ für einen Rest ausgewählt aus der Gruppe bestehend aus Methyl, Ethyl, n-Propyl, $-(CH_2)-N(CH_3)_2$, $-(CH_2)-(CH_2)-N(CH_3)_2$, $-(CH_2)-(CH_2)-(CH_2)-N(CH_3)_2$, $-(CH_2)-(CH_2)-N(C_2H_5)_2$, $-(CH_2)-(CH_2)-(CH_2)-N(C_2H_5)_2$, $-(CH_2)-(CH_2)-N(CH_3)$(Phenyl) und $-(CH_2)-(CH_2)-(CH_2)-N(CH_3)$(Phenyl) steht;
für einen Rest ausgewählt aus der Gruppe bestehend aus

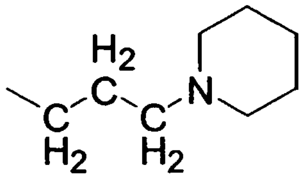 , 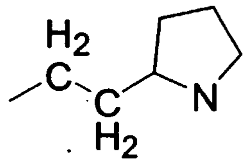 , 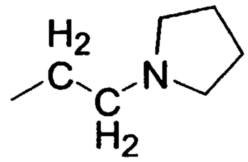 ,

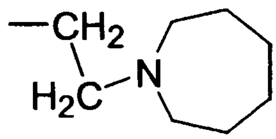 und 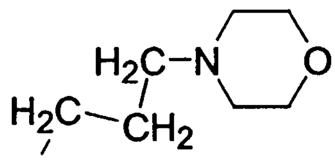 ,

steht, der mit einem Methyt-Rest substituiert sein kann;
für den folgenden Rest,

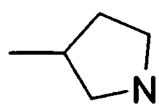 ,

steht, der am Stickstoffatom mit einem Substituenten ausgewählt aus der Gruppe bestehend aus $-(CH_2)$-Benzo[b]furanyl und Benzyl substituiert sein kann, wobei jeweils der zyklische Teil der Reste $-(CH_2)$- Benzo[b]furanyl und Benzyl mit 1, 2, oder 3 Substituenten unabhängig voneinander ausgewählt aus der Gruppe bestehend aus F, Cl, Br, , $-CF_3$, $-O-CH_3$, $-O-C_2H_5$, $-O-C_3H_7$, $-S-CH_3$, $-S-C_2H_5$, $-S-C_3H_7$, $-S-C(CH_3)_3$, Methyl, Ethyl, n-Propyl, Isopropyl, n-Butyl, Phenoxy und Benzyloxy substituiert sein kann;
oder für einen Rest ausgewählt aus der Gruppe bestehend aus Phenyl und Benzyl steht, wobei der zyklische Teil der Reste Phenyl und Benzyl mit 1, 2 oder 3 Substituenten unabhängig voneinander ausgewählt aus der Gruppe bestehend aus $-O-CH_3$, $-O-C_2H_5$, $-O-C_3H_7$, $-N-(CH_3)_2$, $-N(C_2H_5)_2$ und Morpholinyl substituiert sein kann;
$R^5$ und $R^6$ zusammen mit dem sie verbindenden Stickstoffatom als Ringglied einen der folgenden Reste bilden

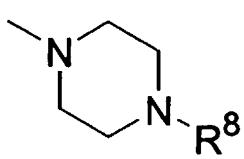 , 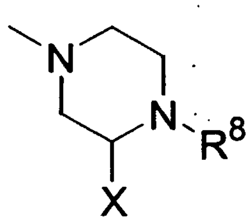 , 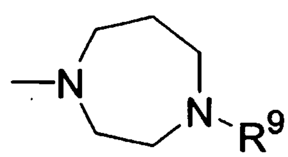 ,

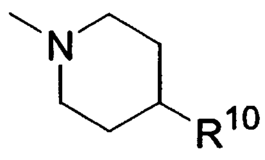 , 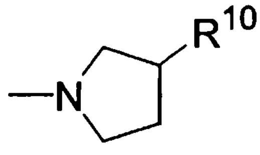 und 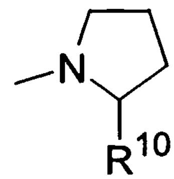 ;

X für einen Methyl-Rest steht,
$R^8$ für einen Rest ausgewählt aus der Gruppe bestehend aus Methyl, Ethyl, n-Propyl, iso-Propyl und $-(CH_2)-(CH_2)-N(CH_3)_2$,
für einen Pyrrolidinyl-Rest steht, der über eine $-(CH_2)-(C{=}O)-$Gruppe gebunden ist,
oder für einen Rest ausgewählt aus der Gruppe bestehend aus Phenyl, 1-Naphthyl, 2-Naphthyl, 2-Thiophenyl, 3-Thiophenyl, 1-Pyridinyl, 2- Pyridinyl, 3-Pyridinyl, Benzo[b]furanyl, (1,3)-Benzodioxolyl und (1,4)- Benzodioxanyl steht, der über eine $-(C{=}O)-$, $-(CH_2)-$, $-(CH_2)_2-$, $-CH(CH_3)-$, $-(CH{=}CH)-$, $-(CH_2)-(C{=}O)-$, $-(CH_2)-(CH{=}CH)-$ oder $-(CH_2)_3-$ Gruppe gebunden und/oder mit 1, 2 oder 3 Substituenten unabhängig voneinander ausgewählt aus der Gruppe bestehend aus F, Cl, Br, -CN, $-CF_3$, $-CHF_2$, $-CH_2F$, $-(C{=}O)-CH_3$, $-O-CH_3$ und $-O-C_2H_5$ substituiert sein kann,
$R^9$ für einen Methyl- oder Ethyl-Rest steht und
$R^{10}$ für einen Pyrrolidinyl-Rest steht, der über eine $-(CH_2)-$Gruppe gebunden ist,

oder für einen Benzyhydryl-Rest steht.

**16.** Verbindungen gemäß einem oder mehreren der Ansprüche 1 bis 15 ausgewählt aus der Gruppe bestehend aus.
2-Cyclohexyl-N-{4-[4-(2-dimethylamino-ethyl)-piperazin-1-carbonyl]-4,5,6,7-tetrahydro-benzothiazol-2-yl}-acetamid,
N-[4-(4-Methyl-[1,4]diazepan-1-carbonyl)-4,5,6,7-tetrahydro-benzothiazol-2-yl]-2-phenoxy-acetamid,
2-(2-Phenyl-propionylamino)-4,5,6,7-tetrahydro-benzothiazol-4-carbonsäure (3-dimethylamino-propyl)-amid,
Naphthalin-2-carbonsäure [4-(4-methyl-piperäzin-1-carbonyl)-4,5,6,7-tetrahydro-benzothiazol-2-yl]-amid,
N-[4-(2-Pyrrolidin-1-ylmethyl-pyrrolidin-1-carbonyl)-4,5,6,7-tetrahydro-benzothiazol-2-yl]-2-thiophen-2-yl-acetamid,
N-{4-[4-(7-Methoxy-benzo[1,3]dioxol-5-ylmethyl)-piperazin-1-carbonyl]-4,5,6,7-tetrahydro-benzothiazol-2-yl}-2-(2-methoxy-phenyl)-acetamid,
N-{4-[4-(4-Methoxy-phenyl)-3-methyl-piperazin-1-carbonyl]-4,5,6,7-tetrahydro-benzothiazol-2-yl}-benzamid,
Furan-2-carbonsäure-{4-[4-(4-acetyl-phenyl)-piperazin-1-carbonyl]-4,5,6,7-tetrahydro-benzothiazol-2-yl}-amid,
2-[(Furan-2-carbonyl)-amino]-4,5,6,7-tetrahydro-benzothiazol-4-carbonsäure-[1-(2,4,6-trimethoxy-benzyl)-pyrrolidin-3-yl]-amid,
N-[4-(4-Isopropyl-piperazin-1-carbonyl)-4,5,6,7-tetrahydro-benzothiazol-2-yl]-2-phenyl-propionamid,
3-Furan-2-yl-N-[4-(4-thiophen-3-ylmethyl-piperazin-1-carbonyl)-4,5,6,7-tetrahydro-benzothiazol-2-yl]-acrylamid,
2-(4-Methoxy-benzoylamino)-4,5,6,7-tetrahydro-benzothiazol-4-carbonsäure-[1-(2-chlor-6-fluor-benzyl)pyrrolidin-3-yl]-methyl-amid,
Hexansäure-[4-(4-pyridin-4-yl-piperazin-1-carbonyl)-4,5,6,7-tetrahydro-benzothiazol-2-yl]-amid,
2-(2-Thiophen-2-yl-acetylamino)-4,5,6,7-tetrahydro-benzothiazol-4-carbonsäure-(3-morpholin-4-yl-propyl)-amid,
2-(2-Phenyl-propionylamino)-4,5,6,7-tetrahydro-benzothiazol-4-carbonsäure [1-(4-phenoxy-benzyl)-pyrrolidin-3-yl]-amid,
2-[(Furan-3-carbonyl)-amino]-4,5,6,7-tetrahydro-benzothiazol-4-carbonsäure (2-azepan-1-yl-ethyl)-amid,
Furan-3-carbonsäure [4-(4-benzofuran-2-ylmethyl-piperazin-1-carbonyl)-4,5,6,7-tetrahydro-benzothiazol-2-yl]-amid,
2-Hexanylamino-4,5,6,7-tetrahydro-benzothiazol-4-carbonsäure [3-(2-methylpiperidin-1-yl)-propyl]-amid,
2-(2-Phenyl-propionylamino)-4,5,6,7-tetrahydro-benzothiazol-4-carbonsäure-(2-dimethylamino-ethyl)-amid,
2-Ethoxy-N-[4-(4-phenethyl-piperazin-1-carbonyl)-4,5,6,7-tetrahydro-benzothiazol-2-yl]-benzamid,
2-(4-Fluor-phenoxy)-N-[4-(4-phenyl-piperazin-1-carbonyl)-4,5,6,7-tetrahydro-benzothiazol-2-yl]-acetamid,
2-(2-Phenyl-propionylamino)-4,5,6,7-tetrahydro-benzothiazol-4-carbonsäure [2-(1-methyl-pyrrolidin-2-yl)-ethyl]-amid,
2-(3-Furan-2-yl-acryloylamino)-4,5,6,7-tetrahydro-benzothiazol-4-carbonsäure [3-(2-methyl-piperidin-1-yl)-propyl]-amid,
2-(3,3-Dimethyl-butyrylamino)-4,5,6,7-tetrahydro-benzothiazol-4-carbonsäure-(3-dimethylamino-propyl)-amid,

N-{4-[4-(4-Chlor-benzyl)-piperazin-1-carbonyl]-4,5,6,7-tetrahydro-benzothiazol-2-yl}-4-methoxy-benzamid,
Naphthalin-2-carbonsäure-[4-(2-pyrrolidin-1-ylmethyl-pyrrolidin-1-carbonyl)-4,5,6,7-tetrahydro-benzothiazol-2-yl]-amid,
N-[4-(4-Benzofuran-2-ylmethyl-piperazin-1-carbonyl)4,5,6,7-tetrahydro-benzothiazol-2-yl]-butyramid,
N-{4-[4-(3-Methoxy-phenyl)-piperazin-1-carbonyl]-4,5,6,7-tetrahydro-benzothiazol-2-yl}-benzamid,
2-Butyrylamino-4,5,6,7-tetrahydro-benzothiazol-4-carbonsäure-(1-biphenyl-4-ylmethyl-pyrrolidin-3-yl)-methylamid,
2-(2-Phenoxy-acetylamino)-4,5,6,7-tetrahydro-benzothiazol-4-carbonsäure (2-dimethylamino-ethyl)-amid,
2-Ethoxy-N-[4-(2-pyrrolidin-1-ylmethyl-pyrrolidin-1-carbonyl)-4,5,6,7-tetrahydro-benzothiazol-2-yl]-benzamid,
Naphthalin-2-carbonsäure {4-[4-(2-oxo-2-pyrrolidin-1-yl-ethyl)-piperazin-1-carbonyl]-4,5,6,7-tetrahydro-benzothiazol-2-yl}-amid,
2-(2-Phenyl-propionylamino)-4,5,6,7-tetrahydro-benzothiazol-4-carbonsäure [1-(2-brom-4,5-dimethoxy-benzyl)-pyrrolidin-3-yl]-amid,
Hexansäure {4-[4-(2,4,6-trimethoxy-benzyl)-piperazin-1-carbonyl]-4,5,6,7-tetrahydro-benzothiazol-2-yl}-amid,
2-(3,5-Dimethyl-benzoylamino)-4,5,6,7-tetrahydro-benzothiazol-4-carbonsäure [1-(2-tert-butylsulfanyl-benzyl)-pyrrolidin-3-yl]-amid,
2-(2-Phenyl-propionylamino)-4,5,6,7-tetrahydro-benzothiazol-4-carbonsäure [1-(2-benzyloxy-benzyl)-pyrrolidin-3-yl]-methyl-amid,
2-Hexa-2,4-dienoylamino-4,5,6,7-tetrahydro-benzothiazol-4-carbonsäure (1-biphenyl-4-ylmethyl-pyrrolidin-3-yl)-methyl-amid,
2-(3-Phenyl-propionylamino)-4,5,6,7-tetrahydro-benzothiazol-4-carbonsäure (2-diethylamino-ethyl)-amid,
2-(2-Thiophen-2-yl-acetylamino)-4,5,6,7-tetrahydro-benzothiazol-4-carbonsäure (2-azepan-1-yl-ethyl)-amid,
2-(2-Thiophen-2-yl-acetylamino)-4,5,6,7-tetrahydro-benzothiazol-4-carbonsäure [1-(2-ethoxy-benzyl)-pyrrolidin-3-yl]-methyl-amid,
2-Hexanylamino-4,5,6,7-tetrahydro-benzothiazol-4-carbonsäure (1-benzofuran-2-ylmethyl-pyrrolidin-3-yl)-methylamid,
2-(2-Phenoxy-acetylamino)-4,5,6,7-tetrahydro-benzothiazol-4-carbonsäure [3-(2-methyl-piperidin-1-yl)-propyl]-amid,
2-[(Naphthalin-2-carbonyl)-amino]-4,5,6,7-tetrahydro-benzothiazol-4-carbonsäure (2-pyrrolidin-1-yl-ethyl)-amid,
2-[2-(3,5-Difluor-phenyl)-acetylamino]-4,5,6,7-tetrahydro-benzothiazol-4-carbonsäure [1-(2-ethoxy-benzyl)-pyrrolidin-3-yl]-methyl-amid,
2-[(Naphthalin-2-carbonyl)-amino]-4,5,6,7-tetrahydro-benzothiazol-4-carbonsäure 4-dimethylamino-benzylamid,
2-[2-(4-Fluor-phenoxy)-acetylamino]-4,5,6,7-tetrahydro-benzothiazol-4-carbonsäure [2-(1-methyl-pyrrolidin-2-yl)-ethyl]-amid,
Furan-2-carbonsäure {4-[4-(2-cyano-phenyl)-piperazin-1-carbonyl]-4,5,6,7-tetrahydro-benzothiazol-2-yl}-amid,
2-(3-Phenyl-propionylamino)-4,5,6,7-tetrahydro-benzothiazol-4-carbonsäure (1-benzofuran-2-ylmethyl-pyrrolidin-3-yl)-methyl-ämid,
2-Hexa-2,4-dienoylamino-4,5,6,7-tetrahydro-benzothiazol-4-carbonsäure [1-(2,6-dichlor-benzyl)-pyrrolidin-3-yl]-methyl-amid,
2-[(Furan-2-carbonyl)-aminol-4,5,6,7-tetrahydro-benzothiazol-4-carbonsäure [1-(5-brom-2-ethoxy-benzyl)-pyrrolidin-3-yl]-amid,
2-(2-Phenoxy-acetylamino)-4,5,6,7-tetrahydro-benzothiazol-4-carbonsäure [1-(2-ethoxy-benzyl)-pyrrolidin-3-yl]-methyl-amid,
Hexansäure {4-[4-(2-fluor-phenyl)-piperazin-1-carbonyl]-4,5,6,7-tetrahydro-benzothiazol-2-yl}-amid,
Naphthalin-2-carbonsäure {4-[4-(2-fluor-benzyl)-piperazin-1-carbonyl]-4,5,6,7-tetrahydro-benzothiazol-2-yl}-amid,
2-(2-Phenoxy-acetylamino)-4,5,6,7-tetrahydro-benzothiazol-4-carbonsäure methyl-[1-(2-trifluormethyl-benzyl)-pyrrolidin-3-yl]-amid,
2-(3-Phenyl-propionylamino)-4,5,6,7-tetrahydro-benzothiazol-4-carbonsäure (1-biphenyl-4-ylmethyl-pyrrolidin-3-yl)-methyl-amid,
2-[2-(2-Methoxy-phenyl)-acetylamino]-4,5,6,7-tetrahydro-benzothiazol-4-carbonsäure (1-benzofuran-2-ylmethyl-pyrrolidin-3-yl)-methyl-amid,
2-(3,5-Difluor-phenyl)-N-[4-(2-pyrrolidin-1-ylmethyl-pyrrolidin-1-carbonyl)-4,5,6,7-tetrahydro-benzothiazol-2-yl]-acetamid,
2-[(Furan-2-carbonyl)-amino]-4,5,6,7-tetrahydro-benzothiazol-4-carbonsäure 4-dimethylamino-benzylamid,
Hexansäure {4-[4-(3-fluor-4-methoxy-benzyl)-piperazin-1-carbonyl]-4,5,6,7-tetrahydro-benzothiazol-2-yl}-amid,
N-{4-[4-(4-Methoxy-phenyl)-3-methyl-piperazin-1-carbonyl]-4,5,6,7-tetrahydro-benzothiazol-2-yl}-2-phenyl-propionamid,
2-Hexa-2,4-dienoylamino-4,5,6,7-tetrahydro-benzothiazol-4-carbonsäure [1-(2-chlor-6-fluor-benzyl)-pyrrolidin-3-yl]-methyl-amid,
2-[2-(3,5-Difluor-phenyl)-acetylamino]-4,5,6,7-tetrahydro-benzothiazol-4-carbonsäure[1-(2,6-dichlor-benzyl)-pyrrolidin-3-yl]-methyl-amid,
2-[2-(4-Fluor-phenoxy)-acetylamino]-4,5,6,7-tetrahydro-benzothiazol-4-carbonsäure [1-(2-ethoxy-benzyl)-pyrrolidin-3-yl]-methyl-amid,
Furan-3-carbonsäure [4-(4-pyridin-2-yl-piperazin-1-carbonyl)-4,5,6,7-tetrahydro-benzothiazol-2-yl]-amid,
Hexansäure [4-(4-benzofuran-2-ylmethyl-piperazin-1-carbonyl)-4,5,6,7-tetrahydro-benzothiazol-2-yl]-amid,
2-Phenoxy-N-{4-[4-(1-phenyl-ethyl)-piperazin-1-carbonyl]-4,5,6,7-tetrahydro-benzothiazol-2-yl}-acetamid,
2-(2-Cyclohexyl-acetylamino)-4,5,6,7-tetrahydro-benzothiazol-4-carbonsäure [1-(2,6-dichlor-benzyl)-pyrrolidin-3-yl]-methyl-amid,
2-(2-Cyclohexyl-acetylamino)-4,5,6,7-tetrahydro-benzothiazol-4-carbonsäure [3-(methyl-phenyl-amino)-propyl]-amid,
2-(2-Ethoxy-benzoylamino)-4,5,6,7-tetrahydro-benzothiazol-4-carbonsäure [3-(methyl-phenyl-amino)-propyl]-amid,
3-Phenyl-N-{4-[4-(5-trifluormethyl-pyridin-2-yl)-piperazin-1-carbonyl]-4,5,6,7-tetrahydro-benzothiazol-2-yl}-propionamid,
Furan-3-carbonsäure [4-(4-phenethyl-piperazin-1-carbonyl)-4,5,6,7-tetrahydro-benzothiazol-2-yl]-amid,
3-Phenyl-N-{4-[4-(3-trifluormethyl-phenyl)-piperazin-1-carbonyl]-4,5,6,7-tetrahydro-benzothiazol-2-yl}-propionamid,
2-(3,3-Dimethyl-butyrylamino)-4,5,6,7-tetrahydro-benzothiazol-4-carbonsäure [1-(2-benzyloxy-benzyl)-pyrrolidin-3-yl]-amid,
2-(3,5-Difluor-phenyl)-N-{4-[4-(3-trifluor-methyl-phenyl)-piperazin-1-carbonyl]-4,5,6,7-tetrahydro-benzothiazol-2-yl}-acetamid,
2-Ethoxy-N-{4-[4-(1-phenyl-ethyl)-piperazin-1-carbonyl]-4,5,6,7-tetrahydro-benzothiazol-2-yl}-benzamid,
3-Furan-2-yl-N-{4-[4-(3-trifluormethyl-phenyl)-piperazin-1-carbonyl]-4,5,6,7-tetrahydro-benzothiazol-2-yl}-acrylamid,
N-{4-[4-(2-Cyano-phenyl)-piperazin-1-carbonyl]-4,5,6,7-tetrahydro-benzothiazol-2-yl}-3-phenyl-propionamid,
2-(2-Cyclohexyl-acetylamino)-4,5,6,7-tetrahydro-benzothiazol-4-carbonsäure[1-(2-chlor-6-fluor-benzyl)-pyrrolidin-3-yl]-methyl-amid,
N-{4-[4-(2-Methoxy-naphthalin-1-ylmethyl)-piperazin-1-carbonyl]-4,5,6,7-tetrahydro-benzothiazol-2-yl}-3,3-dimethyl-butyramid,
2-(3,3-Dimethyl-butyrylamino)-4,5,6,7-tetrahydro-benzothiazol-4-carbonsäure (2,5-diethoxy-4-morpholin-4-yl-phenyl)-amid,
N-{4-[4-(2-Chlor-phenyl)-piperazin-1-carbonyl]-4,5,6,7-tetrahydro-benzothiazol-2-yl}-2-(4-fluor-phenyl)-acetamid,
N-[4-(4-Benzyl-piperazin-1-carbonyl)-4,5,6,7-tetrahydro-benzothiazol-2-yl]-3,3-dimethyl-butyramid,
N-[4-(4-Benzhydryl-piperazin-1-carbony)-4,5,6,7-tetrahydro-benzothiazol-2-yl]-2-(3,5-difluor-phenyl)-acetamid,
N-[4-(4-Benzhydryl-piperazin-1-carbonyl)-4,5,6,7-tetrahydro-benzothiazol-2-yl]-2-thiophen-2-yl-acetamid,
N-{4-[4-(2-Chlor-phenyl)-piperazin-1-carbonyl]-4,5,6,7-tetrahydro-benzothiazol-2-yl}-2-ethoxy-benzamid,
3,3-Dimethyl-N-{4-[4-(5-trifluormethyl-pyridin-2-yl)-piperazin-1-carbonyl]-4,5,6,7-tetrahydro-benzothiazol-2-yl}-butyramid,
3,3-Dimethyl-N-{4-[4-(3-phenyl-allyl)-piperazin-1-carbonyl]-4,5,6,7-tetrahydro-benzothiazol-2-yl}-butyramid,
N-[4-(4-Benzhydryl-piperazin-1-carbonyl)-4,5,6,7-tetrahydro-benzothiazol-2-yl]-2-cyclohexyl-acetamid und
Furan-2-carbonsäure-[4-(4-phenyl-piperazin-1-carbonyl)-4,5,6,7-tetrahydro-benzothiazol-2-yl]-amid,
ggf. in Form ihrer reinen Stereoisomeren, insbesondere Enantiomeren oder Diastereomeren, ihrer Racemate oder in Form von Mischungen der Stereoisomeren, insbesondere der Enantiomeren und/oder Diastereomeren, in einem beliebigen Mischungsverhältnis, oder jeweils ggf. in Form entsprechender Salze oder jeweils ggf. in Form entsprechender Solvate.

**17.** Verfahren zur Herstellung substituierter 4,5,6,7-Tetrahydrobenzothiazol-2-yl-amin-Verbindungen gemäß einem oder mehreren der Ansprüche 1 bis 16, **dadurch gekennzeichnet, daß** wenigstens eine Verbindung der Formel II

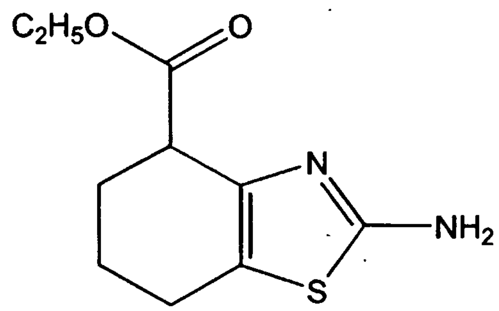

II,

ggf. in Form ihres Salzes, durch Umsetzung mit wenigstens einem Acylierungsreagenz der allgemeinen Formel III,

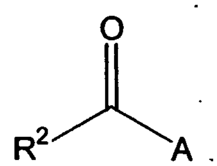

III

worin $R^2$ die Bedeutung gemäß einem oder mehreren der Ansprüche 1 bis 16 hat und A für eine vom Acyl-Rest $R^2$-(C=O)- abspaltbare Gruppe, vorzugsweise für - OH, -Cl oder -O-(C=O)-$R^2$ steht,
in eine Verbindung der allgemeinen Formel IV

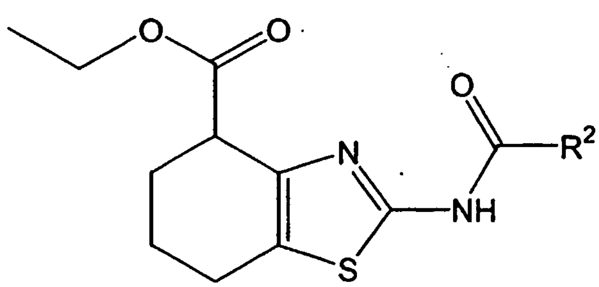

IV

überführt wird, welche ggf. gereinigt und/oder ggf. isoliert wird, und ggf. durch Spaltung des Ethylesters in eine Verbindung der allgemeinen Formel V

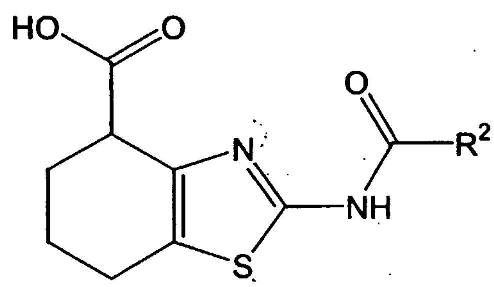

V

überführt wird, welche ggf. gereinigt und/oder ggf. isoliert wird, und wenigstens eine Verbindung der allgemeinen Formel IV und/oder wenigstens eine Verbindung der allgemeinen Formel V durch Umsetzung mit wenigstens einer Verbindung der allgemeinen Formel $R^1$-H, worin $R^1$ die Bedeutung gemäß einem oder mehreren der Ansprüche 1 bis 16 hat, in eine Verbindung der allgemeinen Formel I gemäß Anspruch 1 überführt wird, welche ggf. gereinigt und/oder ggf. isoliert wird.

**18.** Arzneimittel enthaltend wenigstens eine substituierte 4,5,6,7-Tetrahydro-benzothiazol-2-ylamin-Verbindung gemäß einem der Ansprüche 1 bis 16 und ggf. einen oder mehrere pharmazeutisch verträgliche Hilfsstoffe.

**19.** Arzneimitttel gemäß Anspruch 18 zur Regulation der Noradrenalin-Wiederaufnahme (Noradrenalin-Uptake), zur Regulation der 5-Hydroxy-Tryptophan-Wiederaufnahme (5-HT-Uptake), zur Behandlung von Schmerzen, zur Behandlung von Akohol- und/oder Drogen- und/oder Medikamentenmißbrauch, zur Prophylaxe und/oder Behandlung von Akohol- und/oder Drogen- und/oder Medikamentenabhängigkeit, zur Prophylaxe und/oder Behandlung von Entzündungen, zur Prophylaxe und/oder Behandlung von Depressionen, zur Prophylaxe und/oder Behandlung von Antriebslosigkeit, zur Prophylaxe und/oder Behandlung von Störungen der Nahrungsmittelaufnahme, vorzugsweise ausgewählt aus der Gruppe bestehend aus Bulimie, Anorexie, Fettsucht und Kachexie, zur Prophylaxe und/oder Behandlung von Katalepsie, zur Vigilanzsteigerung, zur Libidosteigerung oder zur Anxiolyse.

**20.** Verwendung wenigstens einer substituierten 4,5,6,7-Tetrahydro-benzothiazol-2-ylamin-Verbindung gemäß einem der Ansprüche 1 bis 16 zur Herstellung eines Arzneimittels zur Regulation der Noradrenalin-Wiederaufnahme (Noradrenalin-Uptake).

**21.** Verwendung wenigstens einer substituierten 4,5,6,7-Tetrahydro-benzothiazol-2-ylamin-Verbindung gemäß einem der Ansprüche 1 bis 16 zur Herstellung eines Arzneimittels zur Regulation der 5-Hydroxy-Tryptophan-Wiederaufnahme (5-HT-Uptake).

**22.** Verwendung wenigstens einer substituierten 4,5,6,7-Tetrahydro-benzothiazol-2-ylamin-Verbindung gemäß einem der Ansprüche 1 bis 16 zur Herstellung eines Arzneimittels zur Behandlung von Schmerzen.

**23.** Verwendung wenigstens einer substituierten 4,5,6,7-Tetrahydro-benzothiazol-2-ylamin-Verbindung gemäß einem der Ansprüche 1 bis 16 zur Herstellung eines Arzneimittels zur Behandlung von Akoholmißbrauch und/oder Drogenmißbrauch und/oder Medikamentenmißbrauch.

**24.** Verwendung wenigstens einer substituierten 4,5,6,7-Tetrahydro-benzothiazol-2-ylamin-Verbindung gemäß einem der Ansprüche 1 bis 16 zur Herstellung eines Arzneimittels zur Prophylaxe und/oder Behandlung von Akoholsucht und/oder Drogensucht und/oder Medikamentensucht.

**25.** Verwendung wenigstens einer substituierten 4,5,6,7-Tetrahydro-benzothiazol-2-ylamin-Verbindung gemäß einem der Ansprüche 1 bis 16 zur Herstellung eines Arzneimittels zur Prophylaxe und/oder Behandlung von Entzündungen.

**26.** Verwendung wenigstens einer substituierten 4,5,6,7-Tetrahydro-benzothiazol-2-ylamin-Verbindung gemäß einem der Ansprüche 1 bis 16 zur Herstellung eines Arzneimittels zur Prophylaxe und/oder Behandlung von Depressionen.

**27.** Verwendung wenigstens einer substituierten 4,5,6,7-Tetrahydro-benzothiazol-2-ylamin-Verbindung gemäß einem der Ansprüche 1 bis 16 zur Herstellung eines Arzneimittels zur Behandlung von Antriebslosigkeit.

**28.** Verwendung wenigstens einer substituierten 4,5,6,7-Tetrahydro-benzothiazol-2-ylamin-Verbindung gemäß einem der Ansprüche 1 bis 16 zur Herstellung eines Arzneimittels zur Prophylaxe und/oder Behandlung von Störungen der Nahrungsmittelaufnahme, vorzugsweise ausgewählt aus der Gruppe bestehend aus Bulimie, Anorexie, Fettsucht und Kachexie.

**29.** Verwendung wenigstens einer substituierten 4,5,6,7-Tetrahydro-benzothiazol-2-ylamin-Verbindung gemäß einem der Ansprüche 1 bis 16 zur Herstellung eines Arzneimittels zur Prophylaxe und/oder Behandlung von Katalepsie.

**30.** Verwendung wenigstens einer substituierten 4,5,6,7-Tetrahydro-benzothiazol-2-ylamin-Verbindung gemäß einem der Ansprüche 1 bis 16 zur Herstellung eines Arzneimittels zur Vigilanzsteigerung.

**31.** Verwendung wenigstens einer substituierten 4,5,6,7-Tetrahydro-benzothiazol-2-ylamin-Verbindung gemäß einem der Ansprüche 1 bis 16 zur Herstellung eines Arzneimittels zur Libidosteigerung.

**32.** Verwendung wenigstens einer substituierten 4,5,6,7-Tetrahydro-benzothiazol-2-ylamin-Verbindung gemäß einem der Ansprüche 1 bis 16 zur Herstellung eines Arzneimittels zur Anxiolyse.

**33.** Verwendung wenigstens einer substituierten 4,5,6,7-Tetrahydro-benzothiazol-2-ylamin-Verbindung gemäß einem der Ansprüche 1 bis 16 zur Herstellung eines Arzneimittels zur gleichzeitigen Behandlung von Schmerzen und Depressionen.

## Claims

**1.** Substituted 4,5,6,7-tetrahydrobenzothiazol-2-ylamine compounds of the general formula I:

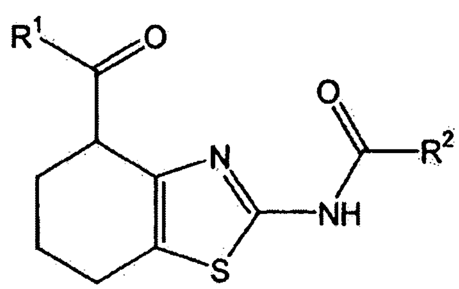

**I**

in which
$R^1$ stands for a group $-NR^3R^4$ or for a group $-NR^5R^6$,
$R^2$ stands for a linear or branched, saturated or unsaturated, optionally at least monosubstituted aliphatic residue, or for a saturated or unsaturated, optionally at least monosubstituted cycloaliphatic residue, which optionally comprises at least one heteroatom as ring member and which may be attached via an optionally at least monosubstituted alkylene group, alkenylene group, or alkynylene group, which groups optionally comprise at least one heteroatom as chain member, or for an optionally at least monosubstituted aryl residue or heteroaryl residue which may be attached via an optionally at least monosubstituted alkylene group, alkenylene group, or alkynylene group, optionally comprising at least one heteroatom as chain member,
$R^3$ stands for a hydrogen residue, or for a linear or branched, saturated or unsaturated, optionally at least monosubstituted aliphatic residue, or for a saturated or unsaturated, optionally at least monosubstituted, cycloaliphatic residue which optionally comprises at least one heteroatom as ring member and which may be attached via an optionally at least monosubstituted alkylene group, alkenylene group, or alkynylene group, optionally comprising at

least one heteroatom as chain member, or for an optionally at least monosubstituted aryl residue or heteroaryl residue which may be attached via an optionally at least monosubstituted alkylene group, alkenylene group, or alkynylene group, which groups optionally comprise at least one heteroatom as chain member,
$R^4$ stands for a hydrogen residue, or for a linear or branched, saturated or unsaturated, optionally at least monosubstituted aliphatic residue, or for a saturated or unsaturated, optionally at least monosubstituted cycloaliphatic residue which optionally comprises at least one heteroatom as ring member and which may be attached via an optionally at least monosubstituted alkylene group, alkenylene group, or alkynylene group, which groups optionally comprise at least one heteroatom as chain member, or for an optionally at least monosubstituted aryl residue or heteroaryl residue which may be attached via an optionally at least monosubstituted alkylene group, alkenylene group, or alkynylene group, which groups optionally comprise at least one heteroatom as chain member,
$R^5$ and $R^6$ form, together with the nitrogen atom bridging them as ring member, a saturated, unsaturated, or aromatic, optionally at least monosubstituted heterocyclic residue optionally comprising at least one further heteroatom as ring member,
optionally in the form of their pure stereoisomers, particularly enantiomers or diastereoisomers, of their racemates or in the form of mixtures of the stereoisomers, particularly the enantiomers and/or diastereoisomers, in any desired mixture ratio, or in each case optionally in the form of corresponding salts or in each case optionally in the form of corresponding solvates.

**2.** Compounds according to claim 1, **characterized in that** $R^2$ stands for a linear or branched, saturated or unsaturated, optionally at least monosubstituted aliphatic $C_1$-$C_{10}$ residue, or for a saturated or unsaturated, three-membered to seven-membered, optionally at least monosubstituted cycloaliphatic residue, which optionally comprises at least one heteroatom as ring member and which may be attached via an optionally at least monosubstituted $C_1$-$C_{10}$ alkylene group, $C_2$-$C_{10}$ alkenylene group, or $C_2$-$C_{10}$ alkynylene group, which groups optionally comprise at least one heteroatom as chain member, or for an optionally at least monosubstituted five-membered to twelve-membered aryl residue or heteroaryl residue which may be attached via an optionally at least monosubstituted $C_1$-$C_{10}$ alkylene group, $C_2$-$C_{10}$ alkenylene group, or $C_2$-$C_{10}$ alkynylene group, optionally comprising at least one heteroatom as chain member,
preferably for a linear or branched $C_{1-}C_{10}$ alkyl residue, for a linear or branched $C_{2-}C_{10}$ alkenyl residue, or for a saturated or unsaturated, optionally at least monosubstituted five-membered or six-membered cycloaliphatic residue, optionally comprising at least one heteroatom as ring member, which cycloaliphatic residue may be attached via an optionally at least monosubstituted $C_{1-}C_5$ alkylene group or $C_{2-}C_5$ alkenylene group, which groups optionally comprise at least one heteroatom as chain member, or for a phenyl, 1-naphthyl, 2-naphthyl, 2-furanyl, 3-furanyl, 2-thiophenyl, or 3-thiophenyl residue, which may in each case be at least monosubstituted and/or may in each case be attached via an optionally at least monosubstituted $C_{1-}C_5$ alkylene group or $C_{2-}C_5$ alkenylene group, which groups optionally comprise at least one heteroatom as chain member,
particularly preferably for a linear or branched $C_{1-}C_5$ alkyl residue, a linear or branched $C_{2-}C_5$ alkenyl residue, for a saturated or unsaturated, optionally at least monosubstituted six-membered cycloaliphatic residue optionally comprising at least one heteroatom as ring member, which six-membered cycloaliphatic residue may be attached via a $C_{1-}C_3$ alkylene group, or for a phenyl, 1-naphthyl, 2-naphthyl, 2-furanyl, 3-furanyl, 2-thiophenyl or 3-thiophenyl residue, which may in each case be at least monosubstituted and/or may in each case be attached via an optionally at least monosubstituted $C_{1-}C_5$ alkylene group or $C_2$-$C_5$alkenylene group, which groups optionally comprise at least one oxygen atom as chain member,

**3.** Compounds according to claim 1 or claim 2, **characterized in that** $R^3$ stands for a hydrogen residue, for a linear or branched, saturated or unsaturated, optionally at least monosubstituted aliphatic $C_1$-$C_{10}$ residue, for a saturated or unsaturated, optionally at least monosubstituted three-membered to seven-membered cycloaliphatic residue optionally comprising at least one heteroatom as ring member, which cycloaliphatic residue may be attached via an optionally at least monosubstituted $C_{1-}C_{10}$ alkylene group, $C_{2-}C_{10}$ alkenylene group, or $C_2$-$C_{10}$ alkynylene group, which groups optionally comprise at least one heteroatom as chain member, or for an optionally at least monosubstituted five-membered to twelve-membered aryl residue or heteroaryl residue, which may be attached via an optionally at least monosubstituted $C_{1-}C_{10}$ alkylene group, $C_{2-}C_{10}$ alkenylene group, or $C_{2-}C_{10}$ alkynylene group, which groups optionally comprise at least one heteroatom as chain member,
preferably for a hydrogen residue, or for a linear or branched, optionally at least monosubstituted $C_{1-}C_{10}$ alkyl residue, or for a saturated or unsaturated, optionally at least monosubstituted five-membered, six-membered, or seven-membered cycloaliphatic residue optionally comprising at least one heteroatom as ring member, which cycloaliphatic residue may be attached via an optionally at least monosubstituted $C_{1-}C_{10}$ alkylene group, $C_{2-}C_{10}$ alkenylene group, or $C_{2-}C_{10}$ alkynylene group, which groups optionally comprise at least one heteroatom as chain member, or for an optionally at least monosubstituted five-membered to twelve-membered aryl residue or heteroaryl

residue, which may be attached via an optionally at least monosubstituted $C_1$-$C_{10}$ alkylene group, $C_2$-$C_{10}$ alkenylene group, or $C_2$-$C_{10}$ alkynylene group, which groups optionally comprise at least one heteroatom as chain member, particularly preferably for a hydrogen residue, or for a linear or branched, optionally at least monosubstituted $C_{1-3}$ alkyl residue, or for a cycloaliphatic residue attached via a $C_{1-3}$ alkylene group and selected from the group consisting of

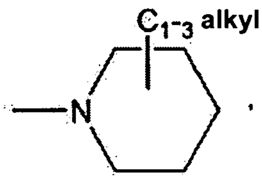, 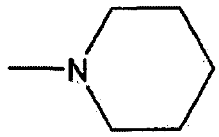, 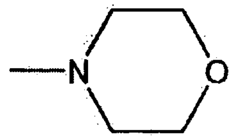,

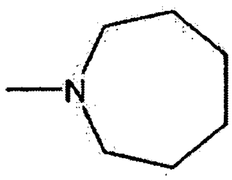, 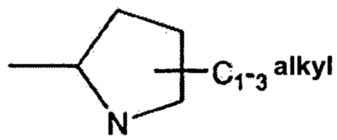, 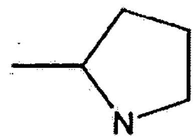,

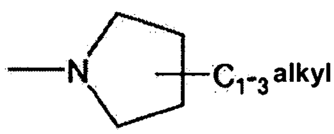 and 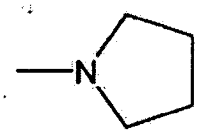,

or for a cycloaliphatic residue

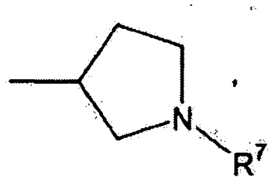,

in which the residue $R^7$ stands for a phenyl residue or benzofuranyl residue attached via a methylene group and optionally monosubstituted or polysubstituted, identically or differently, with a substituent selected from the group consisting of F, Cl, Br, $CF_3$, $CHF_2$, $CH_2F$, phenoxy, benzyloxy, phenyl, $C_{1-4}$ alkoxy or $C_{1-4}$ alkylthio,
or the residue $R^3$ stands for a phenyl residue optionally attached via a methylene group, which phenyl residue may be monosubstituted or polysubstituted, identically or differently, with a substituent selected from the group consisting of di-($C_{1-3}$)-alkylamino, $C_{1-3}$ methoxy, and morpholynyl.

4. Compounds according to any one of claims 1 to 3, **characterized in that** the residue $R^4$ stands for a hydrogen residue, or for a linear or branched, saturated or unsaturated, optionally at least monosubstituted aliphatic $C_1$-$C_{10}$ residue, or for a saturated or unsaturated, optionally at least monosubstituted three-membered to seven-membered cycloaliphatic residue which optionally comprises at least one heteroatom as ring member and which may be attached

via an optionally at least monosubstituted $C_1$-$C_{10}$ alkylene group, $C_2$-$C_{10}$ alkenylene group, or $C_2$-$C_{10}$ alkynylene group, which groups optionally comprise at least one heteroatom as chain member, or for an optionally at least monosubstituted five-membered to twelve-membered aryl residue or heteroaryl residue which may be attached via an optionally at least monosubstituted $C_1$-$C_{10}$ alkylene group, $C_2$-$C_{10}$ alkenylene group, or $C_2$-$C_{10}$ alkynylene group, which groups optionally comprise at least one heteroatom as chain member,

preferably for a hydrogen residue, or for a linear or branched, optionally at least monosubstituted $C_1$-$C_{10}$ alkyl residue, or for a saturated or unsaturated, optionally at least monosubstituted five-membered, six-membered, or seven-membered cycloaliphatic residue which optionally comprises at least one heteroatom as ring member and which may be attached via an optionally at least monosubstituted $C_{1-}C_{10}$ alkylene group, $C_{2-}C_{10}$ alkenylene group, or $C_{2-}C_{10}$ alkynylene group, which groups optionally comprise at least one heteroatom as chain member, or for an optionally at least monosubstituted five-membered to twelve-membered aryl residue or heteroaryl residue which may be attached via an optionally at least monosubstituted $C_{1-}C_{10}$ alkylene group, $C_{2-}C_{10}$ alkenylene group, or $C_{2-}C_{10}$ alkynylene group, which groups optionally comprise at least one heteroatom as chain member,

particularly preferably for a linear or branched, optionally at least monosubstituted $C_{1-3}$ alkyl residue, or for a cycloaliphatic residue attached via a $C_{1-3}$ alkylene group and selected from the group consisting of

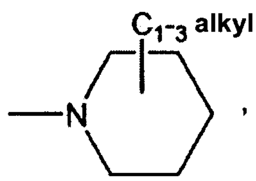 , 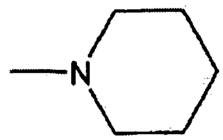 , 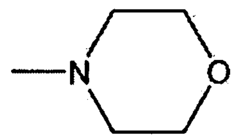 ,

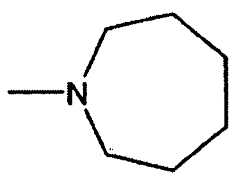 , 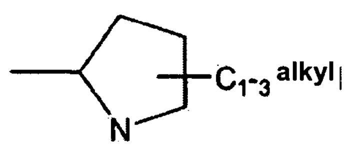 , 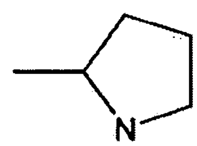 ,

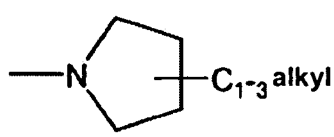 and 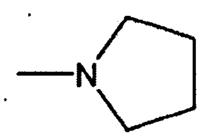 ,

or for a cycloaliphatic residue

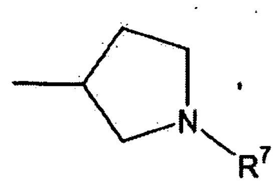 ,

in which the residue $R^7$ stands for a phenyl residue or benzofuranyl residue which is attached via a methylene group

and which may be monosubstituted or polysubstituted, identically or differently, with a substituent selected from the group consisting of F, Cl, Br, $CF_3$, $CHF_2$, $CH_2F$, phenoxy, benzyloxy, phenyl, $C_{1-4}$ alkoxy, or $C_{1-4}$ alkylthio, or the residue $R^4$ stands for a phenyl residue optionally attached via a methylene group, which phenyl residue may be monosubstituted or polysubstituted, identically or differently, with a substituent selected from the group consisting of di-($C_{1-3}$)-alkylamino, $C_{1-3}$ methoxy, and morpholynyl.

**5.** Compounds according to any one of claims 1 to 4, **characterized in that** $R^5$ and $R^6$ form, together with the nitrogen atom bridging them as ring member, a saturated, unsaturated or aromatic, optionally at least monosubstituted five-membered, six-membered or seven-membered heterocyclic residue optionally comprising at least one further heteroatom as ring member,
preferably form, together with the nitrogen atom bridging them as ring member, a residue selected from the group

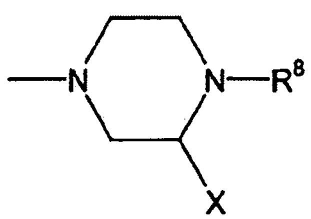 , 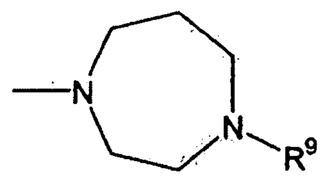

and

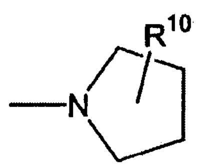

in which
X stands for hydrogen or a linear or optionally branched $C_{1-3}$ alkyl residue, and
$R^8$, $R^9$, and $R^{10}$ independently stand in each case for a linear or branched, saturated or unsaturated, optionally at least monosubstituted aliphatic $C_{1-6}$ residue, or for a saturated or unsaturated, optionally at least monosubstituted five-membered, six-membered or seven-membered cycloaliphatic residue optionally comprising at least one heteroatom as ring member and optionally attached via an optionally at least monosubstituted, $C_{1-6}$ alkylene group, $C_{2-6}$ alkenylene group or $C_{2-6}$ alkynylene group, which groups optionally comprise at least one heteroatom or optionally at least one carbonyl group (C=O) as chain member, which cycloaliphatic residue may be fused with an optionally at least monosubstituted, monocyclic or polycyclic ring system, or for an optionally at least monosubstituted five-membered or six-membered aryl residue or heteroaryl residue optionally attached via an optionally at least monosubstituted $C_{1-6}$ alkylene, $C_{2-6}$ alkenylene or $C_{2-6}$ alkynylene group, which groups optionally comprise at least one heteroatom as chain member, which aryl or heteroaryl residue may be fused with an optionally at least monosubstituted, monocyclic or polycyclic ring system.

**6.** Compounds according to claim 5, **characterized in that**
X stands for a hydrogen residue or a methyl residue,
$R^8$ stands for a $C_{1-3}$ alkyl residue optionally substituted with a di-($C_{1-3}$ alkyl)amino group, or for an optionally at least monosubstituted phenyl residue, an optionally at least monosubstituted naphthyl residue, an optionally at least monosubstituted pyridinyl residue, an optionally at least monosubstituted furanyl residue, an optionally at least monosubstituted thiophenyl residue, an optionally at least monosubstituted pyrrolidinyl residue, an optionally at least monosubstituted benzo[1,3]dioxolyl residue, or for an optionally at least monosubstituted benzofuranyl residue, wherein the cyclic residues may in each case be attached via a $C_{1-3}$ alkylene group, or a $C_{2-3}$ alkenylene group, which groups optionally comprise a carbonyl-(C=O) group as chain member and/or may be monosubstituted or polysubstituted, identically or differently, with a substituent selected from the group consisting of -(C=O)-$C_{1-3}$ alkyl, $C_{1-3}$ alkoxy, F, Cl, Br, -CN, $CF_3$, $CF_2H$, and $CFH_2$,
$R^9$ stands for a linear or optionally branched $C_{1-3}$ alkyl residue,
$R^{10}$ stands for a pyrrolidinyl residue attached via a $C_{1-2}$ alkylene group.

**7.** Compounds according to any one of claims 1 to 6, **characterized in that** $R^1$ stands for a group $-NR^3R^4$ or for a group $-NR^5R^6$, $R^2$ stands for a linear or branched $C_{1-5}$ alkyl residue, or for a linear or branched $C_{2-5}$ alkenyl residue, or for a cyclohexyl residue optionally attached via a $-(CH_2)$ group, for a 1-naphthyl residue or 2-naphthyl residue optionally attached via a $-(CH_2)$ group or $-(CH{=}CH)$ group, or for a 2-furanyl residue or 3-furanyl residue optionally attached via a $-(CH_2)$ group or $-(CH{=}CH)$ group, or for a 2-thienyl residue or 3-thienyl residue optionally attached via a $-(CH_2)$ group or $-(CH{=}CH)$ group, or for an unsubstituted or at least monosubstituted phenyl residue optionally attached via a $-(CH_2)$ group, $-(CH_2)_2$ group, $-C(H)(CH_3)$ group or $-(CH_2)-O$ group, the substituents thereof being preferably independently selected from the group consisting of -F, -Cl, -Br, $-OCH_3$, $-OC_2H_5$, $CH_3$, and $C_2H_5$, $R^3$ stands for a hydrogen residue or a methyl residue, $R^4$ stands for a linear or branched, optionally at least monosubstituted $C_{1-3}$ alkyl residue, or for a cycloaliphatic residue attached via a $C_{1-3}$ alkylene group selected from the group consisting of

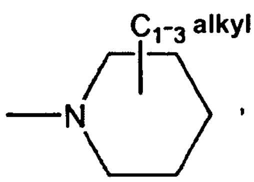 , 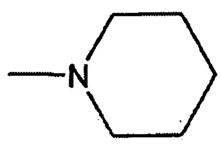 , 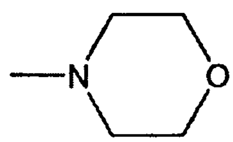 ,

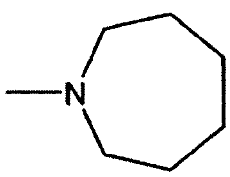 , 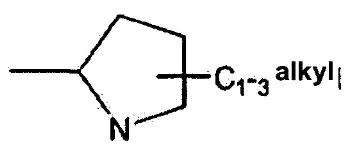 , 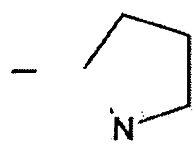 ,

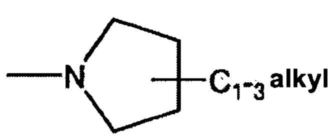 and 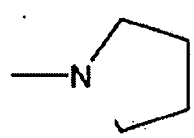 ,

or for a cycloaliphatic residue

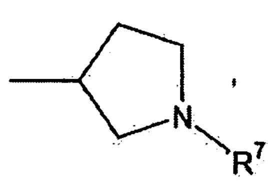 ,

wherein the residue $R^7$ stands for a phenyl residue or benzofuranyl residue attached via a methylene group and monosubstituted or polysubstituted, identically or differently, with a substituent selected from the group consisting of F, Cl, Br, $CF_3$, $CHF_2$, $CH_2F$, phenoxy, benzyloxy, phenyl, $C_{1-4}$ alkoxy and $C_{1-4}$ alkylthio, or the residue $R^4$ stands for a phenyl residue optionally attached via a methylene group, which phenyl residue may

be monosubstituted or polysubstituted, identically or differently, with a substituent selected from the group consisting of di-($C_{1-3}$)alkylamino, $C_{1-3}$ methoxy, and morpholinyl,
$R^5$ and $R^6$ form, together with the nitrogen atom bridging them, a residue selected from the group

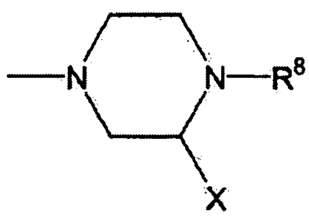 , 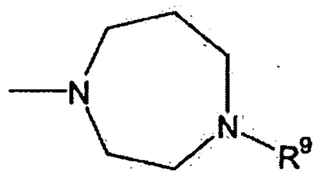

and

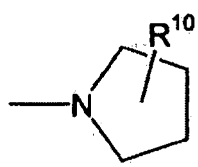

wherein
X stands for a hydrogen residue or a methyl group,
$R^8$ stands for a linear or branched $C_{1-3}$ alkyl residue optionally substituted with a dimethylamino group, an optionally at least monosubstituted phenyl residue, an optionally at least monosubstituted naphthyl residue, an optionally at least monosubstituted pyridinyl residue, an optionally at least monosubstituted furanyl residue, an optionally at least monosubstituted thiophenyl residue, an optionally at least monosubstituted pyrrolidinyl residue, an optionally at least monosubstituted benzo[1,3]dioxolyl residue, or for an optionally at least monosubstituted benzofuranyl residue, wherein the cyclic residues may in each case be attached via a $C_{1-3}$ alkylene group or a $C_{2-3}$ alkenylene group, which groups optionally comprise a carbonyl-(C=O) group as chain member and/or may be monosubstituted or polysubstituted, identically or differently, with a substituent selected from the group consisting of -(C=O)$C_{1-3}$ alkyl, $C_{1-3}$ alkoxy, F, Cl, Br, -CN, $CF_3$, $CF_2H$, and $CFH_2$,
$R^9$ stands for a methyl residue or ethyl residue, and
$R^{10}$ stands for a pyrrolidinyl residue attached via a -($CH_2$) group.

**8.** Compounds according to any one or more of claims 1 to 7, **characterized in that**

$R^1$ stands for a group -$NR^3R^4$ or for a group -$NR^5R^6$,
$R^2$ stands for a linear or branched, saturated or unsaturated, optionally substituted $C_{1-10}$ aliphatic residue; for an unsaturated or saturated, optionally substituted 3-membered, 4-membered, 5-membered, 6- membered, 7-membered, 8-membered, or 9-membered cycloaliphatic residue, which may be attached via a linear or branched, optionally substituted $C_{1-5}$ alkylene group, $C_{2-5}$ alkenylene group or $C_{2-5}$ alkynylene group, each of these groups optionally comprising 1 or 2 heteroatom(s) as chain member(s),
or for an optionally substituted 5-membered to 14- membered aryl residue or heteroaryl residue, which may be attached via a linear or branched, optionally substituted $C_{1-5}$ alkylene group, $C_{2-5}$ alkenylene group, or $C_{2-5}$ alkynylene group, which groups optionally comprise 1 or 2 heteroatom(s) as chain member(s);
$R^3$ stands for a hydrogen residue;
or for a linear or branched, saturated or unsaturated, optionally substituted $C_{1-10}$ aliphatic residue;
or for an unsaturated or saturated, optionally substituted three-membered, four-membered, five- membered, six-membered, seven-membered, eight- membered, or nine-membered cycloaliphatic residue, which may be attached via a linear or branched, optionally substituted $C_{1-5}$ alkylene group, $C_{2-5}$ alkenylene group or $C_{2-5}$ alkynylene group, which groups optionally comprise 1 or 2 heteroatom(s) as chain member(s),
or for an optionally substituted five-membered to fourteen-membered aryl residue or heteroaryl residue, which may be attached via a linear or branched, optionally substituted $C_{1-5}$ alkylene group, $C_{2-5}$ alkenylene group or $C_{2-5}$ alkynylene group, which groups optionally comprise 1 or 2 heteroatom(s) as chain member(s);

$R^4$ stands for a hydrogen residue;
or for a linear or branched, saturated or unsaturated, optionally substituted $C_{1-10}$ aliphatic residue;
or for an unsaturated or saturated, optionally substituted three-membered, four-membered, five- membered, six-membered, seven-membered, eight- membered, or nine-membered cycloaliphatic residue, which may be attached via a linear or branched, optionally substituted $C_{1-5}$ alkylene group, $C_{2-5}$ alkenylene group or $C_{2-5}$ alkynylene group, which groups optionally comprise 1 or 2 heteroatom(s) as chain member(s),
or for an optionally substituted five-membered to fourteen-membered aryl residue or heteroaryl residue, which may be attached via a linear or branched, optionally substituted $C_{1-5}$ alkylene group, $C_{2-5}$ alkenylene group or $C_{2-5}$ alkynylene group, which groups optionally comprise 1 or 2 heteroatom(s) as chain member(s);
$R^5$ and $R^6$ form, together with the nitrogen atom connecting them as ring member, a saturated or unsaturated, optionally substituted four-membered, five-membered, six-membered, seven-membered, eight-membered or nine- membered heterocycloaliphatic residue, wherein in each case the heterocycloaliphatic residue may be substituted with a residue $R^8$ and optionally with a residue X or a residue $R^9$ or a residue $R^{10}$ and/or may have further 1, 2, or 3 heteroatom(s) independently selected from the group consisting of oxygen, nitrogen, and sulfur as ring member(s);
X stands for a linear or branched, saturated or unsaturated $C_{1-10}$ aliphatic residue;
$R^8$, $R^9$, and $R^{10}$ independently stand in each case for a linear or branched, saturated or unsaturated, optionally substituted $C_{1-10}$ aliphatic residue,
an unsaturated or saturated, optionally substituted three-membered, four-membered, five-membered, six- membered, seven-membered, eight-membered or nine- membered cycloaliphatic residue, which residue may be attached via a linear or branched, optionally substituted $C_{1-5}$ alkylene group, $C_{2-5}$ alkenylene group or $C_{2-5}$ alkynylene group, which groups optionally comprise 1 or 2 heteroatom(s) as chain member(s) and/or optionally comprise a carbonyl-(C=O) group as chain member, and/or which residue may be fused with a saturated, unsaturated or aromatic, optionally substituted mono or polycyclic ring system,
or for an optionally substituted five-membered to fourteen-membered aryl residue or heteroaryl residue, which residue may be attached via a linear or branched, optionally substituted $C_{1-5}$ alkylene group, $C_{2-5}$ alkenylene group or $C_{2-5}$ alkynylene group, which groups optionally comprise 1 or 2 heteroatom(s) as chain member(s) and/or optionally comprise a carbonyl- (C=O) group as chain member, and/or which residue may be fused with a saturated or unsaturated, optionally substituted monocyclic or polycyclic ring system;

wherein
the aforementioned $C_{1-10}$ aliphatic residues may in each case be substituted with 1, 2, 3, 4, or 5 substituents independently selected from the group consisting of F, Cl, Br, -CN, $-NO_2$, hydroxy, $C_{1-6}$ alkoxy, $-NH_2$, -NH-($C_{1-3}$ alkyl),- N($C_{1-3}$ alkyl)($C_{1-3}$ alkyl), -NH-phenyl, -N($C_{1-3}$ alkyl)-phenyl, and $-N(phenyl)_2$;
the aforementioned cycloaliphatic residues may in each case be substituted with 1, 2, 3, 4, or 5 substituents independently selected from the group consisting of F, Cl, Br, -CN, $-CF_3$, $-CHF_2$, $-CH_2F$, hydroxy, $-C_{1-6}$ alkoxy, $-NH_2$, -NH-($C_{1-3}$ alkyl), -N($C_{1-3}$ alkyl) ($C_{1-3}$ alkyl), -NH-phenyl, -N($C_{1-3}$ alkyl)-phenyl, $-N(phenyl)_2$, SH, $-C_{1-6}$ alkylthio, $-C_{1-6}$ alkyl, $-(CH_2)$benzo[b]furanyl, phenoxy, benzyloxy, phenyl, and benzyl, wherein in each case the cyclic moiety of the residues phenoxy, benzyloxy, phenyl, $-(CH_2)$-benzo[b]furanyl and benzyl may be substituted with 1, 2, 3, 4, or 5 substituents independently selected from the group consisting of F, Cl, Br, $-C_{1-4}$ alkyl, $-CF_3$, $-CHF_2$, $-CH_2F$, $-C_{1-4}$ alkoxy, $-C_{1-4}$ alkylthio, phenoxy, phenyl, and benzyloxy,
and the aforementioned cycloaliphatic residues may in each case comprise 1, 2, or 3 heteroatom(s) independently selected from the group consisting of oxygen, nitrogen, and sulfur;
the aforementioned $C_{1-5}$ alkylene groups, $C_{2-5}$ alkenylene groups or $C_{2-5}$ alkynylene groups may in each case be substituted with 1, 2, 3, 4, or 5 substituents independently selected from the group consisting of F, Cl, Br, -CN, $-NO_2$, hydroxy, $C_{1-6}$ alkoxy, $-NH_2$, -NH-($C_{1-3}$ alkyl), -N($C_{1-3}$ alkyl)($C_{1-3}$ alkyl), -NH-phenyl, -N($C_{1-3}$ alkyl)-phenyl, $N(phenyl)_2$, and phenyl, and
the aforementioned $C_{1-5}$ alkylene groups, $C_{2-5}$ alkenylene groups or $C_{2-5}$ alkynylene groups may in each case comprise 1 or 2 heteroatom(s) independently selected from the group consisting of oxygen, nitrogen, and sulfur;
the aforementioned aryl residues or heteroaryl residues may in each case be substituted with 1, 2, 3, 4, or 5 substituents independently selected from the group consisting of F, Cl, Br, -CN, $-CF_3$, $-CHF_2$, $-CH_2F$, hydroxy, $-C_{1-6}$ alkoxy, $(C=O)-CH_3$, $-O-CF_3$, $-S-CF_3$, SH, $-C_{1-6}$ alkylthio, $-C_{1-6}$ alkyl, $-NH_2$, -NH-($C_{1-3}$ alkyl), -N($C_{1-3}$ alkyl)($C_{1-3}$ alkyl), -NH-phenyl, -N($C_{1-3}$ alkyl)-phenyl, $N(phenyl)_2$, phenoxy, benzyloxy, phenyl, benzyl, and morpholinyl, wherein in each case the cyclic moiety of the residues phenoxy, benzyloxy, phenyl, and benzyl may be substituted with 1, 2, 3, 4, or 5 substituents independently selected from the group consisting of F, Cl, Br, hydroxy, $-CF_3$, $-SF_5$, -CN, $-NO_2$, $-C_{1-6}$ alkoxy, $-O-CF_3$, $-S-CF_3$, phenyl, and benzyloxy,
and the aforementioned heteroaryl residues may in each case comprise 1, 2, 3, 4, or 5 heteroatom(s) as ring member (s) independently selected from the group consisting of oxygen, nitrogen, and sulfur;

and the rings of the aforementioned monocyclic or polycyclic ring systems may optionally in each case be substituted with 1, 2, 3, 4, or 5 substituents independently selected from the group consisting of F, Cl, Br, -CN, $-CF_3$, $-CHF_2$, $-CH_2F$, hydroxy, $-C_{1-6}$ alkoxy, $-O-CF_3$, $-S-CF_3$, SH, $-C_{1-6}$ alkylthio, $-C_{1-6}$ alkyl, $-NH_2$, $-NH-(C_{1-3}$ alkyl), $-N(C_{1-3}$ alkyl) $(C_{1-3}$ alkyl), -NH-phenyl, $-N(C_{1-3}$ alkyl)-phenyl, $N(phenyl)_2$, phenoxy, benzyloxy, phenyl, benzyl, and morpholinyl, wherein in each case the cyclic moiety of the residues phenoxy, benzyloxy, phenyl, and benzyl may be substituted with 1, 2, 3, 4, or 5 substituents independently selected from the group consisting of F, Cl, Br, hydroxy, $-CF_3$, $-SF_5$, -CN, $-NO_2$, $-C_{1-6}$ alkoxy, $-O-CF_3$, $-S-CF_3$, phenyl, and benzyloxy,
and the rings of the aforementioned monocyclic or polycyclic ring systems are each five-membered, six-membered, or seven-membered rings and may in each case comprise 1, 2, 3, 4, or 5 heteroatom(s) as ring member(s) which are independently selected from the group consisting of oxygen, nitrogen, and sulfur;
in each case optionally in the form of the pure stereoisomers thereof, particularly enantiomers or diastereoisomers, the racemates thereof or in the form of a mixture of stereoisomers, particularly the enantiomers and/or diastereoisomers, in any desired mixture ratio, or in each case in the form of corresponding salts, or in each case in the form of corresponding solvates.

**9.** Compounds according to any one or more of claims 1 to 8, **characterized in that** the residue
$R^2$ stands for an alkyl residue selected from the group consisting of methyl, ethyl, n-propyl, isopropyl, n-butyl, sec-butyl, isobutyl, tert-butyl, n-pentyl, sec-pentyl, (1,1)-dimethylpropyl, and n-hexyl, wherein the alkyl residue may in each case be substituted with 1, 2, 3, 4, or 5 substituents independently selected from the group consisting of F, Cl, Br, -CN, $-NO_2$, hydroxy, $C_{1-6}$ alkoxy, $-NH_2$, $-NH-(C_{1-3}$ alkyl), $-N(C_{1-3}$ alkyl) $(C_{1-3}$ alkyl), -NH-phenyl, $-N(C_{1-3}$ alkyl)-phenyl, and $-N(phenyl)_2$;
or for an alkenyl residue selected from the group consisting of vinyl, 1-propenyl, 2-propenyl, 1-butenyl, 2-butenyl, 3-butenyl, 1-pentenyl, 2-pentenyl, and pent-1,3-dienyl, wherein the alkenyl residue may in each case be substituted with 1, 2, 3, 4, or 5 substituents independently selected from the group consisting of F, Cl, Br, -CN, $-NO_2$, hydroxy, $C_{1-6}$ alkoxy, $-NH_2$, $-NH-(C_{1-3}$ alkyl), $-N(C_{1-3}$ alkyl)$(C_{1-3}$ alkyl), -NH-phenyl, $-N(C_{1-3}$ alkyl)-phenyl, and $-N(phenyl)_2$;
or for a (hetero)cycloaliphatic residue selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, and dithiolanyl, wherein the (hetero)cycloaliphatic residue may in each case be attached via a linear or branched, unsubstituted $C_{1-5}$ alkylene group and/or be substituted with 1, 2, 3, 4, or 5 substituents independently selected from the group consisting of F, Cl, Br, -CN, $-CF_3$, $-CHF_2$, $-CH_2F$, hydroxy, $-C_{1-6}$ alkoxy, $-NH_2$, $-NH-(C_{1-3}$ alkyl), $-N(C_{1-3}$ alkyl)$(C_{1-3}$ alkyl), -NH-phenyl, $-N(C_{1-3}$ alkyl)-phenyl, $-N(phenyl)_2$, SH, $-C_{1-6}$ alkylthio, $-C_{1-6}$ alkyl, $-(CH_2)$-benzo[b]furanyl, phenoxy, benzyloxy, phenyl, and benzyl, wherein in each case the cyclic moiety of the residues phenoxy, benzyloxy, phenyl, $-(CH_2)$-benzo[b]furanyl, and benzyl may be substituted with 1, 2, 3, 4, or 5 substituents independently selected from the group consisting of F, Cl, Br, $-C_{1-4}$ alkyl, $-CF_3$, $-CHF_2$, $-CH_2F$, $-C_{1-4}$ alkoxy, $-C_{1-4}$ alkylthio, phenoxy, phenyl, and benzyloxy;
or for a residue selected from the group consisting of phenyl, naphthyl, thiophenyl, furanyl, pyrrolyl, pyrazolyl, pyrazinyl, pyranyl, triazolyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinazolinyl, quinolinyl, and isoquinolinyl, wherein the residue may in each case be attached via a linear or branched, unsubstituted, $C_{1-5}$ alkylene group or $C_{2-5}$ alkenylene group, which groups optionally comprise an oxygen atom as chain member, and/or may be substituted with 1, 2, 3, 4, or 5 substituents independently selected from the group consisting of F, Cl, Br, -CN, $-CF_3$, $-CHF_2$, $-CH_2F$, hydroxy, $-C_{1-6}$ alkoxy, $-O-CF_3$, $-S-CF_3$, SH, $-C_{1-6}$ alkylthio, $-C_{1-6}$ alkyl, $-NH_2$, $-NH-(C_{1-3}$ alkyl), $-N(C_{1-3}$ alkyl) $(C_{1-3}$ alkyl), -NH-phenyl, $-N(C_{1-3}$ alkyl)-phenyl, $N(phenyl)_2$, phenoxy, benzyloxy, phenyl, benzyl, and morpholinyl, wherein in each case the cyclic moiety of the residues phenoxy, benzyloxy, phenyl, and benzyl may be substituted with 1, 2, 3, 4, or 5 substituents independently selected from the group consisting of F, Cl, Br, hydroxy, $-CF_3$, $-SF_5$, -CN, $-NO_2$, $-C_{1-6}$ alkoxy, $-O-CF_3$, $-S-CF_3$, phenyl, and benzyloxy;
preferably $R^2$ stands
for an alkyl residue selected from the group consisting of methyl, ethyl, n-propyl, isopropyl, n-butyl, sec-butyl, isobutyl, tert-butyl, n-pentyl, sec-pentyl, (1,1)-dimethylpropyl, and n-hexyl;
or for an alkenyl residue selected from the group consisting of vinyl, 1-propenyl, 2-propenyl, 1-butenyl, 2-butenyl, 3-butenyl, 1-pentenyl, 2-pentenyl, and pent-1,3-dienyl;
or for a cycloaliphatic residue selected from the group consisting of cyclopropyl, cyclobutyl, cyclopentyl, cyclohexyl, cycloheptyl, cyclopentenyl, cyclohexenyl, and cycloheptenyl, which may be attached via a $-(CH_2)$ group, $-(CH_2)_2$ group, $-CH(CH_3)$ group, or $-(CH_2)_3$ group and/or may be substituted with 1, 2, 3, 4, or 5 substituents independently selected from the group consisting of F, Cl, Br, -CN, $-CF_3$, $-CHF_2$, $-CH_2F$, hydroxy, $-O-CH_3$, $-O-C_2H_5$, $-0-C_3H_7$, $-NH_2$, $-NH-CH_3$, $-NH-C_2H_5$, $-N-(CH_3)_2$, $-N(C_2H_5)_2$, $-N(CH_3)(C_2H_5)$, -NH-phenyl, $-N(CH_3)$-phenyl, $-N(C_2H_5)$-phenyl, $-N(phenyl)_2$, SH, $-S-CH_3$, $-S-C_2H_5$, $-S-C_3H_7$, $-S-C(CH_3)_3$, methyl, ethyl, n-propyl, isopropyl, n-butyl, sec-butyl, and

tert-butyl;
or for a residue selected from the group consisting of phenyl, 1-naphthyl, 2-naphthyl, 2-furanyl, 3-furanyl, 2-thiophenyl, or 3-thiophenyl, which may be attached via a $-(CH_2)$ group, $-(CH_2)_2$ group, $-(CH_2)$-O group, $-CH(CH_3)$ group, -(CH=CH) group, or $-(CH_2)_3$ group and/or may be substituted with 1, 2, 3, 4, or 5 substituents independently selected from the group consisting of F, Cl, Br, -CN, $-CF_3$, $-CHF_2$, $-CH_2F$, hydroxy, $-O-CH_3$, $-O-C_2H_5$, $-O-C_3H_7$, $-O-CF_3$, $-S-CF_3$, -SH, $-S-CH_3$, $-S-C_2H_5$, $-S-C_3H_7$, $-S-C(CH_3)_3$, methyl, ethyl, n-propyl, isopropyl, n-butyl, sec-butyl, tert-butyl, $-NH_2$, $-NH-CH_3$, $-NH-C_2H_5$, $-N-(CH_3)_2$, $-N(C_2H_5)_2$, $-N(CH_3)(C_2H_5)$, -NH-phenyl, $-N(CH_3)$-phenyl, $-N(C_2H_5)$-phenyl, $-N(phenyl)_2$, phenoxy, benzyloxy, phenyl, benzyl, and morpholinyl;
particularly preferably $R^2$ stands
for an alkyl residue selected from the group consisting of n-propyl, isopropyl, n-butyl, sec-butyl, isobutyl, tert-butyl, n-pentyl, sec-pentyl, and (1,1)-dimethylpropyl;
or for an alkenyl residue selected from the group consisting of 1-pentenyl, 2-pentenyl and pent-1,3-dienyl;
or for a cycloaliphatic residue selected from the group consisting of cyclopentyl, cyclohexyl, and cycloheptyl, which is attached via a $-(CH_2)$ group;
or for a residue selected from the group consisting of phenyl, 1-naphthyl, 2-naphthyl, 2-furanyl, 3-furanyl, 2-thiophenyl, or 3-thiophenyl, which may be attached via a $-(CH_2)$ group, $-(CH_2)_2$ group, $-(CH_2)$-O group, $-CH(CH_3)$ group, -(CH=CH) group, or $-(CH_2)_3$ group and/or may be substituted with 1, 2, or 3 substituents independently selected from the group consisting of F, Cl, Br, $-O-CH_3$, $-O-C_2H_5$, methyl, ethyl, n-propyl, and isopropyl.

**10.** Compounds according to any one or more of claims 1 to 9, **characterized in that** the residue
$R^3$ stands for a hydrogen residue;
or for an alkyl residue selected from the group consisting of methyl, ethyl, n-propyl, isopropyl, n-butyl, sec-butyl, isobutyl, tert-butyl, n-pentyl, sec-pentyl, (1,1)-dimethylpropyl, and n-hexyl, wherein the alkyl residue may in each case be substituted with 1, 2, 3, 4, or 5 substituents independently selected from the group consisting of F, Cl, Br, -CN, $-NO_2$, hydroxy, $C_{1-6}$ alkoxy,- $NH_2$, -NH-($C_{1-3}$ alkyl), -N($C_{1-3}$ alkyl)($C_{1-3}$ alkyl), -NH-phenyl, -N($C_{1-3}$ alkyl)-phenyl, and $-N(phenyl)_2$;
or for an alkenyl residue selected from the group consisting of vinyl, 1-propenyl, 2-propenyl, 1-butenyl, 2-butenyl, 3-butenyl, 1-pentenyl, 2-pentenyl, and pent-1,3-dienyl, wherein the alkenyl residue may in each case be substituted with 1, 2, 3, 4, or 5 substituents independently selected from the group consisting of F, Cl, Br, -CN, $-NO_2$, hydroxy, $C_{1-6}$ alkoxy, $-NH_2$, -NH-($C_{1-3}$ alkyl), -N($C_{1-3}$ alkyl) ($C_{1-3}$ alkyl), -NH-phenyl, -N($C_{1-3}$ alkyl)-phenyl, and $-N(phenyl)_2$;;
or for a (hetero)cycloaliphatic residue selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, and dithiolanyl, wherein the (hetero)cycloaliphatic residue may in each case be attached via a linear or branched, unsubstituted $C_{1-5}$ alkylene group and/or may be substituted with 1, 2, 3, 4, or 5 substituents independently selected from the group consisting of F, Cl, Br, -CN, $-CF_3$, $-CHF_2$, $-CH_2F$, hydroxy, $-C_{1-6}$ alkoxy, $-NH_2$, -NH-($C_{1-3}$ alkyl), -N ($C_{1-3}$ alkyl)($C_{1-3}$ alkyl), -NH-phenyl, -N($C_{1-3}$ alkyl)-phenyl, $-N(phenyl)_2$, -SH, $-C_{1-6}$ alkylthio, $-C_{1-6}$ alkyl, $-(CH_2)$-benzo [b]furanyl, phenoxy, benzyloxy, phenyl, and benzyl, wherein in each case the cyclic moiety of the residues phenoxy, benzyloxy, phenyl, $-(CH_2)$-benzo[b]furanyl and benzyl may be substituted with 1, 2, 3, 4, or 5 substituents independently selected from the group consisting of F, Cl, Br, $-C_{1-4}$ alkyl, $-CF_3$, $-CHF_2$,- $CH_2F$, $-C_{1-4}$ alkoxy, $-C_{1-4}$ alkylthio, phenoxy, phenyl, and benzyloxy;
or for a residue selected from the group consisting of phenyl, naphthyl, thiophenyl, furanyl, pyrrolyl, pyrazolyl, pyrazinyl, pyranyl, triazolyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinazolinyl, quinolinyl, and isoquinolinyl, wherein the residue may in each case be attached via a linear or branched, unsubstituted, $C_{1-5}$ alkylene group or $C_{2-5}$ alkenylene group, which groups optionally comprise an oxygen atom as chain member, and/or said residue may in each case be substituted with 1, 2, 3, 4, or 5 substituents independently selected from the group consisting of F, Cl, Br, -CN, $-CF_3$, $-CHF_2$, $-CH_2F$, hydroxy, $-C_{1-6}$ alkoxy, $-O-CF_3$, $-S-CF_3$, -SH, $-C_{1-6}$ alkylthio, $-C_{1-6}$ alkyl, $-NH_2$, -NH-($C_{1-3}$ alkyl), -N($C_{1-3}$ alkyl)($C_{1-3}$ alkyl), -NH-phenyl, -N($C_{1-3}$ alkyl)-phenyl, $N(phenyl)_2$, phenoxy, benzyloxy, phenyl, benzyl, and morpholinyl, wherein in each case the cyclic moiety of the residues phenoxy, benzyloxy, phenyl, and benzyl may be substituted with 1, 2, 3, 4, or 5 substituents independently selected from the group consisting of F, Cl, Br, hydroxy, $-CF_3$, $-SF_5$, -CN, $-NO_2$, $-C_{1-6}$ alkoxy, $-O-CF_3$, $-S-CF_3$, phenyl, and benzyloxy;
preferably $R^3$ stands
for a hydrogen residue;
or for an alkyl residue selected from the group consisting of methyl, ethyl, n-propyl, n-butyl and n-pentyl, wherein the alkyl residue may in each case be substituted with 1 or 2 substituents independently selected from the group consisting of $NH_2$, $-NH-CH_3$, $-NH-C_2H_5$, $-N-(CH_3)_2$, $-N(C_2H_5)_2$, $-N(CH_3)(C_2H_5)$, -NH-phenyl, $-N(CH_3)$-phenyl, -N ($C_2H_5$)-phenyl, and $-N(phenyl)_2$;

or for a residue selected from the group consisting of

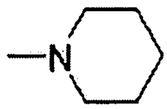 , 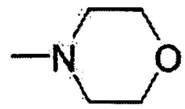

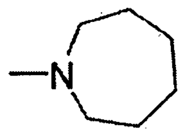 , 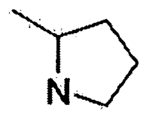

and

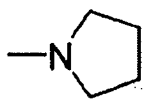 ,

which may be attached via a $-(CH_2)$ group, $-(CH_2)_2$ group, $-(CH_2)$-O group, $-CH(CH_3)$ group, -(CH=CH) group, or $-(CH_2)_3$ group and/or may be substituted with 1, 2, or 3 substituents independently selected from the group consisting of methyl, ethyl, and n-propyl;
or for the following residue

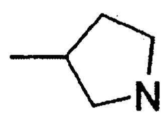 ,

which may be substituted with a substituent selected from the group consisting of $-(CH_2)$-benzo[b]furanyl and benzyl, wherein in each case the cyclic moiety of the residues $-(CH_2)$-benzo[b]furanyl and benzyl may be substituted with 1, 2, 3, 4 or 5 substituents independently selected from the group consisting of F, Cl, Br, $-C_{1-4}$ alkyl, $-CF_3$, $-CHF_2$, $-CH_2F$, $-C_{1-4}$ alkoxy, $-C_{1-4}$ alkylthio, phenoxy, phenyl, and benzyloxy;
or for a residue selected from the group consisting of phenyl and naphthyl, wherein the residue may in each case be attached via a $-(CH_2)$ group, $-(CH_2)_2$ group, or $-(CH_2)_3$ group and/or may be substituted with 1, 2, or 3 substituents independently selected from the group consisting of $-C_{1-6}$ alkoxy, $-C_{1-6}$ alkyl, $-NH_2$, $-NH-(C_{1-3}$ alkyl), $-N(C_{1-3}$ alkyl) $(C_{1-3}$ alkyl), -NH-phenyl, $-N(C_{1-3}$ alkyl)-phenyl, $N(phenyl)_2$, and morpholinyl;
particularly preferably $R^3$ stands
for a hydrogen residue;
or for a residue selected from the group consisting of methyl, ethyl, n-propyl, $-(CH_2)-N(CH_3)_2$, $-(CH_2)-(CH_2)-N(CH_3)_2$, $-(CH_2)-(CH_2)-(CH_2)-N(CH_3)_2$, $-(CH_2)-(CH_2)-N(C_2H_5)_2$, $-(CH_2)-(CH_2)-(CH_2)-N(C_2H_5)_2$, $-(CH_2)-(CH_2)-N(CH_3)$ (phenyl), and $-(CH_2)-(CH_2)-(CH_2)-N(CH_3)$ (phenyl);
or for a residue selected from the group consisting of

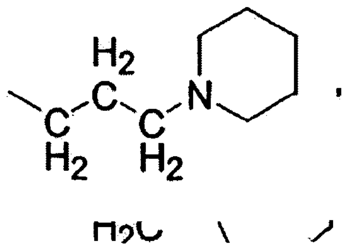, 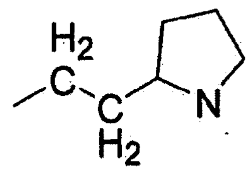, 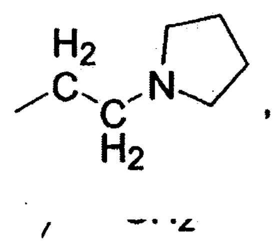, ,

which may be substituted with a methyl residue;
or for the following residue

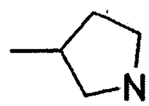,

which may be substituted on the nitrogen atom with a substituent selected from the group consisting of -$(CH_2)$-benzo[b]furanyl and benzyl, wherein in each case the cyclic moiety of the residues -$(CH_2)$benzo[b]furanyl and benzyl may be substituted with 1, 2, or 3 substituents independently selected from the group consisting of F, Cl, Br, -$CF_3$, -0-$CH_3$, -O-$C_2H_5$, -O-$C_3H_7$, -S-$CH_3$, -S-$C_2H_5$, -S-$C_3H_7$, -S-$C(CH_3)_3$, methyl, ethyl, n-propyl, isopropyl, n-butyl, phenoxy and benzyloxy;
or for a residue selected from the group consisting of phenyl and benzyl, wherein the cyclic moiety of the residues phenyl and benzyl may be substituted with 1, 2, or 3 substituents independently selected from the group consisting of -O-$CH_3$, -O-$C_2H_5$, -O-$C_3H_7$, -N-$(CH_3)_2$, -$N(C_2H_5)_2$ and morpholinyl;
very particularly preferably
$R^3$ stands for a hydrogen residue
or for a methyl residue.

**11.** Compounds according to any one or more of claims 1 to 10, **characterized in that** the residue
$R^4$ stands for a hydrogen residue;
or for an alkyl residue selected from the group consisting of methyl, ethyl, n-propyl, isopropyl, n-butyl, sec-butyl, isobutyl, tert-butyl, n-pentyl, sec-pentyl, (1,1)-dimethylpropyl, and n-hexyl, wherein the alkyl residue may in each case be substituted with 1, 2, 3, 4 or 5 substituents independently selected from the group consisting of F, Cl, Br, -CN, -$NO_2$, hydroxy, $C_{1-6}$ alkoxy, -$NH_2$, -NH-($C_{1-3}$ alkyl), -N($C_{1-3}$ alkyl)($C_{1-3}$ alkyl), -NH-phenyl, -N($C_{1-3}$ alkyl)-phenyl, and -N(phenyl)$_2$;
or for an alkenyl residue selected from the group consisting of vinyl, 1-propenyl, 2-propenyl, 1-butenyl, 2-butenyl, 3-butenyl, 1-pentenyl, 2-pentenyl, and pent-1,3-dienyl, wherein the alkenyl residue may in each case be substituted with 1, 2, 3, 4, or 5 substituents independently selected from the group consisting of F, Cl, Br, -CN, -$NO_2$, hydroxy, $C_{1-6}$ alkoxy, -$NH_2$, -NH-($C_{1-3}$ alkyl), -N($C_{1-3}$ alkyl)($C_{1-3}$ alkyl), -NH-phenyl, -N($C_{1-3}$ alkyl)-phenyl, and -N(phenyl)$_2$;
or for a (hetero)cycloaliphatic residue selected from the group consisting of cyclopropyl, cyclobutyl, cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, and dithiolanyl, wherein the (hetero)cycloaliphatic residue may in each case be attached via a linear or branched, unsubstituted $C_{1-5}$ alkylene group and/or may be substituted with 1, 2, 3, 4, or 5 substituents independently selected from the group consisting of F, Cl, Br, -CN, -$CF_3$, -$CHF_2$, -$CH_2F$, hydroxy, -$C_{1-6}$ alkoxy, -$NH_2$, -NH-($C_{1-3}$ alkyl), -N($C_{1-3}$ alkyl)($C_{1-3}$ alkyl), -NH-phenyl, -N($C_{1-3}$ alkyl)-phenyl, -N(phenyl)$_2$, -SH, -$C_{1-6}$ alkylthio, -$C_{1-6}$ alkyl, -$(CH_2)$-benzo[b]furanyl, phenoxy, benzyloxy, phenyl, and benzyl, wherein in each case the cyclic moiety of the residues phenoxy, benzyloxy, phenyl, -$(CH_2)$-benzo[b]furanyl, and benzyl may be substituted with 1, 2, 3, 4, or 5 substituents independently selected from the group consisting of F, Cl, Br, -$C_{1-4}$ alkyl, -$CF_3$, -$CHF_2$, -$CH_2F$, -$C_{1-4}$ alkoxy, -$C_{1-4}$ alkylthio, phenoxy, phenyl, and benzyloxy;
or for a residue selected from the group consisting of phenyl, naphthyl, thiophenyl, furanyl, pyrrolyl, pyrazolyl, pyrazinyl, pyranyl, triazolyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinazolinyl, quinolinyl, and isoquinolinyl, wherein the residue may in each case be attached via a linear or branched, unsubstituted $C_{1-5}$ alkylene group or $C_{2-5}$

alkenylene group, which groups optionally comprise an oxygen atom as chain member, and/or said residue may be substituted with 1, 2, 3, 4, or 5 substituents independently selected from the group consisting of F, Cl, Br, -CN, $-CF_3$, $-CHF_2$, $-CH_2F$, hydroxy, $-C_{1-6}$ alkoxy, $-O-CF_3$, $-S-CF_3$, -SH, $-C_{1-6}$ alkylthio, $-C_{1-6}$ alkyl, $-NH_2$, $-NH-(C_{1-3}$ alkyl), $-N(C_{1-3}$ alkyl)($C_{1-3}$ alkyl), -NH-phenyl, $-N(C_{1-3}$ alkyl)-phenyl, $N(phenyl)_2$, phenoxy, benzyloxy, phenyl, benzyl, and morpholinyl, wherein in each case the cyclic moiety of the residues phenoxy, benzyloxy, phenyl, and benzyl may be substituted with 1, 2, 3, 4, or 5 substituents independently selected from the group consisting of F, Cl, Br, hydroxy, $-CF_3$, $-SF_5$, -CN, $-NO_2$, $-C_{1-6}$ alkoxy, $-O-CF_3$, $-S-CF_3$, phenyl, and benzyloxy;
preferably $R^4$ stands
for a hydrogen residue;
or for an alkyl residue selected from the group consisting of methyl, ethyl, n-propyl, n-butyl, and n-pentyl, wherein the alkyl residue may n each case be substituted with 1 or 2 substituents independently selected from the group consisting of $NH_2$, $-NH-CH_3$, $-NH-C_2H_5$, $-N-(CH_3)_2$, $-N(C_2H_5)_2$, $-N(CH_3)(C_2H_5)$, -NH-phenyl, $-N(CH_3)$-phenyl, -N ($C_2H_5$)-phenyl, and $-N(phenyl)_2$,
or for a residue selected from the group consisting of

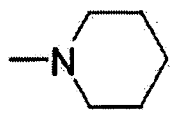 , 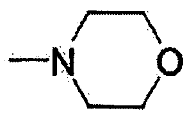 ,

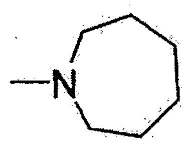 , 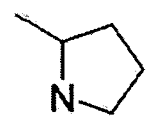

and

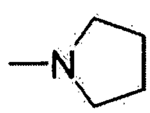 ,

which may be attached via a - ($CH_2$) group, $-(CH_2)_2$ group, $-(CH_2)-O$ group, $-CH(CH_3)$ group, -(CH=CH) group, or $-(CH_2)_3$ group and/or may be substituted with 1, 2, or 3 substituents independently selected from the group consisting of methyl, ethyl, and n-propyl;
or for the following residue

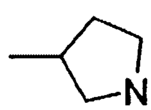 ,

which may be substituted with a substituent selected from the group consisting of $-(CH_2)$-benzo[b]furanyl and benzyl, wherein in each case the cyclic moiety of the residues $-(CH_2)$-benzo[b]furanyl and benzyl may be substituted with 1, 2, 3, 4 or 5 substituents independently selected from the group consisting of F, Cl, Br, $-C_{1-4}$ alkyl, $-CF_3$, $-CHF_2$, $-CH_2F$, $-C_{1-4}$ alkoxy, $-C_{1-4}$ alkylthio, phenoxy, phenyl, and benzyloxy;
or for a residue selected from the group consisting of phenyl and naphthyl, wherein the residue may in each case be attached via a $-(CH_2)$ group, $-(CH_2)_2$ group, or $-(CH_2)_3$ group and/or may be substituted with 1, 2, or 3 substituents independently selected from the group consisting of $-C_{1-6}$ alkoxy, $-C_{1-6}$ alkyl, $-NH_2$, $-NH-(C_{1-3}$ alkyl), $-N(C_{1-3}$ alkyl)-($C_{1-3}$ alkyl), -NH-phenyl, $-N(C_{1-3}$ alkyl)-phenyl, $N(phenyl)_2$, and morpholinyl;

particularly preferably $R^4$ stands
for a residue selected from the group consisting of methyl, ethyl, n-propyl, $-(CH_2)-N(CH_3)_2$, $-(CH_2)-(CH_2)-N(CH_3)_2$ $-(CH_2)-(CH_2)-(CH_2)-N(CH_3)_2$, $-(CH_2)-(CH_2)-N(C_2H_5)_2$, $-(CH_2)-(CH_2)-(CH_2)-N(C_2H_5)_2$,- $(CH_2)-(CH_2)-N(CH_3)$(phenyl), and $-(CH_2)-(CH_2)-(CH_2)-N(CH_3)$(phenyl) ;
or for a residue selected from the group consisting of

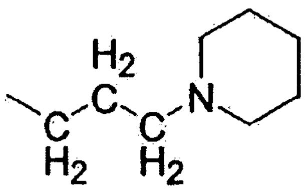
,
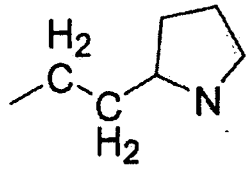
,
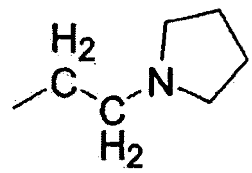
,

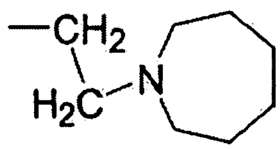
and
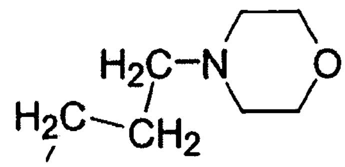
,

which may be substituted with a methyl residue;
or for the following residue

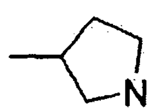
,

which may be substituted on the nitrogen atom with a substituent selected from the group consisting of $-(CH_2)$-benzo[b]furanyl and benzyl, wherein in each case the cyclic moiety of the residues $-(CH_2)$-benzo[b]furanyl and benzyl may be substituted with 1, 2, or 3 substituents independently selected from the group consisting of F, Cl, Br, -CF3, $-O-CH_3$, $-O-C_2H_5$, $-O-C_3H_7$, $-S-CH_3$, $-S-C_2H_5$, $-S-C_3H_7$, $-S-C(CH_3)_3$, methyl, ethyl, n-propyl, isopropyl, n-butyl, phenoxy and benzyloxy;
or for a residue selected from the group consisting of phenyl and benzyl, wherein the cyclic moiety of the residues phenyl and benzyl may be substituted with 1, 2, or 3 substituents independently selected from the group consisting of $-O-CH_3$, $-O-C_2H_5$, $-O-C_3H_7$, $-N-(CH_3)_2$, $-N(C_2H_5)_2$, and morpholinyl.

**12.** Compounds according to any one or more of claims 1 to 11, **characterized in that**
$R^5$ and $R^6$ form, together with the nitrogen atom joining them together as ring member, a heterocycloaliphatic residue selected from the group consisting of piperazinyl, morpholinyl, thiomorpholinyl, pyrrolidinyl, azepanyl, diazepanyl, and piperidinyl, wherein the heterocycloaliphatic residues may be substituted with a residue $R^8$ and optionally a residue X or a residue $R^9$ or a residue $R^{10}$;
particularly preferably $R^5$ and $R^6$ form, together with the nitrogen atom joining them together as ring member, one of the following residues

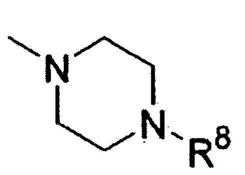, 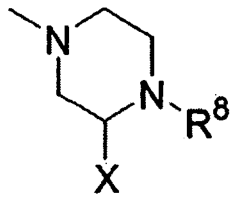, 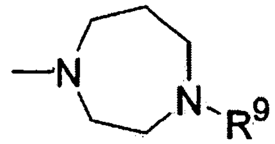,

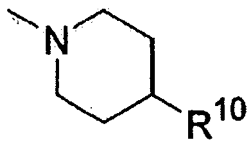, 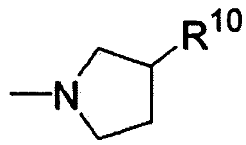

and

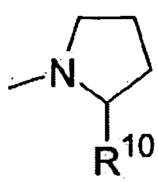.

**13.** Compounds according to any one or more of claims 1 to 12, **characterized in that** the residue X stands for an alkyl residue selected from the group consisting of methyl, ethyl, n-propyl, isopropyl, n-butyl, sec-butyl, isobutyl, tert-butyl, n-pentyl, sec-pentyl, (1,1)-dimethylpropyl, and n-hexyl;
preferably X stands for a methyl residue.

**14.** Compounds according to any one or more of claims 1 to 13, **characterized in that** the residues $R^8$, $R^9$, and $R^{10}$ each independently stand for an alkyl residue selected from the group consisting of methyl, ethyl, n-propyl, isopropyl, n-butyl, sec-butyl, isobutyl, tert-butyl, n-pentyl, sec-pentyl, (1,1)-dimethylpropyl, and n-hexyl, wherein the alkyl residue may in each case be substituted with 1, 2, 3, 4, or 5 substituents independently selected from the group consisting of F, Cl, Br, -CN, $-NO_2$, hydroxy, $C_{1-6}$ alkoxy, $-NH_2$, -NH-($C_{1-3}$ alkyl), -N($C_{1-3}$ alkyl) ($C_{1-3}$ alkyl), -NH-phenyl, -N($C_{1-3}$ alkyl)-phenyl, and $-N(phenyl)_2$;
or for an alkenyl residue selected from the group consisting of vinyl, 1-propenyl, 2-propenyl, 1-butenyl, 2-butenyl, 3-butenyl, 1-pentenyl, 2-pentenyl, and pent-1,3-dienyl, wherein the alkenyl residue may in each case be substituted with 1, 2, 3, 4, or 5 substituents independently selected from the group consisting of F, Cl, Br, -CN, $-NO_2$, hydroxy, $C_{1-6}$ alkoxy, $-NH_2$, -NH-($C_{1-3}$ alkyl), -N($C_{1-3}$ alkyl) ($C_{1-3}$ alkyl), -NH-phenyl, -N($C_{1-3}$ alkyl)-phenyl, and $-N(phenyl)_2$;
or for a (hetero)cycloaliphatic residue selected from the group consisting of cyclopropyl, cyclobutyl, cyclopentyl, cyclohexyl, cycloheptyl, cyclopentenyl, cyclohexenyl, cycloheptenyl, imidazolidynyl, tetrahydrofuranyl, tetrahydrothiophenyl, pyrrolidinyl, piperidinyl, morpholinyl, piperazinyl, thiomorpholinyl, tetrahydropyranyl, azepanyl, diazepanyl, and dithiolanyl, wherein the (hetero)cycloaliphatic residue may in each case be attached via a linear or branched $C_{1-5}$ alkylene group optionally comprising a carbonyl-(C=O) group as chain member and/or may be substituted with 1, 2, 3, 4, or 5 substituents independently selected from the group consisting of F, Cl, Br, -CN, $-CF_3$, $-CHF_2$, $-CH_2F$, hydroxy, $-C_{1-6}$ alkoxy, $-NH_2$, -NH-($C_{1-3}$ alkyl), -N($C_{1-3}$ alkyl)($C_{1-3}$ alkyl), -NH-phenyl, -N($C_{1-3}$ alkyl)-phenyl, $-N(phenyl)_2$, -SH, $-C_{1-6}$ alkylthio, $-C_{1-6}$ alkyl, $-(CH_2)$-benzo[b]furanyl, phenoxy, benzyloxy, phenyl, and benzyl, wherein in each case the cyclic moiety of the residues phenoxy, benzyloxy, phenyl, $-(CH_2)$-benzo[b]furanyl, and benzyl may be substituted with 1, 2, 3, 4, or 5 substituents independently selected from the group consisting of F, Cl, Br, $-C_{1-4}$ alkyl, $-CF_3$, $-CHF_2$, $-CH_2F$, $-C_{1-4}$ alkoxy, $-C_{1-4}$ alkylthio, phenoxy, phenyl, and benzyloxy;
or for a residue selected from the group consisting of phenyl, naphthyl, (1,3)-benzodioxolyl, (1,4)benzodioxanyl, thiophenyl, furanyl, pyrrolyl, pyrazolyl, pyrazinyl, pyranyl, triazolyl, pyridinyl, imidazolyl, indolyl, isoindolyl, benzo[b]furanyl, benzo[b]thiophenyl, thiazolyl, oxazolyl, isoxazolyl, pyridazinyl, pyrazinyl, pyrimidinyl, indazolyl, quinazolinyl, quinolinyl, and isoquinolinyl, wherein the residue may in each case be attached via a linear or branched $C_{1-5}$ alkylene group or $C_{2-5}$ alkenylene group, optionally substituted with a phenyl residue and optionally comprising a carbonyl-(C=O) group as chain member and/or may in each case be substituted with 1, 2, 3, 4, or 5 substituents independently selected from the group consisting of F, Cl, Br, -CN, $-CF_3$, $-CHF_2$, $-CH_2F$, hydroxy, $-C_{1-6}$ alkoxy, $-O-CF_3$, $-S-CF_3$, $)C=O)-CH_3$, -SH, $-C_{1-6}$ alkylthio, $-C_{1-6}$ alkyl, $-NH_2$, -NH-($C_{1-3}$ alkyl), -N($C_{1-3}$ alkyl) ($C_{1-3}$ alkyl), -NH-phenyl, -N ($C_{1-3}$ alkyl)-phenyl, $N(phenyl)_2$, phenoxy, benzyloxy, phenyl, benzyl, and morpholinyl, wherein in each case the cyclic moiety of the residues phenoxy, benzyloxy, phenyl, and benzyl may be substituted with 1, 2, 3, 4, or 5 substituents independently selected from the group consisting of F, Cl, Br, hydroxy, $-CF_3$, $-SF_5$, -CN, $-NO_2$, $-C_{1-6}$ alkoxy, $-O-CF_3$, $-S-CF_3$, phenyl, and benzyloxy;

preferably

$R^8$ stands for an alkyl residue selected from the group consisting of methyl, ethyl, n-propyl, isopropyl, n-butyl, tert-butyl, and n-pentyl, wherein the alkyl residue may in each case be substituted with 1 or 2 substituents independently selected from the group consisting of $NH_2$, $-NH-CH_3$, $-NH-C_2H_5$, $-N-(CH_3)_2$, $-N(C_2H_5)_2$, $-N(CH_3)(C_2H_5)$, -NH-phenyl, $-N(CH_3)$-phenyl, $-N(C_2H_5)$-phenyl, and $-N(phenyl)_2$;

or for a cycloaliphatic residue selected from the group consisting of pyrrolidinyl and piperidinyl, which cycloaliphatic residue may be attached via a -(C=O) group or a $-(CH_2)-(C=O)$ group and/or may be substituted with 1, 2, 3, 4, or 5 substituents independently selected from the group consisting of F, Cl, Br, -CN, $-CF_3$, $-CHF_2$, $-CH_2F$, hydroxy, $-O-CH_3$, $-O-C_2H_5$, $-O-C_3H_7$, $-NH_2$, $-NH-CH_3$, $-NH-C_2H_5$, $-N-(CH_3)_2$, $-N(C_2H_5)_2$, $-N(CH_3)(C_2H_5)$, -NH-phenyl, -N $(CH_3)$-phenyl, $-N(C_2H_5)$-phenyl, $-N(phenyl)_2$, -SH, $-S-CH_3$, $-S-C_2H_5$, $-S-C_3H_7$, $-S-C(CH_3)_3$, methyl, ethyl, n-propyl, isopropyl, n-butyl, sec-butyl, and tert-butyl;

or for a residue selected from the group consisting of phenyl, 1-naphthyl, 2-naphthyl, 2-furanyl, 3-furanyl, 2-thiophenyl, 3-thiophenyl, 1-pyridinyl, 2-pyridinyl, 3-pyridinyl, benzo[b]furanyl, (1,3)benzodioxolyl, and (1,4)benzodioxanyl, which residue may be attached via a -(C=O) group, $-(CH_2)$ group, $-(CH_2)_2$ group, $-CH(CH_3)$ group, -(CH=CH) group, $-(CH_2)-(C=O)$ group, $-(CH_2)$-(CH=CH) group, or $-(CH_2)_3$ group and/or may be substituted with 1, 2, 3, 4, or 5 substituents independently selected from the group consisting of F, Cl, Br, -CN, $-CF_3$, $-CHF_2$, $-CH_2F$, hydroxy, $(C=O)-CH_3$, $-O-CH_3$, $-O-C_2H_5$, $-O-C_3H_7$, $-O-CF_3$, $-S-CF_3$, -SH, $-S-CH_3$, $-S-C_2H_5$, $-S-C_3H_7$, $-S-C(CH_3)_3$, methyl, ethyl, n-propyl, isopropyl, n-butyl, sec-butyl, tert-butyl, $-NH_2$, $-NH-CH_3$, $-NH-C_2H_5$, $-N-(CH_3)_2$, $-N(C_2H_5)_2$, $-N(CH_3)$ $(C_2H_5)$, -NH-phenyl, $-N(CH_3)$-phenyl, $-N(C_2H_5)$-phenyl, $-N(phenyl)_2$, phenoxy, benzyloxy, phenyl, benzyl, and morpholinyl;

$R^9$ stands for an alkyl residue selected from the group consisting of methyl, ethyl, n-propyl, isopropyl, n-butyl, sec-butyl, isobutyl, tert-butyl, n-pentyl, sec-pentyl, (1,1)-dimethylpropyl, and n-hexyl,

and

$R^{10}$ stands for a cycloaliphatic residue selected from the group consisting of pyrrolidinyl and piperidinyl, which cycloaliphatic residue may be attached via a $-(CH_2)$ group, $-(CH_2)_2$ group, $-CH(CH_3)$ group, or $-(CH_2)_3$ group and/or may be substituted with 1, 2, 3, 4, or 5 substituents independently selected from the group consisting of F, Cl, Br, -CN, $-CF_3$, $-CHF_2$, $-CH_2F$, hydroxy, $-O-CH_3$, $-O-C_2H_5$, $-0-C_3H_7$, $-NH_2$, $-NH-CH_3$, $-NH-C_2H_5$, $-N-(CH_3)_2$, $-N(C_2H_5)_2$, $-N(CH_3)(C_2H_5)$, -NH-phenyl, $-N(CH_3)$phenyl, $-N(C_2H_5)$phenyl, $-N(phenyl)_2$, -SH, $-S-CH_3$, $-S-C_2H_5$, $-S-C_3H_7$, -S-C $(CH_3)_3$, methyl, ethyl, n-propyl, isopropyl, n-butyl, sec-butyl, and tert-butyl;

or for a residue selected from the group consisting of phenyl, 1-naphthyl, and 2-naphthyl, which residue may be attached via a $-(CH_2)$ group, -[(CH)phenyl] group, $-(CH_2)_2$ group, and $-(CH_2)_3$ group and/or may be substituted with 1, 2, 3, 4, or 5 substituents independently selected from the group consisting of F, Cl, Br, -CN, $-CF_3$, $-CHF_2$, $-CH_2F$, hydroxy, $-O-CH_3$, $-O-C_2H_5$, $-O-C_3H_7$, $-O-CF_3$, $-S-CF_3$, thio, $-S-CH_3$, $-S-C_2H_5$, $-S-C_3H_7$, $-S-C(CH_3)_3$, methyl, ethyl, n-propyl, isopropyl, n-butyl, sec-butyl, tert-butyl, $-NH_2$, $-NH-CH_3$, $-NH-C_2H_5$, $-N-(CH_3)_2$, $-N(C_2H_5)_2$, $-N(CH_3)(C_2H_5)$, -NH-phenyl, $-N(CH_3)$-phenyl, $-N(C_2H_5)$-phenyl, $-N(phenyl)_2$, phenoxy, benzyloxy, phenyl, benzyl, and morpholinyl;

particularly preferably

$R^8$ stands for a residue selected from the group consisting of methyl, ethyl, n-propyl, isopropyl, and $-(CH_2)-(CH_2)-N(CH_3)_2$,

or for a pyrrolidinyl residue which is attached via a $-(CH_2)-(C=O)$ group,

or for a residue selected from the group consisting of phenyl, 1-naphthyl, 2-naphthyl, 2-thiophenyl, 3-thiophenyl, 1-pyridinyl, 2-pyridinyl, 3-pyridinyl, benzo[b]furanyl, (1,3)benzodioxolyl, and (1,4)benzodioxanyl, which residue may be attached via a -(C=O) group, $-(CH_2)$ group, $-(CH_2)_2$ group, $-CH(CH_3)$ group, -(CH=CH) group, $-(CH_2)-(C=O)$ group, $-(CH_2)$-(CH=CH) group, or $-(CH_2)_3$ group and/or may be substituted with 1, 2, or 3 substituents independently selected from the group consisting of F, Cl, Br, -CN, $-CF_3$, $-CHF_2$, $-CH_2F$, $-(C=O)-CH_3$, $-O-CH_3$, and $-O-C_2H_5$,

$R^9$ stands for a methyl residue or ethyl residue

and

$R^{10}$ stands for a pyrrolidinyl residue, which is attached via a $-(CH_2)$ group,

or for a benzhydryl residue.

**15.** Compounds according to any one or more of claims 1 to 14, **characterized in that**

$R^1$ stands for a group $-NR^3R^4$ or for a group $-NR^5R^6$;
$R^2$ stands for an alkyl residue selected from the group consisting of n-propyl, isopropyl, n-butyl, sec-butyl, isobutyl, tert-butyl, n-pentyl, sec-pentyl, and (1,1)- dimethylpropyl;
or for an alkenyl residue selected from the group consisting of 1-pentenyl, 2-pentenyl and pent-1,3- dienyl;
or for a cycloaliphatic residue selected from the group consisting of cyclopentyl, cyclohexyl, and cycloheptyl, which is attached via a $-(CH_2)$ group;
or for a residue selected from the group consisting of phenyl, 1-naphthyl, 2-naphthyl, 2-furanyl, 3-furanyl, 2-thiophenyl, or 3-thiophenyl, which residue may be attached via a - $(CH_2)$ group, $-(CH_2)_2$ group, $-(CH_2)$-O group, $-CH(CH_3)$ group, -(CH=CH) group, or $-(CH_2)_3$ group and/or may be substituted with 1, 2, or 3 substituents independently selected from the group consisting of F, Cl, Br, $-O-CH_3$, $-O-C_2H_5$, methyl, ethyl, n-propyl, and isopropyl;
$R^3$ stands for a hydrogen residue
or for a methyl residue;
$R^4$ stands for a residue selected from the group consisting of methyl, ethyl, n-propyl, $-(CH_2)-N(CH_3)_2$, $-(CH_2)-(CH_2)-N(CH_3)_2$, $-(CH_2)-(CH_2)-(CH_2)-N(CH_3)_2$, $-(CH_2)-(CH_2)-N(C_2H_5)_2$, $-(CH_2)-(CH_2)-(CH_2)-N(C_2H_5)_2$, $-(CH_2)-(CH_2)-N(CH_3)$-(phenyl), and $-(CH_2)-(CH_2)-(CH_2)-N(CH_3)$-(phenyl);
or for a residue selected from the group consisting of

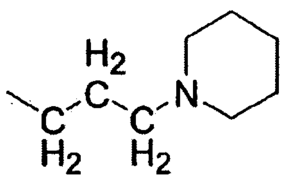 , 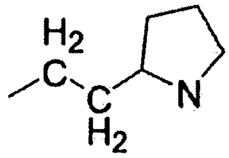 , 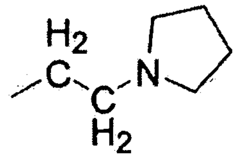 ,

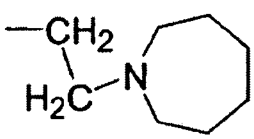 and 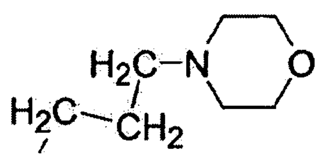 ,

which may be substituted with a methyl residue;
or for the following residue

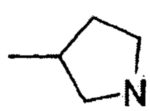 ,

which may be substituted on the nitrogen atom with a substituent selected from the group consisting of $-(CH_2)$-benzo[b]furanyl and benzyl, wherein in each case the cyclic moiety of the residues $-(CH_2)$- benzo[b]furanyl and benzyl may be substituted with 1, 2, or 3 substituents independently selected from the group consisting of F, Cl, Br, $-CF_3$, $-O-CH_3$, $-O-C_2H_5$, $-O-C_3H_7$, $-S-CH_3$, $-S-C_2H_5$, $-S-C_3H_7$, $-S-C(CH_3)_3$, methyl, ethyl, n-propyl, isopropyl, n-butyl, phenoxy, and benzyloxy;
or for a residue selected from the group consisting of phenyl and benzyl, wherein the cyclic moiety of the residues phenyl and benzyl may be substituted with 1, 2, or 3 substituents independently selected from the group consisting of $-O-CH_3$, $-O-C_2H_5$, $-O-C_3H_7$, $-N-(CH_3)_2$, $-N(C_2H_5)_2$, and morpholinyl;
$R^5$ and $R^6$ form, together with the nitrogen atom joining them together as ring member, one of the following residues

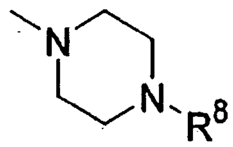, 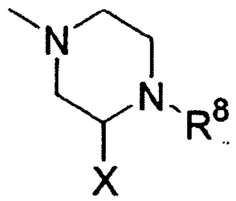, 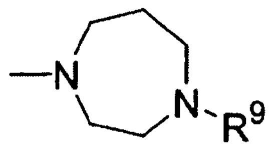,

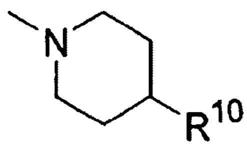, 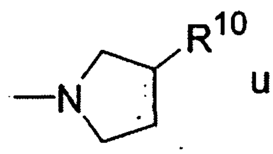 u and 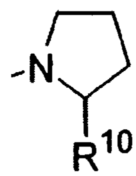;

X stands for a methyl residue,
$R^8$ stands for a residue selected from the group consisting of methyl, ethyl, n-propyl, isopropyl, and $-(CH_2)-(CH_2)-N(CH_3)_2$,
or for a pyrrolidinyl residue which is attached via a $-(CH_2)-(C{=}O)$ group,
or for a residue selected from the group consisting of phenyl, 1-naphthyl, 2-naphthyl, 2-thiophenyl, 3- thiophenyl, 1-pyridinyl, 2-pyridinyl, 3-pyridinyl, benzo[b]furanyl, (1,3)-benzodioxolyl, and (1,4)- benzodioxanyl, which residue may be attached via a -(C=O) group, $-(CH_2)$ group, $-(CH_2)_2$ group, $-CH(CH_3)$ group, -(CH=CH) group, $-(CH_2)-(C{=}O)$ group, $-(CH_2)-$ (CH=CH) group, or $-(CH_2)_3$ group and/or may be substituted with 1, 2, or 3 substituents independently
$R^9$ selected from the group consisting of F, Cl, Br, -CN, $-CF_3$, $-CHF_2$, $-CH_2F$, $-(C{=}O)-CH_3$, $-O-CH_3$, and $-O-C_2H_5$, stands for a methyl residue or ethyl residue; and
$R^{10}$ stands for a pyrrolidinyl residue, which is attached via a $-(CH_2)$ group,

or for a benzhydryl residue.

**16.** Compounds according to any one or more of claims 1 to 15 selected from the group consisting of
2- Cyclohexyl- N- {4-[4-(2- dimethylaminoethyl) piperazin- 1- carbonyl]- 4,5,6,7- tetrahydrobenzothiazol- 2- yl)-acetamide,
N-[4-(4-Methyl[1,4]diazepan-1-carbonyl)-4,5,6,7-tetrahydrobenzothiazol-2-yl]-2-phenoxyacetamide,
2-(2-Phenylpropionylamino)-4,5,6,7-tetrahydrobenzothiazol-4-carboxylic acid (3-dimethylaminopropyl)amide,
Naphthalene-2-carboxylic acid [4-(4-methylpiperazine-1-carbonyl)-4,5,6,7-tetrahydrobenzothiazol-2-yl]amide,
N-[4-(2-Pyrrolidin-1-yl-methylpyrrolidine-1-carbonyl)-4,5,6,7-tetrahydrobenzothiazol-2-yl]-2-thiophen-2-yl-acetamide,
N-{4-[4-(7-Methoxybenzo[1,3]dioxol-5-ylmethyl)piperazine-1-carbonyl]-4,5,6,7-tetrahydrobenzothiazol-2-yl}-2-(2-methoxyphenyl)acetamide,
N-{4-[4-(4-Methoxyphenyl)-3-methylpiperazine-1-carbonyl]-4,5,6,7-tetrahydrobenzothiazol-2-yl}benzamide,
Furan-2-carboxylic acid {4-[4-(4-acetylphenyl)piperazine-1-carbonyl]-4,5,6,7-tetrahydrobenzothiazol-2-yl}amide,
2-[(Furan-2-carbonyl)amino]-4,5,6,7-tetrahydrobenzothiazole-4-carboxylic acid [1-(2,4,6-trimethoxybenzyl)pyrrolidin-3-yl]amide,
N-[4-(4-Isopropylpiperazine-1-carbonyl)-4,5,6,7-tetrahydrobenzothiazol-2-yl]-2-phenylpropionamide,
3-Furan-2-yl-N-[4-(4-thiophen-3-ylmethylpiperazine-1-carbonyl)-4,5,6,7-tetrahydrobenzothiazol-2-yl]acrylamide,
2-(4-Methoxybenzoylamino)-4,5,6,7-tetrahydrobenzothiazole-4-carboxylic acid [1-(2-chloro-6-fluorobenzyl)pyrrolidin-3-yl]methylamide,
Hexanoic acid [4-(4-pyridin-4-ylpiperazine-1-carbonyl)-4,5,6,7-tetrahydrobenzothiazol-2-yl]amide,
2-(2-Thiophen-2-ylacetylamino)-4,5,6,7-tetrahydrobenzothiazole-4-carboxylic acid (3-morpholin-4-ylpropyl)amide,
2-(2-Phenylpropionylamino)-4,5,6,7-tetrahydrobenzothiazol-4-carboxylic acid [1-(4-phenoxybenzyl)pyrrolidin-3-yl] amide,
2-[(Furan-3-carbonyl)amino]-4,5,6,7-tetrahydrobenzothiazol-4-carboxylic acid (2-azepan-1-ylethyl)amide,
Furan-3-carboxylic acid [4-(4-benzofuran-2-ylmethylpiperazine-1-carbonyl)-4,5,6,7-tetrahydrobenzothiazol-2-yl]amide, 2-Hexanylamino-4,5,6,7-tetrahydrobenzothiazol-4-carboxylic acid [3-(2-methylpiperidin-1-yl)propyl]amide, 2-(2-Phenylpropionylamino)-4,5,6,7-tetrahydrobenzothiazole-4-carboxylic acid (2-dimethylamino-ethyl)amide, 2-Ethoxy-N-[4-(4-phenethylpiperazine-1-carbonyl)-4,5,6,7-tetrahydrobenzothiazol-2-yl]benzamide, 2-(4-Fluorophenoxy)-N-[4-(4-phenylpiperazine-1-carbonyl)-4,5,6,7-tetrahydrobenzothiazol-2-yl]acetamide, 2-(2-Phenylpropionylamino)-4,5,6,7-tetrahydrobenzothiazole-4-carboxylic acid [2-(1-methylpyrrolidin-2-yl)ethyl] amide, 2-(3-Furan-2-ylacryloylamino)-4,5,6,7-tetrahydrobenzothiazole-4-carboxylic acid [3-(2-methylpiperidin-1-yl)propyl] amide, 2-(3,3-Dimethylbutyrylamino)-4,5,6,7-tetrahydrobenzothiazole-4-carboxylic acid (3-dimethylaminopropyl)amide, N-{4-[4-(4-chlorobenzyl)piperazine-1-carbonyl]-4,5,6,7-tetrahydrobenzothiazol-2-yl}-4-methoxybenzamide, Naphthalene-2-carboxylic acid [4-(2-pyrrolidin-1-ylmethylpyrrolidine-1-carbonyl)-4,5,6,7-tetrahydrobenzothiazol-2-yl]amide, N-[4-(4-Benzofuran-2-ylmethylpiperazine-1-carbonyl)-4,5,6,7-tetrahydrobenzothiazol-2-yl]butyramide, N-{4-[4-(3-Methoxyphenyl)piperazine-1-carbonyl]-4,5,6,7-tetrahydrobenzothiazol-2-yl}benzamide, 2-Butyrylamino-4,5,6,7-tetrahydrobenzothiazole-4-carboxylic acid (1-biphenyl-4-ylmethylpyrrolidin-3-yl)methylamide, 2-(2-Phenoxyacetylamino)-4,5,6,7-tetrahydrobenzothiazole-4-carboxylic acid (2-dimethylamino-ethyl)amide, 2-Ethoxy-N-[4-(2-pyrrolidin-1-ylmethylpyrrolidine-1-carbonyl)-4,5,6,7-tetrahydrobenzothiazol-2-yl]benzamide, Naphthalene-2-carboxylic acid {4-[4-(2-oxo-2-pyrrolidin-1-ylethyl)piperazin-1-carbonyl]-4,5,6,7-tetrahydrobenzothiazol-2-yl}amide, 2-(2-Phenylpropionylamino)-4,5,6,7-tetrahydrobenzothiazole-4-carboxylic acid [1-(2-brom-4,5-dimethoxybenzyl)-pyrrolidin-3-yl]amide, Hexanoic acid {4-[4-(2,4,6-trimethoxybenzyl)piperazine-1-carbonyl]-4,5,6,7-tetrahydrobenzothiazol-2-yl}amide, 2-(3,5-Dimethylbenzoylamino)-4,5,6,7-tetrahydrobenzothiazole-4-carboxylic acid [1-(2-tert-butylsulfanylbenzyl) pyrrolidin-3-yl]amide, 2-(2-Phenylpropionylamino)-4,5,6,7-tetrahydrobenzothiazole-4-carboxylic acid [1-(2-benzyloxybenzyl)pyrrolidin-3-yl]methylamide, 2-Hexa-2,4-dienoylamino-4,5,6,7-tetrahydrobenzothiazole-4-carboxylic acid (1-biphenyl-4-ylmethylpyrrolidin-3-yl) methylamide, 2-(3-Phenylpropionylamino)-4,5,6,7-tetrahydrobenzothiazole-4-carboxylic acid (2-diethylamino-ethyl)amide, 2-(2-Thiophen-2-ylacetylamino)-4,5,6,7-tetrahydrobenzothiazole-4-carboxylic acid (2-azepan-1-ylethyl)amide, 2-(2-Thiophen-2-ylacetylamino)-4,5,6,7-tetrahydrobenzothiazole-4-carboxylic acid [1-(2-ethoxybenzyl)pyrrolidin-3-yl]methylamide, 2-Hexanylamino-4,5,617-tetrahydrobenzothiazole-4-carboxylic acid (1-benzofuran-2-ylmethylpyrrolidin-3-yl)methylamide, 2-(2-Phenoxyacetylamino)-4,5,6,7-tetrahydrobenzothiazole-4-carboxylic acid [3-(2-methylpiperidin-1-yl)propyl] amide, 2-[(Naphthalene-2-carbonyl)amino]-4,5,6,7-tetrahydrobenzothiazole-4-carboxylic acid (2-pyrrolidin-1-ylethyl) amide, 2-[2-(3,5-Difluorophenyl)acetylamino]-4,5,6,7-tetrahydrobenzothiazole-4-carboxylic acid [1-(2-ethoxybenzyl)pyrrolidin-3-yl]methylamide, 2-[(Naphthalene-2-carbonyl)amino]-4,5,6,7-tetrahydrobenzothiazole-4-carboxylic acid 4-dimethylamino-benzylamide, 2-[2-(4-Fluorophenoxy)acetylamino]-4,5,6,7-tetrahydrobenzothiazole-4-carboxylic acid [2-(1-methylpyrrolidin-2-yl) ethyl]amide, Furan-2-carboxylic acid {4-[4-(2-cyano-phenyl)piperazine-1-carbonyl]-4,5,6,7-tetrahydrobenzothiazol-2-yl}amide, 2-(3-Phenylpropionylamino)-4,5,6,7-tetrahydrobenzothiazole-4-carboxylic acid (1-benzofuran-2-ylmethylpyrrolidin-3-yl)methylamide, 2-Hexa-2,4-dienoylamino-4,5,6,7-tetrahydrobenzothiazole-4-carboxylic acid [1-(2,6-dichlorobenzyl)pyrrolidin-3-yl] methylamide, 2-[(Furan-2-carbonyl)amino]-4,5,6,7-tetrahydrobenzothiazole-4-carboxylic acid [1-(5-bromo-2-ethoxybenzyl)pyrrolidin-3-yl]amide, 2-(2-Phenoxyacetylamino)-4,5,6,7-tetrahydrobenzothiazole-4-carboxylic acid [1-(2-ethoxybenzyl)pyrrolidin-3-yl] methylamide, Hexanoic acid {4-[4-(2-fluorophenyl)piperazine-1-carbonyl]-4,5,6,7-tetrahydrobenzothiazol-2-yl}amide, Naphthalene-2-carboxylic acid {4-[4-(2-fluorobenzyl)-piperazine-1-carbonyl]-4,5,6,7-tetrahydrobenzothiazol-2-yl} amide, 2-(2-Phenoxyacetylamino)-4,5,6,7-tetrahydrobenzothiazole-4-carboxylic acid methyl-[1-(2-trifluoromethylbenzyl)-pyrrolidin-3-yl]amide,

2-(3-Phenylpropionylamino)-4,5,6,7-tetrahydrobenzothiazole-4-carboxylic acid (1-biphenyl-4-ylmethylpyrrolidin-3-yl)methylamide,
2-[2-(2-Methoxyphenyl)acetylamino]-4,5,6,7-tetrahydrobenzothiazole-4-carboxylic acid (1-benzofuran-2-ylmethyl-pyrrolidin-3-yl)methylamide,
2-(3,5- Difluorophenyl)-N-[4-(2- pyrrolidin- 1- ylmethylpyrrolidin- 1- carbonyl)- 4,5,6,7- tetrahydrobenzothiazol- 2- yl] acetamide,
2-[(Furan-2-carbonyl)amino]-4,5,6,7-tetrahydrobenzothiazole-4-carboxylic acid 4-dimethylamino-benzylamide,
Hexanoic acid {4-[4-(3-fluoro-4-methoxybenzyl)piperazin-l-carbonyl]-4,5,6,7-tetrahydrobenzothiazol-2-yl}amide,
N-{4-[4-(4-Methoxyphenyl)-3-methylpiperazin-1-carbonyl]-4,5,6,7-tetrahydrobenzothiazol-2-yl}-2-phenylpropionamide,
2-Hexa-2,4-dienoylamino-4,5,6,7-tetrahydrobenzothiazol-4-carboxylic acid [1-(2-chloro-6-fluorobenzyl)pyrrolidin-3-yl]methylamide,
2-[2-(3,5-Difluorophenyl)acetylamino]-4,5,6,7-tetrahydrobenzothiazole-4-carboxylic acid [1-(2,6-dichlorobenzyl) pyrrolidin-3-yl]methylamide,
2-[2-(4-fluorophenoxy)acetylamino]-4,5,6,7-tetrahydrobenzothiazole-4-carboxylic acid [1-(2-ethoxybenzyl)pyrrolidin-3-yl]methylamide,
Furan-3-carboxylic acid [4-(4-pyridin-2-ylpiperazine-1-carbonyl)-4,5,6,7-tetrahydrobenzothiazol-2-yl]amide,
Hexanoic acid [4-(4-benzofuran-2-ylmethylpiperazine-1-carbonyl)-4,5,6,7-tetrahydrobenzothiazol-2-yl]amide,
2-Phenoxy-N-{4-[4-(1-phenylethyl)piperazine-1-carbonyl]-4,5,6,7-tetrahydrobenzothiazol-2-yl}acetamide,
2-(2-Cyclohexylacetylamino)-4,5,6,7-tetrahydrobenzothiazole-4-carboxylic acid [1-(2,6-dichlorobenzyl)pyrrolidin-3-yl]methylamide,
2-(2-Cyclohexylacetylamino)-4,5,6,7-tetrahydrobenzothiazole-4-carboxylic acid [3-(methylphenylamino)propyl] amide,
2-(2-Ethoxybenzoylamino)-4,5,6,7-tetrahydrobenzothiazole-4-carboxylic acid [3-(methylphenylamino)propyl] amide,
3-Phenyl-N-{4-[4-(5-trifluoromethylpyridin-2-yl)piperazine-1-carbonyl]-4,5,6,7-tetrahydrobenzothiazol-2-yl}propionamide,
Furan-3-carboxylic acid [4-(4-phenethylpiperazine-1-carbonyl)-4,5,6,7-tetrahydrobenzothiazol-2-yl]amide,
3-Phenyl-N-{4-[4-(3-trifluoromethylphenyl)piperazine-1-carbonyl]-4,5,6,7-tetrahydrobenzothiazol-2-yl}propionamide,
2-(3,3-Dimethylbutyrylamino)-4,5,6,7-tetrahydrobenzothiazole-4-carboxylic acid [1-(2-benzyloxybenzyl)pyrrolidin-3-yl]amide,
2-(3,5-Difluorophenyl)-N-{4-[4-(3-trifluoromethylphenyl)piperazine-1-carbonyl]-4,5,6,7-tetrahydrobenzothiazol-2-yl}acetamide,
2-Ethoxy-N-{4-[4-(1-phenylethyl)piperazine-1-carbonyl]-4,5,6,7-tetrahydrobenzothiazol-2-yl}benzamide,
3-Furan-2-yl-N-{4-[4-(3-trifluoromethylphenyl)piperazine-1-carbonyl]-4,5,6,7-tetrahydrobenzothiazol-2-yl}acrylamide,
N-{4-[4-(2-Cyano-phenyl)piperazine-1-carbonyl]-4,5,6,7-tetrahydrobenzothiazol-2-yl}-3-phenylpropionamide,
2-(2-Cyclohexylacetylamino)-4,5,6,7-tetrahydrobenzothiazole-4-carboxylic acid [1-(2-chloro-6-fluorobenzyl)pyrrolidin-3-yl]methylamide,
N- {4-[4-(2- Methoxynaphthalene- 1- ylmethyl) piperazine- 1- carbonyl]- 4,5,6,7- tetrahydrobenzothiazol- 2- yl}- 3,3-dimethylbutyramid,
2-(3,3-Dimethylbutyrylamino)-4,5,6,7-tetrahydrobenzothiazole-4-carboxylic acid (2,5-diethoxy-4-morpholin-4-yl-phenyl)amide,
N-{4-[4-(2-Chlorophenyl)piperazine-1-carbonyl]-4,5,6,7-tetrahydrobenzothiazol-2-yl}-2-(4-fluorophenyl)acetamide,
N-[4-(4-Benzylpiperazine-1-carbonyl)-4,5,6,7-tetrahydrobenzothiazol-2-yl]-3,3-dimethylbutyramide,
N-[4-(4-Benzhydrylpiperazine-1-carbonyl)-4,5,6,7-tetrahydrobenzothiazol-2-yl]-2-(3,5-difluorophenyl)acetamide,
N-[4-(4-Benzhydrylpiperazine-1-carbonyl)-4,5,6,7-tetrahydrobenzothiazol-2-yl]-2-thiophen-2-ylacetamide,
N-{4-[4-(2-Chlorophenyl)piperazine-1-carbonyl]-4,5,6,7-tetrahydrobenzothiazol-2-yl}-2-ethoxybenzamide,
3,3-Dimethyl-N-{4-[4-(5-trifluoromethylpyridin-2-yl)piperazine-1-carbonyl]-4,5,6,7-tetrahydrobenzothiazol-2-yl]-butyramide,
3,3-Dimethyl-N-{4-[4-(3-phenylallyl)piperazine-1-carbonyl]-4,5,6,7-tetrahydrobenzothiazol-2-yl}butyramide
N-[4-(4-Benzhydrylpiperazine-1-carbonyl)-4,5,6,7-tetrahydrobenzothiazol-2-yl]-2-cyclohexylacetamide,
and
Furan-2-carboxylic acid [4-(4-phenylpiperazine-1-carbonyl)-4,5,6,7-tetrahydrobenzothiazol-2-yl]amide,
optionally in the form of their pure stereoisomers, particularly enantiomers or diastereoisomers, of their racemates or in the form of mixtures of the stereoisomers, particularly the enantiomers and/or diastereoisomers, in any desired mixture ratio, or in each case optionally in the form of corresponding salts or in each case optionally in the form of

corresponding solvates.

**17.** A process for the preparation of substituted 4,5,6,7-tetrahydrobenzothiazol-2-ylamine compounds according to any one or more of claims 1 to 16, **characterized in that** at least one compound of the formula II

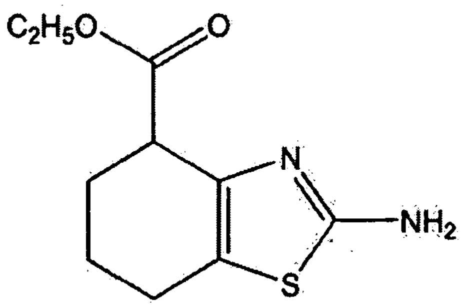

II,

optionally in the form of a salt, is converted, by reaction with at least one acylation reagent of the general formula III,

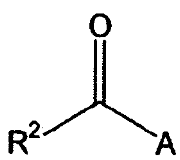

III

in which $R^2$ has the meaning according to one or more of claims 1 to 16, and A stands for a group eliminable from the acyl residue $R^2$-(C=O)-, preferably for -OH, -Cl, or -0-(C=O)-$R^2$, into a compound of the general formula IV,

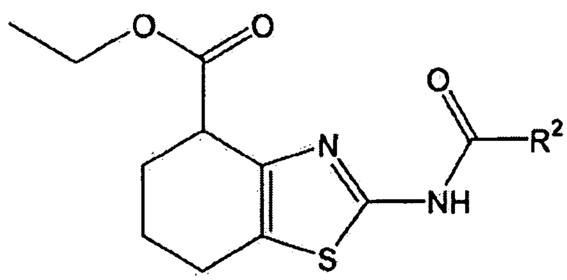

IV

which is optionally purified and/or optionally isolated, and is optionally converted, by cleavage of the ethyl ester, into a compound of the general formula V

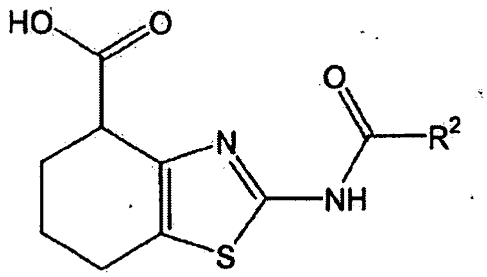

V

which is optionally purified and/or optionally isolated, and at least one compound of the general formula IV and/or at least one compound of the general formula V is converted by reaction with at least one compound of the general formula $R^1$-H, in which $R^1$ has the meaning according to any one or more of claims 1 to 16, into a compound of the general formula I according to claim 1, which compound is optionally purified and/or optionally isolated.

**18.** A medicament containing at least one substituted 4,5,6,7-tetrahydrobenzothiazol-2-ylamine compound according to any one or more of claims 1 to 16 and optionally one or more pharmaceutically acceptable auxiliary substances.

**19.** A medicament according to claim 18 for regulation of noradrenaline reuptake (noradrenaline uptake), for regulation of 5-hydroxytryptophan reuptake (5-HT uptake), for treatment of the abuse of alcohol and/or of drugs and/or of medicines, for the prophylaxis and/or treatment of addiction to alcohol and/or to drugs and/or to medicines, for the prophylaxis and/or treatment of inflammations, for the prophylaxis and/or treatment of depression, for the prophylaxis and/or treatment of lethargy, for the prophylaxis and/or treatment of disturbances of food intake preferably selected from the group consisting of bulimia, anorexia, obesity, and cachexia, for the prophylaxis and/or treatment of catalepsy, for vigilance enhancement, for libido enhancement or for anxiolysis.

**20.** Use of at least one substituted 4,5,6,7-tetrahydrobenzothiazol-2-ylamine compound according to any one of claims 1 to 16 for the production of a medicament for regulation of noradrenaline reuptake (noradrenaline uptake).

**21.** Use of at least one substituted 4,5,6,7-tetrahydrobenzothiazol-2-ylamine compound according to any one of claims 1 to 16 for the production of a medicament for regulation of 5-hydroxy-tryptophan reuptake (5-HT uptake).

**22.** Use of at least one substituted 4,5,6,7-tetrahydrobenzothiazol-2-ylamine compound according to any one of claims 1 to 16 for the production of a medicament for the treatment of pain.

**23.** Use of at least one substituted 4,5,6,7-tetrahydrobenzothiazol-2-ylamine compound according to any one of claims 1 to 16 for the production of a medicament for the treatment of abuse of alcohol and/or abuse of drugs and/or abuse of medicines.

**24.** Use of at least one substituted 4,5,6,7-tetrahydrobenzothiazol-2-ylamine compound according to any one of claims 1 to 16 for the production of a medicament for the prophylaxis und/or treatment of addiction to alcohol and/or addiction to drugs and/or addiction to medicines.

**25.** Use of at least one substituted 4,5,6,7-tetrahydrobenzothiazol-2-ylamine compound according to any one of claims 1 to 16 for the production of a medicament for the prophylaxis and/or treatment of inflammation.

**26.** Use of at least one substituted 4,5,6,7-tetrahydrobenzothiazol-2-ylamine compound according to any one of claims 1 to 16 for the production of a medicament for the prophylaxis and/or treatment of depression.

**27.** Use of at least one substituted 4,5,6,7-tetrahydrobenzothiazol-2-ylamine compound according to any one of claims 1 to 16 for the production of a medicament for the treatment of lethargy.

**28.** Use of at least one substituted 4,5,6,7-tetrahydrobenzothiazol-2-ylamine compound according to any one of claims 1 to 16 for the production of a medicament for the prophylaxis und/or treatment of disturbances of food intake preferably selected from the group consisting of bulimia, anorexia, obesity, and cachexia.

**29.** Use of at least one substituted 4,5,6,7-tetrahydrobenzothiazol-2-ylamine compound according to any one of claims 1 to 16 for the preparation of a medicament for the prophylaxis und/or treatment of catalepsy.

**30.** Use of at least one substituted 4,5,6,7-tetrahydrobenzothiazol-2-ylamine compound according to any one of claims 1 to 16 for the preparation of a medicament for vigilance enhancement.

**31.** Use of at least one substituted 4,5,6,7-tetrahydrobenzothiazol-2-ylamine compound according to any one of claims 1 to 16 for the preparation of a medicament for libido enhancement.

**32.** Use of at least one substituted 4,5,6,7-tetrahydrobenzothiazol-2-ylamine compound according to any one of claims 1 to 16 for the preparation of a medicament for anxiolysis.

**33.** Use of at least one substituted 4,5,6,7-tetrahydrobenzothiazol-2-ylamine compound according to any one of claims 1 to 16 for the preparation of a medicament for simultaneous treatment of depression and pain.

## Revendications

**1.** Composés 4,5,6,7-tétrahydro-benzothiazol-2-ylamine substitués de formule générale I,

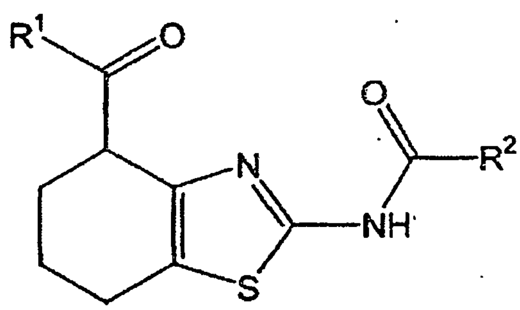

I

dans laquelle
$R^1$ représente un groupe $-NR^3R^4$ ou un groupe $-NR^5R^6$,
$R^2$ représente un radical aliphatique linéaire ou ramifié, saturé ou insaturé, éventuellement au moins une fois substitué, un radical cycloaliphatique saturé ou insaturé, éventuellement au moins une fois substitué, comportant éventuellement au moins un hétéroatome en tant que chaînon formant le cycle, qui peut être lié par un groupe alkylène, alcénylène ou alcynylène éventuellement au moins une fois substitué, comportant éventuellement au moins un hétéroatome en tant que chaînon formant le cycle, ou représente un radical aryle ou hétéroaryle, éventuellement au moins une fois substitué, qui peut être lié par un groupe alkylène, alcénylène ou alcynylène éventuellement au moins une fois substitué, comportant éventuellement au moins un hétéroatome en tant que chaînon,
$R^3$ représente un atome d'hydrogène, un radical aliphatique linéaire ou ramifié, saturé ou insaturé, éventuellement au moins une fois substitué, un radical cycloaliphatique saturé ou insaturé, éventuellement au moins une fois substitué, comportant éventuellement au moins un hétéroatome en tant que chaînon formant le cycle, qui peut être lié par un groupe alkylène, alcénylène ou alcynylène éventuellement au moins une fois substitué, comportant éventuellement au moins un hétéroatome en tant que chaînon, ou représente un radical aryle ou hétéroaryle, éventuellement au moins une fois substitué, qui peut être lié par un groupe alkylène, alcénylène ou alcynylène éventuellement au moins une fois substitué, comportant éventuellement au moins un hétéroatome en tant que chaînon,

$R^4$ représente un atome d'hydrogène, un radical aliphatique linéaire ou ramifié, saturé ou insaturé, éventuellement au moins une fois substitué, un radical cycloaliphatique saturé ou insaturé, éventuellement au moins une fois substitué, comportant éventuellement au moins un hétéroatome en tant que chaînon formant le cycle, qui peut être lié par un groupe alkylène, alcénylène ou alcynylène éventuellement au moins une fois substitué, comportant éventuellement au moins un hétéroatome en tant que chaînon, ou représente un radical aryle ou hétéroaryle, éventuellement au moins une fois substitué, qui peut être lié par un groupe alkylène, alcénylène ou alcynylène éventuellement au moins une fois substitué, comportant éventuellement au moins un hétéroatome en tant que chaînon,

$R^5$ et $R^6$ forment ensemble, avec l'atome d'azote qui les relie en tant que chaînon formant le cycle, un radical hétérocyclique saturé, insaturé ou aromatique, éventuellement au moins une fois substitué, comportant éventuellement au moins un autre hétéroatome en tant que chaînon formant le cycle,

éventuellement sous forme de leurs stéréoisomères, en particulier énantiomères ou diastéréoisomères, purs, de leurs racémates ou sous forme de mélanges des stéréoisomères, en particulier des énantiomères et/ou diastéréoisomères, en un rapport de mélange quelconque, ou chacun éventuellement sous forme des sels correspondants, ou chacun éventuellement sous forme produits de solvatation correspondants.

**2.** Composés selon la revendication 1, **caractérisés en ce que** $R^2$ représente un radical aliphatique en $C_1$-$C_{10}$ linéaire ou ramifié, saturé ou insaturé, éventuellement au moins une fois substitué, un radical cycloaliphatique à 3-7 chaînons, saturé ou insaturé, éventuellement au moins une fois substitué, comportant éventuellement au moins un hétéroatome en tant que chaînon formant le cycle, qui peut être lié par un groupe alkylène en $C_1$-$C_{10}$, alcénylène en $C_2$-$C_{10}$ ou alcynylène en $C_2$-$C_{10}$ éventuellement au moins une fois substitué, comportant éventuellement au moins un hétéroatome en tant que chaînon, ou représente un radical aryle ou hétéroaryle à 5-12 chaînons, éventuellement au moins une fois substitué, qui peut être lié par un groupe alkylène en $C_1$-$C_{10}$, alcénylène en $C_2$-$C_{10}$ ou alcynylène en $C_2$-$C_{10}$ éventuellement au moins une fois substitué, comportant éventuellement au moins un hétéroatome en tant que chaînon,

de préférence représente un radical alkyle en $C_1$-$C_{10}$ linéaire ou ramifié, un radical alcényle en $C_2$-$C_{10}$ linéaire ou ramifié, un radical cycloaliphatique à cinq ou six chaînons, saturé ou insaturé, éventuellement au moins une fois substitué, comportant éventuellement au moins un hétéroatome en tant que chaînon formant le cycle, qui peut être lié par un groupe alkylène en $C_1$-$C_5$ ou alcénylène en $C_2$-$C_5$ éventuellement au moins une fois substitué, comportant éventuellement au moins un hétéroatome en tant que chaînon, ou représente un radical phényle, 1-naphtyle, 2-naphtyle, 2-furannyle, 3-furannyle, 2-thiophényle ou 3-thiophényle, qui chaque fois peut être au moins une fois substitué et/ou lié par un groupe alkylène en $C_1$-$C_5$ ou alcénylène en $C_2$-$C_5$ éventuellement au moins une fois substitué, comportant éventuellement au moins un hétéroatome en tant que chaînon,

de façon particulièrement préférée représente un radical alkyle en $C_1$-$C_5$ linéaire ou ramifié, un radical alcényle en $C_2$-$C_5$ linéaire ou ramifié, un radical cycloaliphatique à six chaînons, saturé ou insaturé, éventuellement au moins une fois substitué, comportant éventuellement au moins un hétéroatome en tant que chaînon formant le cycle, qui peut être lié par un groupe alkylène en $C_1$-$C_3$, ou représente un radical phényle, 1-naphtyle, 2-naphtyle, 2-furannyle, 3-furannyle, 2-thiophényle ou 3-thiophényle, qui chaque fois peut être au moins une fois substitué et/ou lié par un groupe alkylène en $C_1$-$C_5$ ou alcénylène en $C_2$-$C_5$ éventuellement au moins une fois substitué, comportant éventuellement au moins un atome d'oxygène en tant que chaînon,

**3.** Composés selon la revendication 1 ou 2, **caractérisés en ce que** le radical $R^3$ représente un atome d'hydrogène, un radical aliphatique en $C_1$-$C_{10}$ linéaire ou ramifié, saturé ou insaturé, éventuellement au moins une fois substitué, un radical cycloaliphatique à 3-7 chaînons, saturé ou insaturé, éventuellement au moins une fois substitué, comportant éventuellement au moins un hétéroatome en tant que chaînon formant le cycle, qui peut être lié par un groupe alkylène en $C_1$-$C_{10}$, alcénylène en $C_2$-$C_{10}$ ou alcynylène en $C_2$-$C_{10}$ éventuellement au moins une fois substitué, comportant éventuellement au moins un hétéroatome en tant que chaînon, ou représente un radical aryle ou hétéroaryle à 5-12 chaînons, éventuellement au moins une fois substitué, qui peut être lié par un groupe alkylène en $C_1$-$C_{10}$, alcénylène en $C_2$-$C_{10}$ ou alcynylène en $C_2$-$C_{10}$ éventuellement au moins une fois substitué, comportant éventuellement au moins un hétéroatome en tant que chaînon,

de préférence représente un atome d'hydrogène, un radical alkyle en $C_1$-$C_{10}$ linéaire ou ramifié, éventuellement au moins une fois substitué, un radical cycloaliphatique à cinq, six ou sept chaînons, saturé ou insaturé, éventuellement au moins une fois substitué, comportant éventuellement au moins un hétéroatome en tant que chaînon formant le cycle, qui peut être lié par un groupe alkylène en $C_1$-$C_{10}$, alcénylène en $C_2$-$C_{10}$ ou alcynylène en $C_2$-$C_{10}$ éventuellement au moins une fois substitué, comportant éventuellement au moins un hétéroatome en tant que chaînon, ou représente un radical aryle ou hétéroaryle à 5-12 chaînons, éventuellement au moins une fois substitué, qui peut être lié par un groupe alkylène en $C_1$-$C_{10}$, alcénylène en $C_2$-$C_{10}$ ou alcynylène en $C_2$-$C_{10}$ éventuellement au moins une fois substitué, comportant éventuellement au moins un hétéroatome en tant que chaînon,

de façon particulièrement préférée représente un atome d'hydrogène, un radical alkyle en $C_1$-$C_3$ linéaire ou ramifié, éventuellement au moins une fois substitué, un radical cycloaliphatique lié par un groupe alkylène en $C_1$-$C_3$, choisi dans l'ensemble constitué par

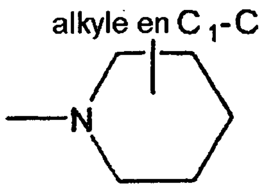 , 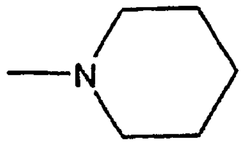 , 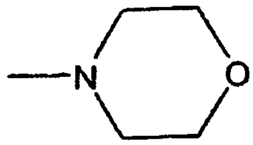 ,

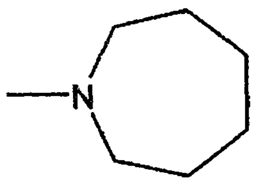 , 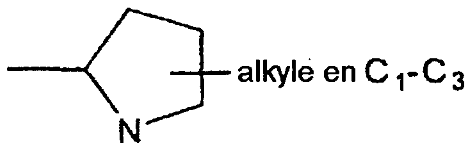 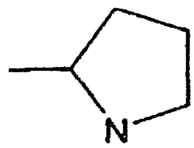 ,

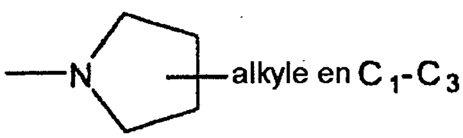 et 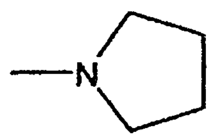 ,

ou représente un radical cycloaliphatique

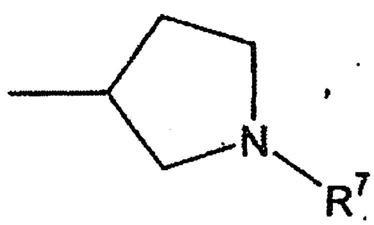 ,

le radical $R^7$ représentant un radical phényle ou benzofurannyle lié par un groupe méthylène, qui peut porter un ou plusieurs substituants, identiques ou différents, choisis dans l'ensemble constitué par F, Cl, Br, $CF_3$, $CHF_2$, $CH_2F$, phénoxy, benzyloxy, phényle, alcoxy en $C_1$-$C_4$ ou alkyl($C_1$-$C_4$)thio,
ou le radical $R^3$ représente un radical phényle éventuellement lié par un groupe méthylène, qui peut porter un ou plusieurs substituants, identiques ou différents, choisis dans l'ensemble constitué par les groupes dialkyl($C_1$-$C_3$) amino, méthoxy en $C_1$-$C_3$ et morpholinyle.

4. Composés selon l'une quelconque des revendication 1 à 3, **caractérisés en ce que** le radical $R^4$ représente un atome d'hydrogène, un radical aliphatique en $C_1$-$C_{10}$ linéaire ou ramifié, saturé ou insaturé, éventuellement au moins une fois substitué, un radical cycloaliphatique à 3-7 chaînons, saturé ou insaturé, éventuellement au moins une fois substitué, comportant éventuellement au moins un hétéroatome en tant que chaînon formant le cycle, qui peut être lié par un groupe alkylène en $C_1$-$C_{10}$, alcénylène en $C_2$-$C_{10}$ ou alcynylène en $C_2$-$C_{10}$ éventuellement au moins une fois substitué, comportant éventuellement au moins un hétéroatome en tant que chaînon, ou représente

un radical aryle ou hétéroaryle à 5-12 chaînons, éventuellement au moins une fois substitué, qui peut être lié par un groupe alkylène en $C_1$-$C_{10}$, alcénylène en $C_2$-$C_{10}$ ou alcynylène en $C_2$-$C_{10}$ éventuellement au moins une fois substitué, comportant éventuellement au moins un hétéroatome en tant que chaînon,
de préférence représente un atome d'hydrogène, un radical alkyle en $C_1$-$C_{10}$ linéaire ou ramifié, éventuellement au moins une fois substitué, un radical cycloaliphatique à cinq, six ou sept chaînons, saturé ou insaturé, éventuellement au moins une fois substitué, comportant éventuellement au moins un hétéroatome en tant que chaînon formant le cycle, qui peut être lié par un groupe alkylène en $C_1$-$C_{10}$, alcénylène en $C_2$-$C_{10}$ ou alcynylène en $C_2$-$C_{10}$ éventuellement au moins une fois substitué, comportant éventuellement au moins un hétéroatome en tant que chaînon, ou représente un radical aryle ou hétéroaryle à 5-12 chaînons, éventuellement au moins une fois substitué, qui peut être lié par un groupe alkylène en $C_1$-$C_{10}$, alcénylène en $C_2$-$C_{10}$ ou alcynylène en $C_2$-$C_{10}$ éventuellement au moins une fois substitué, comportant éventuellement au moins un hétéroatome en tant que chaînon,
de façon particulièrement préférée représente un radical alkyle en $C_1$-$C_3$ linéaire ou ramifié, éventuellement au moins une fois substitué, un radical cycloaliphatique lié par un groupe alkylène en $C_1$-$C_3$, choisi dans l'ensemble constitué par

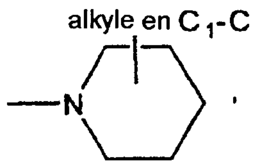 , 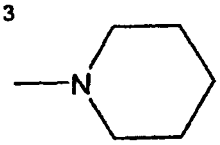 , 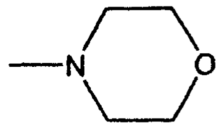 ,

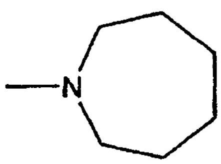 , 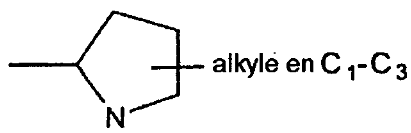 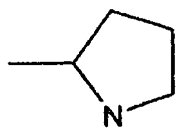 ,

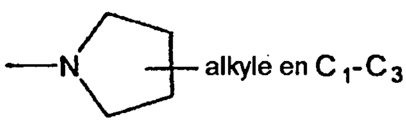 et 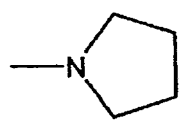 ,

ou représente un radical cycloaliphatique

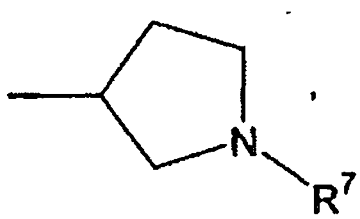 ,

le radical $R^7$ représentant un radical phényle ou benzofurannyle éventuellement lié par un groupe méthylène, qui peut porter un ou plusieurs substituants, identiques ou différents, choisis dans l'ensemble constitué par F, Cl, Br, $CF_3$, $CHF_2$, $CH_2F$, phénoxy, benzyloxy, phényle, alcoxy en $C_1$-$C_4$ ou alkyl($C_1$-$C_4$)thio,
ou le radical $R^4$ représente un radical phényle éventuellement lié par un groupe méthylène, qui peut porter un ou

plusieurs substituants, identiques ou différents, choisis dans l'ensemble constitué par les groupes dialkyl($C_1$-$C_3$) amino, méthoxy en $C_1$-$C_3$ et morpholinyle.

5. Composés selon l'une quelconque des revendications 1 à 4, **caractérisés en ce que** $R^5$ et $R^6$ forment ensemble, avec l'atome d'azote qui les relie en tant que chaînon formant le cycle, un radical hétérocyclique à 5, 6 ou 7 chaînons, saturé, insaturé ou aromatique, éventuellement au moins une fois substitué, comportant éventuellement au moins un autre hétéroatome en tant que chaînon formant le cycle,
de préférence forment ensemble, avec l'atome d'azote qui les relie en tant que chaînon formant le cycle, un radical choisi dans l'ensemble constitué par

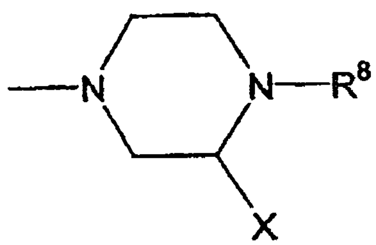 , 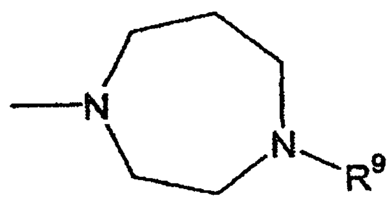

et

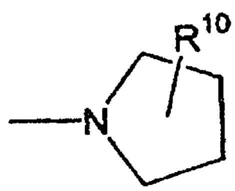

X représentant un atome d'hydrogène ou un radical alkyle en $C_1$-$C_3$ linéaire ou éventuellement ramifié, et
$R^8$, $R^9$ et $R^{10}$ représentant chacun indépendamment un radical aliphatique en $C_1$-$C_6$ linéaire ou ramifié, saturé ou insaturé, éventuellement au moins une fois substitué, un radical cycloaliphatique à 5, 6 ou 7 chaînons, saturé ou insaturé, éventuellement au moins une fois substitué, comportant éventuellement au moins un hétéroatome en tant que chaînon formant le cycle, éventuellement lié par un groupe alkylène en $C_1$-$C_6$, alcénylène en $C_2$-$C_6$ ou alcynylène en $C_2$-$C_6$ éventuellement au moins une fois substitué, comportant éventuellement au moins un hétéroatome ou éventuellement au moins un groupe carbonyle (C=O) en tant que chaînon, radical cycloaliphatique qui peut être condensé avec un système cyclique mono- ou polycyclique éventuellement au moins une fois substitué, ou représentent un radical aryle ou hétéroaryle à 5 ou 6 chaînons, éventuellement au moins une fois substitué, éventuellement lié par un groupe alkylène en $C_1$-$C_6$, alcénylène en $C_2$-$C_6$ ou alcynylène en $C_2$-$C_6$ éventuellement au moins une fois substitué, comportant éventuellement au moins un hétéroatome en tant que chaînon, radical aryle ou hétéroaryle qui peut être condensé avec un système cyclique mono- ou polycyclique éventuellement au moins une fois substitué.

6. Composés selon la revendication 5, **caractérisés en ce que**
X représente un atome d'hydrogène ou un radical méthyle,
$R^8$ représente un radical alkyle en $C_1$-$C_3$ éventuellement substitué par un groupe dialkyl($C_1$-$C_3$)amino, un radical phényle éventuellement au moins une fois substitué, un radical naphtyle éventuellement au moins une fois substitué, un radical pyridinyle éventuellement au moins une fois substitué, un radical furannyle éventuellement au moins une fois substitué, un radical thiophényle éventuellement au moins une fois substitué, un radical pyrrolidinyle éventuellement au moins une fois substitué, un radical benzo[1,3]dioxolyle éventuellement au moins une fois substitué ou un radical benzofurannyle éventuellement au moins une fois substitué, les radicaux cycliques pouvant être liés chacun par un groupe alkylène en $C_1$-$C_3$ ou alcénylène en $C_2$-$C_3$ comportant éventuellement un groupe carbonyle (C=O) en tant que chaînon et/ou porter chacun un ou plusieurs substituants, identiques ou différents, choisis dans l'ensemble constitué par -(C=O)-alkyle($C_1$-$C_3$), alcoxy en $C_1$-$C_3$, F, Cl, Br, -CN, $CF_3$, $CF_2H$ et $CFH_2$,
$R^9$ représente un radical alkyle en $C_1$-$C_3$ linéaire ou éventuellement ramifié,
$R^{10}$ représente un radical pyrrolidinyle lié par un groupe alkylène en $C_1$-$C_2$.

7. Composés selon l'une quelconque des revendications 1 à 6, **caractérisés en ce que**

$R^1$ représente un groupe $-NR^3R^4$ ou un groupe $-NR^5R^6$,

$R^2$ représente un radical alkyle en $C_1$-$C_5$ linéaire ou ramifié, représente un radical alcényle en $C_2$-$C_5$ linéaire ou ramifié, représente un radical cyclohexyle éventuellement lié par un groupe $-(CH_2)-$, représente un radical 1- ou 2-naphtyle éventuellement lié par un groupe $-(CH_2)-$ ou -(CH=CH)-, représente un radical 2-ou 3-furannyle éventuellement lié par un groupe $-(CH_2)-$ ou -(CH=CH)-, représente un radical 2- ou 3-thiényle éventuellement lié par un groupe $-(CH_2)-$ ou -(CH=CH)-, ou représente un radical phényle non substitué ou au moins une fois substitué, éventuellement lié par un groupe $-(CH_2)-$, $-(CH_2)_2-$, $-C(H)(CH_3)-$ ou $-(CH_2)-O-$, ces substituants pouvant être choisis, indépendamment les uns des autres, dans l'ensemble constitué par -F, -Cl, -Br, $-OCH_3$, $-OC_2H_5$, $CH_3$ et $C_2H_5$,

$R^3$ représente un atome d'hydrogène ou un radical méthyle,

$R^4$ représente un radical alkyle en $C_1$-$C_3$ linéaire ou ramifié, éventuellement au moins une fois substitué, un radical cycloaliphatique lié par une groupe alkylène en $C_1$-$C_3$, choisi dans l'ensemble constitué par

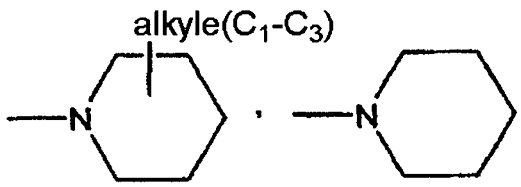

,

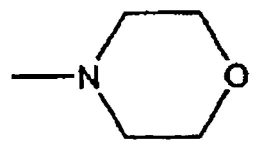

,

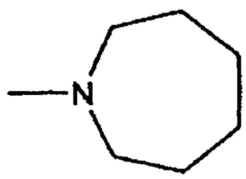

,

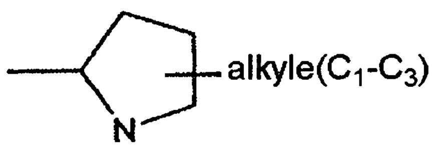

,

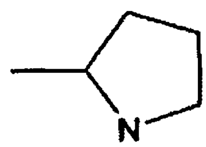

,

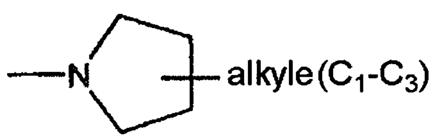

et

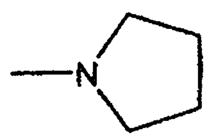

.

ou représente un radical cycloaliphatique

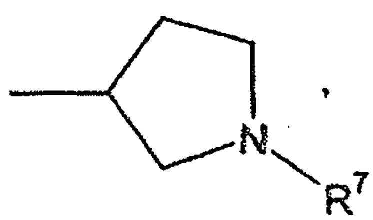

,

le radical $R^7$ représentant un radical phényle ou benzofurannyle lié par un groupe méthylène, qui peut porter un ou plusieurs substituants, identiques ou différents, choisis dans l'ensemble constitué par F, Cl, Br, $CF_3$, $CHF_2$, $CH_2F$, phénoxy, benzyloxy, phényle, alcoxy en $C_1$-$C_4$ ou alkyl($C_1$-$C_4$)thio,

ou le radical $R^4$ représente un radical phényle éventuellement lié par un groupe méthylène, qui peut porter un ou

plusieurs substituants, identiques ou différents, choisis dans l'ensemble constitué par les groupes dialkyl($C_1$-$C_3$) amino, méthoxy en $C_1$-$C_3$ et morpholinyle,
$R^5$ et $R^6$ forment ensemble, avec l'atome d'azote qui les relie, un radical choisi dans l'ensemble

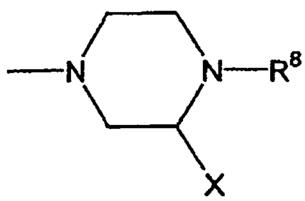 , 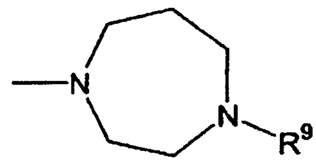

et

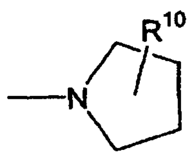

X représentant un atome d'hydrogène ou un groupe méthyle,
$R^8$ représentant un radical alkyle en $C_1$-$C_3$ linéaire ou ramifié, éventuellement substitué par un groupe diméthylamino, un radical phényle éventuellement au moins une fois substitué, un radical naphtyle éventuellement au moins une fois substitué, un radical pyridinyle éventuellement au moins une fois substitué, un radical furannyle éventuellement au moins une fois substitué, un radical thiophényle éventuellement au moins une fois substitué, un radical pyrrolidinyle éventuellement au moins une fois substitué, un radical benzo[1,3]dioxolyle éventuellement au moins une fois substitué ou un radical benzofurannyle éventuellement au moins une fois substitué, les radicaux cycliques pouvant être liés chacun par un groupe alkylène en $C_1$-$C_3$ ou alcénylène en $C_2$-$C_3$ comportant éventuellement un groupe carbonyle (C=O) en tant que chaînon et/ou porter chacun un ou plusieurs substituants, identiques ou différents, choisis dans l'ensemble constitué par -(C=O)-alkyle($C_1$-$C_3$), alcoxy en $C_1$-$C_3$, F, Cl, Br, -CN, $CF_3$, $CF_2H$ et $CFH_2$,
$R^9$ représentant un radical méthyle ou éthyle, et
$R^{10}$ représentant un radical pyrrolidinyle lié par un groupe $-(CH_2)-$.

**8.** Composés selon une ou plusieurs des revendications 1 à 7, **caractérisés en ce que**

$R^1$ représente un groupe $-NR^3R^4$ ou un groupe $-NR^5R^6$,
$R^2$ représente un radical aliphatique en $C_1$-$C_{10}$ linéaire ou ramifié, saturé ou insaturé, éventuellement substitué ; un radical cycloaliphatique saturé ou insaturé, éventuellement substitué, à 3, 4, 5, 6, 7, 8 ou 9 chaînons, qui peut être lié par un groupe alkylène en $C_1$-$C_5$, alcénylène en $C_2$-$C_5$ ou alcynylène en $C_2$-$C_5$ linéaire ou ramifié, éventuellement substitué, comportant éventuellement 1 ou 2 hétéroatome(s) en tant que chaînon(s),
ou un radical aryle ou hétéroaryle à 5-14 chaînons, éventuellement substitué, qui peut être lié par un groupe alkylène en $C_1$-$C_5$, alcénylène en $C_2$-$C_5$ ou alcynylène en $C_2$-$C_5$ linéaire ou ramifié, éventuellement substitué, comportant éventuellement 1 ou 2 hétéroatome(s) en tant que chaînon(s) ;
$R^3$ représente un atome d'hydrogène ;
un radical aliphatique en $C_1$-$C_{10}$ linéaire ou ramifié, saturé ou insaturé, éventuellement substitué ;
un radical cycloaliphatique saturé ou insaturé, éventuellement substitué, à 3, 4, 5, 6, 7, 8 ou 9 chaînons, qui peut être lié par un groupe alkylène en $C_1$-$C_5$, alcénylène en $C_2$-$C_5$ ou alcynylène en $C_2$-$C_5$ linéaire ou ramifié, éventuellement substitué, comportant éventuellement 1 ou 2 hétéroatome(s) en tant que chaînon(s),
ou un radical aryle ou hétéroaryle à 5-14 chaînons, éventuellement substitué, qui peut être lié par un groupe alkylène en $C_1$-$C_5$, alcénylène en $C_2$-$C_5$ ou alcynylène en $C_2$-$C_5$ linéaire ou ramifié, éventuellement substitué, comportant éventuellement 1 ou 2 hétéroatome(s) en tant que chaînon(s) ;
$R^4$ représente un atome d'hydrogène ;
un radical aliphatique en $C_1$-$C_{10}$ linéaire ou ramifié, saturé ou insaturé, éventuellement substitué ;
un radical cycloaliphatique saturé ou insaturé, éventuellement substitué, à 3, 4, 5, 6, 7, 8 ou 9 chaînons, qui peut être lié par un groupe alkylène en $C_1$-$C_5$, alcénylène en $C_2$-$C_5$ ou alcynylène en $C_2$-$C_5$ linéaire ou ramifié,

éventuellement substitué, comportant éventuellement 1 ou 2 hétéroatome(s) en tant que chaînon(s), ou un radical aryle ou hétéroaryle à 5-14 chaînons, éventuellement substitué, qui peut être lié par un groupe alkylène en $C_1$-$C_5$, alcénylène en $C_2$-$C_5$ ou alcynylène en $C_2$-$C_5$ linéaire ou ramifié, éventuellement substitué, comportant éventuellement 1 ou 2 hétéroatome(s) en tant que chaînon(s) ;

$R^5$ et $R^6$ forment ensemble avec l'atome d'azote qui les relie en tant que chaînon formant le cycle, un radical hétérocycloaliphatique à 4, 5, 6, 7, 8 ou 9 chaînons, saturé ou insaturé, éventuellement substitué, le radical hétérocycloaliphatique pouvant chaque fois être substitué par un radical $R^8$ et éventuellement un radical X ou un radical $R^9$ ou un radical $R^{10}$ et/ou comporter chaque fois 1, 2 ou 3 hétéroatome(s) supplémentaire(s) choisis chacun indépendamment parmi les atomes d'oxygène, d'azote et de soufre en tant que chaînon(s) formant le cycle ;

X représente un radical aliphatique en $C_1$-$C_{10}$ linéaire ou ramifié, saturé ou insaturé ;

$R^8$, $R^9$ et $R^{10}$ représentent chacun indépendamment un radical aliphatique en $C_1$-$C_{10}$ linéaire ou ramifié, saturé ou insaturé, éventuellement substitué ; un radical cycloaliphatique à 3, 4, 5, 6, 7, 8 ou 9 chaînons, saturé ou insaturé, éventuellement substitué, qui peut être lié par un groupe alkylène en $C_1$-$C_5$, alcénylène en $C_2$-$C_5$ ou alcynylène en $C_2$-$C_5$ linéaire ou ramifié, éventuellement substitué, comportant éventuellement 1 ou 2 hétéroatome(s) en tant que chaînon(s) et/ou éventuellement un groupe carbonyle (C=O) en tant que chaînon, et/ou être condensé avec un système cyclique mono- ou polycyclique saturé, insaturé ou aromatique éventuellement substitué, ou un radical aryle ou hétéroaryle à 5- 14 chaînons, éventuellement substitué, qui peut être lié par un groupe alkylène en $C_1$-$C_5$, alcénylène en $C_2$-$C_5$ ou alcynylène en $C_2$-$C_5$ linéaire ou ramifié, éventuellement substitué, comportant éventuellement 1 ou 2 hétéroatome(s) en tant que chaînon(s) et/ou éventuellement un groupe carbonyle (C=O) en tant que chaînon, et/ou être condensé avec un système cyclique mono- ou polycyclique saturé ou insaturé, éventuellement substitué ;

les radicaux aliphatiques en $C_1$-$C_{10}$ précités pouvant être substitués chacun par 1, 2, 3, 4 ou 5 substituants choisis indépendamment dans l'ensemble constitué par F, Cl, Br, -CN, $-NO_2$, hydroxy, alcoxy en $C_1$-$C_6$, $-NH_2$, -NH-alkyle ($C_1$-$C_3$), -N[alkyle($C_1$-$C_3$)][alkyle($C_1$-$C_3$)], NH-phényle, -N[alkyle($C_1$-$C_3$)]-phényle et -N(phényle)$_2$ ; les radicaux cycloaliphatiques précités pouvant être substitués chacun par 1, 2, 3, 4 ou 5 substituants choisis, indépendamment les uns des autres, dans l'ensemble constitué par F, Cl, Br, -CN, $-CF_3$, $-CHF_2$, $-CH_2F$, hydroxy, alcoxy en $C_1$-$C_6$, $-NH_2$, -NH-alkyle($C_1$-$C_3$), -N[alkyle($C_1$-$C_3$)][alkyle($C_1$-$C_3$)], NH-phényle, -N[alkyle($C_1$-$C_3$)]-phényle, -N(phényle)$_2$, -SH, -alkyl($C_1$-$C_6$)thio, -alkyle($C_1$-$C_6$), -($CH_2$)-benzo[b]furannyle, phénoxy, benzyloxy, phényle et benzyle, le fragment cyclique des radicaux, phénoxy, benzyloxy, phényle, -($CH_2$)-benzo[b]furannyle et benzyle pouvant chaque fois porter 1, 2, 3, 4 ou 5 substituants choisis, indépendamment les uns des autres, dans l'ensemble constitué par F, Cl, Br, -alkyle($C_1$-$C_4$), $-CF_3$, $-CHF_2$, $-CH_2F$, -alcoxy($C_1$-$C_4$), -alkyl($C_1$-$C_4$)thio, phénoxy, phényle et benzyloxy et les radicaux cycloaliphatiques mentionnés précédemment pouvant comporter chacun 1, 2 ou 3 hétéroatome(s) choisis chacun indépendamment dans l'ensemble constitué par les atomes d'oxygène, d'azote et de soufre ;
les groupes alkylène en $C_1$-$C_5$, alcénylène en $C_2$-$C_5$ ou alcynylène en $C_2$-$C_5$ mentionnés précédemment pouvant porter chacun 1, 2, 3, 4 ou 5 substituants choisis, indépendamment les uns des autres, dans l'ensemble constitué par F, Cl, Br, -CN, $-NO_2$, hydroxy, alcoxy en $C_1$-$C_6$, $-NH_2$, -NH-alkyle ($C_1$-$C_3$), -N[alkyle($C_1$-$C_3$)][alkyle($C_1$-$C_3$)], NH-phényle, -N[alkyle($C_1$-$C_3$)]-phényle, -N(phényle)$_2$ et phényle et
les groupes alkylène en $C_1$-$C_5$, alcénylène en $C_2$-$C_5$ ou alcynylène en $C_2$-$C_5$ mentionnés précédemment pouvant comporter chacun 1 ou 2 hétéroatome(s) choisi(s) indépendamment l'un de l'autre dans l'ensemble constitué par les atomes d'oxygène, d'azote et de soufre ;
les radicaux aryle ou hétéroaryle mentionnés précédemment pouvant être substitués chacun par 1, 2, 3, 4 ou 5 substituants choisis, indépendamment les uns des autres, dans l'ensemble constitué par F, Cl, Br, -CN, $-CF_3$, $-CHF_2$, $-CH_2F$, hydroxy, alcoxy en $C_1$-$C_6$, -(C=O)-$CH_3$, $-O-CF_3$, $-S-CF_3$, -SH, -alkyl($C_1$-$C_6$)thio, -alkyle($C_1$-$C_6$), $-NH_2$, -NH-alkyle($C_1$-$C_3$), -N[alkyle($C_1$-$C_3$)][alkyle($C_1$-$C_3$)], NH-phényle, -N[alkyle($C_1$-$C_3$)]-phényle, -N(phényle)$_2$, phénoxy, benzyloxy, phényle, benzyle et morpholinyle, le fragment cyclique des radicaux phénoxy, benzyloxy, phényle et benzyle pouvant chaque fois être substitué par 1, 2, 3, 4 ou 5 substituants choisis, indépendamment les uns des autres, dans l'ensemble constitué par F, Cl, Br, hydroxy, $-CF_3$, $-SF_5$, -CN, $-NO_2$, -alcoxy($C_1$-$C_6$), $-O-CF_3$, $-S-CF_3$, phényle et benzyloxy,
et les radicaux hétéroaryle mentionnés précédemment pouvant comporter chacun 1, 2, 3, 4 ou 5 hétéroatome(s) choisis chacun indépendamment dans l'ensemble constitué par les atomes d'oxygène, d'azote et de soufre en tant que chaînon(s) formant le cycle ;
et les cycles des systèmes cycliques mono- ou polycycliques mentionnés précédemment pouvant éventuellement être substitués chacun par 1, 2, 3, 4 ou 5 substituants choisis, indépendamment les uns des autres, dans l'ensemble constitué par F, Cl, Br, -CN, $-CF_3$, $-CHF_2$, $-CH_2F$, hydroxy, alcoxy en $C_1$-$C_6$, $-O-CF_3$, $-S-CF_3$, -SH, -alkyl ($C_1$-$C_6$) thio, -alkyle ($C_1$-$C_6$), $-NH_2$, -NH-alkyle($C_1$-$C_3$), -N[alkyle($C_1$-$C_3$)][alkyle($C_1$-$C_3$)], NH-phényle, -N[alkyle($C_1$-$C_3$)]-phényle, -N(phényle)$_2$, phénoxy, benzyloxy, phényle, benzyle et morpholinyle, le fragment cyclique des radicaux phénoxy, benzyloxy, phényle et benzyle pouvant chaque fois être substitué par 1, 2, 3, 4 ou 5 substituants choisis, indépendamment les uns des autres, dans l'ensemble constitué par F, Cl, Br, hydroxy, $-CF_3$, $-SF_5$, -CN, $-NO_2$, -alcoxy($C_1$-$C_6$), $-O-CF_3$, $-S-CF_3$, phényle et benzyloxy,

et les cycles des systèmes cycliques mono- ou polycycliques mentionnés précédemment pouvant comporter chacun en tant que chaînon(s) formant le cycle 1, 2, 3, 4 ou 5 hétéroatome(s) qui sont, indépendamment les uns des autres, choisis dans l'ensemble constitué par les atomes d'oxygène, d'azote et de soufre ;

chacun éventuellement sous forme de ses stéréoisomères, en particulier énantiomères ou diastéréoisomères, purs, de ses racémates ou sous forme d'un mélange de stéréoisomères, en particulier des énantiomères et/ou diastéréoisomères, en un rapport de mélange quelconque, ou chacun sous forme des sels correspondants, ou chacun sous forme de produits de solvatation correspondants.

**9.** Composés selon une ou plusieurs des revendications 1 à 8, **caractérisés en ce que** le radical

$R^2$ représente un radical alkyle choisi dans l'ensemble constitué par les groupes méthyle, éthyle, n-propyle, isopropyle, n-butyle, sec-butyle, isobutyle, tert-butyle, n-pentyle, sec-pentyle, (1,1)-diméthylpropyle et n-hexyle, le radical alkyle pouvant chaque fois être substitué par 1, 2, 3, 4 ou 5 substituants choisis, indépendamment les uns des autres, dans l'ensemble constitué par F, Cl, Br, -CN, $-NO_2$, hydroxy, alcoxy en $C_1$-$C_6$, $-NH_2$, -NH-alkyle($C_1$-$C_3$), -N[alkyle($C_1$-$C_3$)][alkyle($C_1$-$C_3$)], NH-phényle, -N[alkyle($C_1$-$C_3$)]-phényle et -N(phényle)$_2$ ;

un radical alcényle choisi dans l'ensemble constitué par les groupes vinyle, 1-propényle, 2-propényle, 1-butényle, 2-butényle, 3-butényle, 1-pentényle, 2-pentényle et pent-1,3-diényle, le radical alcényle pouvant chaque fois être substitué par 1, 2, 3, 4 ou 5 substituants choisis, indépendamment les uns des autres, dans l'ensemble constitué par F, Cl, Br, -CN, $-NO_2$, hydroxy, alcoxy en $C_1$-$C_6$, $-NH_2$, -NH-alkyle($C_1$-$C_3$), -N[alkyle($C_1$-$C_3$)][alkyle($C_1$-$C_3$)], NH-phényle, -N[alkyle($C_1$-$C_3$)]-phényle et -N(phényle)$_2$ ;

un radical (hétéro)cycloaliphatique choisi dans l'ensemble constitué par les groupes cyclopropyle, cyclobutyle, cyclopentyle, cyclohexyle, cycloheptyle, cyclopentényle, cyclohexényle, cycloheptényle, imidazolidinyle, tétrahydrofurannyle, tétrahydrothiophényle, pyrrolidinyle, pipéridinyle, morpholinyle, pipérazinyle, thiomorpholinyle, tétrahydropyrannyle, azépanyle, diazépanyle et dithiolanyle, le radical (hétéro)cycloaliphatique pouvant chaque fois être lié par un groupe alkylène en $C_1$-$C_5$ linéaire ou ramifié, non substitué, et/ou être substitué par 1, 2, 3, 4 ou 5 substituants choisis, indépendamment les uns des autres, dans l'ensemble constitué par F, Cl, Br, -CN, $-CF_3$, $-CHF_2$, $-CH_2F$, hydroxy, alcoxy en $C_1$-$C_6$, $-NH_2$, -NH-alkyle($C_1$-$C_3$), -N[alkyle($C_1$-$C_3$)][alkyle($C_1$-$C_3$)], NH-phényle, -N[alkyle ($C_1$-$C_3$)]-phényle, -N(phényle)$_2$, -SH, -alkyl($C_1$-$C_6$)thio, -alkyle($C_1$-$C_6$), -($CH_2$)-benzo[b]furannyle, phénoxy, benzyloxy, phényle et benzyle, le fragment cyclique des radicaux phénoxy, benzyloxy, phényle, -($CH_2$)-benzo[b]furannyle et benzyle pouvant chaque fois être substitué par 1, 2, 3, 4 ou 5 substituants choisis, indépendamment les uns des autres, dans l'ensemble constitué par F, Cl, Br, -alkyle($C_1$-$C_4$), $-CF_3$, $-CHF_2$, $-CH_2F$, -alcoxy($C_1$-$C_4$), -alkyl($C_1$-$C_4$) thio, phénoxy, phényle et benzyloxy ;

ou un radical choisi dans l'ensemble constitué par les groupes phényle, naphtyle, thiophényle, furannyle, pyrrolyle, pyrazolyle, pyrazinyle, pyrannyle, triazolyle, pyridinyle, imidazolyle, indolyle, iso-indolyle, benzo[b]furannyle, benzo [b]thiophényle, thiazolyle, oxazolyle, isoxazolyle, pyridazinyle, pyrazinyle, pyrimidinyle, indazolyle, quinazolinyle, quinolinyle et isoquinolinyle, le radical pouvant chaque fois être lié par un groupe alkylène en $C_1$-$C_5$ ou alcénylène en $C_2$-$C_5$ linéaire ou ramifié, non substitué, comportant éventuellement un atome d'oxygène en tant que chaînon et/ou être substitué par 1, 2, 3, 4 ou 5 substituants choisis, indépendamment les uns des autres, dans l'ensemble constitué par F, Cl, Br, -CN, $-CF_3$, $-CHF_2$, $-CH_2F$, hydroxy, alcoxy en $C_1$-$C_6$, $-O-CF_3$, $-S-CF_3$, -SH, -alkyl($C_1$-$C_6$) thio, -alkyle($C_1$-$C_6$), $-NH_2$, -NH-alkyle($C_1$-$C_3$), -N[alkyle($C_1$-$C_3$)][alkyle($C_1$-$C_3$)], NH-phényle, -N[alkyle($C_1$-$C_3$)]-phényle, -N(phényle)$_2$, phénoxy, benzyloxy, phényle, benzyle et morpholinyle, le fragment cyclique des radicaux phénoxy, benzyloxy, phényle et benzyle pouvant chaque fois être substitué par 1, 2, 3, 4 ou 5 substituants choisis, indépendamment les uns des autres, dans l'ensemble constitué par F, Cl, Br, -hydroxy, $-CF_3$, $-SF_5$, -CN, $-NO_2$, -alcoxy($C_1$-$C_6$), $-O-CF_3$, $-S-CF_3$, phényle et benzyloxy ;

de préférence $R^2$

représente un radical alkyle choisi dans l'ensemble constitué par les groupes méthyle, éthyle, n-propyle, isopropyle, n-butyle, sec-butyle, isobutyle, tert-butyle, n-pentyle, sec-pentyle, (1,1)-diméthylpropyle et n-hexyle ;

un radical alcényle choisi dans l'ensemble constitué par les groupes vinyle, 1-propényle, 2-propényle, 1-butényle, 2-butényle, 3-butényle, 1-pentényle, 2-pentényle et pent-1,3-diényle ;

un radical cycloaliphatique choisi dans l'ensemble constitué par les groupes cyclopropyle, cyclobutyle, cyclopentyle, cyclohexyle, cycloheptyle, cyclopentényle, cyclohexényle et cycloheptényle, qui peut chaque fois être lié par un groupe $-(CH_2)-$, $-(CH_2)_2-$, $-CH(CH_3)-$ ou $(CH_2)_3$ et/ou être substitué par 1, 2, 3, 4 ou 5 substituants choisis, indépendamment les uns des autres, dans l'ensemble constitué par F, Cl, Br, -CN, $-CF_3$, $-CHF_2$, $-CH_2F$, hydroxy, $-O-CH_3-$, $-O-C_2H_5$, $-O-C_3H_7$, $-NH_2$, $-NH-CH_3$, $-NH-C_2H_5$, $-N-(CH_3)_2$, $-N(C_2H_5)_2$, $-N(CH_3)(C_2H_5)$, -NH-phényle, -N ($CH_3$)-phényle, -N($C_2H_5$)-phényle, -N(phényle)$_2$, -SH, $-S-CH_3$, $-S-C_2H_5$, $-S-C_3H_7$, $-S-C(CH_3)_3$, méthyle, éthyle, n-propyle, isopropyle, n-butyle, sec-butyle et tert-butyle ;

ou un radical choisi dans l'ensemble constitué par les groupes phényle, 1-naphtyle, 2-naphtyle, 2-furannyle, 3-furannyle, 2-thiophényle ou 3-thiophényle, qui peut être lié par un groupe $-(CH_2)-$, $-(CH_2)_2-$, $-(CH_2)-O-$, $-CH(CH_3)-$, -(CH=CH)- ou $-(CH_2)_3-$ et/ou être substitué par 1, 2, 3, 4 ou 5 substituants choisis, indépendamment les uns des autres, dans l'ensemble constitué par F, Cl, Br, -CN, $-CF_3$, $-CHF_2$, $-CH_2F$, hydroxy, $-O-CH_3-$, $-O-C_2H_5$, $-O-C_3H_7$, $-O-CF_3$, $-S-CF_3$, -SH, $-S-CH_3$, $-S-C_2H_5$, $-S-C_3H_7$, $-S-C(CH_3)_3$, méthyle, éthyle, n-propyle, isopropyle, n-butyle, sec-butyle, tert-butyle, $-NH_2$, $-NH-CH_3$, $-NH-C_2H_5$, $-N-(CH_3)_2$, $-N(C_2H_5)_2$, $-N(CH_3)(C_2H_5)$, -NH-phényle, $-N(CH_3)$-phényle, $-N(C_2H_5)$-phényle, $-N(phényle)_2$, phénoxy, benzyloxy, phényle, benzyle et morpholinyle ;
de façon particulièrement préférée $R^2$
représente un radical alkyle choisi dans l'ensemble constitué par les groupes n-propyle, isopropyle, n-butyle, sec-butyle, isobutyle, tert-butyle, n-pentyle, sec-pentyle, (1,1)-diméthylpropyle ;
un radical alcényle choisi dans l'ensemble constitué par les groupes 1-pentényle, 2-pentényle et pent-1,3-diényle ;
un radical cycloaliphatique choisi dans l'ensemble constitué par les groupes cyclopentyle, cyclohexyle et cycloheptyle, qui est lié par un groupe $-(CH_2)-$ ;
ou un radical choisi dans l'ensemble constitué par les groupes phényle, 1-naphtyle, 2-naphtyle, 2-furannyle, 3-furannyle, 2-thiophényle ou 3-thiophényle, qui peut être lié par un groupe $-(CH_2)-$, $-(CH_2)_2-$, $(CH_2)-O-$, $-CH(CH_3)-$, -(CH=CH)- ou $-(CH_2)_3-$ et/ou être substitué par 1, 2 ou 3 substituants choisis, chacun indépendamment, dans l'ensemble constitué par F, Cl, Br, $-O-CH_3-$, $-O-C_2H_5$, méthyle, éthyle, n-propyle, et isopropyle.

**10.** Composés selon une ou plusieurs des revendications 1 à 9, **caractérisés en ce que** le radical
$R^3$ représente un atome d'hydrogène ;
un radical alkyle choisi dans l'ensemble constitué par les groupes méthyle, éthyle, n-propyle, isopropyle, n-butyle, sec-butyle, isobutyle, tert-butyle, n-pentyle, sec-pentyle, (1,1)-diméthylpropyle et n-hexyle, le radical alkyle pouvant chaque fois être substitué par 1, 2, 3, 4 ou 5 substituants choisis, indépendamment les uns des autres, dans l'ensemble constitué par F, Cl, Br, -CN, $-NO_2$, hydroxy, alcoxy en $C_1$-$C_6$, $-NH_2$,- NH-alkyle($C_1$-$C_3$), -N[alkyle($C_1$-$C_3$)][alkyle($C_1$-$C_3$)], NH-phényle, -N[alkyle($C_1$-$C_3$)]-phényle et $-N(phényle)_2$ ;
un radical alcényle choisi dans l'ensemble constitué par les groupes vinyle, 1-propényle, 2-propényle, 1-butényle, 2-butényle, 3-butényle, 1-pentényle, 2-pentényle et pent-1,3-diényle, le radical alcényle pouvant chaque fois être substitué par 1, 2, 3, 4 ou 5 substituants choisis, indépendamment les uns des autres, dans l'ensemble constitué par F, Cl, Br, -CN, $-NO_2$, hydroxy, alcoxy en $C_1$-$C_6$, $-NH_2$, -NH-alkyle($C_1$-$C_3$), -N[alkyle($C_1$-$C_3$)][alkyle($C_1$-$C_3$)], NH-phényle, -N[alkyle($C_1$-$C_3$)]-phényle et $-N(phényle)_2$ ;
un radical (hétéro)cycloaliphatique choisi dans l'ensemble constitué par les groupes cyclopropyle, cyclobutyle, cyclopentyle, cyclohexyle, cycloheptyle, cyclopentényle, cyclohexényle, cycloheptényle, imidazolidinyle, tétrahydrofurannyle, tétrahydrothiophényle, pyrrolidinyle, pipéridinyle, morpholinyle, pipérazinyle, thiomorpholinyle, tétrahydropyrannyle, azépanyle, diazépanyle et dithiolanyle, le radical (hétéro)cycloaliphatique pouvant chaque fois être lié par un groupe alkylène en $C_1$-$C_5$ linéaire ou ramifié, non substitué et/ou être substitué par 1, 2, 3, 4 ou 5 substituants choisis, indépendamment les uns des autres, dans l'ensemble constitué par F, Cl, Br, -CN, $-CF_3$, $-CHF_2$, $-CH_2F$, hydroxy, alcoxy en $C_1$-$C_6$, $-NH_2$, -NH-alkyle ($C_1$-$C_3$), -N[alkyle($C_1$-$C_3$)][alkyle($C_1$-$C_3$)], NH-phényle, -N[alkyle ($C_1$-$C_3$)]-phényle, $-N(phényle)_2$, -SH, -alkyl($C_1$-$C_6$)thio, -alkyle($C_1$-$C_6$), $-(CH_2)$-benzo[b]furannyle, phénoxy, benzyloxy, phényle et benzyle, le fragment cyclique des radicaux phénoxy, benzyloxy, phényle, $-(CH_2)$-benzo[b]furannyle et benzyle pouvant chaque fois être substitué par 1, 2, 3, 4 ou 5 substituants choisis, indépendamment les uns des autres, dans l'ensemble constitué par F, Cl, Br, -alkyle($C_1$-$C_4$), $-CF_3$, $-CHF_2$, $-CH_2F$, -alcoxy($C_1$-$C_4$), -alkyl($C_1$-$C_4$) thio, phénoxy, phényle et benzyloxy ;
ou un radical choisi dans l'ensemble constitué par les groupes phényle, naphtyle, thiophényle, furannyle, pyrrolyle, pyrazolyle, pyrazinyle, pyrannyle, triazolyle, pyridinyle, imidazolyle, indolyle, iso-indolyle, benzo[b]furannyle, benzo [b]thiophényle, thiazolyle, oxazolyle, isoxazolyle, pyridazinyle, pyrazinyle, pyrimidinyle, indazolyle, quinazolinyle, quinolinyle et isoquinolinyle, le radical pouvant chaque fois être lié par un groupe alkylène en $C_1$-$C_5$ ou alcénylène en $C_2$-$C_5$ linéaire ou ramifié, non substitué, comportant éventuellement un atome d'oxygène en tant que chaînon et/ou être substitué par 1, 2, 3, 4 ou 5 substituants choisis, indépendamment les uns des autres, dans l'ensemble constitué par F, Cl, Br, -CN, $-CF_3$, $-CHF_2$, $-CH_2F$, hydroxy, alcoxy en $C_1$-$C_6$, $-O-CF_3$, $-S-CF_3$, -SH, -alkyl($C_1$-$C_6$) thio, -alkyle($C_1$-$C_6$), $-NH_2$, -NH-alkyle($C_1$-$C_3$), -N[alkyle($C_1$-$C_3$)][alkyle($C_1$-$C_3$)], NH-phényle, -N[alkyle($C_1$-$C_3$)]-phényle, $-N(phényle)_2$, phénoxy, benzyloxy, phényle, benzyle et morpholinyle, le fragment cyclique des radicaux phénoxy, benzyloxy, phényle et benzyle pouvant chaque fois être substitué par 1, 2, 3, 4 ou 5 substituants choisis, indépendamment les uns des autres, dans l'ensemble constitué par F, Cl, Br, -hydroxy, $-CF_3$, $-SF_5$, -CN, $-NO_2$, -alcoxy($C_1$-$C_6$), $-O-CF_3$, $-S-CF_3$, phényle et benzyloxy ;
de préférence $R^3$
représente un atome d'hydrogène ;
un radical alkyle choisi dans l'ensemble constitué par les groupes méthyle, éthyle, n-propyle, n-butyle et n-pentyle, le radical alkyle pouvant chaque fois être substitué par 1 ou 2 substituants choisis, indépendamment l'un de l'autre,

dans l'ensemble constitué par $-NH_2$, $-NH-CH_3$, $-NH-C_2H_5$, $-N-(CH_3)_2$,- $N(C_2H_5)_2$, $-N(CH_3)(C_2H_5)$, -NH-phényle, -N ($CH_3$)-phényle, $-N(C_2H_5)$-phényle et -N(phényle)$_2$ ;
un radical choisi dans l'ensemble constitué par

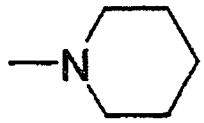 , 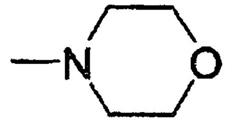 ,

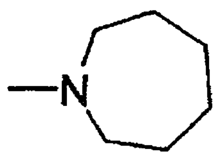 , 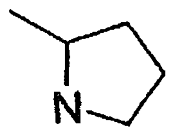 et 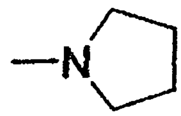 ,

qui peut être lié par un groupe $-(CH_2)-$, $-(CH_2)_2-$, $-(CH_2)-O-$, $-CH(CH_3)-$, $-(CH=CH)-$ ou $-(CH_2)_3-$ et/ou être substitué par 1, 2 ou 3 substituants choisis, chacun indépendamment, dans l'ensemble constitué par les groupes méthyle, éthyle et n-propyle ;
représente le radical suivant,

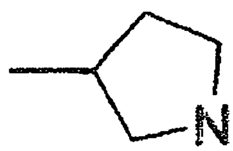 ,

qui peut être substitué par un substituant choisi dans l'ensemble constitué par les groupes $-(CH_2)$-benzo[b]furannyle et benzyle, le fragment cyclique de chacun des radicaux $-(CH_2)$-benzofurannyle et benzyle pouvant être substitué par 1, 2, 3, 4 ou 5 substituants choisis, indépendamment les uns des autres, dans l'ensemble constitué par F, Cl, Br, -alkyle($C_1$-$C_4$), $-CF_3$, $-CHF_2$, $-CH_2F$, -alcoxy($C_1$-$C_4$), -alkyl($C_1$-$C_4$)thio, phénoxy, phényle et benzyloxy ;
ou un radical choisi dans l'ensemble constitué par les groupes phényle et naphtyle, le radical pouvant être lié chaque fois par un groupe $-(CH_2)-$, $-(CH_2)_2-$ ou- $(CH_2)_3-$ et/ou substitué par 1, 2 ou 3 substituants choisis, chacun indépendamment, dans l'ensemble constitué par -alcoxy($C_1$-$C_6$), -alkyle($C_1$-$C_6$), $-NH_2$, -NH-alkyle($C_1$-$C_3$), -N[alkyle($C_1$-$C_3$)] [alkyle($C_1$-$C_3$)], NH-phényle, -N[alkyle($C_1$-$C_3$)]-phényle, -N(phényle)$_2$ et morpholinyle ;
de façon particulièrement préférée $R^3$
représente un atome d'hydrogène ;
un radical choisi dans l'ensemble constitué par les groupes méthyle, éthyle, n-propyle, $-(CH_2)-N(CH_3)_2$, $-(CH_2-(CH_2)-N(CH_3)_2$, $-(CH_2)-(CH_2)-(CH_2)-N(CH_3)_2$, $(CH_2)-(CH_2)-N(C_2H_5)_2$, $-(CH_2)-(CH_2)-(CH_2)-N(C_2H_5)_2$, $-(CH_2)-(CH_2)-N(CH_3)$ (phényle) et $-(CH_2)-(CH_2)-(CH_2)-N(CH_3)$(phényle) ;
un radical choisi dans l'ensemble constitué par

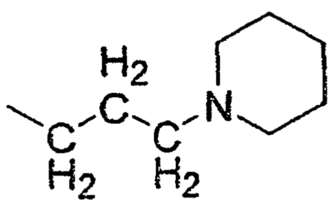 , 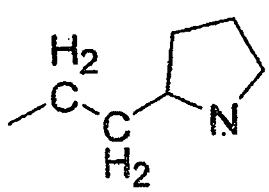 , 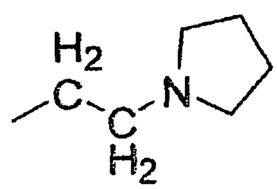 ,

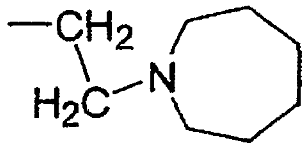 et 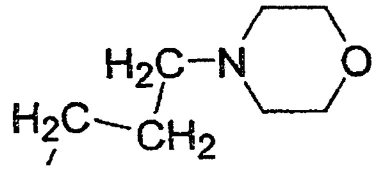 ,

qui peut être substitué par un radical méthyle ;
le radical suivant,

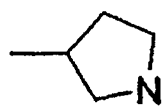 ,

qui peut être substitué sur l'atome d'azote par un substituant choisi dans l'ensemble constitué par les groupes $-(CH_2)$-benzo[b]furannyle et benzyle, le fragment cyclique respectif des radicaux $-(CH_2)$-benzo[b]furannyle et benzyle pouvant être substitué par 1, 2 ou 3 substituants choisis, chacun indépendamment, dans l'ensemble constitué par F, Cl, Br, $-CF_3$, $-O-CH_3$, $-O-C_2H_5$, $-O-C_3H_7$, $-S-CH_3$, $-S-C_2H_5$, $-S-C_3H_7$, $-S-C(CH_3)_3$, méthyle, éthyle, n-propyle, isopropyle, n-butyle, phénoxy et benzyloxy ;
ou un radical choisi dans l'ensemble constitué par les groupes phényle et benzyle, le fragment cyclique des radicaux phényle et benzyle pouvant être substitué par 1, 2 ou 3 substituants choisis, chacun indépendamment, dans l'ensemble constitué par les groupes $-O-CH_3$, $-O-C_2H_5$, $-O-C_3H_7$, $-N-(CH_3)_2$, $-N(C_2H_5)_2$ et morpholinyle ;
de façon particulièrement préférée
$R^3$ représente un atome d'hydrogène
ou un radical méthyle.

**11.** Composés selon une ou plusieurs des revendications 1 à 10, **caractérisés en ce que** le radical
$R^4$ représente un atome d'hydrogène ;
un radical alkyle choisi dans l'ensemble constitué par les groupes méthyle, éthyle, n-propyle, isopropyle, n-butyle, sec-butyle, isobutyle, tert-butyle, n-pentyle, sec-pentyle, (1,1)-diméthylpropyle et n-hexyle, le radical alkyle pouvant chaque fois être substitué par 1, 2, 3, 4 ou 5 substituants choisis, indépendamment les uns des autres, dans l'ensemble constitué par F, Cl, Br, -CN, $-NO_2$, hydroxy, alcoxy en $C_1$-$C_6$, $-NH_2$, -NH-alkyle ($C_1$-$C_3$), -N[alkyle($C_1$-$C_3$)] [alkyle($C_1$-$C_3$)], NH-phényle, -N[alkyle($C_1$-$C_3$)]-phényle et -N(phényle)$_2$ ;
un radical alcényle choisi dans l'ensemble constitué par les groupes vinyle, 1-propényle, 2-propényle, 1-butényle, 2-butényle, 3-butényle, 1-pentényle, 2-pentényle et pent-1,3-diényle, le radical alcényle pouvant chaque fois être substitué par 1, 2, 3, 4 ou 5 substituants choisis, indépendamment les uns des autres, dans l'ensemble constitué par F, Cl, Br, -CN, $-NO_2$, hydroxy, alcoxy en $C_1$-$C_6$, $-NH_2$, -NH-alkyle($C_1$-$C_3$), -N[alkyle($C_1$-$C_3$)][alkyle($C_1$-$C_3$)], NH-phényle, -N[alkyle($C_1$-$C_3$)]-phényle et -N(phényle)$_2$ ;
un radical (hétéro)cycloaliphatique choisi dans l'ensemble constitué par les groupes cyclopropyle, cyclobutyle, cyclopentyle, cyclohexyle, cycloheptyle, cyclopentényle, cyclohexényle, cycloheptényle, imidazolidinyle, tétrahydrofurannyle, tétrahydrothiophényle, pyrrolidinyle, pipéridinyle, morpholinyle, pipérazinyle, thiomorpholinyle, tétrahydropyrannyle, azépanyle, diazépanyle et dithiolanyle, le radical (hétéro)cycloaliphatique pouvant chaque fois être lié par un groupe alkylène en $C_1$-$C_5$ linéaire ou ramifié, non substitué et/ou être substitué par 1, 2, 3, 4 ou 5 substituants choisis, indépendamment les uns des autres, dans l'ensemble constitué par F, Cl, Br, -CN, $-CF_3$, $-CHF_2$, $-CH_2F$, hydroxy, alcoxy en $C_1$-$C_6$, $-NH_2$, -NH-alkyle($C_1$-$C_3$), -N[alkyle($C_1$-$C_3$)][alkyle($C_1$-$C_3$)], NH-phényle, -N[alkyle ($C_1$-$C_3$)]-phényle, -N(phényle)$_2$, -SH, -alkyl($C_1$-$C_6$)thio, -alkyle($C_1$-$C_6$), $-(CH_2)$-benzo[b]furannyle, phénoxy, benzyloxy, phényle et benzyle, le fragment cyclique des radicaux phénoxy, benzyloxy, phényle, $-(CH_2)$-benzo[b]furannyle et benzyle pouvant chaque fois être substitué par 1, 2, 3, 4 ou 5 substituants choisis, indépendamment les uns des autres, dans l'ensemble constitué par F, Cl, Br, -alkyle($C_1$-$C_4$), $-CF_3$, $-CHF_2$, $-CH_2F$, -alcoxy ($C_1$-$C_4$), -alkyl($C_1$-$C_4$) thio, phénoxy, phényle et benzyloxy ;
ou un radical choisi dans l'ensemble constitué par les groupes phényle, naphtyle, thiophényle, furannyle, pyrrolyle, pyrazolyle, pyrazinyle, pyrannyle, triazolyle, pyridinyle, imidazolyle, indolyle, iso-indolyle, benzo[b]furannyle, benzo [b]thiophényle, thiazolyle, oxazolyle, isoxazolyle, pyridazinyle, pyrazinyle, pyrimidinyle, indazolyle, quinazolinyle, quinolinyle et isoquinolinyle, le radical pouvant chaque fois être lié par un groupe alkylène en $C_1$-$C_5$ ou alcénylène

en $C_2$-$C_5$ linéaire ou ramifié, non substitué, comportant éventuellement un atome d'oxygène en tant que chaînon et/ou être substitué par 1, 2, 3, 4 ou 5 substituants choisis, indépendamment les uns des autres, dans l'ensemble constitué par F, Cl, Br, -CN, $-CF_3$, $-CHF_2$, $-CH_2F$, hydroxy, alcoxy en $C_1$-$C_6$, $-O-CF_3$, $-S-CF_3$, -SH, -alkyl($C_1$-$C_6$) thio, -alkyle($C_1$-$C_6$), $-NH_2$, -NH-alkyle($C_1$-$C_3$), -N[alkyle($C_1$-$C_3$)][alkyle($C_1$-$C_3$)], NH-phényle, -N[alkyle($C_1$-$C_3$)]-phényle, -N(phényle)$_2$, phénoxy, benzyloxy, phényle, benzyle et morpholinyle, le fragment cyclique des radicaux phénoxy, benzyloxy, phényle et benzyle pouvant chaque fois être substitué par 1, 2, 3, 4 ou 5 substituants choisis, indépendamment les uns des autres, dans l'ensemble constitué par F, Cl, Br, hydroxy, $-CF_3$, $-SF_5$, -CN, $-NO_2$, -alcoxy($C_1$-$C_6$), $-O-CF_3$, $-S-CF_3$, phényle et benzyloxy ;

de préférence $R^4$

représente un atome d'hydrogène ;

un radical alkyle choisi dans l'ensemble constitué par les groupes méthyle, éthyle, n-propyle, n-butyle et n-pentyle, le radical alkyle pouvant chaque fois être substitué par 1 ou 2 substituants choisis, indépendamment l'un de l'autre, dans l'ensemble constitué par $-NH_2$, $-NH-CH_3$, $-NH-C_2H_5$, $-N-(CH_3)_2$, $-N(C_2H_5)_2$, $-N(CH_3)(C_2H_5)$, -NH-phényle, -N ($CH_3$)-phényle, -N($C_2H_5$)-phényle et -N(phényle)$_2$ ;

un radical choisi dans l'ensemble constitué par

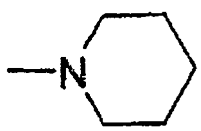 , 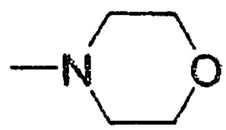 ,

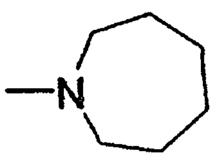 , 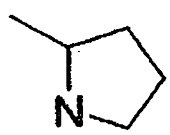 et 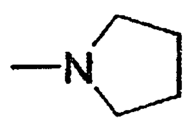 ,

qui peut être lié par un groupe $-(CH_2)-$, $-(CH_2)_2-$, $-(CH_2)-O-$, $-CH(CH_3)-$, $-(CH=CH)-$ ou $-(CH_2)_3-$ et/ou être substitué par 1, 2 ou 3 substituants choisis, chacun indépendamment, dans l'ensemble constitué par les groupes méthyle, éthyle et n-propyle ;

représente le radical suivant,

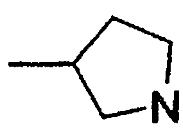 ,

qui peut être substitué par un substituant choisi dans l'ensemble constitué par les groupes $-(CH_2)$-benzo[b]furannyle et benzyle, le fragment cyclique de chacun des radicaux $-(CH_2)$-benzofurannyle et benzyle pouvant être substitué par 1, 2, 3, 4 ou 5 substituants choisis, indépendamment les uns des autres, dans l'ensemble constitué par F, Cl, Br, -alkyle($C_1$-$C_4$), $-CF_3$, $-CHF_2$, $-CH_2F$, -alcoxy($C_1$-$C_4$), -alkyl($C_1$-$C_4$)thio, phénoxy, phényle et benzyloxy ;

ou un radical choisi dans l'ensemble constitué par les groupes phényle et naphtyle, le radical pouvant être lié chaque fois par un groupe $-(CH_2)-$, $-(CH_2)_2-$ ou $-(CH_2)_3-$ et/ou substitué par 1, 2 ou 3 substituants choisis, indépendamment les uns des autres, dans l'ensemble constitué par -alcoxy($C_1$-$C_6$), -alkyle($C_1$-$C_6$), $-NH_2$, -NH-alkyle($C_1$-$C_3$), -N[alkyle ($C_1$-$C_3$)][alkyle($C_1$-$C_3$)], NH-phényle,- N[alkyle($C_1$-$C_3$)]-phényle, -N(phényle)$_2$ et morpholinyle ;

de façon particulièrement préférée $R^4$

représente un radical choisi dans l'ensemble constitué par les groupes méthyle, éthyle, n-propyle, $-(CH_2)-N(CH_3)_2$, $-(CH_2)-(CH_2)-N(CH_3)_2$, $-(CH_2)-(CH_2)-(CH_2)-N(CH_3)_2$, $-(CH_2)-(CH_2)-N(C_2H_5)_2$, $-(CH_2)-(CH_2)-(CH_2)-N(C_2H_5)_2$, $-(CH_2)-(CH_2)-N(CH_3)$ (phényle) et $-(CH_2)-(CH_2)-(CH_2)-N(CH_3)$(phényle) ;

un radical choisi dans l'ensemble constitué par

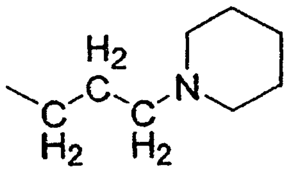 , 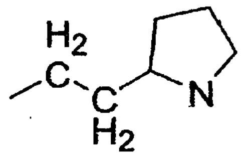 , 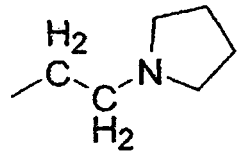 ,

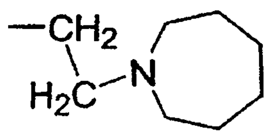 et 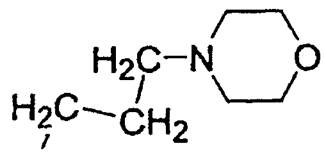 ,

qui peut être substitué par un radical méthyle ; le radical suivant,

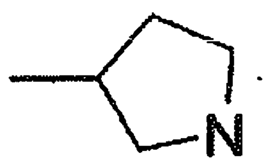 ,

qui peut être substitué sur l'atome d'azote par un substituant choisi dans l'ensemble constitué par les groupes $-(CH_2)$-benzo[b]furannyle et benzyle, le fragment cyclique respectif des radicaux $-(CH_2)$-benzo[b]furannyle et benzyle pouvant être substitué par 1, 2 ou 3 substituants choisis, chacun indépendamment, dans l'ensemble constitué par F, Cl, Br, $-CF_3$, $-O-CH_3$, $-O-C_2H_5$, $-O-C_3H_7$, $-S-CH_3$, $-S-C_2H_5$, $-S-C_3H_7$, $-S-C(CH_3)_3$, méthyle, éthyle, n-propyle, isopropyle, n-butyle, phénoxy et benzyloxy ;
ou un radical choisi dans l'ensemble constitué par les groupes phényle et benzyle, le fragment cyclique des radicaux phényle et benzyle pouvant être substitué par 1, 2 ou 3 substituants choisis, chacun indépendamment, dans l'ensemble constitué par les groupes $-O-CH_3$, $-O-C_2H_5$, $-O-C_3H_7$, $-N-(CH_3)_2$, $-N(C_2H_5)_2$ et morpholinyle.

**12.** Composés selon une ou plusieurs des revendications 1 à 11, **caractérisés en ce que** les radicaux $R^5$ et $R^6$ forment ensemble, avec l'atome d'azote qui les relie en tant que chaînon formant le cycle, un radical hétérocycloaliphatique choisi dans l'ensemble constitué par les groupes pipérazinyle, morpholinyle, thiomorpholinyle, pyrrolidinyle, azépanyle, diazépanyle et pipéridinyle, les radicaux hétérocycloaliphatique pouvant être substitués par un radical $R^8$ et éventuellement un radical X ou un radical $R^9$ ou un radical $R^{10}$ ;
de façon particulièrement préférée $R^5$ et $R^6$ forment ensemble, avec l'atome d'azote qui les relie en tant que chaînon formant le cycle, l'un des radicaux suivants

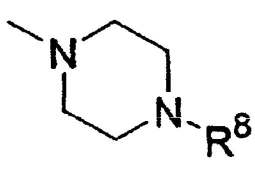 , 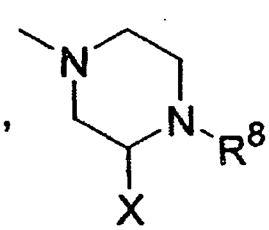 , 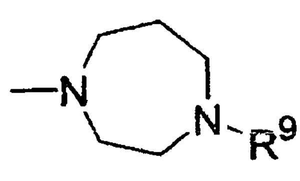 ,

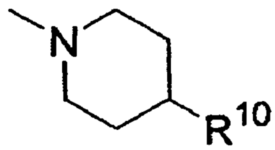,
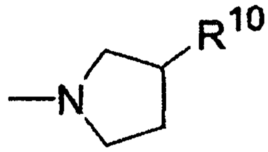
et
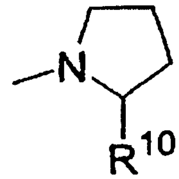.

**13.** Composés selon une ou plusieurs des revendications 1 à 12, **caractérisés en ce que** le radical X représente un radical alkyle choisi dans l'ensemble constitué par les groupes méthyle, éthyle, n-propyle, isopropyle, n-butyle, sec-butyle, isobutyle, tert-butyle, n-pentyle, sec-pentyle, (1,1)-diméthylpropyle et n-hexyle ;
X représente de préférence un radical méthyle.

**14.** Composés selon une ou plusieurs des revendications 1 à 13, **caractérisés en ce que** les radicaux $R^8$, $R^9$ et $R^{10}$, chacun indépendamment, représentent chacun un radical alkyle choisi dans l'ensemble constitué par les groupes méthyle, éthyle, n-propyle, isopropyle, n-butyle, sec-butyle, isobutyle, tert-butyle, n-pentyle, sec-pentyle, (1,1)-diméthylpropyle et n-hexyle, le radical alkyle pouvant chaque fois être substitué par 1, 2, 3, 4 ou 5 substituants choisis, indépendamment les uns des autres, dans l'ensemble constitué par F, Cl, Br, -CN, $-NO_2$, hydroxy, alcoxy en $C_1$-$C_6$, $-NH_2$, -NH-alkyle ($C_1$-$C_3$), -N [alkyle ($C_1$-$C_3$)][alkyle($C_1$-$C_3$)], NH-phényle, -N[alkyle ($C_1$-$C_3$)]-phényle et -N(phényle)$_2$ ;
un radical alcényle choisi dans l'ensemble constitué par les groupes vinyle, 1-propényle, 2-propényle, 1-butényle, 2-butényle, 3-butényle, 1-pentényle, 2-pentényle et pent-1,3-diényle, le radical alcényle pouvant chaque fois être substitué par 1, 2, 3, 4 ou 5 substituants choisis, indépendamment les uns des autres, dans l'ensemble constitué par F, Cl, Br, -CN, $-NO_2$, hydroxy, alcoxy en $C_1$-$C_6$, $-NH_2$, -NH-alkyle($C_1$-$C_3$),- N[alkyle($C_1$-$C_3$)][alkyle($C_1$-$C_3$)], NH-phényle, -N[alkyle($C_1$-$C_3$)]-phényle et -N(phényle)$_2$ ;
un radical (hétéro)cycloaliphatique choisi dans l'ensemble constitué par les groupes cyclopropyle, cyclobutyle, cyclopentyle, cyclohexyle, cycloheptyle, cyclopenténylе, cyclohexényle, cycloheptényle, imidazolidinyle, tétrahydrofurannyle, tétrahydrothiophényle, pyrrolidinyle, pipéridinyle, morpholinyle, pipérazinyle, thiomorpholinyle, tétrahydropyrannyle, azépanyle, diazépanyle et dithiolanyle, le radical (hétéro)cycloaliphatique pouvant chaque fois être lié par un groupe alkylène en $C_1$-$C_5$ linéaire ou ramifié, comportant éventuellement un groupe cabonyle (C=O) en tant que chaînon et/ou être substitué par 1, 2, 3, 4 ou 5 substituants choisis, indépendamment les uns des autres, dans l'ensemble constitué par F, Cl, Br, -CN, $-CF_3$, $-CHF_2$, $-CH_2F$, hydroxy, alcoxy en $C_1$-$C_6$, $-NH_2$, -NH-alkyle ($C_1$-$C_3$), -N [alkyle($C_1$-$C_3$)][alkyle($C_1$-$C_3$)], NH-phényle, -N[alkyle($C_1$-$C_3$)]-phényle, -N(phényle)$_2$, -SH, -alkyl($C_1$-$C_6$) thio, -alkyle($C_1$-$C_6$), -($CH_2$)-benzo[b]furannyle, phénoxy, benzyloxy, phényle et benzyle, le fragment cyclique des radicaux phénoxy, benzyloxy, phényle, -($CH_2$)-benzo[b]furannyle et benzyle pouvant chaque fois être substitué par 1, 2, 3, 4 ou 5 substituants choisis, indépendamment les uns des autres, dans l'ensemble constitué par F, Cl, Br, -alkyle($C_1$-$C_4$), $-CF_3$, $-CHF_2$, $-CH_2F$, -alcoxy($C_1$-$C_4$), -alkyl($C_1$-$C_4$)thio, phénoxy, phényle et benzyloxy ;
ou un radical choisi dans l'ensemble constitué par les groupes phényle, naphtyle, (1,3)-benzodioxolyle, (1,4)-benzodioxannyle, thiophényle, furannyle, pyrrolyle, pyrazolyle, pyrazinyle, pyrannyle, triazolyle, pyridinyle, imidazolyle, indolyle, iso-indolyle, benzo[b]furannyle, benzo[b]thiophényle, thiazolyle, oxazolyle, isoxazolyle, pyridazinyle, pyrazinyle, pyrimidinyle, indazolyle, quinazolinyle, quinolinyle et isoquinolinyle, le radical pouvant chaque fois être lié par un groupe alkylène en $C_1$-$C_5$ ou alcénylène en $C_2$-$C_5$ linéaire ou ramifié, éventuellement substitué par un radical phényle, comportant éventuellement un groupe carbonyle (C=O) en tant que chaînon, et/ou être substitué par 1, 2, 3, 4 ou 5 substituants choisis, indépendamment les uns des autres, dans l'ensemble constitué par F, Cl, Br, -CN, $-CF_3$, $-CHF_2$, $-CH_2F$, hydroxy, alcoxy en $C_1$-$C_6$, $-O-CF_3$, $-S-CF_3$, -(C=O)-$CH_3$, -SH, -alkyl($C_1$-$C_6$)thio, -alkyle($C_1$-$C_6$), $-NH_2$, -NH-alkyle($C_1$-$C_3$), -N[alkyle($C_1$-$C_3$)][alkyle($C_1$-$C_3$)], NH-phényle, -N[alkyle($C_1$-$C_3$)]-phényle, -N(phényle)$_2$, phénoxy, benzyloxy, phényle, benzyle et morpholinyle, le fragment cyclique des radicaux phénoxy, benzyloxy, phényle et benzyle pouvant chaque fois être substitué par 1, 2, 3, 4 ou 5 substituants choisis, indépendamment les uns des autres, dans l'ensemble constitué par F, Cl, Br, hydroxy, $-CF_3$, $-SF_5$, -CN, $-NO_2$, -alcoxy($C_1$-$C_6$), $-O-CF_3$, $-S-CF_3$, phényle et benzyloxy ;
de préférence
$R^8$ représente un radical alkyle choisi dans l'ensemble constitué par les groupes méthyle, éthyle, n-propyle, isopropyle, n-butyle, tert-butyle et n-pentyle, le radical alkyle pouvant chaque fois être substitué par 1 ou 2 substituants choisis, indépendamment l'un de l'autre, dans l'ensemble constitué par $-NH_2$, $-NH-CH_3$, $-NH-C_2H_5$, $-N-(CH_3)_2$, $-N(C_2H_5)_2$, $-N(CH_3)(C_2H_5)$, -NH-phényle, -N($CH_3$)-phényle, -N($C_2H_5$)-phényle et -N(phényle)$_2$ ;

un radical cycloaliphatique choisi dans l'ensemble constitué par les groupes pyrrolidinyle et pipéridinyle, qui peut être lié par un groupe -(C=O)- ou $(CH_2)$-(C=O)- et/ou être substitué par 1, 2, 3, 4 ou 5 substituants choisis, indépendamment les uns des autres, dans l'ensemble constitué par F, Cl, Br, -CN, $-CF_3$, $-CHF_2$, $-CH_2F$, hydroxy, -O-$CH_3$-, -O-$C_2H_5$, -O-$C_3H_7$, $-NH_2$, -NH-$CH_3$, -NH-$C_2H_5$, -N-$(CH_3)_2$, -N$(C_2H_5)_2$,- N$(CH_3)(C_2H_5)$, -NH-phényle, -N$(CH_3)$-phényle, -N$(C_2H_5)$-phényle, -N(phényle)$_2$, -SH, -S-$CH_3$, -S-$C_2H_5$, -S-$C_3H_7$, -S-C$(CH_3)_3$, méthyle, éthyle, n-propyle, isopropyle, n-butyle, sec-butyle et tert-butyle ; ou un radical choisi dans l'ensemble constitué par les groupes phényle, 1-naphtyle, 2-naphtyle, 2-furannyle, 3-furannyle, 2-thiophényle, 3-thiophényle, 1-pyridinyle, 2-pyridinyle, 3-pyridinyle, benzo[b]-furannyle, (1,3-benzodioxolyle et (1,4)-benzodioxannyle, qui peut être lié par un groupe -(C=O)-, -$(CH_2)$-, -$(CH_2)_2$-, -CH$(CH_3)$-, -(CH=CH)-, -$(CH_2)$-(C=O)-, -$(CH_2)$-(CH=CH)- ou -$(CH_2)_3$- et/ou être substitué par 1, 2, 3, 4 ou 5 substituants choisis, indépendamment les uns des autres, dans l'ensemble constitué par F, Cl, Br, -CN, $-CF_3$, $-CHF_2$, $-CH_2F$, hydroxy, -(C=O)-$CH_3$, -O-$CH_3$, -O-$C_2H_5$, -O-$C_3H_7$, -O-$CF_3$, -S-$CF_3$, -SH, -S-$CH_3$, -S-$C_2H_5$, -S-$C_3H_7$, -S-C$(CH_3)_3$, méthyle, éthyle, n-propyle, isopropyle, n-butyle, sec-butyle, tert-butyle, $-NH_2$, -NH-$CH_3$, -NH-$C_2H_5$, -N-$(CH_3)_2$, -N$(C_2H_5)_2$, -N$(CH_3)(C_2H_5)$, -NH-phényle, -N$(CH_3)$-phényle, -N$(C_2H_5)$-phényle, -N(phényle)$_2$, phénoxy, benzyloxy, phényle, benzyle et morpholinyle ;

$R^9$ représente un radical alkyle choisi dans l'ensemble constitué par les groupes méthyle, éthyle, n-propyle, isopropyle, n-butyle, sec-butyle, isobutyle, tert-butyle, n-pentyle, sec-pentyle, (1,1)-diméthylpropyle et n-hexyle

et

$R^{10}$ représente un radical cycloaliphatique choisi dans l'ensemble constitué par les groupes pyrrolidinyle et pipéridinyle, qui peut être lié par un groupe -$(CH_2)$-, -$(CH_2)_2$-, -CH$(CH_3)$- ou -$(CH_2)_3$- et/ou être substitué par 1, 2, 3, 4 ou 5 substituants choisis, indépendamment les uns des autres, dans l'ensemble constitué par F, Cl, Br, -CN, $-CF_3$, $-CHF_2$, $-CH_2F$, hydroxy, -O-$CH_3$, -0-$C_2H_5$,- O-$C_3H_7$, $-NH_2$, -NH-$CH_3$, -NH-$C_2H_5$, -N-$(CH_3)_2$, -N$(C_2H_5)_2$, -N$(CH_3)$ $(C_2H_5)$, -NH-phényle, -N$(CH_3)$-phényle, -N$(C_2H_5)$-phényle, -N(phényle)$_2$, -SH, -S-$CH_3$, -S-$C_2H_5$, -S-$C_3H_7$, -S-C $(CH_3)_3$, méthyle, éthyle, n-propyle, isopropyle, n-butyle, sec-butyle et tert-butyle ;

ou un radical choisi dans l'ensemble constitué par les groupes phényle, 1-naphtyle et 2-naphtyle, qui peut être lié par un groupe -$(CH_2)$-, -[(CH)phényle]-, -$(CH_2)_2$-, -$(CH_2)_3$- et/ou être substitué par 1, 2, 3, 4 ou 5 substituants choisis, indépendamment les uns des autres, dans l'ensemble constitué par F, Cl, Br, -CN, $-CF_3$, $-CHF_2$, $-CH_2F$, hydroxy, -O-$CH_3$, -O-$C_2H_5$, -O-$C_3H_7$, -O-$CF_3$, -S-$CF_3$, thio, -S-$CH_3$, -S-$C_2H_5$, -S-$C_3H_7$, -S-C$(CH_3)_3$, méthyle, éthyle, n-propyle, isopropyle, n-butyle, sec-butyle, tert-butyle, $-NH_2$, -NH-$CH_3$, -NH-$C_2H_5$, -N-$(CH_3)_2$, -N$(C_2H_5)_2$, -N$(CH_3)(C_2H_5)$, -NH-phényle, -N$(CH_3)$-phényle, -N$(C_2H_5)$-phényle, -N(phényle)$_2$, phénoxy, benzyloxy, phényle, benzyle et morpholinyle ;

de façon particulièrement préférée

$R^8$ représente un radical choisi dans l'ensemble constitué par les groupes méthyle, éthyle, n-propyle, isopropyle et n-$(CH_2)$-$(CH_2)$-N$(CH_3)_2$,

un radical pyrrolidinyle qui est lié par un groupe -$(CH_2)$-(C=O),

ou un radical choisi dans l'ensemble constitué par les groupes phényle, 1-naphtyle, 2-naphtyle, 2-thiophényle, 3-thiophényle, 1-pyridinyle, 2-pyridinyle, 3-pyridinyle, benzo[b]furannyle, (1,3)-benzodioxolyle et (1,4)-benzodioxannyle, qui peut être lié par un groupe -(C=O)-, -$(CH_2)$-, -$(CH_2)_2$-, -CH$(CH_3)$-, -(CH=CH)-, -$(CH_2)$-(C=O)-, -$(CH_2)$-(CH=CH)- ou -$(CH_2)_3$- et/ou être substitué par 1, 2 ou 3 substituants choisis, chacun indépendamment, dans l'ensemble constitué par F, Cl, Br, -CN, $-CF_3$, $-CHF_2$, $-CH_2F$, -(C=O)-$CH_3$, -O-$CH_3$ et -0-$C_2H_5$,

$R^9$ représente un radical méthyle ou éthyle

et

$R^{10}$ représente un radical pyrrolidinyle qui est lié par un groupe -$(CH_2)$-,

ou représente un radical benzhydryle.

**15.** Composés selon une ou plusieurs des revendications 1 à 14, **caractérisés en ce que**

$R^1$ représente un groupe -$NR^3R^4$ ou un groupe -$NR^5R^6$ ;

$R^2$ représente un radical alkyle choisi dans l'ensemble constitué par les groupes n-propyle, isopropyle, n-butyle, sec-butyle, isobutyle, tert- butyle, n-pentyle, sec-pentyle et (1,1)- diméthylpropyle ;

représente un radical alcényle choisi dans l'ensemble constitué par les groupes 1-penténylе, 2-pentényle et pent-1,3-diényle ;

représente un radical cycloaliphatique choisi dans l'ensemble constitué par les groupes cyclopentyle, cyclohexyle et cycloheptyle, qui est lié par un groupe -$(CH_2)$ ;

ou représente un radical choisi dans l'ensemble constitué par les groupes phényle, 1-naphtyle, 2-naphtyle, 2-furannyle, 3-furannyle, 2-thiophényle ou 3-thiophényle, qui peut être lié par un groupe -$(CH_2)$-, -$(CH_2)_2$-, -$(CH_2)$-O-, -CH$(CH_3)$-, -(CH=CH)- ou -$(CH_2)_3$- et/ou être substitué par 1, 2 ou 3 substituants choisis, chacun indépendamment, dans l'ensemble constitué par F, Cl, Br, -O-$CH_3$, -O-$C_2H_5$, méthyle, éthyle, n- propyle et isopropyle ;

$R^3$ représente un atome d'hydrogène

ou un radical méthyle ;
$R^4$ représente un radical choisi dans l'ensemble constitué par les groupes méthyle, éthyle, n-propyle, $-(CH_2)-N(CH_3)_2$, $-(CH_2)-(CH_2)-N(CH_3)_2$, $-(CH_2)-(CH_2)-(CH_2)-N(CH_3)_2$, $(CH_2)-(CH_2)-N(C_2H_5)_2$, $-(CH_2)-(CH_2)-(CH_2)-N(C_2H_5)_2$, $-(CH_2)-(CH_2)-N(CH_3)$(phényle) et $-(CH_2)-(CH_2)-(CH_2)-N(CH_3)$(phényle) ;
représente un radical choisi dans l'ensemble constitué par

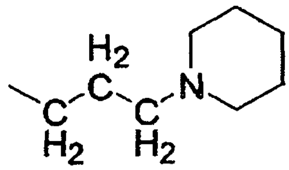 , 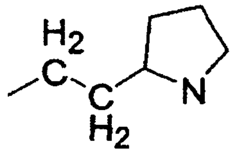 , 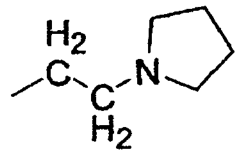 ,

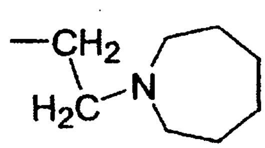 et 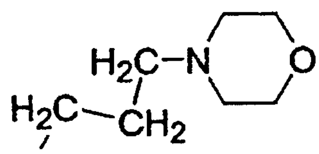 ,

qui peut être substitué par un radical méthyle ;
représente le radical suivant,

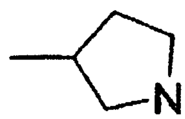 ,

qui peut être substitué sur l'atome d'azote par un substituant choisi dans l'ensemble constitué par les groupes $-(CH_2)$-benzo[b]furannyle et benzyle, le fragment cyclique respectif des radicaux $-(CH_2)$- benzo[b]furannyle et benzyle pouvant être substitué par 1, 2 ou 3 substituants choisis, chacun indépendamment, dans l'ensemble constitué par F, Cl, Br, $-CF_3$, $-O-CH_3$, $-O-C_2H_5$, $-O-C_3H_7$, $-S-CH_3$, $-S-C_2H_5$, $-S-C_3H_7$, $-S-C(CH_3)_3$, méthyle, éthyle, n-propyle, isopropyle, n-butyle, phénoxy et benzyloxy ;
ou un radical choisi dans l'ensemble constitué par les groupes phényle et benzyle, le fragment cyclique des radicaux phényle et benzyle pouvant être substitué par 1, 2 ou 3 substituants choisis, chacun indépendamment, dans l'ensemble constitué par les groupes $-O-CH_3$, $-O-C_2H_5$, $-O-C_3H_7$, $-N-(CH_3)_2$, $-N(C_2H_5)_2$ et morpholinyle.
$R^5$ et $R^6$ forment ensemble, avec l'atome d'azote qui les relie en tant que chaînon formant le cycle, l'un des radicaux suivants

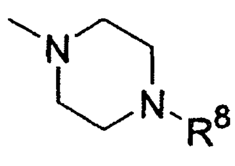 , 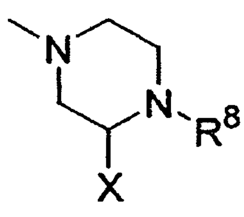 , 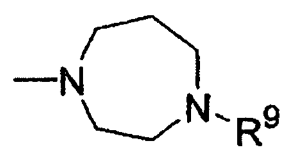 ,

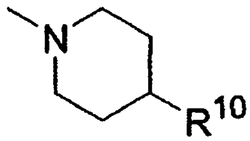 , 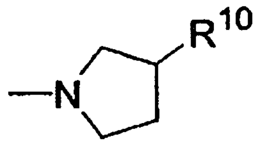 et 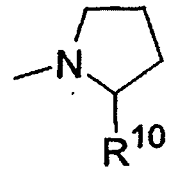 ;

X représente un groupe méthyle,
$R^8$ représente un radical choisi dans l'ensemble constitué par les groupes méthyle, éthyle, n-propyle, isopropyle et $-(CH_2)-(CH_2)-N(CH_3)_2$,
un radical pyrrolidinyle qui est lié par un groupe $-(CH_2)-(C=O)$,
ou un radical choisi dans l'ensemble constitué par les groupes phényle, 1-naphtyle, 2-naphtyle, 2-thiophényle, 3-thiophényle, 1-pyridinyle, 2-pyridinyle, 3-pyridinyle, benzo[b]furannyle, (1,3)-benzodioxolyle et (1,4)-benzodioxannyle, qui peut être lié par un groupe $-(C=O)-$, $-(CH_2)-$, $-(CH_2)_2-$, $-CH(CH_3)-$, $-(CH=CH)-$, $-(CH_2)-(C=O)-$, $-(CH_2)-(CH=CH)-$ ou $-(CH_2)_3-$ et/ou être substitué par 1, 2 ou 3 substituants choisis, chacun indépendamment, dans l'ensemble constitué par F, Cl, Br, -CN, $-CF_3$, $-CHF_2$, $-CH_2F$, $-(C=O)-CH_3$, $-O-CH_3$ et $-O-C_2H_5$,
$R^9$ représente un radical méthyle ou éthyle et
$R^{10}$ représente un radical pyrrolidinyle qui est lié par un groupe $-(CH_2)$,

ou représente un radical benzhydryle.

**16.** Composés selon une ou plusieurs des revendications 1 à 15, choisis dans l'ensemble constitué par les composés suivants :

2- cyclohexyl- N- {4-[4-(2- diméthylamino- éthyl)-pipérazine- 1- carbonyl]- 4,5,6,7- tétrahydro- benzothiazol- 2-yl}-acétamide,
N-[4-(4-méthyl[1,4]diazépane-1-carbonyl)-4,5,6,7-tétrahydro-benzothiazol-2-yl]-2-phénoxy-acétamide,
(3-diméthylamino-propyl)-amide d'acide 2-(2-phényl-propionylamino)-4,5,6,7-tétrahydro-benzothiazole-4-carboxylique,
[4-(4-méthyl-pipérazine-1-carbonyl)-4,5,6,7-tétrahydro-benzothiazol-2-yl]-amide d'acide naphtalène-2-carboxylique
N-[4-(2-pyrrolidin-1-ylméthyl-pyrrolidine-1-carbonyl)-4,5,6,7-tétrahydro-benzothiazol-2-yl]-2-thiophén-2-yl-acétamide,
N-{4-[4-(7-méthoxy-benzo[1,3]dioxol-5-ylméthyl)-pipérazine-1-carbonyl]-4,5,6,7-tétrahydro-benzothiazol-2-yl}-2-(2-méthoxy-phényl)-acétamide,
N-{4-[4-(4-méthoxy-phényl)-3-méthyl-pipérazine-1-carbonyl]-4,5,6,7-tétrahydro-benzothiazol-2-yl}-benzamide,
{4-[4-(4-acétyl-phényl)-pipérazine-1-carbonyl]-4,5,6,7-tétrahydro-benzothiazol-2-yl)-amide d'acide furanne-2-carboxylique,
[1-(2,4,6-triméthoxy-benzyl)-pyrrolidin-3-yl]-amide d'acide 2-[(furanne-2-carbonyl)-amino]-4,5,6,7-tétrahydro-benzothiazole-4-carboxylique,
N-[4-(4-isopropyl-pipérazine-1-carbonyl)-4,5,6,7-tétrahydro-benzothiazol-2-yl]1-2-phényl-propionamide,
3-furann-2-yl-N-[4-(4-thiophén-3-ylméthyl-pipérazine-1-carbonyl)-4,5,6,7-tétrahydro-benzothiazol-2-yl]-acrylamide
[1-(2-chloro-6-fluoro-benzyl)-pyrrolidin-3-yl]-méthyl-amide d'acide 2-(4-méthoxy-benzoylamino)-4,5,6,7-tétrahydro-benzothiazole-4-carboxylique
[4-(4-pyridin-4-yl-pipérazine-1-carbonyl)-4,5,6,7-tétrahydro-benzothiazol-2-yl]-amide d'acide hexanoïque,
(3-morpholin-4-yl-propyl)-amide d'acide 2-(2-thiophén-2-yl-acétylamino)-4,5,6;7-tétrahydro-benzothiazole-4-carboxylique,
[1-(4-phénoxy-benzyl)-pyrrolidin-3-yl]-amide d'acide 2-(2-phényl-propionylamino)-4,5,6,7-tétrahydro-benzothiazole-4-carboxylique,
(2-azépan-1-yl-éthyl)-amide d'acide 2-[(furanne-3-carbonyl)-amino]-4,5,6,7-tétrahydro-benzothiazole-4-carboxylique,
[4-(4-benzofurann-2-ylméthyl-pipérazine-1-carbonyl)-4,5,6,7-tétrahydro-benzothiazol-2-yl]-amide d'acide furanne-3-carboxylique,
[3-(2-méthyl-pipéridin-1-yl)-propyl]-amide d'acide 2-hexanylamino-4,5,6,7-tétrahydro-benzothiazole-4-carboxylique,
(2-diméthylamino-éthyl)-amide d'acide 2-(2-phényl-propionylamino)-4,5,6,7-tétrahydro-benzothiazole-4-carboxylique,
2-éthoxy-N-[4-(4-phénéthyl-pipérazine-1-carbonyl)-4,5,6,7-tétrahydro-benzothiazol-2-yl]-benzamide,
2-(4-fluoro-phénoxy)-N-[4-(4-phényl-pipérazine-1-carbonyl)-4,5,6,7-tétrahydro-benzothiazol-2-yl]-acétamide,
[2-(1-méthyl-pyrrolidin-2-yl)-éthyl]-amide d'acide 2-(2-phényl-propionylamino)-4,5,6,7-tétrahydro-benzothiazole-4-carboxylique,
[3-(2-méthyl-pipéridin-1-yl)-propyl]-amide d'acide 2-(3-furann-2-yl-acryloylamino)-4,5,6,7-tétrahydro-benzothiazole-4-carboxylique,
(3-diméthylamino-propyl)-amide d'acide 2-(3,3-diméthyl-butyrylamino)-4,5,6,7-tétrahydro-benzothiazole-4-carboxylique,
N-{4-[4-(4-chloro-benzyl)-pipérazine-1-carbonyl]-4,5,6,7-tétrahydro-benzothiazol-2-yl}-4-méthoxy-benzamide,
[4-(2-pyrrolidin-1-ylméthyl-pyrrolidine-1-carbonyl)-4,5,6,7-tétrahydro-benzothiazol-2-yl]-amide d'acide naphtalène-2-carboxylique,
N-[4-(4-benzofurann-2-ylméthyl-pipérazine-1-carbonyl)-4,5,6,7-tétrahydro-benzothiazol-2-yl]-butyramide,
N-{4-[4-(3-méthoxy-phényl)-pipérazine-1-carbonyl]-4,5,6,7-tétrahydro-benzothiazol-2-yl}-benzamide,
(1-biphényl-4-ylméthyl-pyrrolidin-3-yl)-méthyl-amide d'acide 2-butyrylamino-4,5,6,7-tétrahydro-benzothiazole-4-carboxylique,
(2-diméthylamino-éthyl)-amide d'acide 2-(2-phénoxy-acétylamino)-4,5,6,7-tétrahydro-benzothiazole-4-carboxylique,
2-éthoxy-N-[4-(2-pyrrolidin-1-ylméthyl-pyrrolidine-1-carbonyl)-4,5,6,7-tétrahydro-benzothiazol-2-yl]-benzamide,
{4-[4-(2-oxo-2-pyrrolidin-1-yl-éthyl)-pipérazine-1-carbonyl]-4,5,6,7-tétrahydro-benzothiazol-2-yl}-amide d'acide naphtalène-2-carboxylique,
[1-(2-bromo-4,5-diméthoxy-benzyl)-pyrrolidin-3-yl]-amide d'acide 2-(2-phényl-propionylamino)-4,5,6,7-tétrahydro-benzothiazole-4-carboxylique,
{4-[4-(2,4,6-triméthoxy-benzyl)-pipérazine-1-carbonyl]-4,5,6,7-tétrahydro-benzothiazol-2-yl}-amide d'acide hexanoïque,
[1-(2-tert-butylsulfanyl-benzyl)-pyrrolidin-3-yl]-amide d'acide 2-(3,5-diméthyl-benzoylamino)-4,5,6,7-tétrahydro-benzothiazole-4-carboxylique,
[1-(2-benzyloxy-benzyl)-pyrrolidin-3-yl]-méthylamide d'acide 2-(2-phényl-propionylamino)-4,5,6,7-tétrahydrobenzothiazole-4-carboxylique,
(1-biphényl-4-ylméthyl-pyrrolidin-3-yl)-méthyl-amide d'acide 2-hexa-2,4-diénoylamino-4,5,6,7-tétrahydro-benzothiazole-4-carboxylique,
(2-diéthylamino-éthyl)-amide d'acide 2-(3-phényl-propionylamino)-4,5,6,7-tétrahydro-benzothiazole-4-carboxylique,
(2-azépan-1-yl-éthyl)-amide d'acide 2-(2-thiophén-2-yl-acétylamino)-4,5,6,7-tétrahydro-benzothiazole-4-carboxylique,
1-(2-éthoxy-benzyl)-pyrrolidin-3-yl]-méthyl-amide d'acide 2-(2-thiophén-2-yl-acétylamino)-4,5,6,7-tétrahydrobenzothiazole-4-carboxylique,
(1-benzofurann-2-ylméthyl-pyrrolidin-3-yl)-méthyl-amide d'acide 2-hexanylamino-4,5,6,7-tétrahydro-benzothiazole-4-carboxylique,
[3-(2-méthyl-pipéridin-1-yl)-propyl]-amide d'acide 2-(2-phénoxy-acétylamino)-4,5,6,7-tétrahydro-benzothiazole-4-carboxylique,
(2-pyrrolidin-1-yl-éthyl)-amide d'acide 2-[(naphtalène-2-carbonyl)-amino]-4,5,6,7-tétrahydro-benzothiazole-4-carboxylique,
[1-(2-éthoxy-benzyl)-pyrrolidin-3-yl]-méthyl-amide d'acide 2-[2-(3,5-difluoro-phényl)-acétylamino]-4,5,6,7-tétrahydro-benzothiazole-4-carboxylique,
4-diméthylamino-benzylamide d'acide 2-[(naphtalène-2-carbonyl)-amino]-4,5,6,7-tétrahydro-benzothiazole-4-carboxylique,
[2-(1-méthyl-pyrrolidin-2-yl)-éthyl]-amide d'acide 2-[2-(4-fluoro-phénoxy)-acétylamino]-4,5,6,7-tétrahydro-benzothiazole-4-carboxylique,
{4-[4-(2-cyano-phényl)-pipérazine-1-carbonyl]-4,5,6,7-tétrahydro-benzothiazol-2-yl)-amide d'acide furanne-2-carboxylique,
(1-benzofurann-2-ylméthyl-pyrrolidin-3-yl)-méthyl-amide d'acide 2-(3-phényl-propionylamino)-4,5,6,7-tétrahydro-benzothiazole-4-carboxylique,
[1-(2,6-dichloro-benzyl)-pyrrolidin-3-yl]-méthyl-amide d'acide 2-hexa-2,4-diénoylamino-4,5,6,7-tétrahydrobenzothiazole-4-carboxylique,

[1-(5-bromo-2-éthoxy-benzyl)-pyrrolïdin-3-yl]-amide d'acide 2-[(furanne-2-carbonyl)-amino]-4,5,6,7-tétrahydro-benzothiazole-4-carboxylique,
[1-(2-éthoxy-benzyl)-pyrrolidin-3-yl]-méthyl-amide d'acide 2-(2-phénoxy-acétylamino)-4,5,6,7-tétrahydro-benzothiazole-4-carboxylique,
{4-[4-(2-fluoro-phényl)-pipérazine-1-carbonyl]-4,5,6,7-tétrahydro-benzothiazol-2-yl}-amide d'acide hexanoïque,
{4-[4-(2-fluoro-benzyl)-pipérazine-1-carbonyl]-4,5,6,7-tétrahydro-benzothiazol-2-yl}-amide d'acide naphtalène-2-carboxylique,
méthyl-[1-(2-trifluorométhyl-benzyl)-pyrrolidin-3-yl]-amide d'acide 2-(2-phénoxy-acétylamino)-4,5,6,7-tétrahydro-4-benzothiazole-4-carboxylique,
(1-biphényl-4-ylméthyl-pyrrolidin-3-yl)-méthyl-amide d'acide 2-(3-phényl-propionylamino)-4,5,6,7-tétrahydro-benzothiazole-4-carboxylique,
(1-benzofurann-2-ylméthyl-pyrrolidin-3-yl)-méthyl-amide d'acide 2-[2-(2-méthoxy-phényl)-acétylamino]-4,5,6,7-tétrahydro-benzothiazole-4-carboxylique,
2-(3,5-difluoro-phényl)-N-[4-(2-pyrrolidin-1-ylméthyl-pyrrolidine-1-carbonyl)-4,5,6,7-tétrahydro-benzothiazol-2-yl]-acétamide,
4-diméthylamino-benzylamide d'acide 2-[(furann-2-carbonyl)-amino]-4,5,6,7-tétrahydro-benzothiazole-4-carboxylique,
{4-[4-(3-fluoro-4-méthoxy-benzyl)-pipérazine-1-carbonyl]-4,5,6,7-tétrahydro-benzothiazol-2-yl}-amide d'acide hexanoïque,
N-{4-[4-(4-méthoxy-phényl)-3-méthyl-pipérazin-1-carbonyl)-4,5,6,7-tétrahydro-benzothiazol-2-yl}-2-phényl-propionamide,
[1-(2-chloro-6-fluoro-benzyl)-pyrrolidin-3-yl]-méthyl-amide d'acide 2-hexa-2,4-diénoylamino-4,5,6,7-tétrahydro-benzothiazole-4-carboxylique,
[1-(2,6-dichloro-benzyl)-pyrrolidin-3-yl]-méthyl-amide d'acide 2-[2-(3,5-difluoro-phényl)-acétylamino]-4,5,6,7-tétrahydro-benzothiazole-4-carboxylique,
[1-(2-éthoxy-benzyl)-pyrrolidin-3-yl]-méthyl-amide d'acide 2-[2-(4-fluoro-phénoxy)-acétylamino]-4,5,6,7-tétrahydro-benzothiazole-4-carboxylique,
[4-(4-pyridin-2-yl-pipérazine-1-carbonyl)-4,5,6,7-tétrahydro-benzothiazol-2-yl]-amide d'acide furanne-3-carboxylique,
[4-(4-benzofurann-2-ylméthyl-pipérazine-1-carbonyl)-4,5,6,7-tétrahydro-benzothiazol-2-yl]-amide d'acide hexanoïque,
2-phénoxy-N-{4-[4-(1-phényl-éthyl)-pipérazine-1-carbonyl]-4,5,6,7-tétrahydro-benzothiazol-2-yl}-acétamide,
[1-(2,6-dichloro-benzyl)-pyrrolidin-3-yl]-méthyl-amide d'acide 2-(2-cyclohexyl-acétylamino)-4,5,6,7-tétrahydro-benzothiazole-4-carboxylique,
[3-(méthyl-phényl-amino)-propyl]-amide d'acide 2-(2-cyclohexyl-acétylamino)-4,5,6,7-tétrahydro-benzothiazole-4-carboxylique,
[3-(méthyl-phényl-amino)-propyl]-amide d'acide 2-(2-éthoxy-benzoylamino)-4,5,6,7-tétrahydro-benzothiazole-4-carboxylique,
3-phényl-N-{4-[4-(5-trifluorométhyl-pyridin-2-yl)-pipérazine-1-carbonyl]-4,5,6,7-tétrahydro-benzothiazol-2-yl}-propionamide,
[4-(4-phénéthyl-pipérazine-1-carbonyl)-4,5,6,7-tétrahydro-benzothiazol-2-yl]-amide d'acide furanne-3-carboxylique,
3-phényl-N-{4-[4-(3-trifluorométhyl-phényl)-pipérazine-1-carbonyl]-4,5,6,7-tétrahydro-benzothiazol-2-yl}-propionamide,
[1-(2-benzyloxy-benzyl)-pyrrolidin-3-yl]-amide d'acide 2-(3,3-diméthyl-butyrylamino)-4,5,6,7-tétrahydro-benzothiazole-4-carboxylique,
2-(3,5-difluoro-phényl)-N-{4-[4-(3-trifluoro-méthyl-phényl)-pipérazine-1-carbonyl]-4,5,6,7-tétrahydro-benzothiazol-2-yl}-acétamide,
2-éthoxy-N-{4-[4-(1-phényl-éthyl)-pipérazine-1-carbonyl]-4,5,6,7-tétrahydro-benzothiazol-2-yl}-benzamide,
3-furann-2-yl-N-{4-[4-(3-trifluorométhyl-phényl)-pipérazin-1-carbonyl]-4,5,6,7-tétrahydro-benzothiazol-2-yl}-acrylamide,
N-{4-[4-(2-cyano-phényl)-pipérazine-1-carbonyl]-4,5,6,7-tétrahydro-benzothiazol-2-yl}-3-phényl-propionamide,
[1-(2-chloro-6-fluoro-benzyl)-pyrrolidin-3-yl]-méthyl-amide d'acide 2-(2-cyclohexyl-acétylamino)-4,5,6,7-tétrahydro-benzothiazole-4-carboxylique,
N-{4-[4-(2-méthoxy-naphtalén-1-ylméthyl)-pipérazine-1-carbonyl]-4,5,6,7-tétrahydro-benzothiazol-2-yl}-3,3-diméthyl-butyramide,

(2,5-diéthoxy-4-morpholin-4-yl-phényl)-amide d'acide 2-(3,3-diméthyl-butyrylamino)-4,5,6,7-tétrahydro-benzothiazole-4-carboxylique,
N- {4-[4-(2- chloro- phényl)-pipérazine- 1- carbonyl]- 4,5,6,7- tétrahydro- benzothiazol- 2- yl}- 2-(4- fluoro- phényl)-acétamide,
N[4-(4-benzyl-pipérazine-1-carbonyl)-4,5,6,7-tétrahydro-benzothiazol-2-yl]-3,3-diméthyl-butyramide,
N-[4-(4-benzhydryl-pipérazine-1-carbonyl)-4,5,6,7-tétrahydro-benzothiazol-2-yl]-2-(3,5-difluoro-phényl)-acétamide,
N-[4-(4-benzhydryl-pipérazine-1-carbonyl)-4,5,6,7-tétrahydro-benzothiazol-2-yl]-2-thiophén-2-yl-acétamide,
N-{4-[4-(2-chloro-phényl)-pipérazine-1-carbonyl]-4,5,6,7-tétrahydro-benzothiazol-2-yl}-2-éthoxy-benzamide,
3,3-diméthyl-N-{4-[4-(5-trifluorométhyl-pyridin-2-yl)-pipérazine-1-carbonyl]-4,5,6,7-tétrahydro-benzothiazol-2-yl}-butyramide,
3,3-diméthyl-N-{4-[4-(3-phényl-allyl)-pipérazine-1-carbonyl]-4,5,6,7-tétrahydro-benzothiazol-2-yl}-butyramide,
N-[4-(4-benzhydryl-pipérazine-1-carbonyl)-4,5,6,7-tétrahydro-benzothiazol-2-yl]-2-cyclohexyl-acétamide et
[4-(4-phényl-pipérazine-1-carbonyl)-4,5,6,7-tétrahydro-benzothiazol-2-yl]amide d'acide furanne-2-carboxylique,
éventuellement sous forme de leurs stéréoisomères, en particulier énantiomères ou diastéréoisomères, purs, de leurs racémates ou sous forme de mélanges des stéréoisomères, en particulier des énantiomères et/ou diastéréoisomères, en un rapport de mélange quelconque, ou chacun éventuellement sous forme des sels correspondants, ou chacun éventuellement sous forme de produits de solvatation correspondants.

**17.** Procédé pour la préparation de composés 4,5,6,7-tétrahydro-benzothiazol-2-yl-amine substitués, selon une ou plusieurs des revendications 1 à 16, **caractérisé en ce qu'**on convertit au moins un composé de formule II

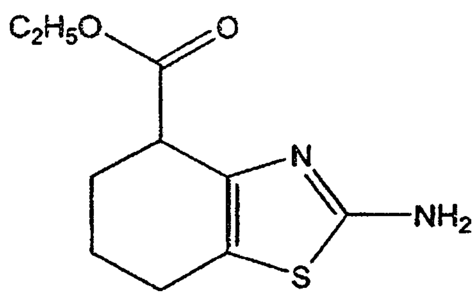

II,

éventuellement sous forme de son sel, par mise en réaction avec au moins un réactif d'acylation de formule générale III

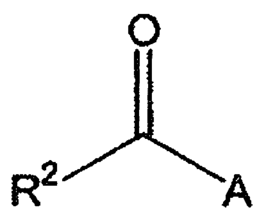

III

dans laquelle $R^2$ a la signification selon une ou plusieurs des revendications 1 à 16 et A représente un groupe séparable du radical acyle $R^2$-(C=O), de préférence -OH, -Cl ou -O-(C=O)-$R^2$,
en un composé de formule générale IV,

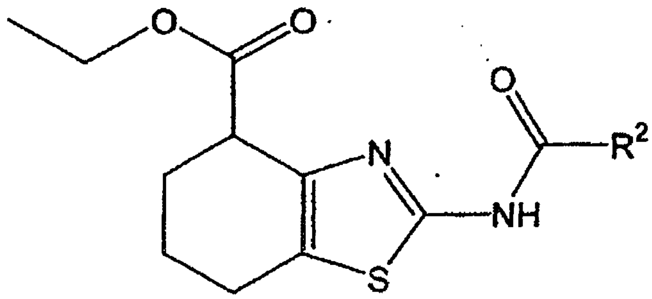

IV

qui est éventuellement purifié et/ou éventuellement isolé, et éventuellement on convertit ce dernier, par dissociation de l'ester éthylique, en un composé de formule générale V

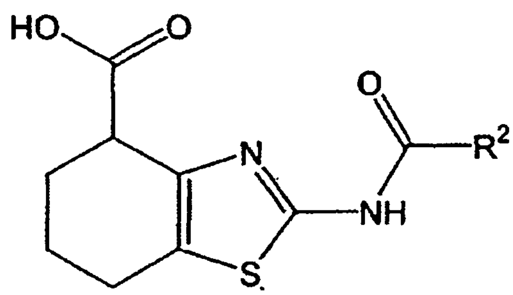

V

qui est éventuellement purifié et/ou éventuellement isolé, et on convertit au moins un composé de formule générale IV et/ou au moins un composé de formule générale V, par mise en réaction avec au moins un composé de formule générale $R^1$-H, dans laquelle $R^1$ a la signification selon une ou plusieurs des revendications 1 à 16, en un composé de formule générale 1 selon la revendication 1, qui est éventuellement purifié et/ou éventuellement isolé.

**18.** Médicament contenant au moins un composé 4,5,6,7-tétrahydro-benzothiazol-2-yl-amine substitué, selon l'une quelconque des revendications 1 à 16, et éventuellement un ou plusieurs adjuvants physiologiquement acceptables.

**19.** Médicament selon la revendication 18, pour la régulation de la recapture de noradrénaline (*Noradrenalin-Uptake*), pour la régulation de la recapture du 5-hydroxytryptophane (*5-HT-Uptake*), pour le traitement de douleurs, pour le traitement du mésusage d'alcool et/ou de drogues et/ou de médicaments, pour la prophylaxie et/ou le traitement de la dépendance à l'alcool et/ou aux drogues et/ou de la pharmacodépendance, pour la prophylaxie et/ou le traitement d'inflammations, pour la prophylaxie et/ou le traitement de dépressions, pour la prophylaxie et/ou le traitement de la perte de tonus, pour la prophylaxie et/ou le traitement de troubles de la prise alimentaire, de préférence choisis dans le groupe constitué par la boulimie, l'anorexie, l'obésité et la cachexie, pour la prophylaxie et/ou le traitement de la catalepsie, pour l'accroissement de la vigilance, pour l'accroissement de la libido ou pour l'anxiolyse.

**20.** Utilisation d'au moins un composé 4,5,6,7-tétrahydro-benzothiazol-2-yl-amine substitué, selon l'une quelconque des revendications 1 à 16, pour la fabrication d'un médicament destiné à la régulation de la recapture de noradrénaline (*Noradrenalin-Uptake*).

**21.** Utilisation d'au moins un composé 4,5,6,7-tétrahydro-benzothiazol-2-yl-amine substitué, selon l'une quelconque des revendications 1 à 16, pour la fabrication d'un médicament destiné à la régulation de la recapture du 5-hydroxytryptophane (*5-HT-Uptake*).

**22.** Utilisation d'au moins un composé 4,5,6,7-tétrahydro-benzothiazol-2-yl-amine substitué, selon l'une quelconque des revendications 1 à 16, pour la fabrication d'un médicament destiné au traitement de douleurs.

**23.** Utilisation d'au moins un composé 4,5,6,7-tétrahydro-benzothiazol-2-yl-amine substitué, selon l'une quelconque des revendications 1 à 16, pour la fabrication d'un médicament destiné au traitement du mésusage d'alcool et/ou du mésusage de drogues et/ou du mésusage de médicaments.

**24.** Utilisation d'au moins un composé 4,5,6,7-tétrahydro-benzothiazol-2-yl-amine substitué, selon l'une quelconque des revendications 1 à 16, pour la fabrication d'un médicament destiné à la prophylaxie et/ou au traitement du besoin d'alcool et/ou du besoin de drogues et/ou du besoin de médicaments.

**25.** Utilisation d'au moins un composé 4,5,6,7-tétrahydro-benzothiazol-2-yl-amine substitué, selon l'une quelconque des revendications 1 à 16, pour la fabrication d'un médicament destiné à la prophylaxie et/ou au traitement d'inflammations.

**26.** Utilisation d'au moins un composé 4,5,6,7-tétrahydro-benzothiazol-2-yl-amine substitué, selon l'une quelconque des revendications 1 à 16, pour la fabrication d'un médicament destiné à la prophylaxie et/ou au traitement de dépressions.

**27.** Utilisation d'au moins un composé 4,5,6,7-tétrahydro-benzothiazol-2-yl-amine substitué, selon l'une quelconque des revendications 1 à 16, pour la fabrication d'un médicament destiné au traitement de la perte de tonus.

**28.** Utilisation d'au moins un composé 4,5,6,7-tétrahydro-benzothiazol-2-yl-amine substitué, selon l'une quelconque des revendications 1 à 16, pour la fabrication d'un médicament destiné à la prophylaxie et/ou au traitement de troubles de la prise alimentaire, de préférence choisis dans le groupe constitué par la boulimie, l'anorexie, l'obésité et la cachexie.

**29.** Utilisation d'au moins un composé 4,5,6,7-tétrahydro-benzothiazol-2-yl-amine substitué, selon l'une quelconque des revendications 1 à 16, pour la fabrication d'un médicament destiné à la prophylaxie et/ou au traitement de la catalepsie.

**30.** Utilisation d'au moins un composé 4,5,6,7-tétrahydro-benzothiazol-2-yl-amine substitué, selon l'une quelconque des revendications 1 à 16, pour la fabrication d'un médicament destiné à l'accroissement de la vigilance.

**31.** Utilisation d'au moins un composé 4,5,6,7-tétrahydro-benzothiazol-2-yl-amine substitué, selon l'une quelconque des revendications 1 à 16, pour la fabrication d'un médicament destiné à l'accroissement de la libido.

**32.** Utilisation d'au moins un composé 4,5,6,7-tétrahydro-benzothiazol-2-yl-amine substitué, selon l'une quelconque des revendications 1 à 16, pour la fabrication d'un médicament destiné à l'anxiolyse.

**33.** Utilisation d'au moins un composé 4,5,6,7-tétrahydro-benzothiazol-2-yl-amine substitué, selon l'une quelconque des revendications 1 à 16, pour la fabrication d'un médicament destiné au traitement simultané de douleurs et de dépressions.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- NA and 5-HT Reuptake Inhibitors and α2 agonists. **Tzschentke.** Analgesics: From Chemistry and Pharmacology to Clinical Application. Wiley, 2002, 265-284
- **Berard.** *Int. Med. J.,* 1996, vol. 3/4, 257-259
- **Veröffentlichung von I.C. Hendershot ; J. Forsaith.** *J. Pharmacol. Exp. Ther.,* 1959, vol. 125, 237-240
- **Veröffentlichung ; von E.G. Gray ; V.P. Whittaker.** The isolation of nerve endings from brain. *J. Anatomy,* 1962, vol. 96, 79-88
- **Lowry et al.** Protein measurement with the folin phenol reagent. *J. Biol. Chem.,* 1951, vol. 193, 265-275
- **M.Ch. Frink ; H.-H. Hennies ; W. Engelberger ; M. Haurand ; B. Witffert.** *Arzneim.-Forsch./Drug Res.,* 1996, vol. 46 (III), 1029-1036